# EUROPEAN PATENT SPECIFICATION

(11) **EP 2 870 246 B1**
(45) Date of publication and mention of the grant of the patent: **11.09.2019**
(21) Application number: 13739847.5
(22) Date of filing: 03.07.2013
(51) Int. Cl.: C12N 15/113, A61K 31/7088

(54) **OLIGONUCLEOTIDE FOR THE TREATMENT OF MUSCULAR DYSTROPHY PATIENTS**
OLIGONUKLEOTID ZUR BEHANDLUNG VON PATIENTEN MIT MUSKULÄRER DYSTROPHIE
OLIGONUCLÉOTIDE POUR LE TRAITEMENT DE PATIENTS ATTEINTS DE DYSTROPHIE MUSCULAIRE

(30) Priority: 03.07.2012 EP 12174781; 03.07.2012 US 201261667517 P
(43) Date of publication of application: 13.05.2015
(60) Divisional application: 19189136.5
(73) Proprietor: BioMarin Technologies B.V., 2333 CH Leiden (NL)
(72) Inventor: VAN DEUTEKOM, Judith Christina Theodora, 3329 AA Dordrecht (NL)
(74) Representative: Nederlandsch Octrooibureau
(86) International application number: PCT/NL2013/050487
(87) International publication number: WO 2014/007620

(56) References cited:
- WO-A1-00/76554
- WO-A1-2006/000057
- WO-A1-2011/057350
- WO-A1-2012/109296
- WO-A1-2013/112053
- WO-A2-2004/083446
- WO-A2-2011/078797
- US-A1- 2012 046 348
- ANNEMIEKE AARTSMA-RUS ET AL: "Exonic Sequences Provide Better Targets for Antisense Oligonucleotides Than Splice Site Sequences in the Modulation of Duchenne Muscular Dystrophy Splicing", OLIGONUCLEOTIDES, vol. 20, no. 2, 1 April 2010 (2010-04-01), pages 69-77, XP055024186, ISSN: 1545-4576, DOI: 10.1089/oli.2009.0215
- YOKOTA TOSHIFUMI ET AL: "Antisense oligo-mediated multiple exon skipping in a dog model of duchenne muscular dystrophy.", METHODS IN MOLECULAR BIOLOGY, vol. 709, 2011, pages 299-312, XP008158625, ISSN: 1940-6029 cited in the application

## Description

### Field of the invention

The invention relates to the field of human genetics, more specifically to a method for designing a single oligonucleotide which is preferably capable of inducing the skipping of two or more exons of a pre-mRNA. The invention further provides said oligonucleotide, a pharmaceutical composition comprising said oligonucleotide, and the use of said oligonucleotide as identified herein.

### Background of the invention

Oligonucleotides are emerging in medicine for treating genetic disorders like muscular dystrophy. Muscular dystrophy (MD) refers to genetic diseases that are characterized by progressive weakness and degeneration of skeletal muscles. Duchenne muscular dystrophy (DMD) and Becker muscular dystrophy (BMD) are the most common childhood forms of muscular dystrophy and are used herein to illustrate the invention. DMD is a severe, lethal neuromuscular disorder resulting in a dependency on wheelchair support before the age of 12 and DMD patients often die before the age of thirty due to respiration or heart failure.

DMD is caused by mutations in the DMD gene; mainly frame-shifting deletions or duplications of one or more exons, small nucleotide insertions or deletions, or by nonsense point mutations, which typically result in the absence of functional dystrophin. During the last decade, specifically induced modification of splicing in order to restore the disrupted reading frame of the DMD transcript has emerged as a promising therapy for Duchenne muscular dystrophy (DMD) (van Ommen G.J. et al, Yokota T., et al, van Deutekom et al., Goemans N.M., et al.,). Using sequence-specific Antisense OligoNucleotides (AONs) which target a specific exon flanking or containing the mutation and interfere with its splicing signals, the skipping of that exon can be induced during the processing of the DMD pre-mRNA. Despite the resulting truncated transcript, the open reading frame is restored and a protein is introduced which is similar to those found in the typically milder Becker muscular dystrophy patients. AON-induced exon skipping provides a mutation-specific, and thus potentially personalized therapeutic approach for DMD patients and specific severe BMD patients. Since most mutations cluster around exons 45 to 55 in the DMD gene, the skipping of one specific exon in that region may be therapeutic for a subpopulation of patients with a variety of mutations. The skipping of exon 51 affects the largest subpopulations of patients (approximately 13%), including those with deletions of exons 45 to 50, 48 to 50, 50, or 52. For some mutations, the skipping of more than one exon is required to restore the open reading frame. For instance, for DMD patients with a deletion of exon 46 to exon 50 in the DMD gene, only the skipping of both exons 45 and 51 would be corrective. To treat these patients, administration of two oligonucleotides, one targeting exon 45 and the other targeting exon 51, is required. The feasibility of skipping two or multiple consecutive exons by using a combination of AONs, either in a cocktail or in virally delivered gene constructs, has been studied extensively (Aartsma-Rus A. et al., 2004; Béroud C., et al.; Van Vliet L., et al.; Yokota T., et al.; Goyenvalle A., et al.,). Multi-exon skipping would be applicable to combined subpopulations of patients, allow the mimicking of deletions known to be associated with relatively mild phenotypes, and would provide a tool to address rare mutations outside the hot spot deletion region in the DMD gene. A drawback for development of drugs comprising multiple oligonucleotides is however that drug regulation authorities may regard oligonucleotides of different sequences as different drugs, each requiring prove of stable production, toxicity- and clinical testing. Therefore, there is a need for a single molecular compound capable of inducing the skipping of at least two exons to facilitate the treatment of combined subgroups of DMD patients.

### Description of the invention

The invention provides a method for designing a single oligonucleotide, wherein said oligonucleotide is capable of binding to a region of a first exon and to a region of a second exon within the same pre-mRNA, wherein said region of said second exon has at least 50% identity with said region of said first exon. The oligonucleotides obtainable by said method are preferably capable of inducing the skipping of said first exon and said second exon of said pre-mRNA. Preferably, the skipping of additional exon(s) is also induced, wherein said additional exon(s) is/are preferably located in between said first and said second exon. The resulting transcript of said pre-mRNA, wherein said exons are skipped, is in-frame.

### Oligonucleotide

In a first aspect, the invention relates to an oligonucleotide that is capable of binding to a region of a first exon and to a region of a second exon within the same pre-mRNA, wherein said region of said second exon has at least 50% identity with said region of said first exon.

This oligonucleotide is preferably capable of inducing the skipping of said first and second exons of said pre-mRNA; more preferably the skipping of additional exon(s) is induced, wherein said additional exon(s) is/are preferably located in between said first and said second exons, and wherein the resulting transcript is in frame.

Exon skipping interferes with the natural splicing processes occurring within a eukaryotic cell. In higher eukaryotes the genetic information for proteins in the DNA of the cell is encoded in exons which are separated from each other by intronic sequences. These introns are in some cases very long. The transcription machinery of eukaryotes generates a pre-mRNA which contains both exons and introns, while the splicing machinery, often already during the ongoing production of the pre-mRNA, generates the actual coding mRNA for the protein by removing the introns and connecting the exons present in the pre-mRNA during a process called splicing.

An oligonucleotide of the invention that is capable of binding to a region of a first exon from a pre-mRNA and to a region of a second exon within the same pre-mRNA is to be construed as an oligonucleotide suitable for binding to a region of a first exon from a pre-mRNA and suitable for binding to a region of a second exon within the same pre-mRNA. Such oligonucleotide of the invention is characterized by its binding feature (i.e. capable of binding), when used with or when used in combination with a pre-mRNA, preferably in a cell. Within this context "capable of' may be replaced by "able to". Thus, the person skilled in the art will appreciate that an oligonucleotide capable of binding to a region of a first exon and capable of binding to a region of a second exon within the same pre-mRNA, defined by a nucleotide sequence, defines said oligonucleotide structurally, i.e. said oligonucleotide has a sequence such that it is reverse-complementary with the sequence of said region of said first exon and also reverse-complementary with the sequence of said region of said second exon within the same pre-mRNA. The degree of reverse-complementarity with said regions of said first and/or said second exon which is needed for an oligonucleotide of the invention may be less than 100%. A certain amount of mismatches or one or two gaps may be allowed, as is addressed further herein. The nucleotide sequence of a region of a first exon which has at least 50% identity with said region of said second exon (or the nucleotide sequence of a region of a second exon which has at least 50% identity with said region of said first exon), to which the oligonucleotide of the invention is capable of binding, could be designed using a method of the invention as explained later herein. Preferred pre-mRNA is a dystrophin pre-mRNA. Preferred combinations of first and second exons of the dystrophin pre-mRNA and preferred regions of said first and second dystrophin exons are defined in table 2. The oligonucleotide of the invention is preferably capable of inducing the skipping of said first and second exons of said dystrophin pre-mRNA; more preferably the skipping of additional exon(s) is induced, wherein said additional exon(s) is/are preferably located in between said first and said second exons, and wherein the resulting dystrophin transcript is in frame, preferably as in Table 1.

A transcript is in frame when it has an open reading frame that allows for the production of a protein. The in-frame status of an mRNA can be assessed by sequence and/or RT-PCR analysis as known to a person skilled in the art. The resulting protein that results from translation of the in-frame transcript can be analysed by immunofluorescence and/or western blot analysis using antibodies that cross-react with said protein, as known to a person skilled in the art. Throughout the invention, an oligonucleotide as identified herein may be said functional if a resulting in frame transcript is identified by RT-PCR and/or sequence analysis, or if the protein resulting from said in frame transcript is identified by immunofluorescence and/or Western blot analysis, in a relevant *in vitro* or *in vivo* system depending on the identity of the transcript. If the transcript is the dystrophin transcript, a relevant system may be a muscle cell, or myotube, or muscle fiber or myofiber, of a healthy donor or a DMD patient as explained later herein.

In an embodiment, a region of a second exon (present within the same pre-mRNA as a region of a first exon) has at least 50%, 55%, 60%, 65%, 70%, 75%, 80%, 85%, 90%, 95% or 100% identity with a region of a first exon as identified above (preferreds regions of a first and of a second dystrophin exon are identified in Table 2). The identity percentage may be assessed over the entire length of said first and/or second exon or over a region of 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 90, 100, 110, 120, 130,140, 150, 160, 170, 180, 190, 200 or more nucleotides as exemplified for dystrophin exons herein. It is clear for the skilled person that a first and a second exon as identified herein are two distinct exons of one single pre-mRNA or two distinct exons within the same pre-mRNA. A first exon as identified herein may be located upstream of (i.e. 5' of) the second exon within the same pre-mRNA as identified herein, or said second exon may be upstream of said first exon. Preferably, said first exon is located upstream from said second exon. It is clear for a skilled person that an oligonucleotide of the invention may be primarily designed to be capable of binding to a region of a first exon; in view of the identity of a region of said first and said second exons, said oligonucleotide may secondary also be capable of binding to said region of said second exon. The reverse design is possible: an oligonucleotide of the invention may be primarily designed to be capable of binding to a region of a second exon; in view of the identity of a region of said first and said second exons, said oligonucleotide may secondary also be capable of binding to said region of said first exon.

The identity percentage between a region of the first and a region of the second exon may be assessed over the whole region of said first exon, wherein that region may be shorter, longer or equally long as the part of that region to which the oligonucleotide of the invention is capable of binding. The region of the first exon and the region of the second exon as used herein may also be identified as the identity region(s). Preferably the region of the first exon which defines the identity with the region of the second exon is equally long or longer than the part of that region to which the oligonucleotide of the invention is capable of binding. It has to be understood that the oligonucleotide of the invention may be capable of binding to a smaller part, or a partly overlapping part, of said regions used to assess sequence identity of the first and/or second exon. It is therefore to be understood that an oligonuceotide which is capable of binding to a region of a first and of a second exon may bind a part of said region of said first exon and of said second exon. Said part may be as long as said region of said first and/or said second exon. Said part may be shorter or longer as said region of said first and/or said second exon. Said part may be comprised within said region of said first and/or said second exon. Said part may overlap with said region of said first and/or said second exon. This overlap may be of 1, 2, 3, 4, 5, or more nucleotides at the 5' and/or at the 3' side of the region of said first and/or second exon. The oligonucleotide may be at least 1, 2, 3, 4, 5, or more nucleotides longer or shorter than the region of said first and/or said second exon and may be at the 5' or 3' side of said region of the first and/or second region.

The region, being 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or up to 90, 100, 110, 120, 130,140, 150, 160, 170, 180, 190, 200 or more nucleotides, used to calculate the percentage of identity between a first exon and a second exon may be a continuous stretch or may be interrupted by one, two, three, four or more gaps as long as the identity percentage over the whole region is at least 50%.

The identity percentage between the region of the first and the region of the second exons may be assessed using any program known to the skilled person. Preferably, said identity is assessed as follows: the best pair-wise alignment between the first and second exons using the online tool EMBOSS Matcher using default settings (Matrix: EDNAFULL, Gap_penalty: 16, Extend_penalty: 4).

An oligonucleotide as used herein preferably refers to an oligomer that is capable of binding to, targeting, hybridizing to, and/or is reverse-complementary to, a region or to a part of a region of a first and a second exon within the same pre-mRNA.

An oligonucleotide as identified herein (i.e. which is capable of binding to a region of a first exon and to a region of another exon (i.e, a second exon) within the same pre-mRNA, wherein said region of said second exon has at least 50% identity with said region of said first exon) is also preferably at least 80% reverse complementary to said region of said first exon and at least 45% reverse complementary to said region of said second exon. More preferably, said oligonucleotide is at least 85%, 90%, 95% or 100% reverse complementary to said region of said first and at least 50%, 55%, 60%, 65%, 70%, 75% ,80%, 85%, 90%, 95% or 100% reverse complementary to said region of said second exon. The reverse complementarity is preferably but not necessarily assessed over the whole length of the oligonucleotide.

An oligonucleotide encompassed by the invention may comprise at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides. An oligonucleotide encompassed by the invention may comprise at most 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10 nucleotides. The length of the oligonucleotide of the invention is defined by the total number of nucleotides encompassed in said oligonucleotide, irrespective of any modifications present in said oligonucleotide. As discussed further below, nucleotides may contain certain chemical modifications, but such modified nucleotides are still considered nucleotides in the context of the present invention. Depending on the chemistry of an oligonucleotide, the optimal length of an oligonucleotide may be distinct. For example the length of a 2'-*O*-methyl phosphorothioate oligonucleotide may be from 15 till 30. If this oligonucleotide is further modified as exemplified herein, the optimal length may be shortened to 14, 13 or even lower.

In a preferred embodiment, the oligonucleotide of the invention is not longer than 30 nucleotides, to limit the chance for reduced synthesis efficiency, yield, purity or scalability, reduced bioavailability and/or cellular uptake and trafficking, reduced safety, and to limit cost of goods. In a more preferred embodiment an oligonucleotide is from 15 and 25 nucleotides. Most preferably an oligonucleotide encompassed by the invention consists of 20, 21, 22, 23, 24, or 25 nucleotides. The length of the oligonucleotide of the invention is preferably such that the functionality or activity of the oligonucleotide is defined by inducing at least 5% of skipping of the first and second exons (and any exon(s) in between), or by facilitating that at least 5% of an in frame transcript is formed, when at least 100 nM of said oligonucleotide is being used to transfect a relevant cell culture *in vitro.* The assessment of the presence of said transcript has already been explained herein. A relevant cell culture is a cell culture wherein the pre-mRNA comprising said first and said exon is transcribed and spliced into an mRNA transcript. If the pre-mRNA is the dystrophin pre-mRNA, a relevant cell culture comprises (differentiated) muscle cells. In this case, at least 20% of an in frame transcript is formed when at least 250 nM of said oligonucleotide is being used.

A region of a first exon may be at least 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or up to 90, 100, 110, 120, 130, 140, 150, 160, 170, 180, 190, 200, or more nucleotides. A region of a first exon may also be defined as being at least 1%, 5%, 10%, 20%, 30%, 40%, 50%, 60%, 70%, 80%, 90% or 100% of the length of said exon. A region of a first exon may be called an identity region.

A region of a second exon may be at least 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79,80 or up to 90, 100, 110, 120, 130, 140, 150, 160, 170, 180, 190, 200, or more nucleotides. A region of a second exon may be defined as being at least 1%, 5%, 10%, 20%, 30%, 40%, 50%, 60%, 70%, 80%, 90% or 100% of the length of said exon. A region of a second exon may be called an identity region.

In one embodiment, the oligonucleotide of the invention is capable of binding to a region of exon U+1 (first exon) from a pre-mRNA, wherein a region of another exon D-1 (second exon) within the same pre-mRNA has at least 50% identity with said region of said (U+1) exon, wherein said oligonucleotide is for the skipping of said U+1 and said D-1 exons (and of additional exon(s) preferably located in between said first and said second exon) of said pre-mRNA, to obtain an in-frame transcript in which exons U and D are spliced together (eg. for DMD preferably as in Table 1). An oligonucleotide of the invention is also identified herein as a compound. An oligonucleotide of the invention is preferably an antisense oligonucleotide (i.e. AON). An oligonucleotide is preferably for the skipping of said two exons (i.e. said first (U+1) and said second (D-1) exons) of said pre-mRNA, and wherein the resulting transcript (in which U is directly spliced to D) is in-frame (eg. for DMD preferably as in Table 1). One may say that said oligonucleotide induces the skipping of said two exons in one single pre-mRNA. Optionally the skipping of additional exon(s) is induced wherein said additional exon(s) is/are preferably located in between said first and said second exons and the resulting transcript is in frame.

An oligonucleotide is more preferably for the skipping of said two exons (i.e. said first and said second exon), and the entire stretch of exons in between said first and said second exon, in said pre-mRNA, in order to remove any mutation within said stretch, and to obtain a transcript which is shorter but which has a restored open reading frame allowing protein production.

Without wishing to be bound by any theory, it is believed that due to the at least 50% sequence identity or similarity between said two exons, a single oligonucleotide of the invention is able to bind to and induce the skipping of both exons, and, preferably, the entire stretch of exons in between in order to obtain a shorter transcript, which is in frame. Said two exons may thus be adjacent in a pre-mRNA or may be separated by at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 exons. The region encompassing one or more exons present between said first and said second exons may also be called a stretch of (multiple) exons or a multiexon stretch. Preferably, the first exon of this multiexon stretch is the first exon identified earlier herein and the last exon of this multiexon stretch is the second exon identified earlier herein. An oligonucleotide of the invention may also be identified as an oligonucleotide which is able to induce the skipping of said two exons or the skipping of a stretch of (multiple) exons or the skipping of said multiexon stretch. In a preferred embodiment, skipping of both the first exon and the second exons is induced by using one single oligonucleotide of the invention. In a preferred embodiment, the skipping of more than one, more than 2, more than 3, more than 4, more than 5, more than 6, more than 7, more than 8, more than 9, more than 10, more than 11, more than 12, more than 13, more than 14, more than 15, more than 16, more than 17 exons, more than 18, more than 19, more than 20, more than 21, more than 22, more than 23, more than 24, more than 25, more than 26, more than 27, more than 28, more than 29, more than 30, more than 31, more than 32, more than 33, more than 34, more than 35, more than 36 more than 37, more than 38, more than 39, more than 40, more than 41, more than 42, more than 43, more than 44, more than 45, more than 46, more than 47, more than 48, more than 49, more than 50 exons using one single oligonucleotide is carried out and therefore this skipping of more than 1, 2, 3, 4, 5, 6, 7, 8, 9, 10, 11, 12, 13, 14, 15,16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 exons is carried out, not using a mixture or cocktail of two or more distinct oligonucleotides, or not using two or more distinct oligonucleotides that may be linked with one or more linker(s), or not using a gene construct transcribing two or more distinct oligonucleotides. In an embodiment, it is therefore provided that the invention encompasses one single oligonucleotide and does not comprise two or more distinct oligonucleotides, said single oligonucleotide being capable of binding to said first and said second exons, and able of inducing the skipping of at least said first and said second exons within a single pre-mRNA as explained herein. In this context the skilled person understands that the word "single" does not refer to the number of molecules needed in order to induce exon skipping. Single refers to the sequence of an oligonucleotide: the invention encompasses one single oligonucleotide sequence and its use and does not comprise two or more distinct oligonucleotide sequences, said single oligonucleotide sequence being capable of binding to said first and said second exons and able of inducing the skipping of at least said first and said second exons within a single pre-mRNA as explained herein. This is the first invention allowing the skipping of more than one exon with only one single oligonucleotide in order to treat a disease caused by a (rare) mutation in a gene, provided that different exons in said gene comprise regions that have at least 50% sequence identity.

An oligonucleotide of the invention is preferably used as a part of therapy based on RNA-modulating activity as later herein defined. Depending on the identity of the transcript wherein the first and second exons are present, one may design an oligonucleotide for preventing, treating or delaying a given disease.

It has been shown that targeting two exons within a single pre-mRNA with a single oligonucleotide capable of binding to both exons, results in mRNA lacking the targeted exons and, additionally, the entire stretch of exons in between. An advantage of such single oligonucleotide as defined herein is that defects caused by different mutations within this multiexon stretch can be treated. If one would choose to use two or more distinct oligonucleotides to induce the skipping of two or more exons, one should for example take into account that each oligonucleotide may have its own PK profile and that thus conditions would have to be found wherein each of them will be similarly present in a same cell. Therefore another advantage of using only one single oligonucleotide able to bind to two different exons as identified above, is that production, toxicity, dose finding and clinical testing is majorly facilitated as these can be reduced to the straightforward production and testing of one single compound.

Below additional features of the oligonucleotide of the invention are defined.

Within the context of the invention, in a preferred embodiment, an oligonucleotide is capable of binding to, targeting, hybridizing to, is reverse-complementary with and/or is capable of inhibiting the function of at least one splicing regulatory sequence within at least said first exon and/or said second exon and/or is affecting the structure of at least said first exon and/or said second exon:
wherein said oligonucleotide comprises a sequence that is capable of binding to, targeting, hybridizing to and/or is reverse-complementary to a binding site for a serine-arginine (SR) protein in said first and/or second exon
   and/or
wherein said oligonucleotide is capable of binding to, targeting, hybridizing and/or is reverse-complementary to an exonic splicing enhancer (ESE), an exon recognition sequence (ERS), and/or an exonic splicing silencer (ESS) in said first and/or second exon.

More preferably, said oligonucleotide which is capable of binding to, targeting, hybridizing to and/or being reverse-complementary to a region of a first pre-mRNA exon and/or to a region of a second pre-mRNA exon is capable of specifically inhibiting at least one splicing regulatory sequence and/or affecting the structure of at least said first and/or second exon in said pre-mRNA. Interfering with such splicing regulatory sequences and/or structures has the advantage that such elements are located within the exon. By providing such an oligonucleotide as defined herein, it is possible to effectively mask at least said first and second exon, and preferably the entire stretch of exons in between, from the splicing apparatus. The failure of the splicing apparatus to recognize these exons thus leads to the skipping or exclusion of these exons from the final mRNA. This embodiment focuses on coding sequences only. It is thought that this allows the method to be more specific and thus reliable. The reverse complementarity of said oligonucleotide to said region of said first and/or second exon of a pre-mRNA is preferably at least 45%, 50%, 55%, 60% ,65%, 70%, 75%, 80%, 85%, 90%, 95% or 100%.

Therefore, the present invention relates to an antisense oligonucleotide for use as a medicament for preventing, delaying, ameliorating and/or treating a disease in a subject, wherein said oligonucleotide is capable of binding to a region of a first exon and a region of a second exon, wherein said region of said second exon has at least 50% identity with said region of said first exon,
wherein said first and said second exons are within a same pre-mRNA in said subject, wherein said binding results in the skipping of said first exon and said second exon and prefereably in the skipping of a multi-exon stretch starting with said first exon and encompassing one or more exons present between said first and said second exons and at the most in the skipping of the entire stretch of exons in between said first and said second exons, and
wherein an in-frame transcript is obtained allowing production of a functional or semi-functional protein.

Preferably, as explained herein, said oligonucleotide is capable of inducing the skipping of the entire stretch of exons between said first exon and said second exon.

More preferably, as explained herein said binding of said oligonucleotide is capable of interfering with at least one splicing regulatory sequence in said regions of said first and second exons and/or with the secondary structure of said first and/or said second exons and/or with the secondary strucutre encompassing at least said first and/or said second exons in said pre-mRNA. Preferred splicing regulatory sequences are presented later herein.

One preferred embodiment therefore relates to an oligonucleotide of the invention that is capable of binding to a region of a first exon from a pre-mRNA and/or to a region or a second exon within the same pre-mRNA, wherein said region of said second exon within the same pre-mRNA has at least 50% identity with said region of said first exon (eg. for DMD preferably as in Table 2), wherein said oligonucleotide is capable of inducing the skipping of said first and second exons of said pre-mRNA; resulting in a transcript which is in frame (eg. for DMD preferably as in Table 1 or 6). Said oligonucleotide providing said individual with a functional or semi-functional protein, and said oligonucleotide further comprising:
- a sequence which is capable of binding to, targeting, hybridizing to and/or is reverse-complementary to a region of a first and/or second pre-mRNA exon that is hybridized to another part of a first and/or second pre-mRNA exon (closed structure), and/or
- a sequence which is capable of binding to, targeting, hybridizing to and/or is reverse-complementary to a region of said first and/or second pre-mRNA exon that is not hybridized in said pre-mRNA (open structure).

For this embodiment, reference is made to WO 2004/083446 patent application. RNA molecules exhibit strong secondary structures, mostly due to base pairing of reverse-complementary or partly reverse-complementary stretches within the same RNA. It has long since been thought that structures in the RNA play a role in the function of the RNA. Without being bound by theory, it is believed that the secondary structure of the RNA of an exon plays a role in structuring the splicing process. Through its structure, an exon is recognized as a part that needs to be included in the mRNA. In an embodiment, an oligonucleotide is capable of interfering with the structure of at least said first exon and probably also said second exon and possibly also the stretch of exons in between, and therefore capable of interfering with the splicing of said first and probably also said second exon and possibly also the stretch of exons in between, by masking said exons from the splicing apparatus and thereby resulting in the skipping of said exons. Without being bound by theory, it is thought that the overlap with an open structure improves the invasion efficiency of an oligonucleotide (i.e. increases the efficiency with which the oligonucleotide can enter the structure), whereas the overlap with the closed structure subsequently increases the efficiency of interfering with the secondary structure of the RNA of the exon. It is found that the length of the partial reverse-complementarity to both the closed and the open structure is not extremely restricted. We have observed high efficiencies with an oligonucleotide with variable lengths of reverse-complementarity in either structure. The term reverse-complementarity is used herein to refer to a stretch of nucleic acids that can hybridize to another stretch of nucleic acids under physiological conditions. Hybridization conditions are later defined herein. It is thus not absolutely required that all the bases in the region of reverse-complementarity are capable of pairing with bases in the opposing strand. For instance, when designing an oligonucleotide, one may want to incorporate for instance one or more residues that do not base pair with the bases on the reverse-complementary strand. Mismatches may to some extent be allowed, if under the circumstances in the cell, the stretch of nucleotides is still capable of hybridizing to the reverse-complementary part. In the context of this invention the presence of a mismatch in the oligonucleotide of the invention is preferred since in an embodiment, said oligonucleotide is at least 45%, 50%, 55%, 60%, 65%, 70%, 75%, 80%, 85%, 90%, 95%, 100% reverse complementary to a region of a first exon and/or to a region of a second exon. The presence of a mismatch in said oligonucleotide is a preferred characteristic of the invention since said oligonucleotide is able to bind to a region of said first and to a region of said second exon as earlier identified herein.

Other advantages of allowing the presence of a mismatch in an antisense oligonucleotide of the invention are defined herein and are similar to those provided by the presence of an inosine (hypoxanthine) and/or a universal base and/or a degenerate base and/or a nucleotide containing a base able to form a wobble base pair: avoid the presence of a CpG, avoid or decrease a potential multimerisation or aggregation, avoid quadruplex structures, allow to design an oligonucleotide with improved RNA binding kinetics and/or thermodynamic properties.

Preferably, the reverse-complementarity of the oligonucleotide to the region of identity between said first and/or second exon is from 45% to 65%, 50% to 75%, but more preferably from 65% to 100% or from 70% to 90% or from 75% to 85%, or from 80% to 95%. In general this allows for 1, 2, 3, 4, 5, 6, 7, 8, 9, 10, or 11 mismatch(es) in an oligonucleotide of 20 nucleotides. Therefore, we may have 1, 2, 3, 4, 5, 6, 7, 8, 9, 10, 11, 12, 13, 14, 15, 16, 18, 19, 20, 21, or 22 mismatch(es) in an oligonucleotide of 40 nucleotides. Preferably, less than 14 mismatches are present in an oligonucleotide of 40 nucleotides. The number of mismatches is such that an oligonucleotide of the invention is still able of binding to, hybridizing to, targeting a region of said first exon and to a region of said second exon, thereby inducing the skipping of at least said first and said second exons and inducing the production of an in frame transcript as explained herein. Preferably the production of an in frame transcript is obtained with at least 5% efficiency using at least 100 nM of said oligonucleotide to transfect a relevant cell culture *in vitro* as earlier explained herein.

It should be pointed out that the invention encompasses an oligonucleotide that does not have any mismatch with a region of a first exon and that may have 1, 2, 3, 4, 5, 6, 7, 8, 9, 10, 11, 12, 13, 14, 15, 16, 18, 19, 20, 21, or 22 mismatches with the corresponding region of a second exon as defined herein. However, the invention also encompasses an oligonucleotide that does not have any mismatch with a region of a second exon and that may have 1, 2, 3, 4, 5, 6, 7, 8, 9, 10, 11, 12, 13, 14, 15, 16, 18, 19, 20, 21, or 22 mismatches with the corresponding region of a first exon as defined herein. Finally, the invention encompasses an oligonucleotide that may have 1, 2, 3, 4, 5, 6, 7, 8, 9, 10, 11, 12, 13, 14, 15, 16, 18, 19, 20, 21, or 22 mismatches with a region of a first exon and that may have 22, 21, 20, 19, 18, 17, 16, 15, 14, 13, 12, 11, 10, 9, 8, 7, 6, 5, 4, 3, 2, 1 mismatches with the corresponding region of a second exon as defined herein. Here again, it is to be understood that the number of mismatches in an oligonucleotide of the invention is such that said oligonucleotide is still able of binding to, hybridizing to, targeting a region of said first exon and a region of said second exon as explained herein.

An oligonucleotide of the invention preferably does not cross a gap in the alignment of a region of a first exon and a region of a second exon as identified herein. The alignment is preferably carried out using the online tool EMBOSS Matcher as explained earlier herein. In specific cases however an oligonucleotide of the invention may need to cross a gap, as earlier mentioned herein. Said gap is preferably just one gap. Said gap is preferably spanning less than 3 nucleotides, most preferably only one nucleotide. The number and length of gaps is such that an oligonucleotide of the invention is still able of binding to, hybridizing to, targeting a region of said first exon and a region of said second exon, thereby inducing the skipping of at least said first and said second exons and inducing the production of an in frame transcript as explained herein.

The structure (i.e. open and closed structures) is best analyzed in the context of the pre-mRNA wherein the exons reside. Such structure may be analyzed in the actual RNA. However, it is currently possible to predict the secondary structure of an RNA molecule (at lowest energy costs) quite well using structure-modeling programs. A non-limiting example of a suitable program is Mfold web server (Zuker, M.).

A person skilled in the art will be able to predict, with suitable reproducibility, a likely structure of an exon, given a nucleotide sequence. Best predictions are obtained when providing such modeling programs with both said exon and flanking intron sequences. It is typically not necessary to model the structure of the entire pre-mRNA.

The annealing of an oligonucleotide of the invention may affect the local folding or 3D structure or conformation of the target RNA (i.e. of the region encompassing at least the first and/or second exons). The different conformation may result in the disruption of a structure recognized by the splicing machinery. However, when potential (cryptic) splice acceptor and/or donor sequences are present within the first and/or second targeted exon, occasionally a new structure is generated defining a different (neo) exon, i.e. with a different 5' end, a different 3' end, or both. This type of activity is within the scope of the present invention as the targeted exon is excluded from the mRNA. The presence of a new exon, containing part of said first and/or second targeted exon, in the mRNA does not alter the fact that the targeted exon, as such, is excluded. The inclusion of a neo-exon can be seen as a side effect which occurs only occasionally. There are two possibilities when exon skipping is used to restore (part of) an open reading frame of a transcript that is disrupted as a result of a mutation. One is that the neo-exon is functional in the restoration of the reading frame, whereas in the other case the reading frame is not restored. When selecting an oligonucleotide for restoring an open reading frame by means of multiple exon-skipping it is of course clear that under these conditions only those oligonucleotides are selected that indeed result in exon-skipping that restore the open reading frame of a given transcript, with or without a neo-exon.

Further in another preferred embodiment is provided an oligonucleotide of the invention that is capable of binding to a region of a first exon from a pre-mRNA and to a region of a second exon within the same pre-mRNA, wherein said region of said second exon has at least 50% identity with said region of said first exon (preferred regions for dystrophin exons are identified in Table 2 or Table 6), wherein said oligonucleotide is capable of inducing the skipping of said first and second exons of said pre-mRNA; resulting in a transcript which is in frame (for DMD preferably as in Table 1 or Table 6). Said oligonucleotide providing said individual with a functional or semi-functional protein, and said oligonucleotide further comprising: a sequence that is capable of binding to, targeting, hybridizing to, is reverse-complementary to and/or is able to inhibit a function of one or more binding sites for a serine-arginine (SR) protein in RNA of an exon of a pre-mRNA.

In WO 2006/112705 patent application we have disclosed the presence of a correlation between the effectiveness of an exon-internal antisense oligonucleotide (AON) in inducing exon skipping and the presence of a putative SR binding site in the target pre-mRNA site of said AON. Therefore, in one embodiment, said oligonucleotide as defined herein is generated comprising determining one or more (putative) binding sites for an SR protein in RNA of said first and/or said exon and producing a corresponding oligonucleotide that is capable of binding to, targeting, hybridizing to and/or is reverse-complementary to said RNA and that at least partly overlaps said (putative) binding site. The term "at least partly overlaps" is defined herein as to comprise an overlap of only a single nucleotide of an SR binding site as well as multiple nucleotides of one or more said binding sites as well as a complete overlap of one or more said binding sites. This embodiment preferably further comprises determining from a secondary structure of a first and/or second exon, a region that is hybridized to another part of said first and/or second exon (closed structure) and a region that is not hybridized in said structure (open structure), and subsequently generating an oligonucleotide that at least partly overlaps one or more said (putative) binding sites and that overlaps at least part of said closed structure and overlaps at least part of said open structure and with binding to, targeting, hybridizing with and/or being reverse-complementary to both first and second exons. In this way we increase the chance of obtaining an oligonucleotide that is capable of interfering with the inclusion of said first and second exons, and if applicable the entire stretch of exons in between, from the pre-mRNA into mRNA. Without wishing to be bound by any theory it is currently thought that use of an oligonucleotide directed to an SR protein binding site results in (at least partly) impairing of the binding of an SR protein to said binding site which results in disrupted or impaired splicing.

Preferably, a region of a first exon and/or a region of a second exon within the same pre-mRNA an oligonucleotide of the invention is capable of binding to, comprises an open/closed structure and/or an SR protein binding site, more preferably said open/closed structure and said SR protein binding site partly overlap and even more preferred said open/closed structure completely overlaps an SR protein binding site or an SR protein binding site completely overlaps an open/closed structure. This allows for a further improved disruption of exon inclusion.

Besides consensus splice sites sequences, many (if not all) exons contain splicing regulatory sequences such as exonic splicing enhancer (ESE) sequences to facilitate the recognition of genuine splice sites by the spliceosome (Cartegni L, et al. 2002; and Cartegni L, et al, 2003). A subgroup of splicing factors, called the SR proteins, can bind to these ESEs and recruit other splicing factors, such as U1 and U2AF to (weakly defined) splice sites. The binding sites of the four most abundant SR proteins (SF2/ASF, SC35, SRp40 and SRp55) have been analyzed in detail (Cartegni L, et al. 2002) and Cartegni L, et al, 2003). There is a correlation between the effectiveness of an oligonucleotide and the presence/absence of an SF2/ASF, SC35, SRp40 and SRp55 binding site in a part of a first exon bound by, hybridized by and/or targeted by said oligonucleotide. In a preferred embodiment, the invention thus provides an oligonucleotide, which binds to, hybridizes with, targets and/or is reverse-complementary to a binding site for a SR protein. Preferably, said SR protein is SF2/ASF or SC35, SRp40 or SRp55. In one embodiment the oligonucleotide binds to, hybridizes with, targets and/or is reverse-complementary to a binding site for a SF2/ASF, SC35, SRp40, or SRp55 protein in a first exon and to a binding site for a different SF2/ASF, SC35, SRp40, or SRp55 protein in a second exon. In a more preferred embodiment the oligonucleotide binds to, hybridizes with, targets and/or is reverse-complementary to a binding site for a SF2/ASF, SC35, SRp40, or SRp55 protein in a first exon and to a corresponding binding site for a similar SF2/ASF, SC35, SRp40, or SRp55 protein in a second exon.

In one embodiment a patient is provided with a functional or semi-functional protein by using an oligonucleotide which is capable of binding, targeting a regulatory RNA sequence present in a first and/or second exon which is required for the correct splicing of said exon(s) in a transcript. Several cis-acting RNA sequences are required for the correct splicing of exons in a transcript. In particular, supplementary elements such as exonic splicing enhancers (ESEs) are identified to regulate specific and efficient splicing of constitutive and alternative exons. Using a compound comprising an oligonucleotide that binds to or is capable of binding to one of the supplementary elements in said first and/or second exon(s), their regulatory function is disturbed so that the exons are skipped. Hence, in one preferred embodiment, an oligonucleotide of the invention is capable of binding to a region of a first exon from a pre-mRNA and to region of a second exon within the same pre-mRNA wherein said region of said second exon has at least 50% identity with said region of said first exon, wherein said oligonucleotide is capable of inducing the skipping of said first and second exons of said pre-mRNA; wherein said region of said first exon and/or said region of said second exon comprises an exonic splicing enhancer (ESE), an exon recognition sequence (ERS), and/or a splicing enhancer sequence (SES) and/or wherein said oligonucelotide is capable of binding to, targeting, is capable of inhibiting and/or is reverse-complementary to said exonic splicing enhancer (ESE), an exon recognition sequence (ERS), and/or a splicing enhancer sequence (SES).

Below preferred chemistries of the oligonucleotide of the invention are disclosed.

An oligonucleotide is commonly known as an oligomer that has basic hybridization characteristics similar to natural nucleic acids. Hybridization has been defined in the part dedicated to the definitions at the end of the description of the invention. Within this application, the term oligonucleotide and oligomer are used interchangeably. Different types of nucleosides may be used to generate said oligonucleotide of the invention. An oligonucleotide may comprise at least one modified internucleoside linkage and/or at least one sugar modification and/or at least one base modification, compared to a naturally occurring ribonucleotide- or deoxyribonucleotide-based oligonucleotide.

A "modified internucleoside linkage" indicates the presence of a modified version of the phosphodiester as naturally occurring in RNA and DNA. Examples of internucleoside linkage modifications, which are compatible with the present invention, are phosphorothioate (PS), chirally pure phosphorothioate, phosphorodithioate (PS2), phosphonoacetate (PACE), phosphonoacetamide (PACA), thiophosphonoacetate, thiophosphonoacetamide, phosphorothioate prodrug, H-phosphonate, methyl phosphonate, methyl phosphonothioate, methyl phosphate, methyl phosphorothioate, ethyl phosphate, ethyl phosphorothioate, boranophosphate, boranophosphorothioate, methyl boranophosphate, methyl boranophosphorothioate, methyl boranophosphonate, methyl boranophosphonothioate, and their derivatives. Another modification includes phosphoramidite, phosphoramidate, N3'→5' phosphoramidate, phosphorodiamidate, phosphorthioamidate, phosphorothiodiamidate, sulfamate, dimethylenesulfoxide, sulfonate, methyleneimino (MMI), oxalyl and thioacetamido nucleic acid (TANA); and their derivatives. Depending on its length, an oligonucleotide of the invention may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 or 39 backbone modifications. It is also encompassed by the invention to introduce more than one distinct backbone modification in said oligonucleotide.

Also encompassed within the invention is an oligonucleotide that comprises a internucleoside linkage that may be different in respect of the atoms of the nucleosides that are connect to each other, when compared to the naturally occurring internucleoside linkage. In this respect, the oligonucleotide of the invention may comprise at least one internucleoside linkage construed as 3'-3', 5'-5', 2'-3', 2'-5', 2'-2' linked monomers. Position numbering might differ for other chemistries, but the idea remains within the scope of the invention.

In one embodiment, the oligonucleotide of the invention comprises at least one phosphorothioate modification. In a more preferred embodiment, an oligonucleotide of the invention is fully phosphorothioate modified.

A "sugar modification" indicates the presence of a modified version of the ribosyl moiety as naturally occurring in RNA and DNA (i.e. the furanosyl moiety), such as bicyclic sugars, tetrahydropyrans, morpholinos, 2'-modified sugars, 3'-modified sugars, 4'-modified sugars, 5'-modified sugars, and 4'-subsituted sugars. Examples of suitable sugar modifications include, but are not limited to, 2'-*O*-modified RNA nucleotide residues, such as 2'-*O*-alkyl or 2'-*O-*(substituted)alkyl *e.g*. 2'-*O*-methyl, 2'-*O*-(2-cyanoethyl), 2'-*O*-(2-methoxy)ethyl (2'-MOE), 2'-*O*-(2-thiomethyl)ethyl, 2'-*O*-butyryl, 2'-*O*-propargyl, 2'-*O*-allyl, 2'-*O*-(2-amino)propyl, 2'-*O*-(2-(dimethylamino)propyl), 2'-*O*-(3-amino)propyl, 2'-*O*-(3-(dimethylamino)propyl), 2'-*O-*(2-amino)ethyl, 2'-*O*-(3-guanidino)propyl (as described in patent application WO 2013/061295, University of the Witwatersrand), 2'-*O*-(2-(dimethylamino)ethyl); 2'-*O-*(haloalkoxy)methyl (Arai K. *et al*.) *e.g.* 2'-*O*-(2-chloroethoxy)methyl (MCEM), 2'-*O*-(2,2-dichloroethoxy)methyl (DCEM); 2'-*O*-alkoxycarbonyl *e.g*. 2'-*O*-[2-(methoxycarbonyl)ethyl] (MOCE), 2'-*O*-[2-(*N*-methylcarbamoyl)ethyl] (MCE), 2'*-O-*[2-(*N,N-*dimethylcarbamoyl)ethyl] (DMCE); 2'-*O*-[methylaminocarbonyl]methyl; 2'-azido; 2'-amino and 2'-substituted amino; 2'-halo *e.g.* 2'-F, FANA (2'-F arabinosyl nucleic acid); carbasugar and azasugar modifications; 3'-*O*-alkyl *e.g*. 3'-*O*-methyl, 3'-*O*-butyryl, 3'-*O*-propargyl; 2',3'-dideoxy; and their derivatives.

Another sugar modification includes "bridged" or "bicylic" nucleic acid (BNA) modified sugar moieties, such as found in *e.g.* locked nucleic acid (LNA), *xylo*-LNA, α-L-LNA, β-D-LNA, cEt (2'-*O*,4'-*C* constrained ethyl) LNA, cMOEt (2'-*O*,4'-*C* constrained methoxyethyl) LNA, ethylene-bridged nucleic acid (ENA), BNA^{NC}[N-Me] (as described in Chem. Commun. 2007, 3765-3767 Kazuyuki Miyashita et al.), CRNs as described in patent application WO 2013/036868 (Marina Biotech); unlocked nucleic acid (UNA) or other acyclic nucleosides such as described in US patent application US 2013/0130378 (Alnylam Pharmaceuticals); 5'-methyl substituted BNAs (as described in US patent application 13/530,218); cyclohexenyl nucleic acid (CeNA), altriol nucleic acid (ANA), hexitol nucleic acid (HNA), fluorinated HNA (F-HNA), pyranosyl-RNA (p-RNA), 3'-deoxypyranosyl-DNA (p-DNA); or other modified sugar moieties, such as morpholino (PMO), cationic morpholino (PMOPlus), PMO-X; tricycloDNA; tricyclo-PS-DNA; and their derivatives. BNA derivatives are for example described in WO 2011/097641. Examples of PMO-X are described in WO2011150408. Depending on its length, an oligonucleotide of the invention may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 sugar modifications. It is also encompassed by the invention to introduce more than one distinct sugar modification in said oligonucleotide.

In one embodiment, the oligonucleotide according to the invention comprises at least one sugar modification selected from 2'-*O*-methyl, 2'-*O*-(2-methoxy)ethyl, 2'-F, morpholino, a bridged nucleotide or BNA, or the oligonucleotide comprises both bridged nucleotides and 2'-deoxy nucleotides (BNA/DNA mixmers). Oligonucleotides comprising a 2'-fluoro (2-'F) nucleotide have been shown to be able to recruit the interleukin enhancer-binding factor 2 and 3 (ILF2/3) and is thereby able to induce exon skipping in the targeted pre-mRNA (Rigo F, et al, WO2011/097614).

In another embodiment, an oligonucleotide as defined herein comprises or consists of an LNA or a derivative thereof. More preferably, the oligonucleotide according to the invention is modified over its full length with a sugar modification selected from 2'-*O*-methyl, 2'-*O*-(2-methoxy)ethyl, morpholino, bridged nucleic acid (BNA) or BNA/DNA mixmer. In a more preferred embodiment, an oligonucleotide of the invention is fully 2'-*O*-methyl modified.

In a preferred embodiment, the oligonucleotide of the invention comprises at least one sugar modification and at least one modified internucleoside linkage. Such modifications include peptide-base nucleic acid (PNA), boron-cluster modified PNA, pyrrolidine-based oxy-peptide nucleic acid (POPNA), glycol- or glycerol-based nucleic acid (GNA), threose-based nucleic acid (TNA), acyclic threoninol-based nucleic acid (aTNA), morpholino-based oligonucleotides (PMO, PPMO, PMO-X), cationic morpholino-based oligomers (PMOPlus, PMO-X), oligonucleotides with integrated bases and backbones (ONIBs), pyrrolidine-amide oligonucleotides (POMs); and their derivatives. In a preferred embodiment, the oligonucleotide of the invention comprises a peptide nucleic acid backbone and/or a morpholino phosphorodiamidate backbone or a derivative thereof. In a more preferred embodiment, the oligonucleotide according to the invention is 2'-*O*-methyl phosphorothioate modified, i.e. comprises at least one 2'-*O*-methyl phosphorothioate modification, preferably the oligonucleotide according tot he invention is fully 2'-*O*-methyl phosphorothioate modified. Preferably, the 2'-*O*-methyl phosphorothioate modified oligonucleotide or the fully 2'-*O*-methyl phosphorothioate modified oligonucleotide is an RNA oligonucleotide.

The term "base modification" or "modified base" as identified herein refers to the modification of a naturally occurring base in RNA and/or DNA (i.e. pyrimidine or purine base) or to *de novo* synthesized bases. Such *de novo* synthesized base could be qualified as "modified" by comparison to an existing base.

In addition to the modifications described above, the oligonucleotide of the invention may comprise further modifications such as different types of nucleic acid nucleotide residues or nucleotides as described below. Different types of nucleic acid nucleotide residues may be used to generate an oligonucleotide of the invention. Said oligonucleotide may have at least one backbone, and/or sugar modification and/or at least one base modification compared to an RNA or DNA-based oligonucleotide.

An oligonucleotide may comprise the natural bases purines (adenine, guanine), or pyrimidines (cytosine, thymine, uracil) and/or modified bases as defined below. Within the context of the invention, a uracil may be replaced by a thymine.

A base modification includes a modified version of the natural purine and pyrimidine bases (e.g. adenine, uracil, guanine, cytosine, and thymine), such as hypoxanthine, orotic acid, agmatidine, lysidine, pseudouracil, pseudothymine, N1-methyl pseudouracil, 2-thiopyrimidine (e.g. 2-thiouracil, 2-thiothymine), 2,6-diaminopurine, G-clamp and its derivatives, 5-substituted pyrimidine (e.g. 5-halouracil, 5-methyluracil, 5-methylcytosine, 5-propynyluracil, 5-propynylcytosine, 5-aminomethyluracil, 5-hydroxymethyluracil, 5-aminomethylcytosine, 5-hydroxymethylcytosine, Super T), 5-octylpyrimidine, 5-thiophenepyrimidine, 5-octyn-1-ylpyrimidine, 5-ethynylpyrimidine, 5-(pyridylamide), 5-isobutyl, 5-phenyl as described in patent application US 2013/0131141 (RXi); 7-deazaguanine, 7-deazaadenine, 7-aza-2,6-diaminopurine, 8-aza-7-deazaguanine, 8-aza-7-deazaadenine, 8-aza-7-deaza-2,6-diaminopurine, Super G, Super A, and N4-ethylcytosine, or derivatives thereof; N2-cyclopentylguanine (cPent-G), N2-cyclopentyl-2-aminopurine (cPent-AP), and N2-propyl-2-aminopurine (Pr-AP), or derivatives thereof; and degenerate or universal bases, like 2,6-difluorotoluene or absent bases like abasic sites (e.g. 1-deoxyribose, 1,2-dideoxyribose, 1-deoxy-2-*O*-methylribose; or pyrrolidine derivatives in which the ring oxygen has been replaced with nitrogen (azaribose)). Examples of derivatives of Super A, Super G and Super T can be found in US patent 6,683,173 (Epoch Biosciences). cPent-G, cPent-AP and Pr-AP were shown to reduce immunostimulatory effects when incorporated in siRNA (Peacock H. et al.), and similar features were shown for pseudouracil and N1-methylpseudouracil (US patent application 2013/0123481, modeRNA Therapeutics).

'Thymine' and '5-methyluracil' may be interchanged throughout the document. In analogy, '2,6-diaminopurine' is identical to '2-aminoadenine' and these terms may be interchanged throughout the document.

In a preferred embodiment, the oligonucleotide of the invention comprises at least one 5-methylcytosine and/or at least one 5-methyluracil and/or at least one 2,6-diaminopurine base, which is to be understood that at least one of the cytosine nucleobases of said oligonucleotide has been modified by substitution of the proton at the 5-position of the pyrimidine ring with a methyl group (i.e. a 5-methylcytosine), and/or that at least one of the uracil nucleobases of said oligonucleotide has been modified by substitution of the proton at the 5-position of the pyrimidine ring with a methyl group (i.e. a 5-methyluracil), and/or that at least one of the adenine nucleobases of said oligonucleotide has been modified by substitution of the proton at the 2-position with an amino group (i.e. a 2,6-diaminopurine), respectively. Within the context of the invention, the expression "the substitution of a proton with a methyl group in position 5 of the pyrimidine ring" may be replaced by the expression "the substitution of a pyrimidine with a 5-methylpyrimidine," with pyrimidine referring to only uracil, only cytosine or both. Likewise, within the context of the invention, the expression "the substitution of a proton with an amino group in position 2 of adenine" may be replaced by the expression "the substitution of an adenine with a 2,6-diaminopurine." If said oligonucleotide comprises 1, 2, 3, 4, 5, 6, 7, 8, 9 or more cytosines, uracils, and/or adenines, at least 1, 2, 3, 4, 5, 6, 7, 8, 9 or more cytosines, uracils and/or adenines respectively may have been modified this way. In a preferred embodiment, all cytosines, all uracils and/or all adenines have been modified this way or replaced by 5-methylcytosine, 5-methyluracil and/or 2,6-diaminopurine, respectively.

It was found that the presence of a 5-methylcytosine, a 5-methyluracil and/or a 2,6-diaminopurine in an oligonucleotide of the invention has a positive effect on at least one of the parameters or an improvement of at least one parameters of said oligonucleotide. In this context, parameters may include: binding affinity and/or kinetics, silencing activity, biostability, (intra-tissue) distribution, cellular uptake and/or trafficking, and/or immunogenicity of said oligonucleotide, as explained below.

Since several modifications as mentioned above are known to increase the Tm value and thus enhance binding of a certain nucleotide to its counterpart on its target mRNA, these modifications can be explored to promote the binding of an oligonucleotide of the invention with both a region of a first and of a second exon in the context of the invention. Since sequences of an oligonucleotide of the invention may not be 100% reverse complementary to a given region of a first exon and/or of a second exon, Tm-increasing modifications such as a bridged nucleotide or BNA (such as LNA) or a base modification selected from 5-methylpyrimidines and/or 2,6-diaminopurine may preferably be implemented at a nucleotide position that is reverse complementary to saidfirst and /or said second region of said exons.

Binding affinity and/or binding or hybridisation kinetics depend on the AON's thermodynamic properties. These are at least in part determined by the melting temperature of said oligonucleotide (Tm; calculated with e.g. the oligonucleotide properties calculator (http://www.unc.edu/∼cail/biotool/oligo/index.html or http://eu.idtdna.com/analyzer/Applications/OligoAnalyzer/) for single stranded RNA using the basic Tm and the nearest neighbor model), and/or the free energy of the oligonucleotide-target exon complex (using RNA structure version 4.5 or RNA mfold version 3.5). If a Tm is increased, the exon skipping activity typically increases, but when a Tm is too high, the AON is expected to become less sequence-specific. An acceptable Tm and free energy depend on the sequence of the oligonucleotide. Therefore, it is difficult to give preferred ranges for each of these parameters.

An activity of an oligonucleotide of the invention is preferably defined as follows:
- alleviating one or more symptom(s) of a disease associated with a mutation present in a first and/or in a second and/or a mutation present within the stretch starting at said first and ending at said second exon, preferably alleviating one or more symptom(s) of DMD or BMD; and/or
- alleviating one or more characteristics of a cell from a patient, preferably a muscle cell from a patient; and/or
- providing said individual with a functional or semi-functional protein, preferably a functional or semi-functional dystrophin protein; and/or
- at least in part decreasing the production of an aberrant protein in said individual, preferably at least in part decreasing the production of an aberrant dystrophin protein in said individual. Each of these features and assays for assessing them has been defined later herein.

A preferred oligonucleotide of the invention, comprising a 5-methylcytosine and/or a 5-methyluracil and/or a 2,6-diaminopurine base is expected to exhibit an increased activity by comparison to the corresponding activity of an oligonucleotide without any 5-methylcytosine, without any 5-methyluracil and without any 2,6-diaminopurine base. This difference in terms of activity may be of at least 1%, 5%, 10%, 15%, 20%, 25%, 30%, 35%, 40%, 45%, 50%, 55%, 60%, 65%, 70%, 75%, 80%, 85%, 90%, 95%, or 100%. Biodistribution and biostability are preferably at least in part determined by a validated hybridization ligation assay adapted from Yu et al., 2002. In an embodiment, plasma or homogenized tissue samples are incubated with a specific capture oligonucleotide probe. After separation, a DIG-labeled oligonucleotide is ligated to the complex and detection followed using an anti-DIG antibody-linked peroxidase. Non-compartmental pharmacokinetic analysis is performed using WINNONLIN software package (model 200, version 5.2, Pharsight, Mountainview, CA). Levels of AON (ug) per mL plasma or mg tissue are monitored over time to assess area under the curve (AUC), peak concentration (Cmax), time to peak concentration (Tmax), terminal half-life and absorption lag time (tlag). Such a preferred assay has been disclosed in the experimental part. An oligonucleotide may stimulate an innate immune response by activating the Toll-like receptors (TLR), including TLR9 and TLR7 (Krieg A.M., et al., 1995). The activation of TLR9 typically occurs due to the presence of non-methylated CG sequences present in oligodeoxynucleotides (ODNs), by mimicking bacterial DNA which activates the innate immune system through TLR9-mediated cytokine release. The 2'-*O*-methyl modification may however markedly reduce such possible effect. TLR7 has been described to recognize uracil repeats in RNA (Diebold S.S., et al., 2006).

The activation of TLR9 and TLR7 results in a set of coordinated immune responses that include innate immunity (macrophages, dendritic cells (DC), and NK cells) (Krieg A.M., et al., 1995; Krieg A.M., et al 2000). Several chemo- and cytokines, such as IP-10, TNFα, IL-6, MCP-1 and IFNα (Wagner H., et al, 1999; Popovic P.J., et al., 2006) have been implicated in this process. The inflammatory cytokines attract additional defensive cells from the blood, such as T and B cells. The levels of these cytokines can be investigated by in vitro testing. In short, human whole blood is incubated with increasing concentrations of oligonucleotides after which the levels of the cytokines are determined by standard commercially available ELISA kits. A decrease in immunogenicity preferably corresponds to a detectable decrease of concentration of at least one of the cytokines mentioned above by comparison to the concentration of corresponding cytokine in an assay in a cell treated with an oligonucleotide comprising at least one 5-methylcytosine and/or 5-methyluracil, and/or 2,6-diaminopurine compared to a cell treated with a corresponding oligonucleotide having no 5-methylcytosines, 5-methyluracils, or 2,6-diaminopurines.

Accordingly, a preferred oligonucleotide of the invention has an improved parameter, such as an acceptable or a decreased immunogenicity and/or a better biodistribution and/or acceptable or improved RNA binding kinetics and/or thermodynamic properties by comparison to a corresponding oligonucleotide without a 5-methylcytosine, without a 5-methyluracil and without a 2,6-diaminopurine. Each of these parameters could be assessed using assays known to the skilled person.

A preferred oligonucleotide of the invention comprises or consists of an RNA molecule or a modified RNA molecule. In a preferred embodiment, an oligonucleotide is single stranded. The skilled person will understand that it is however possible that a single stranded oligonucleotide may form an internal double stranded structure. However, this oligonucleotide is still named a single stranded oligonucleotide in the context of this invention. A single stranded oligonucleotide has several advantages compared to a double stranded siRNA oligonucleotide: (i) its synthesis is expected to be easier than two complementary siRNA strands; (ii) there is a wider range of chemical modifications possible to enhance uptake in cells, a better (physiological) stability and to decrease potential generic adverse effects; (iii) siRNAs have a higher potential for non-specific effects (including off-target genes) and exaggerated pharmacology (e.g. less control possible of effectiveness and selectivity by treatment schedule or dose) and (iv) siRNAs are less likely to act in the nucleus and cannot be directed against introns.

In another embodiment, an oligonucleotide of the invention comprises an abasic site or an abasic monomer. Within the context of the invention, such monomer may be called an abasic site or an abasic monomer. An abasic monomer is a nucleotide residue or building block that lacks a nucleobase by comparison to a corresponding nucleotide residue comprising a nucleobase. Within the invention, an abasic monomer is thus a building block part of an oligonucleotide but lacking a nucleobase. Such abasic monomer may be present or linked or attached or conjugated to a free terminus of an oligonucleotide.

In a more preferred embodiment, an oligonucleotide of the invention comprises 1-10 or more abasic monomers. Therefore, 1, 2, 3, 4, 5, 6, 7, 8, 9, 10 or more abasic monomers may be present in an oligonucleotide of the invention.

An abasic monomer may be of any type known and conceivable by the skilled person, non-limiting examples of which are depicted below: 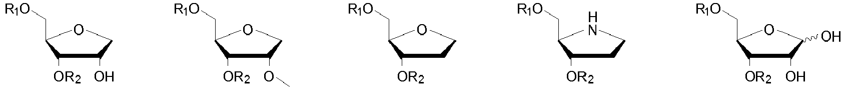 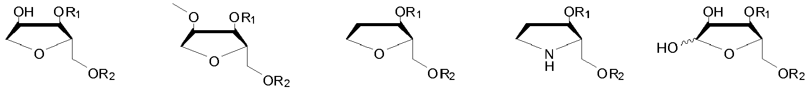 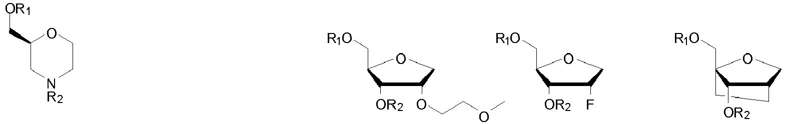 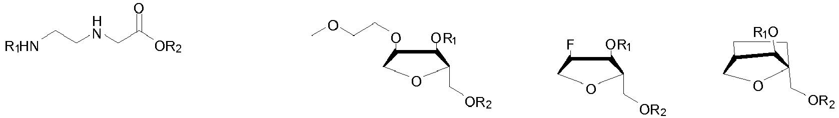 Herein, R₁ and R₂ are independently H, an oligonucleotide or other abasic site(s), provided that not both R₁ and R₂ are H and R₁ and R₂ are not both an oligonucleotide. An abasic monomer(s) can be attached to either or both termini of the oligonucleotide as specified before. It should be noted that an oligonucleotide attached to one or two an abasic site(s) or abasic monomer(s) may comprise less than 10 nucleotides. In this respect, the oligonucleotide according to the invention may comprise at least 10 nucleotides, optionally including one or more abasic sites or abasic monomers at one or both termini. Other examples of abasic sites that are encompassed by the invention are described in US patent application 2013/013378 (Alnylam Pharmaceuticals).

Depending on its length an oligonucleotide of the invention may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 base modifications. It is also encompassed by the invention to introduce more than one distinct base modification in said oligonucleotide.

Thus, in one embodiment the oligonucleotide according to the invention comprises:
(a) at least one base modification selected from 2-thiouracil, 2-thiothymine, 5-methylcytosine, 5-methyluracil, thymine, 2,6-diaminopurine; and/or
(b) at least one sugar modification selected from 2'-*O*-methyl, 2'-*O*-(2-methoxy)ethyl, 2'-O-deoxy (DNA), 2'-F, morpholino, a bridged nucleotide or BNA, or the oligonucleotide comprises both bridged nucleotides and 2'-deoxy modified nucleotides (BNA/DNA mixmers); and/or
(c) at least one backbone modification selected from phosphorothioate or phosphorodiamidate.

In another embodiment the oligonucleotide according to the invention comprises:
(a) at least one base modification selected from 5-methylpyrimidine and 2,6-diaminopurine; and/or
(b) at least one sugar modification, which is 2'-*O*-methyl; and/or
(c) at least one backbone modification, which is phosphorothioate.

In an embodiment, an oligonucleotide of the invention comprises at least one modification compared to a naturally occurring ribonucleotide- or deoxyribonucleotide-based oligonucleotide, more preferably
(a) at least one base modification, preferably selected from 2-thiouracil, 2-thiothymine, 5-methylcytosine, 5-methyluracil, thymine, 2,6-diaminopurine, more preferably selected from 5-methylpyrimidine and 2,6-diaminopurine; and/or
(b) at least one sugar modification, preferably selected from 2'-*O*-methyl, 2'-*O*-(2-methoxy)ethyl, 2'-*O*-deoxy (DNA), 2'-F, morpholino, a bridged nucleotide or BNA, or the oligonucleotide comprises both bridged nucleotides and 2'-deoxy modified nucleotides (BNA/DNA mixmers), more preferably the sugar modification is 2'-*O*-methyl; and/or
(c) at least one backbone modification, preferably selected from phosphorothioate or phosphorodiamidate, more preferably the backbone modification is phosphorothioate.

Thus, an oligonucleotide according to this embodiment of the invention comprises a base modification (a) and no sugar modification (b) and no backbone modification (c). Another preferred oligonucleotide according to this aspect of the invention comprises a sugar modification (b) and no base modification (a) and no backbone modification (c). Another preferred oligonucleotide according to this aspect of the invention comprises a backbone modification (c) and no base modification (a) and no sugar modification (b). Also oligonucleotides having none of the above-mentioned modifications are understood to be covered by the present invention, as well as oligonucleotides comprising two, i.e. (a) and (b), (a) and (c) and/or (b) and (c), or all three of the modifications (a), (b) and (c), as defined above.

In a preferred embodiment, the oligonucleotide according to the invention is modified over its entire length with one or more of the same modification, selected from (a) one of the base modifications; and/or (b) one of the sugar modifications; and/or (c) one of the backbone modifications.

With the advent of nucleic acid mimicking technology, it has become possible to generate molecules that have a similar, preferably the same hybridization characteristics in kind not necessarily in amount as nucleic acid itself. Such functional equivalents are of course also suitable for use in the invention.

In another preferred embodiment, an oligonucleotide comprises an inosine, a hypoxanthine, a universal base, a degenerate base and/or a nucleoside or nucleotide containing a base able to form a wobble base pair or a functional equivalent thereof. The use of an inosine (hypoxanthine) and/or a universal base and/or a degenerate base and/or a nucleotide containing a base able to form a wobble base pair in an oligonucleotide of the invention is very attractive as explained below. Inosine for example is a known modified base which can pair with three bases: uracil, adenine, and cytosine. Inosine is a nucleoside that is formed when hypoxanthine is attached to a ribose ring (also known as a ribofuranose) via a β [beta]-N9-glycosidic bond. Inosine (I) is commonly found in tRNAs and is essential for proper translation of the genetic code in wobble base pairs. A wobble base pair may exist between G and U, or between I on one hand and U, A or C on the other hand. These are fundamental in the formation of RNA secondary structure. Its thermodynamic stability is comparable to that of the Watson-Crick base pair. The genetic code makes up for disparities in the number of amino acids (20) for triplet codons (64), by using modified base pairs in the first base of the anti-codon.

A first advantage of using an inosine (hypoxanthine) and/or a universal base and/or a degenerate base and/or a nucleotide containing a base able to form a wobble base pair in an oligonucleotide of the invention allows one to design an oligonucleotide that is capable of binding to a region of a first exon from a pre-mRNA and is capable of binding to a region of a second exon within the same pre-mRNA, wherein said region from said second exon has at least 50% identity with said region of said first exon. In other words, the presence of an inosine (hypoxanthine) and/or a universal base and/or a degenerate base and/or a nucleotide containing a base able to form a wobble base pair in an oligonucleotide of the invention allows the binding of said oligonucleotide to a region of a first exon and to a region of a second exon of said pre-mRNA.

A second advantage of using an inosine (hypoxanthine) and/or a universal base and/or a degenerate base and/or a nucleotide containing a base able to form a wobble base pair in an oligonucleotide of the invention allows one to design an oligonucleotide that spans a single nucleotide polymorphism (SNP), without concern that the polymorphism will disrupt the oligonucleotide's annealing efficiency. Therefore in the invention, the use of such a base allows to design an oligonucleotide that may be used for an individual having a SNP within the pre-mRNA stretch which is targeted by an oligonucleotide of the invention.

A third advantage of using an inosine (hypoxanthine) and/or a universal base and/or a degenerate base and/or a nucleotide containing a base able to form a wobble base pair in an oligonucleotide of the invention is when said oligonucleotide would normally contain a CpG if one would have designed it as being complementary to a part of a first pre-mRNA exon as identified herein. The presence of a CpG in an oligonucleotide is usually associated with an increased immunogenicity of said oligonucleotide (Dorn A. and Kippenberger S.,). This increased immunogenicity is undesired since it may induce the breakdown of muscle fibers. Replacing the guanine by an inosine in one, two or more CpGs in said oligonucleotide is expected to provide an oligonucleotide with a decreased and/or acceptable level of immunogenicity. Immunogenicity may be assessed in an animal model by assessing the presence of CD4⁺ and/or CD8⁺ cells and/or inflammatory mononucleocyte infiltration in muscle biopsy of said animal. Immunogenicity may also be assessed in blood of an animal or of a human being treated with an oligonucleotide of the invention by detecting the presence of a neutralizing antibody and/or an antibody recognizing said oligonucleotide using a standard immunoassay known to the skilled person. An increase in immunogenicity preferably corresponds to a detectable increase of at least one of these cell types by comparison to the amount of each cell type in a corresponding muscle biopsy of an animal before treatment or treated with a corresponding oligonucleotide having at least one an inosine (hypoxanthine) and/or a universal base and/or a degenerate base and/or a nucleotide containing a base able to form a wobble base pair. Alternatively, an increase in immunogenicity may be assessed by detecting the presence or an increasing amount of a neutralizing antibody or an antibody recognizing said oligonucleotide using a standard immunoassay. A decrease in immunogenicity preferably corresponds to a detectable decrease of at least one of these cell types by comparison to the amount of corresponding cell type in a corresponding muscle biopsy of an animal before treatment or treated with a corresponding oligonucleotide having no inosine (hypoxanthine) and/or universal base and/or degenerate base and/or nucleotide containing a base able to form a wobble base pair. Alternatively a decrease in immunogenicity may be assessed by the absence of or a decreasing amount of said compound and/or neutralizing antibodies using a standard immunoassay.

A fourth advantage of using an inosine (hypoxanthine) and/or a universal base and/or a degenerate base and/or a nucleotide containing a base able to form a wobble base pair in an oligonucleotide of the invention is to avoid or decrease a potential multimerisation or aggregation of oligonucleotides. It is for example known that an oligonucleotide comprising a G- quartet motif has the tendency to form a quadruplex, a multimer or aggregate formed by the Hoogsteen base-pairing of four single-stranded oligonucleotides (Cheng A.J. and Van Dyke M.W.), which is of course not desired: as a result the efficiency of the oligonucleotide is expected to be decreased. Multimerisation or aggregation is preferably assessed by standard polyacrylamid non- denaturing gel electrophoresis techniques known to the skilled person. In a preferred embodiment, less than 20% or 15%, 10%, 7%, 5% or less of a total amount of an oligonucleotide of the invention has the capacity to multimerise or aggregate assessed using the assay mentioned above.

A fifth advantage of using an inosine (hypoxanthine) and/or a universal base and/or a degenerate base and/or a nucleotide containing a base able to form a wobble base pair in an oligonucleotide of the invention is thus also to avoid quadruplex structures which have been associated with antithrombotic activity (Macaya R.F., et al.) as well as with the binding to, and inhibition of, the macrophage scavenger receptor (Suzuki K., et al.,).

A sixth advantage of using an inosine (hypoxanthine) and/or a universal base and/or a degenerate base and/or a nucleotide containing a base able to form a wobble base pair in an oligonucleotide of the invention is to allow to design an oligonucleotide with improved RNA binding kinetics and/or thermodynamic properties. The RNA binding kinetics and/or thermodynamic properties are at least in part determined by the melting temperature of an oligonucleotide (Tm; calculated with the oligonucleotide properties calculator (http://www.unc.edu/∼cail/biotool/oligo/index.html) for single stranded RNA using the basic Tm and the nearest neighbor model), and/or the free energy of the AON-target exon complex (using RNA structure version 4.5). If a Tm is too high, the oligonucleotide is expected to be less specific. An acceptable Tm and free energy depend on the sequence of the oligonucleotide. Therefore, it is difficult to give preferred ranges for each of these parameters. An acceptable Tm may be ranged between 35 and 85 °C and an acceptable free energy may be ranged between 15 and 45 kcal/mol.

Depending on its length, an oligonucleotide of the present invention may comprise at least 1, 2, 3, 4, 5, 6, 7, 8, 9, 10 inosines (hypoxanthine) and/or universal bases and/or degenerate bases and/or nucleotides containing a base able to form a wobble base pair or a functional equivalents thereof.

Preferably, said oligonucleotide of the invention comprises RNA, as RNA/RNA duplexes are very stable. It is preferred that an RNA oligonucleotide comprises a modification providing the RNA with an additional property, for instance resistance to endonucleases, exonucleases, and RNaseH, additional hybridisation strength, increased stability (for instance in a bodily fluid), increased or decreased flexibility, reduced toxicity, increased intracellular transport, tissue-specificity, etc. Preferred modifications have been identified above.

One embodiment thus provides an oligonucleotide which comprises at least one modification. A preferred modified oligonucleotide is fully 2'-*O*-methyl modified. In one embodiment of the invention, an oligonucleotide comprises or consists of a hybrid oligonucleotide comprising a 2'-*O*-methyl phosphorothioate oligoribonucleotide modification and a bridged nucleic acid modification (BNA, as exemplified above). In another embodiment of the invention, an oligonucleotide comprises or consists of a hybrid oligonucleotide comprising a 2'-*O*-methoxyethyl phosphorothioate modification and a bridged nucleic acid (BNA, as exemplified above). In another embodiment of the invention, an oligonucleotide comprises or consists of a hybrid oligonucleotide comprising a bridged nucleic acid (BNA, as exemplified above) modification and an oligodeoxyribonucleotide modification. This particular combination comprises better sequence specificity compared to an equivalent consisting of bridged nucleic acid only, and comprises improved efficacy when compared with an oligonucleotide consisting of 2'-*O*-methylphosphorothioate oligo(deoxy)ribonucleotide modification.

The compound as described in the invention may preferably possess ionizable groups. Ionizable groups may be bases or acids, and may be charged or neutral. Ionizable groups may be present as ion pair with an appropriate counterion that carries opposite charge(s). Examples of cationic counterions are sodium, potassium, cesium, Tris, lithium, calcium, magnesium, trialkylammonium, triethylammonium, and tetraalkylammonium. Examples of anionic counterions are chloride, bromide, iodide, lactate, mesylate, acetate, trifluoroacetate, dichloroacetate, and citrate. Examples of counterions have been described (*e.g*. Kumar L,). Examples of application of di- or trivalent counterions, especially Ca²⁺, have been described as adding positive characteristics to selected oligonucleotides in US Pat. Appl. 2012046348 (Replicor). Preferred divalent or trivalent counterions are selected from the following list or group: calcium, magnesium, cobalt, iron, manganese, barium, nickel, copper, and zinc. A preferred divalent counterion is Calcium. It is therefore encompassed by the present invention to prepare, obtain and use a composition comprising an oligonucleotide of the invention and any other counterion identified above, preferably calcium. Such a method for the preparation of said composition comprising said oligonucleotide and said counterion, preferably calcium, may be as follows: an oligonucleotide of the invention may be dissolved in a pharmaceutically acceptable aqueous excipient, and gradually a solution comprising said counterion is added to the dissolved oligonucleotide such that the oligonucleotide chelate complex remains soluble.

Therefore in a preferred embodiment, an oligonucleotide of the invention has been contacted with a composition comprising such counterion preferably Ca²⁺, to form an oligonucleotide chelate complex comprising two or more identical oligonucleotides linked by such a counterion as identified herein. A composition comprising an oligonucleotide chelate complex comprising two or more identical oligonucleotides linked by a counterion, preferably calcium, is therefore encompassed by the present invention.

### Method for designing an oligonucleotide

Accordingly in a further aspect of the invention, there is provided a method for designing an oligonucleotide wherein said method leads to an oligonucleotide as identified above.

This method comprises the following steps:
(a) identifying an in-frame combination of a first and a second exon in a same pre-mRNA, wherein a region of said second exon has at least 50% identity with a region of said first exon;
(b) designing an oligonucleotide that is capable of binding to said region of said first exon and said region of said second exon,
(c) wherein said binding results in the skipping of said first exon and said second exon, preferably in the skipping of a multi-exon stretch starting with said first exon and encompassing one or more exons present between said first and said second exons and at the most in the skipping of the entire stretch of exons in between said first and said second exons, and
(d) wherein said method leads to an oligonucleotide .

In step b) of such a method said oligonucleotide is preferably designed so that its binding interferes with at least one splicing regulatory sequence in said regions of said first and/or second exons in said pre-mRNA.

Alternatively or in combination with the interference of a splicing regulatory sequence, the binding of said oligonucleotide preferably interferes with the secondary structure encompassing at least said first and/or said second exons in said pre-mRNA.

The oligonucleotide obtainable by this method is capable of inducing the skipping of said first and second exons of said pre-mRNA. Preferably the skipping of additional exon(s) is induced, wherein said additional exon(s) is/are preferably located in between said first and said second exon, and wherein the resulting mRNA transcript is in frame. The oligonucleotide is preferably capable of inducing the skipping of the entire stretch of exons between said first exon and said second exon. In an embodiment, said regions of said first exon and of said second exon comprise a splicing regulatory element, so that the binding of said oligonucleotide is capable of interfering with at least one splicing regulatory sequence in said regions of said first and second exons. It is also encompassed that the binding of said oligonucleotide interferes with the secondary structure encompassing at least said first and/or said second exons in said pre-mRNA. Preferred splicing regulatory sequence comprises a binding site for a serine-arginine (SR) protein, an exonic splicing enhancer (ESE), an exon recognition sequence (ERS) and/or an exonic splicing silencer (ESS).

Each feature of this method has already been defined herein, or is known to the skilled person.

### Preferred oligonucleotides

Preferably, an oligonucleotide of the invention is for use as a medicament, more preferably said compound is for use in RNA modulating therapeutics. More preferred, said RNA modulation leads to correction of a disrupted transcriptional reading frame and/or to restoration of the expression of a desired or anticipated protein. The method of the invention and the oligonucleotide of the invention may thus in principle be applied to any disease linked to the presence of a frame-disrupting mutation, leading to an aberrant transcript and/or to the absence of an encoded protein and/or to the presence of an aberrant protein. In a preferred embodiment the method of the invention and the oligonucleotide of the invention are applied to disease-associated genes carrying repetitive sequences and thus regions with relatively high sequence identity (i.e. at least 50%, 60%, 70%, 80% sequence identity between a region of a second exon and a region of a first exon as defined herein). Not limiting examples are genes with spectrin-like repeats (as the DMD gene involved in Duchenne muscular dystrophy as described herein), Kelch-like repeats (such as the KLHL3 gene involved in familial hyperkalemic hypertension (Louis-Dit-Picard H et al.), the KLHL6 gene involved in chronic lymphocytic leukemia (Puente XS et al.), the KLHL7 gene involved in retinitis pigmentosa (Friedman JS et al.), the KLHL7/12 genes involved in Sjögren's syndrome (Uchida K et al.), the KLHL9 gene involved in distal myopathy (Cirak S et al.), the KLHL16 or GAN gene involved in giant axonal neuropathy (Bomont P et al.), the KLHL19 or KEAP1 gene involved in several cancers (Dhanoa BS et al.), the KLHL20 gene involved in promyelocytic leukemia (Dhanoa BS et al.), or the KLHL37 or ENC1 gene involved in brain tumors (Dhanoa BS et al.)), FGF-like repeats, EGF-like repeats (such as the NOTCH3 gene involved in CADASIL (Chabriat H et al.), the SCUBE genes involved in cancer or metabolic bone disease, the neurexin-1 (NRXN1) gene involved in neuropsychiatric disorders and idiopathic generalized epilepsy (Moller RS et al.), the Del-1 gene, the Tenascin-C (TNC) gene involved in atherosclerosis and coronary artery disease (Mollie A et al.), the THBS3 gene, or the Fibrillin (FBN1) gene involved in Marfan syndrome (Rantamaki T et al.)), Ankyrin-like repeats (such as the ANKRD1 gene involved in dilated cardiomyopathy (Dubosq-Bidot L et al.), the ANKRD2 gene, the ANKRD11 gene involved in KBG syndrome (Sirmaci A et al.), the ANKRD26 gene involved in thrombocytopenia (Noris P et al.), or diabetes (Raciti GA et al.) the ANKRD55 gene involved in multiple sclerosis (Alloza I et al.), or the TRPV4 gene involved in distal spinal muscular atrophy (Fiorillo C et al.)), HEAT-like repeats (such as the htt gene involved in Huntington's disease), Annexin like-repeats, leucine-rich repeats (such as NLRP2 and NLRP7 genes involved in idiopathic recurrent miscarriage (Huang JY et al.), the LRRK2 gene involved in Parkinson's disease (Abeliovich A et al.),the NLRP3 gene involved in Alzheimer's disease or meningitis (Heneka MT et al.), the NALP3 gene involved in renal failure (Knauf F et al.), or the LRIG2 gene involved in urofacial syndrome (Stuart HM et al.)), or serine protease inhibitor domains (such as the SPINK5 gene involved in Netherton syndrome (Hovnanian A et al.), as described in Andrade M.A. et al.

Within the context of the invention a preferred pre-mRNA or transcript is the dystrophin pre-mRNA or transcript. This preferred pre-mRNA or transcript is preferably a human one. A disease linked to the presence of a mutation present in the dystrophin pre-mRNA is DMD or BMD depending on the mutation. Preferred combinations and regions of first and second exons from the dystrophin pre-mRNA to be used in the context of the invention are identified in table 2.

In a preferred embodiment, a compound of the invention is used for inducing exon-skipping in a cell, in an organ, in a tissue and/or in a patient, preferably in a BMD or DMD patient or in a cell, organ, tissue derived from said patient. Exon-skipping results in a mature mRNA that does not contain a skipped exon and thus, when said exon codes for amino acids can lead to the expression of an altered, internally truncated, but partly to largely functional dystrophin product. Technology for exon skipping is currently directed toward the use of AONs or AON transcribing gene constructs. Exon skipping techniques are nowadays explored in order to combat genetic muscular dystrophies. Promising results have recently been reported by us and others on AON-induced exon skipping therapy aimed at restoring the reading frame of the dystrophin pre-mRNA in cells from the *mdx* mouse and DMD patients (Heemskerk H., et al.,; Cirak S., et al.,; Goemans et al.,). By the targeted skipping of a specific exon, a severe DMD phenotype (lacking functional dystrophin) is converted into a milder BMD phenotype (expressing functional or semi-functional dystrophin). The skipping of an exon is preferably induced by the binding of AONs targeting an exon-internal sequence.

Below, the invention is illustrated by a mutated dystrophin pre-mRNA wherein a first and a second exon are present. As defined herein a dystrophin pre-mRNA preferably means a pre-mRNA of a DMD gene coding for a dystrophin protein. A mutated dystrophin pre-mRNA corresponds to a pre-mRNA of a BMD or DMD patient with a mutation when compared to a wild type DMD pre-mRNA of a non-affected person, resulting in (reduced levels of) an aberrant protein (BMD), or in the absence of functional dystrophin (DMD). A dystrophin pre-mRNA is also named a DMD pre-mRNA. A dystrophin gene may also be named a DMD gene. Dystrophin and DMD may be used interchangeably throughout the application.

A patient is preferably intended to mean a patient having DMD or BMD as later defined herein or a patient susceptible to develop DMD or BMD due to his (or her) genetic background. In the case of a DMD patient, a compound used will preferably correct a mutation as present in the DMD gene of said patient and therefore will preferably create a dystrophin protein that will look like a dystrophin protein from a BMD patient: said protein will preferably be a functional or semi-functional dystrophin as later defined herein. In the case of a BMD patient, an antisense oligonucleotide of the invention will preferably modify a mutation as present in the BMD gene of said patient and will preferably create a dystrophin which will be more functional than the dystrophin which was originally present in said BMD patient.

As defined herein, a functional dystrophin is preferably a wild type dystrophin corresponding to a protein having the amino acid sequence as identified in SEQ ID NO: 1. A functional dystrophin is preferably a dystrophin, which has an acting binding domain in its N terminal part (first 240 amino acids at the N terminus), a cysteine-rich domain (amino acids 3361 till 3685) and a C terminal domain (last 325 amino acids at the C terminus) each of these domains being present in a wild type dystrophin as known to the skilled person. The amino acids indicated herein correspond to amino acids of the wild type dystrophin being represented by SEQ ID NO: 1. In other words, a functional or semi-functional dystrophin is a dystrophin which exhibits at least to some extent an activity of a wild type dystrophin. "At least to some extent" preferably means at least 10%, 20%, 30%, 40%, 50%, 60%, 70%, 80%, 90%, 95% or 100% of a corresponding activity of a wild type functional dystrophin. In this context, an activity of a functional dystrophin is preferably binding to actin and interacting with the dystrophin-associated glycoprotein complex (DGC) or (DAGC) (Ehmsen J et al,). Binding of dystrophin to actin and to the DGC complex may be visualized by either co-immunoprecipitation using total protein extracts or immunofluorescence analysis of cross-sections, from a biopsy of a muscle suspected to be dystrophic, as known to the skilled person.

Individuals suffering from DMD typically have a mutation in the gene encoding dystrophin that prevents synthesis of the complete protein, e.g. a premature stop prevents the synthesis of the C-terminus. In BMD the dystrophin gene also comprises a mutation but this mutation does not disrupt the open reading frame and the C-terminus is synthesized. As a result a (semi-)functional or functional dystrophin protein is generated that has a similar activity in kind as the wild type protein, although not necessarily a similar amount of activity. The genome of a BMD individual typically encodes a dystrophin protein comprising the N terminal part (first 240 amino acids at the N terminus), a cysteine-rich domain (amino acid 3361 till 3685) and a C terminal domain (last 325 amino acids at the C terminus) but in most cases its central rod shaped domain may be shorter than the one of a wild type dystrophin (Monaco A.P., et al,). Exon skipping for the treatment of DMD is typically directed to bypass the premature stop in the pre-mRNA by skipping an exon flanking or containing the mutation. This allows correction of the open reading frame and synthesis of a internally truncated dystrophin protein but including the C-terminus. In a preferred embodiment, an individual having DMD and being treated with an oligonucleotide of the invention will synthesize a dystrophin which exhibits at least to some extent a similar activity of a wild type dystrophin. More preferably, if said individual is a DMD patient or is suspected to be a DMD patient, a (semi-) functional dystrophin is a dystrophin of an individual having BMD: typically said dystrophin is able to interact with both actin and the DGC or DAGC (Ehmsen J., et al., Monaco A.P., et al,).

The central rod domain of wild type dystrophin comprises 24 spectrin-like repeats (Ehmsen J., et al,). In many cases, the central rod shaped domain in BMD-like proteins is shorter than the one of a wild type dystrophin (Monaco A.P., et al.). For example, a central rod shaped domain of a dystrophin as provided herein may comprise 5 to 23, 10 to 22 or 12 to 18 spectrin-like repeats as long as it can bind to actin and to the DGC.

Alleviating one or more symptom(s) of DMD or BMD in an individual using a compound of the invention may be assessed by any of the following assays: prolongation of time to loss of walking, improvement of muscle strength, improvement of the ability to lift weight, improvement of the time taken to rise from the floor, improvement in the nine-meter walking time, improvement in the time taken for four-stairs climbing, improvement of the leg function grade, improvement of the pulmonary function, improvement of cardiac function, improvement of the quality of life. Each of these assays is known to the skilled person. As an example, the publication of Manzur et al (Manzur A.Y. et al,) gives an extensive explanation of each of these assays. For each of these assays, as soon as a detectable improvement or prolongation of a parameter measured in an assay has been found, it will preferably mean that one or more symptoms of DMD or BMD has been alleviated in an individual using a compound of the invention. Detectable improvement or prolongation is preferably a statistically significant improvement or prolongation as described in Hodgetts et al (Hodgetts S., et al,). Alternatively, the alleviation of one or more symptom(s) of DMD or BMD may be assessed by measuring an improvement of a muscle fiber function, integrity and/or survival. In a preferred method, one or more symptom(s) of a DMD or a BMD patient is/are alleviated and/or one or more characteristic(s) of one or more muscle cells from a DMD or a BMD patient is/are improved. Such symptoms or characteristics may be assessed at the cellular, tissue level or on the patient self.

An alleviation of one or more characteristics of a muscle cell from a DMD or BMD patient may be assessed by any of the following assays on a myogenic cell or muscle cell from that patient: reduced calcium uptake by muscle cells, decreased collagen synthesis, altered morphology, altered lipid biosynthesis, decreased oxidative stress, and/or improved muscle fiber function, integrity, and/or survival. These parameters are usually assessed using immunofluorescence and/or histochemical analyses of cross sections of muscle biopsies.

The improvement of muscle fiber function, integrity and/or survival may be assessed using at least one of the following assays: a detectable decrease of creatine kinase in blood, a detectable decrease of necrosis of muscle fibers in a biopsy cross-section of a muscle suspected to be dystrophic, and/or a detectable increase of the homogeneity of the diameter of muscle fibers in a biopsy cross-section of a muscle suspected to be dystrophic. Each of these assays is known to the skilled person.

Creatine kinase may be detected in blood as described in Hodgetts et al (Hodgetts S., et al,). A detectable decrease in creatine kinase may mean a decrease of 5%, 10%, 20%, 30%, 40%, 50%, 60%, 70%, 80%, 90% or more compared to the concentration of creatine kinase in a same DMD or BMD patient before treatment.

A detectable decrease of necrosis of muscle fibers is preferably assessed in a muscle biopsy, more preferably as described in Hodgetts et al (Hodgetts S., et al,) using biopsy cross-sections. A detectable decrease of necrosis may be a decrease of 5%, 10%, 20%, 30%, 40%, 50%, 60%, 70%, 80%, 90% or more of the area wherein necrosis has been identified using biopsy cross-sections. The decrease is measured by comparison to the necrosis as assessed in a same DMD or BMD patient before treatment.

A detectable increase of the homogeneity of the diameter of a muscle fiber is preferably assessed in a muscle biopsy cross-section, more preferably as described in Hodgetts et al (Hodgetts S., et al,). The increase is measured by comparison to the homogeneity of the diameter of a muscle fiber in a same DMD or BMD patient before treatment.

Preferably, an oligonucleotide of the invention provides said individual with (higher levels of) a functional and/or a (semi) functional dystrophin protein (both for DMD and BMD) and/or is able to, for at least in part decrease the production of an aberrant dystrophin protein in said individual. In this context, "a" functional and/or "a" semi-functional dystrophin may mean that several forms of functional and/or of semi-functional dystrophin could be generated. This may be expected when a region of at least 50% identity between a first and a second exon overlaps with one or more other region(s) of at least 50% identity between other first and second exons, and when said oligonucleotide is capable of binding to said overlapping part such that several different stretches of exons are skipped and several different in frame transcripts produced. This situation is illustrated in example 4 wherein one single oligonucleotide according to the invention (PS816; SEQ ID NO:1679) is able to induce the production of several in frame transcripts which all share exon 10 as first exon and wherein the second exon may be 13, 14, 15, 18, 20, 27, 30, 31, 32, 35, 42, 44, 47, 48 or 55.

Higher levels refer to the increase of a functional and/or (semi) functional dystrophin protein level by comparison to a corresponding level of a functional and/or a (semi) functional dystrophin protein in a patient before the onset of the treatment with an oligonucleotide of the invention. The level of said functional and/or (semi) functional dystrophin protein is preferably assessed using immunofluorescence or western blot analysis (protein).

Decreasing the production of an aberrant dystrophin mRNA, or aberrant dystrophin protein, preferably means that 90%, 80%, 70%, 60%, 50%, 40%, 30%, 20%, 10%, 5% or less of the initial amount of aberrant dystrophin mRNA, or aberrant dystrophin protein, is still detectable by RT PCR (mRNA) or immunofluorescence or western blot analysis (protein). An aberrant dystrophin mRNA or protein is also referred to herein as a less functional (compared to a wild type functional dystrophin protein as earlier defined herein) or non-functional dystrophin mRNA or protein. A non-functional dystrophin protein is preferably a dystrophin protein which is not able to bind actin and/or members of the DGC protein complex. A non-functional dystrophin protein or dystrophin mRNA does typically not have, or does not encode a dystrophin protein with an intact C-terminus of the protein. In a preferred embodiment an exon skipping (also called RNA-modulating or splice-switching) technique is applied.

Increasing the production of a functional and/or semi-functional dystrophin mRNA and/or protein, preferably means that such a functional and/or semi-functional dystrophin mRNA and/or protein is detectable or is increased of at least 5%, 10%, 20%, 30%, 40%, 50%, 60%, 70%, 80%, 90% or more by comparison to the detectable quantity of said mRNA and/or protein detectable at the onset of the treatment. Said detection may be carried out using RT-PCR (mRNA) or immunofluorescence or western blot analysis (protein).

In another embodiment, a compound of the invention provides said individual with a functional or semi-functional dystrophin protein. This functional or semi-functional dystrophin protein or mRNA may be detected as an aberrant dystrophin protein or mRNA as earlier explained herein.

By the targeted co-skipping of said two exons (said first and said second exons), the skipping of additional exon(s) may be induced wherein said additional exon(s) is/are preferably located in between said first and said second exons, and wherein the resulting dystrophin transcript is in-frame (preferably as in Table 1 or 6), a DMD or severe BMD phenotype is converted into a milder BMD or even asymptomatic phenotype. The co-skipping of said two exons is preferably induced by the binding of an oligonucleotide to a region of a first exon from the dystrophin pre-mRNA and by the binding of an oligonucleotide to a region of a second exon within the dystrophin pre-mRNA, wherein said region of said second exon has at least 50% identity with said region of said first exon. Preferably, said first and said second dystrophin exons, and the identity regions therein, are as identified in Table 2 or 6. Said oligonucleotide preferably exhibits no overlap with non-exon sequences. Said oligonucleotide preferably does not overlap with the splice sites at least not insofar as these are present in an intron. Said oligonucleotide directed toward an exon internal sequence preferably does not contain a sequence reverse-complementary to an adjacent intron. An exon skipping technique is preferably applied such that the absence of said two exons, preferably of additional exon(s), more preferably located in between said first and said second exons, from an mRNA produced from a DMD pre-mRNA generates a coding region for (higher) expression of a more (semi-) functional - albeit shorter - dystrophin protein. In this context (typically a BMD patient), inhibiting inclusion of said two exons, preferably of additional exon(s) located in between said first and said second exons, preferably means that:
- the level of the original, aberrant (less functional) dystrophin mRNA is decreased with at least 5% as assessed by RT-PCR, or that a corresponding aberrant dystrophin protein level is decreased with at least 2% as assessed by immunofluorescence or western blot analysis using anti-dystrophin antibodies; and/or
- an in frame transcript encoding a semi-functional or functional dystrophin protein is detectable or its level is increased of at least 5% as assessed by RT-PCR (mRNA level) or of at least 2% as assessed by immunofluorescence or western blot using anti-dystrophin antibodies (protein level).

The decrease in aberrant, less-functional or non-functional dystrophin protein is preferably at least 5%, 10%, 20%, 30%, 40%, 50%, 60%, 70%, 80%, 90% or 100% and is preferably in line or parallel to the detection or the increased production of a more functional or semi-functional dystrophin transcript or protein. In this context (typically a DMD patient), inhibiting inclusion of said two exons, preferably of additional exon(s) located in between said first and said second exons, preferably means that (higher levels of) a more functional or (semi) functional dystrophin protein or mRNA is provided to said individual.

Once a DMD patient is provided with (higher levels of) a (more) functional or semi-functional dystrophin protein, the cause of DMD is at least in part alleviated. Hence, it would then be expected that the symptoms of DMD are sufficiently reduced. The present invention further provides the insight that the skipping of an entire stretch of at least two dystrophin exons from a pre-mRNA comprising said exons is induced or enhanced, when using a single oligonucleotide directed toward (or capable of binding to or hybridizing to or reverse-complementary to or capable of targeting) both of the outer exons of said stretch. Throughout the application in a preferred embodiment, an oligonucleotide of the invention is therefore at least 80% reverse complementary to said said region of said first exon and at least 45% reverse complementary to said said region of said second exon as defined herein, wherein said first and second exons correspond to the outer exons of the exon stretch that is to be skipped. More preferably, said oligonucleotide is at least 85%, 90% 95% or 100% reverse complementary to said region of said first and at least 50%, 55%, 60%, 65%, 70%, 75%, 80%, 85%, 90%, 95% or 100% reverse complementary to said region of said second exon. The enhanced skipping frequency also increases the level of more (semi-) functional dystrophin protein produced in a muscle cell of a DMD or BMD individual.

An oligonucleotide according to the present invention that is preferably used,
is preferably reverse-complementary to or is capable of binding or hybridizing to or targeting a region of a first dystrophin exon, said region having at least 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 90, 100, 110, 120, 130, 140, 150, 160, 170, 180, 190, 200, or more nucleotides,
is preferably reverse-complementary to or is capable of binding or hybridizing to or targeting a region of a second dystrophin exon, said region having at least 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 90, 100, 110, 120, 130, 140, 150, 160, 170, 180, 190, 200, or more nucleotides, wherein said region of said second exon within the same pre-mRNA has at least 50% identity with said region of said first exon.

Within the context of the invention, an oligonucleotide may comprise or consist of a functional equivalent of an oligonucleotide. A functional equivalent of an oligonucleotide preferably means an oligonucleotide as defined herein wherein one or more nucleotides have been substituted and wherein an activity of said functional equivalent is retained to at least some extent. Preferably, an activity of said compound comprising a functional equivalent of an oligonucleotide is providing a functional or semi-functional dystrophin protein. Said activity of said compound comprising a functional equivalent of an oligonucleotide is therefore preferably assessed by quantifying the amount of a functional or a semi-functional dystrophin protein. A functional or semi-functional dystrophin is herein preferably defined as being a dystrophin able to bind actin and members of the DGC protein complex and to support the muscle fiber membrane structure and flexibility. The assessment of said activity of a compound comprising a functional oligonucleotide or equivalent preferably includes RT-PCR (to detect exon skipping on RNA level) and/or immunofluorescence or Western blot analysis (to detect protein expression and localisation). Said activity is preferably retained to at least some extent when it represents at least 50%, or at least 60%, or at least 70% or at least 80% or at least 90% or at least 95% or more of corresponding activity of said compound comprising an oligonucleotide where the functional equivalent derives from. Throughout this application, when the word oligonucleotide is used it may be replaced by a functional equivalent thereof as defined herein.

Hence, the use of an oligonucleotide, or a functional equivalent thereof, comprising or consisting of a sequence
which is capable of binding to, targeting, hybridizing to and/or is reverse-complementary to a region of a first dystrophin exon,
which is capable of binding targeting, hybridizing to and/or is reverse-complementary to a region of a second dystrophin exon
and
wherein said region of second exon within the dystrophin pre-mRNA has at least 50% identity with said region of said first exon, exhibits DMD therapeutic results by:
- alleviating one or more symptom(s) of DMD or BMD; and/or
- alleviating one or more characteristics of a muscle cell from a patient; and/or
- providing said individual with a functional or semi-functional dystrophin protein; and/or
- at least in part decreasing the production of an aberrant dystrophin protein in said individual.

Each of these features has already been defined herein.

Preferably, an oligonucleotide is comprising or consisting of a sequence which is capable of binding to, targeting, hybridizing and/or is reverse-complementary to a region of a first DMD or dystrophin exon, wherein a region of a second DMD or dystrophin exon within the same pre-mRNA has at least 50% identity with said region of said first exon. Said first and second exons are preferably selected from the group of exons including exons 8 to 60, wherein the stretch of exons starting with the first exon and comprising the second exon as last exon, when skipped, yields an in frame transcript. Preferred in-frame exon combinations in the dystrophin mRNA are given in Table 1, 2 or 6.

Without wishing to be bound by any theory, the identity of the first and second dystrophin exons may be determined by one or more of the following aspects. In one embodiment, one or more introns present between the first exon and the second exon are not exceptionally large. In this context, an exceptionally large intron in the dystrophin gene may be 70, 80, 90, 100, 200 kb or more; for example intron 1 (∼83 kb), intron 2 (∼170 kb), intron 7 (∼110 kb), intron 43 (∼70 kb), intron 44 (∼248 kb), intron 55 (∼119 kb), or intron 60 (∼96 kb). In addition in a further embodiment, there may already be an example of a BMD patient expressing a truncated dystrophin protein, wherein this first, this second and the exon stretch from said first exon to said second exon had been deleted. Another criteron may be the relatively large applicability of the skipped exons for combined subpopulations of DMD (and/or BMD) patients with specific relevant mutations.

In a preferred embodiment, an in-frame stretch of DMD exons is skipped (more preferably entirely skipped within one transcript), wherein the outer exons are defined by a first and a second exon as follows:
- first exon is exon 8 and the second exon is exon 19 (applicable to ∼7% of DMD patients),
- first exon is exon 9 and the second exon is exon 22 (applicable to ∼11% of DMD patients),
- first exon is exon 9 and the second exon is exon 30 (applicable to ∼14% of DMD patients),
- first exon is exon 10 and the second exon is exon 18 (applicable to ∼5% of DMD patients),
- first exon is exon 10 and the second exon is exon 30 (applicable to ∼13% of DMD patients),
- first exon is exon 10 and the second exon is exon 42 (applicable to ∼16% of DMD patients),
- first exon is exon 10 and the second exon is exon 47 (applicable to ∼29% of DMD patients),
- first exon is exon 10 and the second exon is exon 57 (applicable to ∼72% of DMD patients),
- first exon is exon 10 and the second exon is exon 60 (applicable to ∼72% of DMD patients),
- first exon is exon 11 and the second exon is exon 23 (applicable to ∼8% of DMD patients),
- first exon is exon 13 and the second exon is exon 30 (applicable to ∼10% of DMD patients),
- first exon is exon 23 and the second exon is exon 42 (applicable to ∼7% of DMD patients),
- first exon is exon 34 and the second exon is exon 53 (applicable to ∼42% of DMD patients),
- first exon is exon 40 and the second exon is exon 53 (applicable to ∼38% of DMD patients),
- first exon is exon 44 and the second exon is exon 56 (applicable to ∼40% of DMD patients),
- first exon is exon 45 and the second exon is exon 51 (applicable to ∼17% of DMD patients)
- first exon is exon 45 and the second exon is exon 53 (applicable to ∼28% of DMD patients),
- first exon is exon 45 and the second exon is exon 55 (applicable to ∼33% of DMD patients),
- first exon is exon 45 and the second exon is exon 60 (applicable to ∼37% of DMD patients), or
- first exon is exon 56 and the second exon is exon 60 (applicable to ∼2% of DMD patients).

Therefore, in an embodiment, an oligonucleotide of the invention induces the skipping of the following dystrophin exons: exons 8 to 19, exons 9 to 22, exons 9 to 30, exons 10 to 18, exons 10 to 30, exons 10 to 42, exons 10 to 47, exons 10 to 57, exons 10 to 60, exons 11 to 23, exons 13 to 30, exons 23 to 42, exons 34 to 53, exons 40 to 53, exons 44 to 56, exons 45 to 51, exons 45 to 53, exons 45 to 55, exons 45 to 60 or exons 56 to 60.

Preferably, an oligonucleotide of the invention comprises or consists of a sequence that is capable of binding to, targeting, hybridizing and/or is reverse-complementary to a region of a first exon of the dystrophin pre-mRNA such that the reverse-complementary part is at least 30% of the length of said oligonucleotide of the invention, more preferably at least 40%, even more preferably at least 50%, even more preferably at least 60%, even more preferably at least 70%, even more preferably at least 80%, even more preferably at least 90% or even more preferably at least 95%, or even more preferably 98% and most preferably up to 100%. In this context, a first exon is preferably exon 8, 9, 10, 11, 13, 23, 34, 40, 44, 45, or 56 of dystrophin pre-mRNA as defined herein. Said oligonucleotide may comprise additional flanking sequences. In a more preferred embodiment, the length of said reverse-complementary part of said oligonucleotide is at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides. Several types of flanking sequences may be used. Preferably, flanking sequences are used to modify the binding of a protein to said oligonucleotide, or to modify a thermodynamic property of said oligonucleotide, more preferably to modify target RNA binding affinity. In another preferred embodiment, additional flanking sequences are reverse-complementary to sequences of the dystrophin pre-mRNA which are not present in said exon.

In a preferred embodiment, an oligonucleotide comprises or consists of a sequence that is capable of binding to, targeting, hybridizing to, and is reverse-complementary to at least a region of a first and to a region of a second dystrophin exon as present in a dystrophin pre-mRNA wherein said first and second exons are selected from the group of exons 8 (SEQ ID NO:2), 9 (SEQ ID NO:3), 10 (SEQ ID NO:4), 11 (SEQ ID NO:1761), 13 (SEQ ID NO:1762),18 (SEQ ID NO:5), 19 (SEQ ID NO:6), 22 (SEQ ID NO:7), 23 (SEQ ID NO:8), 30 (SEQ ID NO:9), 34 (SEQ ID NO:1763), 40 (SEQ ID NO:1764), 42 (SEQ ID NO:11), 44 (SEQ ID NO:1765), 45 (SEQ ID NO:12), 47 (SEQ ID NO:10), 51 (SEQ ID NO:1760), 53 (SEQ ID NO:13), 55 (SEQ ID NO:14), 56 (SEQ ID NO:15), 57 (SEQ ID NO:1744), or 60 (SEQ ID NO:16), and said regions having at least 10 nucleotides. However, said regions may also have at least 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 90, 100, 110, 120, 130, 140, 150, 160, 170, 180, 190, 200, or more nucleotides. For the preferred exons identified above, the skilled person is able to identify a region of a first exon and a region of a second exon using techniques known in the art. More preferably the online tool EMBOSS Matcher is used as earlier explained herein. Even more preferably, preferred identity regions of first and second dystrophin exons to which an oligonucleotide of the invention preferably binds and/or is at least in part reverse-complementary to, have been provided in Table 2. Reverse complementarity is in this context preferably of at least 45%, 50%, 55%, 60%, 65%, 70%, 75%, 80%, 85%, 90%, 95% or 100%.A preferred oligonucleotide sequence to be used in the invention is capable of binding to, hybridizing to, targeting and/or is reverse-complementary to a region of identity between said first and second exons, preferably from Table 2, and has a length of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides, more preferably less than 40 nucleotides or more preferably less than 30 nucleotides, even more preferably less than 25 nucleotides and most preferably 20 to 25 nucleotides. Reverse complementarity is in this context preferably of at least 45%, 50%, 55%, 60%, 65%, 70%, 75%, 80%, 85%, 90%, 95% or 100%.

In an embodiment, a preferred oligonucleotide is such that the first and second dystrophin exons are as identified in table 1, 2 or 6 and said oligonucleotide is capable of binding to the corresponding first and second exon regions as identified in table 2 or 6 and as defined by SEQ ID NO:17 to 1670, 1742, 1743, or 1766 to 1777.

Preferred oligonucleotides are disclosed in Table 3 and comprise or consist of SEQ ID NO: 1671-1741. Other preferred oligonucleotides comprise or consist SEQ ID NO:1778-1891 as disclosed in Table 6. Preferred oligonucleotides comprise SEQ ID NO: 1671-1741 or 1778-1891 and have 1, 2, 3, 4, or 5 nucleotides more or 1, 2, 3, 4 or 5 nucleotides less than their exact SEQ ID NO as given in table 3 or 6. These additional nucleotides may be present at the 5'or 3' side of a given SEQ ID NO. These missing nucleotides may be nucleotides present at the 5'or 3'side of a given SEQ ID NO. Each of these oligonucleotides may have any of the chemistries as defined earlier herein or combinations thereof. In each of the oligonucleotides identified by a SEQ ID NO herein, a U may be replaced by a T.

More preferred oligonucleotides are depicted below.

If the first dystrophin exon is exon 8 and the second dystrophin exon is exon 19, a preferred region of exon 8 comprises or consists of SEQ ID NO:17 and a preferred region of exon 19 comprises or consists of SEQ ID NO:18. A preferred oligonucleotide consists of SEQ ID NO: 1722, or 1723, or comprises SEQ ID NO: 1722, or 1723 and has a length of 24, 25, 26, 27, 28, 29 or 30 nucleotides.

If the first dystrophin exon is exon 10 and the second dystrophin exon is exon 13, a preferred region of exon 10 comprises or consists of SEQ ID NO:101 and a preferred region of exon 13 comprises or consists of SEQ ID NO:102.

If the first dystrophin exon is exon 10 and the second dystrophin exon is exon 14, a preferred region of exon 10 comprises or consists of SEQ ID NO:103 and a preferred region of exon 14 comprises or consists of SEQ ID NO:104.

If the first dystrophin exon is exon 10 and the second dystrophin exon is exon 15, a preferred region of exon 10 comprises or consists of SEQ ID NO:105 and a preferred region of exon 15 comprises or consists of SEQ ID NO:106.

If the first dystrophin exon is exon 10 and the second dystrophin exon is exon 18, a preferred region of exon 10 comprises or consists of SEQ ID NO:109 and a preferred region of exon 18 comprises or consists of SEQ ID NO:110. Preferred oligonucleotides comprise:
- a base sequence as defined in any one of SEQ ID NO: 1679 to 1681, 1778, 1812, 1813, 1884 to 1886, 1890, or 1891, and have a length of 25, 26, 27, 28, 29 or 30 nucleotides or
- a base sequence as defined in SEQ ID NO: 1814 and have a length of 26, 27, 28, 29 or 30 nucleotides; or
- a base sequence as defined in any one of SEQ ID NO: 1815 to 1819 and have a length of 22, 23, 24, 25, 26, 27, 28, 29 or 30 nucleotides, or
- a base sequence as defined in any one of SEQ ID NO: 1820, 1824, and have a length of 20, 21, 22, 23, 24, 25, 26, 27, 28, 29 or 30 nucleotides, or
- a base sequence as defined in any one of SEQ ID NO: 1826, 1782, 1832 and have a length of 21, 22, 23, 24, 25, 26, 27, 28, 29 or 30 nucleotides, or
- a base sequence as defined in any one of SEQ ID NO: 1821, 1825, 1780 and have a length of 22, 23, 24, 25, 26, 27, 28, 29 or 30 nucleotides, or
- a base sequence as defined in any one of SEQ ID NO: 1822 and have a length of 24, 25, 26, 27, 28, 29 or 30 nucleotides, or
- a base sequence as defined in any one of SEQ ID NO: 1823, 1781, 1829, 1830, 1831 and have a length of 25, 26, 27, 28, 29 or 30 nucleotides,
- a base sequence as defined in any one of SEQ ID NO: 1887 and and have a length of 24, 25, 26, 27, 28, 29 or 30 nucleotides, or
- a base sequence as defined in any one of SEQ ID NO: 1888 or 1889 and and have a length of 26, 27, 28, 29 or 30 nucleotides, or
- a base sequence as defined in SEQ ID NO: 1827 and have a length of 21, 22, 23, 24, 25, 26, 27, 28, 29 or 30 nucleotides, or
- a base sequence as defined in SEQ ID NO: 1828 and have a length of 25, 26, 27, 28, 29 or 30 nucleotides.

If the first dystrophin exon is exon 10 and the second dystrophin exon is exon 18, another preferred region of exon 10 comprises or consists of SEQ ID NO:1766 and another preferred region of exon 18 comprises or consists of SEQ ID NO:1767. Preferred oligonucleotides comprise a base sequence as defined in any one of SEQ ID NO: 1783, 1833, 1834, 1835 and have a length of 17, 18, 19, 20, 21, 22, 23, 24, 25, 26, 27, 28, 29 or 30 nucleotides.

If the first dystrophin exon is exon 10 and the second dystrophin exon is exon 18, another preferred region of exon 10 comprises or consists of SEQ ID NO:1768 and another preferred region of exon 18 comprises or consists of SEQ ID NO:1769. Preferred oligonucleotides comprise a base sequence as defined in any one of SEQ ID NO: 1673 or 1674 and have a length of 25, 26, 27, 28, 29 or 30 nucleotides.

If the first dystrophin exon is exon 10 and the second dystrophin exon is exon 20, a preferred region of exon 10 comprises or consists of SEQ ID NO:111 and a preferred region of exon 20 comprises or consists of SEQ ID NO:112.

If the first dystrophin exon is exon 10 and the second dystrophin exon is exon 27, a preferred region of exon 10 comprises or consists of SEQ ID NO:121 and a preferred region of exon 27 comprises or consists of SEQ ID NO:122.

If the first dystrophin exon is exon 10 and the second dystrophin exon is exon 30, a preferred region of exon 10 comprises or consists of SEQ ID NO:127 and a preferred region of exon 30 comprises or consists of SEQ ID NO:128. Preferred oligonucleotides comprise:
- a base sequence as defined in any one of SEQ ID NO: 1679 to 1681, 1812, 1813, 1884 to 1886, 1890, or 1891 and have a length of 25, 26, 27, 28, 29 or 30 nucleotides or
- a base asequence as defined in SEQ ID NO: 1814 and have a length of 26, 27, 28, 29 or 30 nucleotides; or
- a base sequence as defined in SEQ ID NO: 1675 or 1676 and have a length of 21, 22, 23, 24, 25, 26, 27, 28, 29 or 30 nucleotides; or
- a base sequence as defined in SEQ ID NO: 1677 or 1678 and have a length of 25, 26, 27, 28, 29 or 30 nucleotides, or
- a base sequence as defined in any one of SEQ ID NO: 1784, 1836 and have a length of 21, 22, 23, 24, 25, 26, 27, 28, 29 or 30 nucleotides, or
- a base sequence as defined in any one of SEQ ID NO: 1786, 1838 and have a length of 25, 26, 27, 28, 29 or 30 nucleotides, or
- a base sequence as defined in any one of SEQ ID NO: 1780 and have a length of 22, 23, 24, 25, 26, 27, 28, 29 or 30 nucleotides, or
- a base sequence as defined in any one of SEQ ID NO: 1785, 1837 and have a length of 25, 26, 27, 28, 29 or 30 nucleotides.

If the first dystrophin exon is exon 10 and the second dystrophin exon is exon 30, another preferred region of exon 10 comprises or consists of SEQ ID NO:1772 and another preferred region of exon 30 comprises or consists of SEQ ID NO:1773. Preferred oligonucleotides comprise:
- a base sequence as defined in any one of SEQ ID NO: 1688, 1689, 1839, 1840, 1841, 1842, 1843 or 1844 and have a length of 26, 27, 28, 29 or 30 nucleotides or
- a base sequence as defined in any one of SEQ ID NO: 1845, 1846, 1847, 1848 and have a length of 24, 25, 26, 27, 28, 29 or 30 nucleotides, or
- a base sequence as defined in any one of SEQ ID NO: 1849, 1850 and have a length of 22, 23, 24, 25, 26, 27, 28, 29 or 30 nucleotides, or
- a base sequence as defined in any one of SEQ ID NO: 1787, 1851 and have a length of 26, 27, 28, 29 or 30 nucleotides.

If the first dystrophin exon is exon 10 and the second dystrophin exon is exon 31, a preferred region of exon 10 comprises or consists of SEQ ID NO:129 and a preferred region of exon 31 comprises or consists of SEQ ID NO:130.

If the first dystrophin exon is exon 10 and the second dystrophin exon is exon 32, a preferred region of exon 10 comprises or consists of SEQ ID NO:131 and a preferred region of exon 32 comprises or consists of SEQ ID NO:132.

If the first dystrophin exon is exon 10 and the second dystrophin exon is exon 35, a preferred region of exon 10 comprises or consists of SEQ ID NO:137 and a preferred region of exon 35 comprises or consists of SEQ ID NO:138.

If the first dystrophin exon is exon 10 and the second dystrophin exon is exon 42, a preferred region of exon 10 comprises or consists of SEQ ID NO:151 and a preferred region of exon 42 comprises or consists of SEQ ID NO:152.

If the first dystrophin exon is exon 10 and the second dystrophin exon is exon 44, a preferred region of exon 10 comprises or consists of SEQ ID NO:153 and a preferred region of exon 44 comprises or consists of SEQ ID NO:154.

If the first dystrophin exon is exon 10 and the second dystrophin exon is exon 47, a preferred region of exon 10 comprises or consists of SEQ ID NO:157 and a preferred region of exon 47 comprises or consists of SEQ ID NO:158.

If the first dystrophin exon is exon 10 and the second dystrophin exon is exon 48, a preferred region of exon 10 comprises or consists of SEQ ID NO:159 and a preferred region of exon 48 comprises or consists of SEQ ID NO:160.

If the first dystrophin exon is exon 10 and the second dystrophin exon is exon 55, a preferred region of exon 10 comprises or consists of SEQ ID NO:167 and a preferred region of exon 55 comprises or consists of SEQ ID NO:168.

If the first dystrophin exon is exon 10 and the second dystrophin exon is exon 57, a preferred region of exon 10 comprises or consists of SEQ ID NO:169 and a preferred region of exon 57 comprises or consists of SEQ ID NO:170.

If the first dystrophin exon is exon 10 and the second dystrophin exon is exon 60, a preferred region of exon 10 comprises or consists of SEQ ID NO:173 and a preferred region of exon 60 comprises or consists of SEQ ID NO:174.

A preferred oligonucleotide consists of SEQ ID NO: 1673 or comprises SEQ ID NO:1673 and has a length of 25, 26, 27, 28, 29 or 30 nucleotides.

A preferred oligonucleotide consists of SEQ ID NO: 1675 or comprises SEQ ID NO:1675 and has a length of 21, 22, 23, 24, 25, 26, 27, 28, 29 or 30 nucleotides.

A preferred oligonucleotide consists of SEQ ID NO: 1677 or comprises SEQ ID NO:1677 and has a length of 25, 26, 27, 28, 29 or 30 nucleotides.

A preferred oligonucleotide consists of SEQ ID NO: 1679 or comprises SEQ ID NO:1679 and has a length of 25, 26, 27, 28, 29 or 30 nucleotides. A preferred oligonucleotide comprises the base sequence SEQ ID NO:1679 and has a length of 25, 26, 27, 28, 29 or 30 nucleotides. Optionally 1, 2, 3, 4, 5, 6, 7 of the U of SEQ ID NO:1679 has/been replaced by a T. In a preferred embodiment, all U of SEQ ID NO:1679 have been replaced by T. A preferred oligonucleotide comprising SEQ ID NO: 1679 comprises any of the chemistries defined earlier herein: a base modification and/or a sugar modification and/or a backbone modification.

A preferred oligonucleotide consists of SEQ ID NO: 1681 or comprises SEQ ID NO:1681 and has a length of 25, 26, 27, 28, 29 or 30 nucleotides.

A preferred oligonucleotide consists of SEQ ID NO: 1684 or comprises SEQ ID NO:1684 and has a length of 21, 22, 23, 24, 25, 26, 27, 28, 29 or 30 nucleotides.

A preferred oligonucleotide consists of SEQ ID NO: 1685 or comprises SEQ ID NO:1685 and has a length of 21, 22, 23, 24, 25, 26, 27, 28, 29 or 30 nucleotides.

A preferred oligonucleotide consists of SEQ ID NO: 1686 or comprises SEQ ID NO:1686 and has a length of 21, 22, 23, 24, 25, 26, 27, 28, 29 or 30 nucleotides.

A preferred oligonucleotide consists of SEQ ID NO: 1688 or comprises SEQ ID NO:1688 and has a length of 26, 27, 28, 29 or 30 nucleotides. A preferred oligonucleotide comprises the base sequence SEQ ID NO:1688 and has a length of 26, 27, 28, 29 or 30 nucleotides. Optionally 1, 2, 3, 4, 5, 6 of the U of SEQ ID NO:1688 has/been replaced by a T. In a preferred embodiment, all U of SEQ ID NO:1688 have been replaced by T. A preferred oligonucleotide comprising SEQ ID NO: 1688 comprises any of the chemistries defined earlier herein: a base modification and/or a sugar modification and/or a backbone modification.

For each of the oligonucleotides represented by SEQ ID NO:1673, 1675, 1677, 1679, 1681, 1684, 1685, 1686 and 1688 the first dystrophin exon is exon 10. However, the second dystrophin exon is exon 13, 14, 15, 18, 20, 27, 30, 31, 32, 35, 42, 44, 47, 48, 55, 57, or 60. This means that using any of these oligonucleotides the formation of several in frame transcripts may occur, each leading to the production of a truncated but (semi)functional dystrophin protein.

If the first dystrophin exon is exon 11 and the second dystrophin exon is exon 23, a preferred region of exon 23 comprises or consists of SEQ ID NO:191 and a preferred region of exon 23 comprises or consists of SEQ ID NO:192. Preferred oligonucleotides comprise:
- a base sequence as defined in any one of SEQ ID NO: 1794, 1861, 1795, 1862 and have a length of 21, 22, 23, 24, 25, 26, 27, 28, 29 or 30 nucleotides, or
- a base sequence as defined in any one of SEQ ID NO: 1796, 1863, 1797, 1864 and have a length of 23, 24, 25, 26, 27, 28, 29 or 30 nucleotides, or
- a base sequence as defined in any one of SEQ ID NO: 1798, 1865, 1799, 1866 and have a length of 25, 26, 27, 28, 29 or 30 nucleotides.

If the first dystrophin exon is exon 13 and the second dystrophin exon is exon 30, a preferred region of exon 13 comprises or consists of SEQ ID NO:285 and a preferred region of exon 30 comprises or consists of SEQ ID NO:286. Preferred oligonucleotides comprise:
- a base sequence as defined in any one of SEQ ID NO: 1808, 1867, 1809, 1868, 1810, 1869, 1858, 1873 and have a length of 21, 22, 23, 24, 25, 26, 27, 28, 29 or 30 nucleotides
- a base sequence as defined in any one of SEQ ID NO: 1811, 1870, 1859, 1874 and have a length of 22, 23, 24, 25, 26, 27, 28, 29 or 30 nucleotides, or
- a base sequence as defined in any one of SEQ ID NO: 1856, 1871, 1860, 1875 and having a length of 23, 24, 25, 26, 27, 28, 29 or 30 nucleotides, or
- a base sequence as defined in any one of SEQ ID NO: 1857, 1872 and having a length of 26, 27, 28, 29 or 30 nucleotides.

If the first dystrophin exon is exon 23 and the second dystrophin exon is exon 42, a preferred region of exon 23 comprises or consists of SEQ ID NO:776 and a preferred region of exon 42 comprises or consists of SEQ ID NO:777 Preferred oligonucleotides comprise or consist of SEQ ID NO: 1698-1703.

If the first dystrophin exon is exon 34 and the second dystrophin exon is exon 53, a preferred region of exon 34 comprises or consists of SEQ ID NO:1294 and a preferred region of exon 53 comprises or consists of SEQ ID NO:1295. Preferred oligonucleotides comprise:
- a base sequence as defined in any one of SEQ ID NO: 1800, 1876, 1801, 1877 and have a length of 19, 20, 21, 22, 23, 24, 25, 26, 27, 28, 29 or 30 nucleotides, or
- a base sequence as defined in any one of SEQ ID NO: 1802, 1878, 1803, 1879 and have a length of 23, 24, 25, 26, 27, 28, 29 or 30 nucleotides.

If the first dystrophin exon is exon 40 and the second dystrophin exon is exon 53, a preferred region of exon 40 comprises or consists of SEQ ID NO:1477 and a preferred region of exon 53 comprises or consists of SEQ ID NO:1478. Preferred oligonucleotides comprise:
- a base sequence as defined in any one of SEQ ID NO: 1804, 1880 and have a length of 21, 22, 23, 24, 25, 26, 27, 28, 29 or 30 nucleotides, or
- a base sequence as defined in any one of SEQ ID NO: 1805, 1881 and have a length of 22, 23, 24, 25, 26, 27, 28, 29 or 30 nucleotides.

If the first dystrophin exon is exon 44 and the second dystrophin exon is exon 56, a preferred region of exon 44 comprises or consists of SEQ ID NO:1577 and a preferred region of exon 56 comprises or consists of SEQ ID NO:1558. Preferred oligonucleotides comprise a base sequence as defined in any one of SEQ ID NO: 1806, 1882, 1807, 1883 and have a length of 20, 21, 22, 23, 24, 25, 26, 27, 28, 29 or 30 nucleotides.

If the first dystrophin exon is exon 45 and the second dystrophin exon is exon 51, a preferred region of exon 45 comprises or consists of SEQ ID NO:1567 and a preferred region of exon 51 comprises or consists of SEQ ID NO:1568. Preferred oligonucleotides comprise or consist of SEQ ID NO: 1730-1731.

If the first dystrophin exon is exon 45 and the second dystrophin exon is exon 53, a preferred region of exon 45 comprises or consists of SEQ ID NO:1569 and a preferred region of exon 53 comprises or consists of SEQ ID NO:1570. Preferred oligonucleotides comprise or consist of SEQ ID NO: 1732-1737.

If the first dystrophin exon is exon 45 and the second dystrophin exon is exon 55, a preferred region of exon 45 comprises or consists of SEQ ID NO:1571 and a preferred region of exon 55 comprises or consists of SEQ ID NO:1572. Preferred oligonucleotides comprise or consist of SEQ ID NO: 1704-1719, 1788, 1852, 1789, 1853.

A preferred oligonucleotide consists of SEQ ID NO: 1706 or comprises SEQ ID NO:1706 and has a length of 25, 26, 27, 28, 29 or 30 nucleotides. A preferred oligonucleotide comprising SEQ ID NO:1706 and having a length of 25 nucleotides consists of SEQ ID NO: 1706.

A preferred oligonucleotide consists of SEQ ID NO: 1707 or comprises SEQ ID NO:1707 and has a length of 23, 24, 25, 26, 27, 28, 29 or 30 nucleotides. A preferred oligonucleotide comprising SEQ ID NO:1707 and having a length of 25 nucleotides consists of SEQ ID NO: 1706.

A preferred oligonucleotide consists of SEQ ID NO: 1713 or comprises SEQ ID NO:1713 and has a length of 23, 24, 25, 26, 27, 28, 29 or 30 nucleotides. A preferred oligonucleotide comprising SEQ ID NO:1713 and having a length of 25 nucleotides consists of SEQ ID NO: 1710.

Preferred oligonucleotides comprise a base sequence as defined in any one of SEQ ID NO: 1788, 1852, 1789, 1853 and have a length of 24, 25, 26, 27, 28, 29 or 30 nucleotides

If the first dystrophin exon is exon 45 and the second dystrophin exon is exon 55, another preferred region of exon 45 comprises or consists of SEQ ID NO:1774 and another preferred region of exon 55 comprises or consists of SEQ ID NO:1775. Preferred oligonucleotides comprise a base sequence as defined in any one of SEQ ID NO: 1790, 1854, 1792, 1855 and have a length of 25, 26, 27, 28, 29 or 30 nucleotides.

If the first dystrophin exon is exon 45 and the second dystrophin exon is exon 60, a preferred region of exon 45 comprises or consists of SEQ ID NO:1577 and a preferred region of exon 60 comprises or consists of SEQ ID NO:1578. Preferred oligonucleotides comprise or consist of SEQ ID NO: 1738-1741.

If the first dystrophin exon is exon 56 and the second dystrophin exon is exon 60, a preferred region of exon 56 comprises or consists of SEQ ID NO:1742 and a preferred region of exon 60 comprises or consists of SEQ ID NO:1743. Preferred oligonucleotides comprise or consist of SEQ ID NO: 1720-1721.

More preferred oligonucleotide comprise:
(a) the base sequence as defined in SEQ ID NO: 1673 or 1674 and have a length of 25, 26, 27, 28, 29 or 30 nucleotides; or
(b) the base sequence as defined in SEQ ID NO: 1675 or 1676 and have a length of 21, 22, 23, 24, 25, 26, 27, 28, 29 or 30 nucleotides; or
(c) the base sequence as defined in SEQ ID NO: 1677 or 1678 and have a length of 25, 26, 27, 28, 29 or 30 nucleotides; or
(d) the base sequence as defined in any one of SEQ ID NO: 1679 to 1681 and have a length of 25, 26, 27, 28, 29 or 30 nucleotides; or
(e) the base sequence as defined in any one of SEQ ID NO: 1684 to 1686 and have a length of 21, 22, 23, 24, 25, 26, 27, 28, 29 or 30 nucleotides; or
(f) the base sequence as defined in SEQ ID NO: 1688 or 1689 and have a length of 26, 27, 28, 29 or 30 nucleotides; or
(g) the base sequence as defined in any one of SEQ ID NO: 1704 to 1706 and have a length of 25, 26, 27, 28, 29 or 30 nucleotides,
(h) the base sequence as defined in any one of SEQ ID NO: 1707 to 1709 and have a length of 23, 24, 25, 26, 27, 28, 29 or 30 nucleotides; or
(i) the base sequence as defined in any one of SEQ ID NO: 1710, 1713 to 1717 and have a length of 23, 24, 25, 26, 27, 28, 29 or 30 nucleotides.

Even more preferred oligonucleotides comprise:
(a) the base sequence as defined in any one of SEQ ID NO: 1679 to 1681, 1778, 1812, 1813, 1884 to 1886, 1890, or 1891, and having a length of 25, 26, 27, 28, 29 or 30 nucleotides or SEQ ID NO: 1814 and having a length of 26, 27, 28, 29 or 30 nucleotides; or
(b) the base sequence as defined in SEQ ID NO: 1688, 1689, or 1839 to 1844, and having a length of 26, 27, 28, 29 or 30 nucleotides; or
(c) the base sequence as defined in SEQ ID NO: 1673 or 1674 and having a length of 25, 26, 27, 28, 29 or 30 nucleotides; or
(d) the base sequence as defined in SEQ ID NO: 1675 or 1676 and having a length of 21, 22, 23, 24, 25, 26, 27, 28, 29 or 30 nucleotides; or
(e) the base sequence as defined in SEQ ID NO: 1677 or 1678 and having a length of 25, 26, 27, 28, 29 or 30 nucleotides; or
(f) the base sequence as defined in any one of SEQ ID NO: 1684 to 1686 and having a length of 21, 22, 23, 24, 25, 26, 27, 28, 29 or 30 nucleotides; or
(g) the base sequence as defined in any one of SEQ ID NO: 1704 to 1706 and having a length of 25, 26, 27, 28, 29 or 30 nucleotides,
(h) the base sequence as defined in any one of SEQ ID NO: 1707 to 1709 and having a length of 23, 24, 25, 26, 27, 28, 29 or 30 nucleotides; or
(i) the base sequence as defined in any one of SEQ ID NO: 1710, 1713 to 1717 and having a length of 23, 24, 25, 26, 27, 28, 29 or 30 nucleotides or
(j) the base sequence as defined in any one of SEQ ID NO: 1815 to 1819 and having a length of 22, 23, 24, 25, 26, 27, 28, 29 or 30 nucleotides, or
(k) the base sequence as defined in any one of SEQ ID NO: 1820, 1824, and having a length of 20, 21, 22, 23, 24, 25, 26, 27, 28, 29 or 30 nucleotides, or
(1) the base sequence as defined in any one of SEQ ID NO: 1826, 1782, 1832 and having a length of 21, 22, 23, 24, 25, 26, 27, 28, 29 or 30 nucleotides, or
(m) base sequence as defined in any one of SEQ ID NO: 1821, 1825, 1780 and having a length of 22, 23, 24, 25, 26, 27, 28, 29 or 30 nucleotides, or
(n) base sequence as defined in any one of SEQ ID NO: 1822 and having a length of 24, 25, 26, 27, 28, 29 or 30 nucleotides, or
(o) base sequence as defined in any one of SEQ ID NO: 1823, 1781, 1829, 1830, 1831 and having a length of 25, 26, 27, 28, 29 or 30 nucleotides, or
(p) base sequence as defined in any one of SEQ ID NO: 1783, 1833, 1834, 1835 and having a length of 17, 18, 19, 20, 21, 22, 23, 24, 25, 26, 27, 28, 29 or 30 nucleotides, or
(q) base sequence as defined in any one of SEQ ID NO: 1887 and and have a length of 24, 25, 26, 27, 28, 29 or 30 nucleotides, or
(r) base sequence as defined in any one of SEQ ID NO: 1888 or 1889 and and have a length of 26, 27, 28, 29 or 30 nucleotides, or
(s) base sequence as defined in SEQ ID NO: 1827 and have a length of 21, 22, 23, 24, 25, 26, 27, 28, 29 or 30 nucleotides, or
(t) a base sequence as defined in SEQ ID NO: 1828 and have a length of 25, 26, 27, 28, 29 or 30 nucleotides, or
(u) base sequence as defined in any one of SEQ ID NO: 1784, 1836 and having a length of 21, 22, 23, 24, 25, 26, 27, 28, 29 or 30 nucleotides, or
(v) or base sequence as defined in any one of SEQ ID NO: 1786, 1838 and having a length of 25, 26, 27, 28, 29 or 30 nucleotides, or
(w) base sequence as defined in any one of SEQ ID NO: 1780 and having a length of 22, 23, 24, 25, 26, 27, 28, 29 or 30 nucleotides, or
(x) base sequence as defined in any one of SEQ ID NO: 1785, 1837 and having a length of 25, 26, 27, 28, 29 or 30 nucleotides, or
(y) base sequence as defined in any one of SEQ ID NO: 1845, 1846, 1847, 1848 and having a length of 24, 25, 26, 27, 28, 29 or 30 nucleotides, or
(z) base sequence as defined in any one of SEQ ID NO: 1849, 1850 and having a length of 22, 23, 24, 25, 26, 27, 28, 29 or 30 nucleotides, or
(a1) base sequence as defined in any one of SEQ ID NO: 1787, 1851 and having a length of 26, 27, 28, 29 or 30 nucleotides, or
(b1) base sequence as defined in any one of SEQ ID NO: 1788, 1852, 1789, 1853 and having a length of 24, 25, 26, 27, 28, 29 or 30 nucleotides, or
(c1) base sequence as defined in any one of SEQ ID NO: 1790, 1854, 1792, 1855 and having a length of 25, 26, 27, 28, 29 or 30 nucleotides, or
(d1) base sequence as defined in any one of SEQ ID NO: 1794, 1861, 1795, 1862 and having a length of 21, 22, 23, 24, 25, 26, 27, 28, 29 or 30 nucleotides, or
(e1) base sequence as defined in any one of SEQ ID NO: 1796, 1863, 1797, 1864 and having a length of 23, 24, 25, 26, 27, 28, 29 or 30 nucleotides, or
(f1) base sequence as defined in any one of SEQ ID NO: 1798, 1865, 1799, 1866 and having a length of 25, 26, 27, 28, 29 or 30 nucleotides, or
(g1) base sequence as defined in any one of SEQ ID NO: 1808, 1867, 1809, 1868, 1810, 1869, 1858, 1873 and having a length of 21, 22, 23, 24, 25, 26, 27, 28, 29 or 30 nucleotides, or
(h1) base sequence as defined in any one of SEQ ID NO: 1811, 1870, 1859, 1874 and having a length of 22, 23, 24, 25, 26, 27, 28, 29 or 30 nucleotides, or
(i1) base sequence as defined in any one of SEQ ID NO: 1856, 1871, 1860, 1875 and having a length of 23, 24, 25, 26, 27, 28, 29 or 30 nucleotides, or
(j1) base sequence as defined in any one of SEQ ID NO: 1857, 1872 and having a length of 26, 27, 28, 29 or 30 nucleotides, or
(k1) base sequence as defined in any one of SEQ ID NO: 1800, 1876, 1801, 1877 and having a length of 19, 20, 21, 22, 23, 24, 25, 26, 27, 28, 29 or 30 nucleotides, or
(11) base sequence as defined in any one of SEQ ID NO: 1802, 1878, 1803, 1879 and having a length of 23, 24, 25, 26, 27, 28, 29 or 30 nucleotides, or
(m1) base sequence as defined in any one of SEQ ID NO: 1804, 1880 and having a length of 21, 22, 23, 24, 25, 26, 27, 28, 29 or 30 nucleotides, or
(n1) base sequence as defined in any one of SEQ ID NO: 1805, 1881 and having a length of 22, 23, 24, 25, 26, 27, 28, 29 or 30 nucleotides, or
(o1) base sequence as defined in any one of SEQ ID NO: 1806, 1882, 1807, 1883 and having a length of 20, 21, 22, 23, 24, 25, 26, 27, 28, 29 or 30 nucleotides.

The oligonucleotide according to the invention, which comprises or consists of a sequence as defined by a SEQ ID number is also meant to encompass an oligonucleotide comprising the base sequence as defined in the SEQ ID. Oligonucleotides having a modified backbone (i.e. modified sugar moieties and/or modified internucleoside linkages) with respect to those defined by the SEQ IDs are also encompassed within the invention. Each base U in a SEQ ID NO of an oligonucleotide as identified herein may be modified or replaced by a T.

### Composition

In a further aspect, there is provided a composition comprising an oligonucleotide as described in the previous section entitled "Oligonucleotide". This composition preferably comprises or consists of an oligonucleotide as described above. A preferred composition comprises one single oligonucleotide as defined above. It is therefore clear that the skipping of at least said first and said second exon is obtained using one single oligonucleotide and not using a cocktail of distinct oligonucloetides.

In a preferred embodiment, said composition is for use as a medicament. Said composition is therefore a pharmaceutical composition. A pharmaceutical composition usually comprises a pharmaceutically accepted carrier, diluent and/or excipient. In a preferred embodiment, a composition of the current invention comprises a compound as defined herein and optionally further comprises a pharmaceutically acceptable formulation, filler, preservative, solubilizer, carrier, diluent, excipient, salt, adjuvant and/or solvent. Such pharmaceutically acceptable carrier, filler, preservative, solubilizer, diluent, salt, adjuvant, solvent and/or excipient may for instance be found in Remington. The compound as described in the invention possesses at least one ionizable group. An ionizable group may be a base or acid, and may be charged or neutral. An ionizable group may be present as ion pair with an appropriate counterion that carries opposite charge(s). Examples of cationic counterions are sodium, potassium, cesium, Tris, lithium, calcium, magnesium, trialkylammonium, triethylammonium, and tetraalkylammonium. Examples of anionic counterions are chloride, bromide, iodide, lactate, mesylate, acetate, trifluoroacetate, dichloroacetate, and citrate. Examples of counterions have been described (Kumar L.). Therefore in a preferred embodiment, an oligonucleotide of the invention is contacted with a composition comprising such ionizable groups, preferably Ca²⁺ to form an oligonucleotide chelate complex comprising two or more identical oligonucleotides linked by such a multivalent cation as already defined herein.

A pharmaceutical composition may be further formulated to further aid in enhancing the stability, solubility, absorption, bioavailability, pharmacokinetics and cellular uptake of said compound, in particular formulations comprising excipients or conjugates capable of forming complexes, nanoparticles, microparticles, nanotubes, nanogels, hydrogels, poloxamers or pluronics, polymersomes, colloids, microbubbles, vesicles, micelles, lipoplexes, and/or liposomes. Examples of nanoparticles include polymeric nanoparticles, gold nanoparticles, magnetic nanoparticles, silica nanoparticles, lipid nanoparticles, sugar particles, protein nanoparticles and peptide nanoparticles.

A preferred composition comprises at least one excipient that may further aid in enhancing the targeting and/or delivery of said composition and/or said oligonucleotide to and/or into muscle and/or a cell. A cell may be a muscular cell.

Another preferred composition may comprise at least one excipient categorized as a second type of excipient. A second type of excipient may comprise or contain a conjugate group as described herein to enhance targeting and/or delivery of the composition and/or of the oligonucleotide of the invention to a tissue and/or cell and/or into a tissue and/or cell, as for example muscle tissue or cell. Both types of excipients may be combined together into one single composition as identified herein. Preferred conjugate groups are disclosed in the part dedicated to the definitions.

The skilled person may select, combine and/or adapt one or more of the above or other alternative excipients and delivery systems to formulate and deliver a compound for use in the present invention.

Such a pharmaceutical composition of the invention may be administered in an effective concentration at set times to an animal, preferably a mammal. More preferred mammal is a human being. A compound or a composition as defined herein for use according to the invention may be suitable for direct administration to a cell, tissue and/or an organ *in vivo* of individuals, preferably said individuals are affected by or at risk of developing BMD or DMD, and may be administered directly *in vivo, ex vivo* or *in vitro.* Administration may be *via* systemic and/or parenteral routes, for example intravenous, subcutaneous, intraventricular, intrathecal, intramuscular, intranasal, enteral, intravitreal, intracerebral, epidural or oral route. Preferably, such a pharmaceutical composition of the invention may be encapsulated in the form of an emulsion, suspension, pill, tablet, capsule or soft-gel for oral delivery, or in the form of aerosol or dry powder for delivery to the respiratory tract and lungs.

In an embodiment a compound of the invention may be used together with another compound already known to be used for the treatment of said disease. Such other compounds may be used for slowing down progression of disease, for reducing abnormal behaviors or movements, for reducing muscle tissue inflammation, for improving muscle fiber function, integrity and/or survival and/or improve, increase or restore cardiac function. Examples are, but not limited to, a steroid, preferably a (gluco)corticosteroid, an ACE inhibitor (preferably perindopril), an angiotensin II type 1 receptor blocker (preferably losartan), a tumor necrosis factor-alpha (TNFα) inhibitor, a TGFβ inhibitor (preferably decorin), human recombinant biglycan, a source of mIGF-1, a myostatin inhibitor, mannose-6-phosphate, an antioxidant, an ion channel inhibitor, a protease inhibitor, a phosphodiesterase inhibitor (preferably a PDE5 inhibitor, such as sildenafil or tadalafil), L-arginine, dopamine blockers, amantadine, tetrabenazine, and/or co-enzyme Q10. This combined use may be a sequential use: each component is administered in a distinct composition. Alternatively each compound may be used together in a single composition.

### Use

In a further aspect, there is provided the use of a composition or a compound as described herein for use as a medicament or part of therapy, or applications in which the compound exerts its activity intracellularly.

In a preferred embodiment, a compound or composition of the invention is for use as a medicament wherein the medicament is for preventing, delaying, ameliorating and/or treating a disease as defined herein, preferably DMD or BMD.

### Method for preventing, delaying, ameliorating and/or treating a disease

In the disclosure, there is provided a method for preventing, delaying, ameliorating and/or treating a disease as defined herein, preferably DMD or BMD. Said disease may be prevented, treated, delayed, or ameliorated in an individual, in a cell, tissue or organ of said individual. The method comprising administering an oligonucleotide or a composition of the invention to said individual or a subject in the need thereof.

The method according to the disclosure wherein an oligonucleotide or composition as defined herein may be suitable for administration to a cell, tissue and/or an organ *in vivo* of individuals, preferably individuals affected by BMD or DMD or at risk of developing such disease, and may be administered *in vivo, ex vivo* or *in vitro.* An individual or a subject in need is preferably a mammal, more preferably a human being.

In a method of the disclosure, a concentration of an oligonucleotide or composition is ranged from 0.01 nM to 1 µM. More preferably, the concentration used is from 0.02 to 400 nM, or from 0.05 to 400 nM, or from 0.1 to 400 nM, even more preferably from 0.1 to 200 nM. Dose ranges of an oligonucleotide or composition according to the invention are preferably designed on the basis of escalating dose studies in clinical trials (*in vivo* use) for which rigorous protocol requirements exist. An oligonucleotide as defined herein may be used at a dose which is ranged from 0.01 to 200 mg/kg or 0.05 to 100 mg/kg or 0.1 to 50 mg/kg or 0.1 to 20 mg/kg, preferably from 0.5 to 10 mg/kg.

The ranges of concentration or dose of an oligonucleotide or composition as given above are preferred concentrations or doses for *in vitro* or *ex vivo* uses. The skilled person will understand that depending on the identity of the oligonucleotide used, the target cell to be treated, the gene target and its expression levels, the medium used and the transfection and incubation conditions, the concentration or dose of said oligonucleotide used may further vary and may need to be optimised any further.

### Definitions

"Sequence identity", as known in the art, is a relationship between two or more nucleic acid (polynucleotide or nucleotide) sequences, as determined by comparing the sequences. In the art, the percentage of "identity" or "similarity" indicates the degree of sequence relatedness between nucleic acid sequences as determined by the match between strings of such sequences. "Identity" may be replaced by "similarity" herein. Preferably, the percentage of identity is determined by comparing the whole SEQ ID NO as identified herein. However, part of a sequence may also be used. Part of a sequence in this context may mean at least 5%, 10%, 20%, 30%, 40%, 50%, 60%, 70%, 80%, 90% or 100% of a given sequence or SEQ ID NO.

"Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include *e.g.* the GCG program package (Devereux, J., et al., Nucleic Acids Research 12 (1):387 (1984)), BestFit and FASTA (Altschul, S. F. et al., J. Mol. Biol. 215:403-410 (1990). The BLAST 2.0 family of programs which can be used for database similarity searches includes:eg. BLASTN for nucleotide query sequences against nucleotide database sequences.. The BLAST 2.0 family programs are publicly available from NCBI and other sources (BLAST Manual, Altschul, S., et al., NCBI NLM NIH Bethesda, MD 20894; Altschul, S., et al., J. Mol. Biol. 215:403-410 (1990)). The well-known Smith Waterman algorithm may also be used to determine identity.

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Another preferred method to determine sequence similarity and identity is by using the algorithm Needleman-Wunsch (Needleman, S. B. and Wunsch, C. D. (1970) J. Mol. Biol. 48, 443-453, Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

Another preferred method to determine sequence similarity and identity is by using EMBOSS Matcher and the Waterman-Eggert algorithm (local alignment of two sequences; [Schoniger and Waterman, Bulletin of Mathematical Biology 1992, Vol. 54 (4), pp. 521-536; Vingron and Waterman, J. Mol. Biol. 1994; 235(1), p1-12.]). The following websites could be used: http://www.ebi.ac.uk/Tools/emboss/align/index.html or http://emboss.bioinformatics.nl/cgi-bin/emboss/matcher. Definitions of the parameters used in this algorithm are found at the following website: http://emboss.sourceforge.net/docs/themes/AlignFormats.html#id. Preferably, the default settings (Matrix: EDNAFULL, Gap_penalty: 16, Extend_penalty: 4) are used. Emboss Matcher provides the best local alignments between two sequences, but alternative alignments are provided as well. Table 2 discloses the best local alignments between two different exons, preferably dystrophin exons, which are preferably used for oligonucleotide design. However, also alternative aligments and thus alternative identity regions between two exons may be identified and used for oligonucleotide design, which is also part of this invention.

Throughout the application, the word "binds", "targets", "hybridizes" could be used interchangeably when used in the context of an oligonucleotide which is reverse complementary to a region of a pre-mRNA as identified herein. Similarly, the expressions "is capable of binding", "is capable of targeting" and "is capable of hybridizing" can be used interchangeably to indicate that an oligonucleotide has a certain sequence which allows binding, targeting or hybridization to a target sequence. Whenever a target sequence is defined herein or known in the art, the skilled person is able to construct all possible structures of said oligonucleotide, using the concept of reverse complementarity. In this respect, it will be understood that a limited number of sequence mismatches or gaps between the oligonucleotide of the invention and the target sequences in first and/or second exon is allowable, as long as the binding is not affected, as discussed above. Thus, an oligonucleotide that is capable of binding to a certain target sequence, can be regarded as an oligonucleotide that is reverse complementary to that target sequence. In the context of the invention, "hybridizes" or "is capable of hybridizing" is used under physiological conditions in a cell, preferably a human cell unless otherwise indicated.

As used herein, "hybridization" refers to the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is nucleobase complementary to the natural nucleobases thymine, 5-methyluracil and uracil which pair through the formation of hydrogen bonds. The natural base guanine is nucleobase complementary to the natural bases cytosine and 5-methylcytosine. Hybridization can occur under varying circumstances.

Similarly, "reverse complementarity" is used to identify two nucleotide sequences which are able to hybridize to one another, while one of the sequences is oriented from 3' to 5' and the other in the reverse direction, i.e. from 5' to 3'. Thus, a nucleoside A on a first nucleotide sequence is able to pair with a nucleoside A* on a second nucleotide sequence, via their respective nucleobases, and a nucleoside B, located at 5'-position from the afore mentioned nucleoside A in the first nucleotide sequence, is able to pair with a nucleoside B*, which is located at the 3'-position from the afore mentioned nucleoside A* in the second nucleotide sequence. In the context of the present invention, the first nucleotide sequence is typically an oligonucleotide of the invention, and the second nucleotide sequence part of an exon from a pre-mRNA, preferably the dystrophin pre-mRNA. An oligonucleotide is preferably said to be reverse complementary to a region of an exon (first and/or second exon) when said oligonucleotide is at least 45, 50, 55, 60, 65 70, 75, 80, 85, 90, 95, 100% reverse complementary with said region of said first and/or second exon. Preferably, the reverse complementarity is at least 80%, 85%, 90%, 95% or 100%.

Within the context of the invention, excipients include polymers (e.g. polyethyleneimine (PEI), polypropyleneimine (PPI), dextran derivatives, butylcyanoacrylate (PBCA), hexylcyanoacrylate (PHCA), poly(lactic-co-glycolic acid) (PLGA), polyamines (e.g. spermine, spermidine, putrescine, cadaverine), chitosan, poly(amido amines) (PAMAM), poly(ester amine), polyvinyl ether, polyvinyl pyrrolidone (PVP), polyethylene glycol (PEG) cyclodextrins, hyaluronic acid, colominic acid, and derivatives thereof), dendrimers (e.g. poly(amidoamine)), lipids {*e.g*. 1,2-dioleoyl-3-dimethylammonium propane (DODAP), dioleoyldimethylammonium chloride (DODAC), phosphatidylcholine derivatives [e.g 1,2-distearoyl-sn-glycero-3-phosphocholine (DSPC)], lyso-phosphatidylcholine derivaties [e.g. 1-stearoyl-2-lyso-sn-glycero-3-phosphocholine (S-LysoPC)], sphingomyeline, 2-{3-[Bis-(3-amino-propyl)-amino]-propylamino} -*N*-ditetracedyl carbamoyl methylacetamide (RPR209120), phosphoglycerol derivatives [*e.g.* 1,2-dipalmitoyl-sn-glycero-3-phosphoglycerol,sodium salt (DPPG-Na), phosphaticid acid derivatives [1,2-distearoyl-sn-glycero-3-phosphaticid acid, sodium salt (DSPA), phosphatidylethanolamine derivatives [e.g. dioleoyl--phosphatidylethanolamine (DOPE), 1,2-distearoyl-sn-glycero-3-phosphoethanolamine (DSPE), 2-diphytanoyl-sn-glycero-3-phosphoethanolamine (DPhyPE),], *N*-[1-(2,3-dioleoyloxy)propyl]-*N*,*N*,*N*-trimethylammonium (DOTAP), N-[1-(2,3-dioleyloxy)propyl]-*N*,*N*-*N*-trimethylammonium (DOTMA), 1,3-di-oleoyloxy-2-(6-carboxy-spermyl)-propylamid (DOSPER), (1,2-dimyristyolxypropyl-3-dimethylhydroxy ethyl ammonium (DMRIE), (N1-cholesteryloxycarbonyl-3,7-diazanonane-1,9-diamine (CDAN), dimethyldioctadecylammonium bromide (DDAB), 1-palmitoyl-2-oleoyl-sn-glycerol-3-phosphocholine (POPC), (b-L-arginyl-2,3-L-diaminopropionic acid-*N*-palmityl-*N*-olelyl-amide trihydrochloride (AtuFECT01), *N*,*N*-dimethyl-3-aminopropane derivatives [*e.g. 1,2-*distearoyloxy-*N*,*N*-dimethyl-3-aminopropane (DSDMA), 1,2-dioleyloxy-*N*,*N*-dimethyl-3-aminopropane (DoDMA), 1,2-dilinoleyloxy-*N*,*N*-3-dimethylaminopropane (DLinDMA), 2,2-dilinoleyl-4-dimethylaminomethyl [1,3]-dioxolane (DLin-K-DMA), phosphatidylserine derivatives [1,2-dioleyl-sn-glycero-3-phospho-L-serine, sodium salt (DOPS)], cholesterol}, proteins (*e.g*. albumin, gelatins, atellocollagen), and peptides (e.g. protamine, PepFects, NickFects, polyarginine, polylysine, CADY, MPG).

Within the context of the invention, conjugate groups are selected from the group consisting of targeting moieties, stability enhancing moieties, uptake enhancing moieties, solubility enhancing moieties, pharmacokinetics enhancing moieties, pharmacodynamics enhancing moieties, activity enhancing moieties, reporter molecules and drugs, wherein these moieties may be peptides, proteins, carbohydrates, polymers, ethylene glycol derivatives, vitamins, lipids, polyfluoroalkyl moieties, steroids, cholesterol, fluorescent moieties and radioactively labeled moieties. Conjugate groups may optionally be protected and may be attached directly to an oligonucleotide of the invention or *via* a divalent or multivalent linker.

Conjugate groups include moieties that enhance targeting, uptake, solubility, activity, pharmacodynamics, pharmacokinetics, or that decrease toxicity. Examples of such groups include peptides (*e.g.* glutathione, polyarginine, RXR peptides (see *e.g.* Antimicrob. Agents Chemother. 2009, 53, 525), polyornithine, TAT, TP10, pAntp, polylysine, NLS, penetratin, MSP, ASSLNIA, MPG, CADY, Pep-1, Pip, SAP, SAP(E), Transportan, buforin II, polymyxin B, histatin, CPP5, NickFects, PepFects), vivo-porter, proteins (*e.g*. antibodies, avidin, Ig, transferrin, albumin), carbohydrates (*e.g*. glucose, galactose, mannose, maltose, maltotriose, ribose, trehalose, glucosamine, *N*-acetylglucosamine, lactose, sucrose, fucose, arabinose, talose, sialic acid, hyaluronic acid, neuramidinic acid, rhamnose, quinovose, galactosamine, *N*-acetylgalactosamine, xylose, lyxose, fructose, mannose-6-phosphate, 2-deoxyribose, glucal, cellulobiose, chitobiose, chitotriose), polymers (*e.g*. polyethylene glycol, polyethyleneimine, polylactic acid, poly(amidoamine)), ethylene glycol derivatives (*e.g*. triethyleneglycol, tetraethyleneglycol), watersoluble vitamins (*e.g*. vitamin B, B1, B2, B3, B5, B6, B7, B9, B12, C), fat-soluble vitamins (*e.g*. vitamin A, D, D2, D3, E, K1, K2, K3), lipids (*e.g*. palmityl, myristyl, oleyl, stearyl, batyl, glycerophospholipid, glycerolipid, sphingolipid, ceramide, cerebroside, sphingosine, sterol, prenol, erucyl, arachidonyl, linoleyl, linolenyl, arachidyl, butyryl, sapeinyl, elaidyl, lauryl, behenyl, nonyl, decyl, undecyl, octyl, heptyl, hexyl, pentyl, DOPE, DOTAP, terpenyl, diterpenoid, triterpernoid), polyfluoroalkyl moieties (*e.g*. perfluoro[1H,1H,2H,2H]-alkyl), approved drugs of MW < 1500 Da that have affinity for specific proteins (*e.g*. NSAIDs such as ibuprofen (more are described in US patent 6,656,730), antidepressants, antivirals, antibiotics, alkylating agents, amebicides, analgesics, androgens, ACE inhibitors, anorexiants, antacids, anthelmintics, anti-angiogenics, antiadrenergics, anti-anginals, anticholinergics, anticoagulants, anticonvulsants, antidiabetics, antidiaarheals, antidiuretics, antidotes, antifungals, antiemetics, antivertigos, antigouts, antigonadotropics, antihistamines, antihyperlipidemics, antihypertensives, antimalrials, antimigraines, antineoplastics, antipsychotics, antirheumatics, antithyroids, antitoxins, antitussives, anxiolytics, contraceptives, CNS stimulants, chelators, cardiovascular agents, decongestants, dermatological agents, diuretics, expectorants, diagnostics, gastrointestinal agents, anesthetics, glucocorticoids, antiarrhytmics, immunostimulants, immunosuppressives, laxatives, leprostatics, metabolic agents, respiratory agents, mucolytics, muscle relaxants, neutraceuticals, vasodilators, thrombolytics, uterotonics, vasopressors), natural compounds of MW < 2000 Da (*e.g*. antibiotics, eicosanoids, alkaloids, flavonoids, terpenoids, enzyme cofactors, polyketides), steroids (e.g. prednisone, prednisolone, dexamethasone, lanosterol, cholic acid, estrane, androstane, pregnane, cholane, cholestane, ergosterol, cholesterol, cortisol, cortisone, deflazacort), pentacyclic triterpenoids (*e.g*. 18β-glycyrrhetinic acid, ursolic acid, amyrin, carbenoloxone, enoxolone, acetoxolone, betulinic acid, asiatic acid, erythrodiol, oleanolic acid), polyamines (e*.g*. spermine, spermidine, putrescine, cadaverine), fluorescent moieties (e*.g*. FAM, carboxyfluorescein, FITC, TAMRA, JOE, HEX, TET, rhodamine, Cy3, Cy3.5, Cy5, Cy5.5, CW800, BODIPY, AlexaFluors, Dabcyl, DNP), reporter molecules (*e.g*. acridines, biotins, digoxigenin, (radio)isotopically labeled moieties (with *e.g.* ²H, ¹³C, ¹⁴C, ¹⁵N, ¹⁸O, ¹⁸F, ³²P, ³⁵S, ⁵⁷Co, ^{99m}Tc, ¹²³I, ¹²⁵I, ¹³¹I, ¹⁵³Gd)) and combinations thereof. Such conjugate groups may be connected directly to the compounds of the invention, or *via* a linker. This linker may be divalent (yielding a 1:1 conjugate) or multivalent, yielding an oligomer with more than one conjugate group. Procedures for coupling such a conjugate group, either directly or *via* a linker, to the oligomer according to the invention are known in the art. Also within the context of the invention is the use of nanoparticles to which oligonucleotides of the invention are covalently linked, to the extent that such constructs are called spherical nucleic acids (SNAs), as for example described in J. Am. Chem. Soc. 2008, 130, 12192 (Hurst et al.).

In this document and in its claims, the verb "to comprise" and its conjugations is used in its non-limiting sense to mean that items following the word are included, but items not specifically mentioned are not excluded. In addition the verb "to consist" may be replaced by "to consist essentially of' meaning that an oligonucleotide or a composition as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".

The word "about" or "approximately" when used in association with a numerical value (about 10) preferably means that the value may be the given value of 10 more or less 1% of the value.

Each embodiment as identified herein may be combined together unless otherwise indicated.

The following examples are offered for illustrative purposes only, and are not intended to limit the scope of the present invention in any way.

### Examples

### Tables 1-3

**Table 1. List of possible exon combinations in the DMD gene transcript for which exon U (upstream) has a continued open reading frame with exon D (downstream) if exons U+1 (a first exon) to D-1 (a second exon), and any exons in between, are removed from the transcript.**

| **First** | **Second** |
|---|---|
| **Exon ('U')** | **Exon ('D')** |
| 1 | 8,20,22,51,53,59,62,64,65,67,76,79 |
| 2 | 5,6,9,10,11,13,14,15,16,17,19,21 ,23,24,25,26,27 ,28,29,30,31,32,33,34,35,36,37,38,39,40,41, 42,43,45,47,49,50,52,54,56,58,60,61,68,70 |
| 3 | 6,9,10,11,13,14,15,16,17,19,21 ,23,24,25,26,27 ,28,29,30,31,32,33,34,35,36,37,38,39,40,41,42, 43,45,47,49,50,52,54,56,58,60,61,68,70 |
| 4 | 9,10,11,13,14,15,16,17,19,21,23,24,25,26,27,28,29,30,31,32,33,34,35,36,37,38,39,40,41,42, 43,45,47,49,50,52,54,56,58,60,61,68,70 |
| 5 | 9,10,11,13,14,15,16,17,19,21,23,24,25,26,27,28,29,30,31,32,33,34,35,36,37,38,39,40,41,42, 43,45,47,49,50,52,54,56,58,60,61,68,70 |
| 6 | 12,18,44,46,55,57,63,66,69,71,72,73,74,75,77,78 |
| 7 | 20,22,51,53,59,62,64,65,67,76,79 |
| 8 | 11,13,14,15,16,17,19,21,23,24,25,26,27,28,29,30,31,32,33,34,35,36,37,38,39,40,41,42,43,45, 47,49,50,52,54,56,58,60,61,68,70 |
| 9 | 13,14,15,16,17,19,21,23,24,25,26,27,28,29,30,31,32,33,34,35,36,3 7,3 8,39,40,41,42,43,45,47, 49,50,52,54,56,58,60,61,68,70 |
| 10 | 13,14,15,16,17,19,21,23,24,25,26,27,28,29,30,31,32,33,34,35,36,37,3 8,39,40,41,42,43,45,47, 49,50,52,54,56,58,60,61,68,70 |
| 11 | 18,44,46,55,57,63,66,69,71,72,73,74,75,77,78 |
| 12 | 15,16,17,19,21,23,24,25,26,27,28,29,30,31,32,33,34,35,36,37,38,39,40,41,42,43,45,47,49,50, 52,54,56,58,60,61,68,70 |
| 13 | 16,17,19,21,23,24,25,26,27,28,29,30,31,32,33,34,35,36,37,3 8,39,40,41,42,43,45,47,49,50,52, 54,56,58,60,61,68,70 |
| 14 | 17,19,21,23,24,25,26,27,28,29,30,31,32,33,34,35,36,37,3 8,39,40,41,42,43,45,47,49,50,52,54, 56,58,60,61,68,70 |
| 15 | 19,21,23,24,25,26,27,28,29,30,31,32,33,34,35,36,37,38,39,40,41,42,43,45,4 7,49,50,52,54,56, 58,60,61,68,70 |
| 16 | 19,21,23,24,25,26,27,28,29,30,31,32,33,34,35,36,37,38,39,40,41,42,43,45,4 7,49,50,52,54,56, 58,60,61,68,70 |
| 17 | 44,46,55,57,63,66,69,71,72,73,74,75,77,78 |
| 18 | 21,23,24,25,26,27,28,29,30,3 1,32,3 3,34,3 5,36,37,3 8,39,40,41,42,43,45,47,49,50,52,54,56,58, 60,61,68,70 |
| 19 | 22,51,53,59,62,64,65,67,76,79 |
| 20 | 23,24,25,26,27 ,28,29 ,30,31,32,3 3,34,3 5,3 6,37,3 8,39,40,41,42,43,45,47,49,50,52,54,56,58,60, 61,68,70 |
| 21 | 51,53,59,62,64,65,67,76,79 |
| 22 | 25,26,27,28,29,30,31,32,33,34,35,36,37,38,39,40,41,42,43,45,47,49,50,52,54,56,58,60,61,68, 70 |
| 23 | 26,27,28,29,30,31,32,33,34,3 5,36,37,38,39,40,41,42,43,45,47,49,50,52,54,56,58,60,61,68,70 |
| 24 | 27,28,29,30,31,32,33,34,35,36,37,38,39,40,41 ,42,43,45,47,49,50,52,54,56,58,60,61,68,70 |
| 25 | 28,29,30,31,32,33,34,35,36,37,38,39,40,41,42,43,45,47,49,50,52,54,56,58,60,61,68,70 |
| 26 | 29,30,31,32,33,34,35,36,37,38,39,40,41,42,43,45,47 ,49 ,50,52,54,56,58,60,61,68,70 |
| 27 | 30,31,32,33,34,35,36,37,38,39,40,41,42,43,45,47,49,50,52,54,56,58,60,61,68,70 |
| 28 | 31,32,33,34,35,36,37,38,39,40,41,42,43,45,47,49,50,52,54,56,58,60,61,68,70 |
| 29 | 32,33,34,35,36,37,38,39,40,41,42,43,45,47,49,50,52,54,56,58,60,61,68,70 |
| 30 | 33,34,35,36,37,38,39,40,41,42,43,45,47,49,50,52,54,56,58,60,61,68,70 |
| 31 | 34,3 5,3 6,37,38,39,40,41,42,43,45,47,49,50,52,54,56,58,60,61,68,70 |
| 32 | 35,36,37,38,39,40,41,42,43,45,47,49,50,52,54,56,58,60,61,68,70 |
| 33 | 36,37,38,39,40,41,42,43,45,47,49,50,52,54,56,58,60,61,68,70 |
| 34 | 37,38,39,40,41,42,43,45,47,49,50,52,54,56,58,60,61,68,70 |
| 35 | 38,39,40,41,42,43,45,47,49,50,52,54,56,58,60,61,68,70 |
| 36 | 39,40,41,42,43,45,47,49,50,52,54,56,58,60,61,68,70 |
| 37 | 40,41,42,43,45,47,49,50,52,54,56,58,60,61,68,70 |
| 38 | 41,42,43,45,47,49,50,52,54,56,58,60,61,68,70 |
| 39 | 42,43,45,47,49,50,52,54,56,58,60,61,68,70 |
| 40 | 43,45,47,49,50,52,54,56,58,60,61,68,70 |
| 41 | 45,47,49,50,52,54,56,58,60,61,68,70 |
| 42 | 45,47,49,50,52,54,56,58,60,61,68,70 |
| 43 | 46,55,57,63,66,69,71,72,73,74,75,77,78 |
| 44 | 47,49,50,52,54,56,58,60,61,68,70 |
| 45 | 55,57,63,66,69,71,72,73,74,75,77,78 |
| 46 | 49,50,52,54,56,58,60,61,68,70 |
| 47 | 50,52,54,56,58,60,61,68,70 |
| 48 | 52,54,56,58,60,61,68,70 |
| 49 | 52,54,56,58,60,61,68,70 |
| 50 | 53,59,62,64,65,67,76 |
| 51 | 54,56,58,60,61,68,70 |
| 52 | 59,62,64,65,67,76 |
| 53 | 56,58,60,61,68,70 |
| 54 | 57,63,66,69,71,72,73,74,75,77,78 |
| 55 | 58,60,61,68,70 |
| 56 | 63,66,69,71,72,73,74,75,77,78 |
| 57 | 60,61,68,70 |
| 58 | 62,64,65,67,76,79 |
| 59 | 68,70 |
| 60 | 68,70 |
| 61 | 64,65,67,76,79 |
| 62 | 66,69,71,72,73,74,75,77,78 |
| 63 | 67,76,79 |
| 64 | 67,76,79 |
| 65 | 69,71,72,73,74,75,77,78 |
| 66 | 76,79 |
| 67 | 70 |
| 68 | 71,72,73,74,75,77,78 |
| 70 | 73,74,75,77,78 |
| 71 | 74,75,77,78 |
| 72 | 75,77,78 |
| 73 | 77,78 |
| 74 | 77,78 |

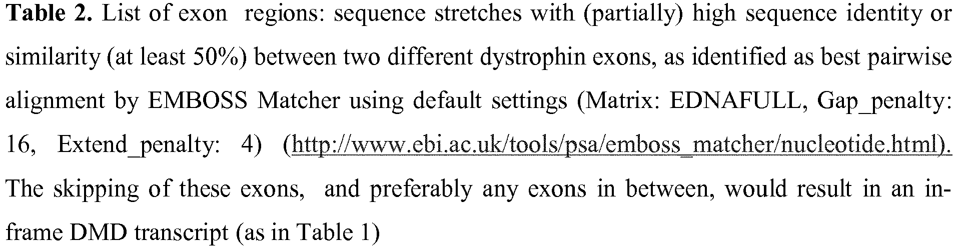

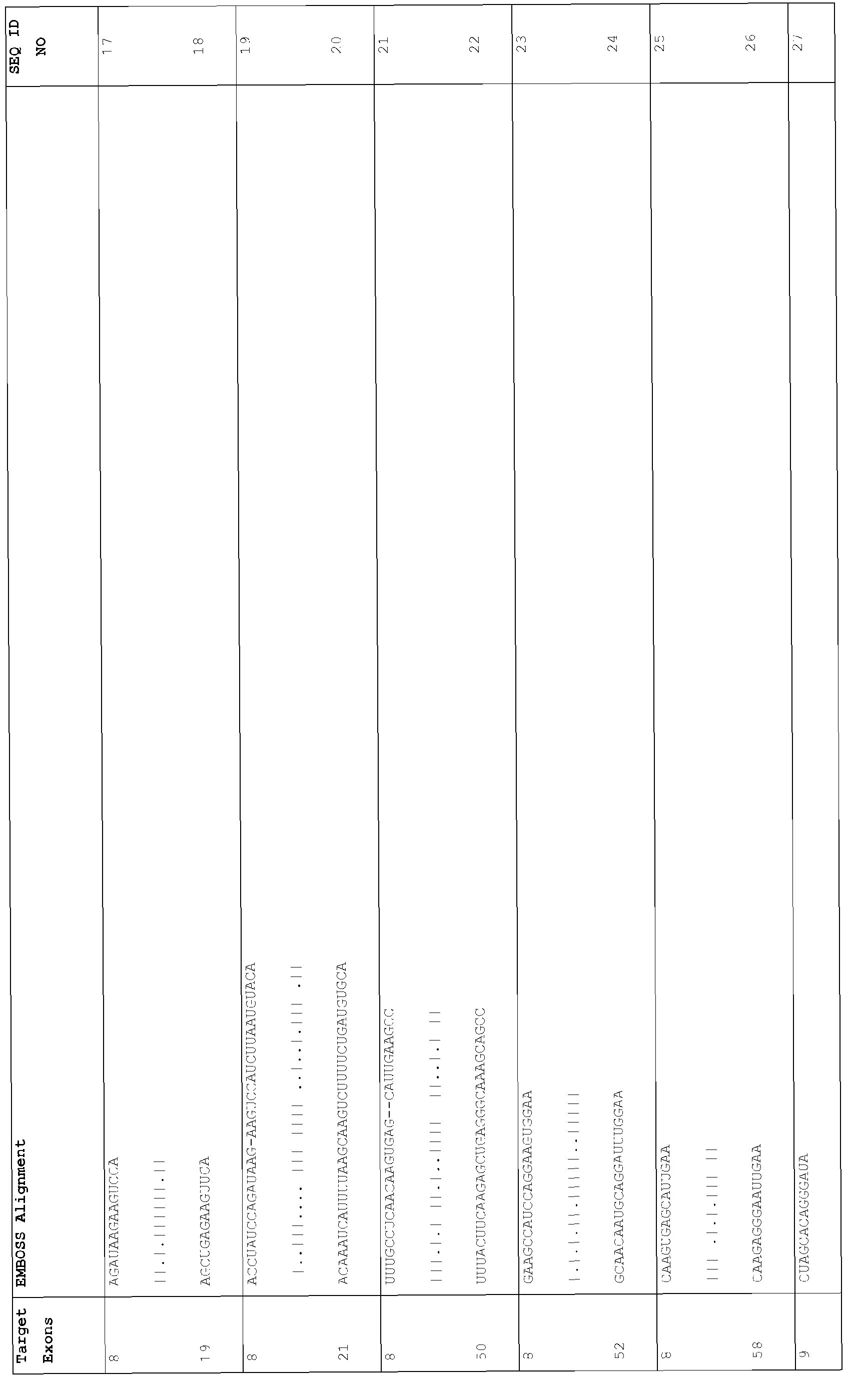

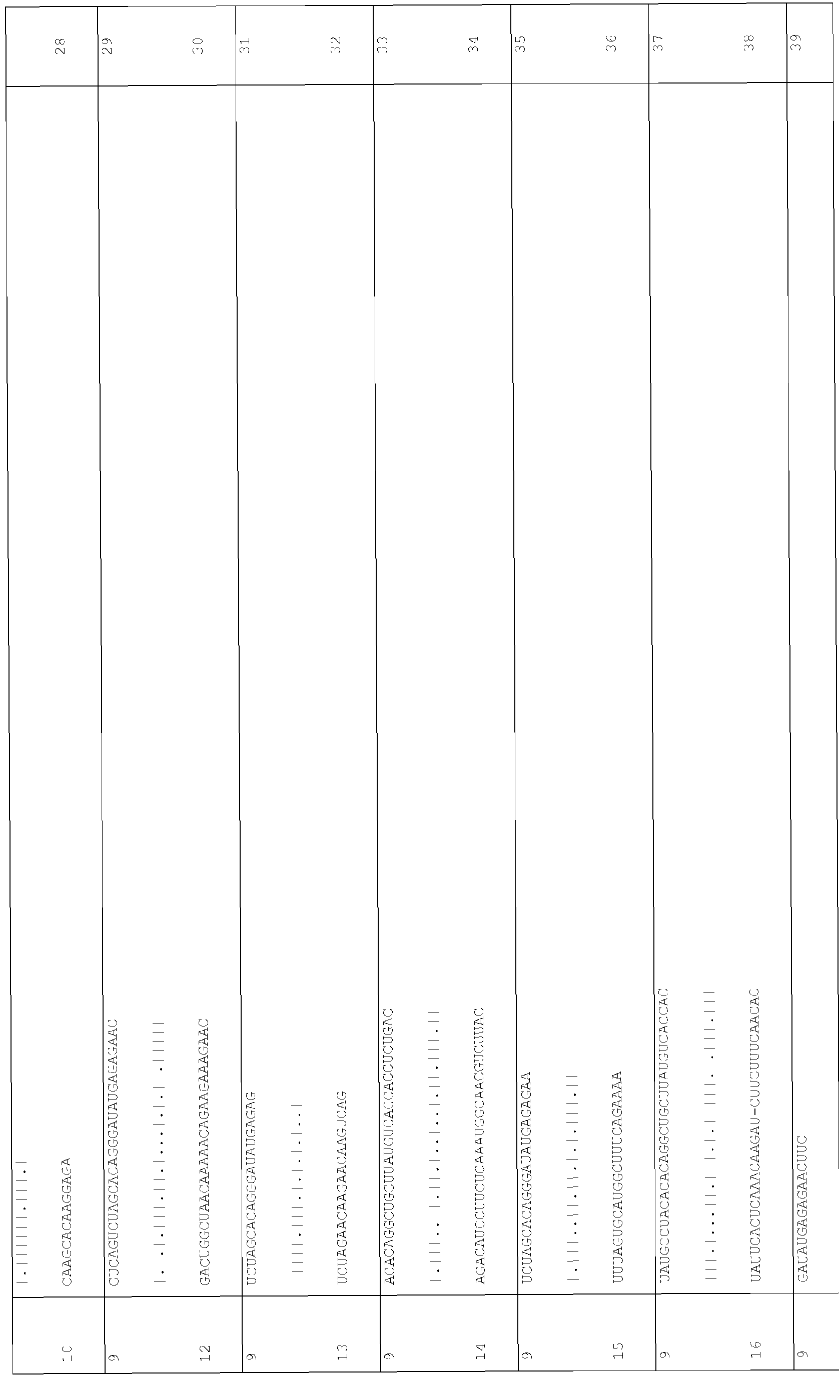

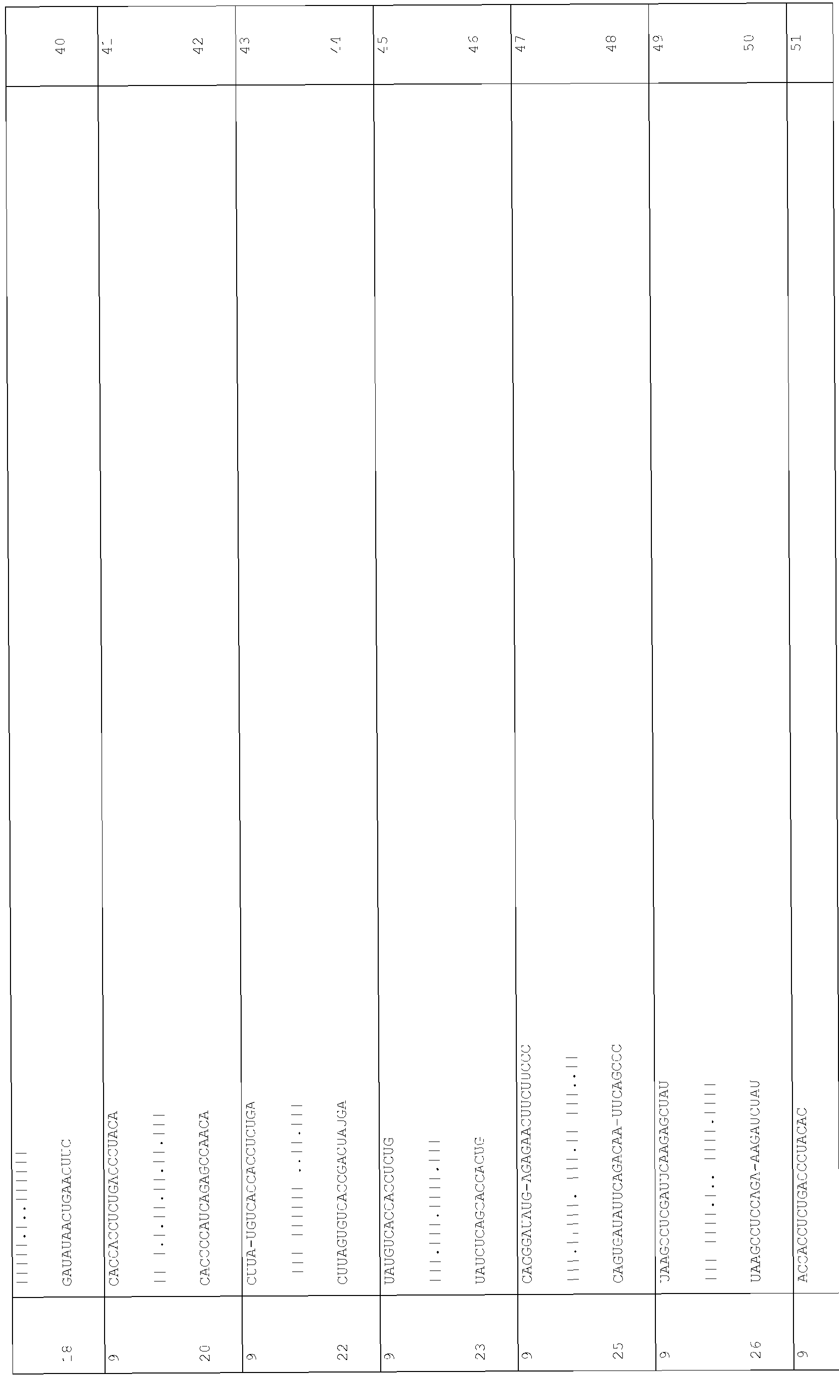

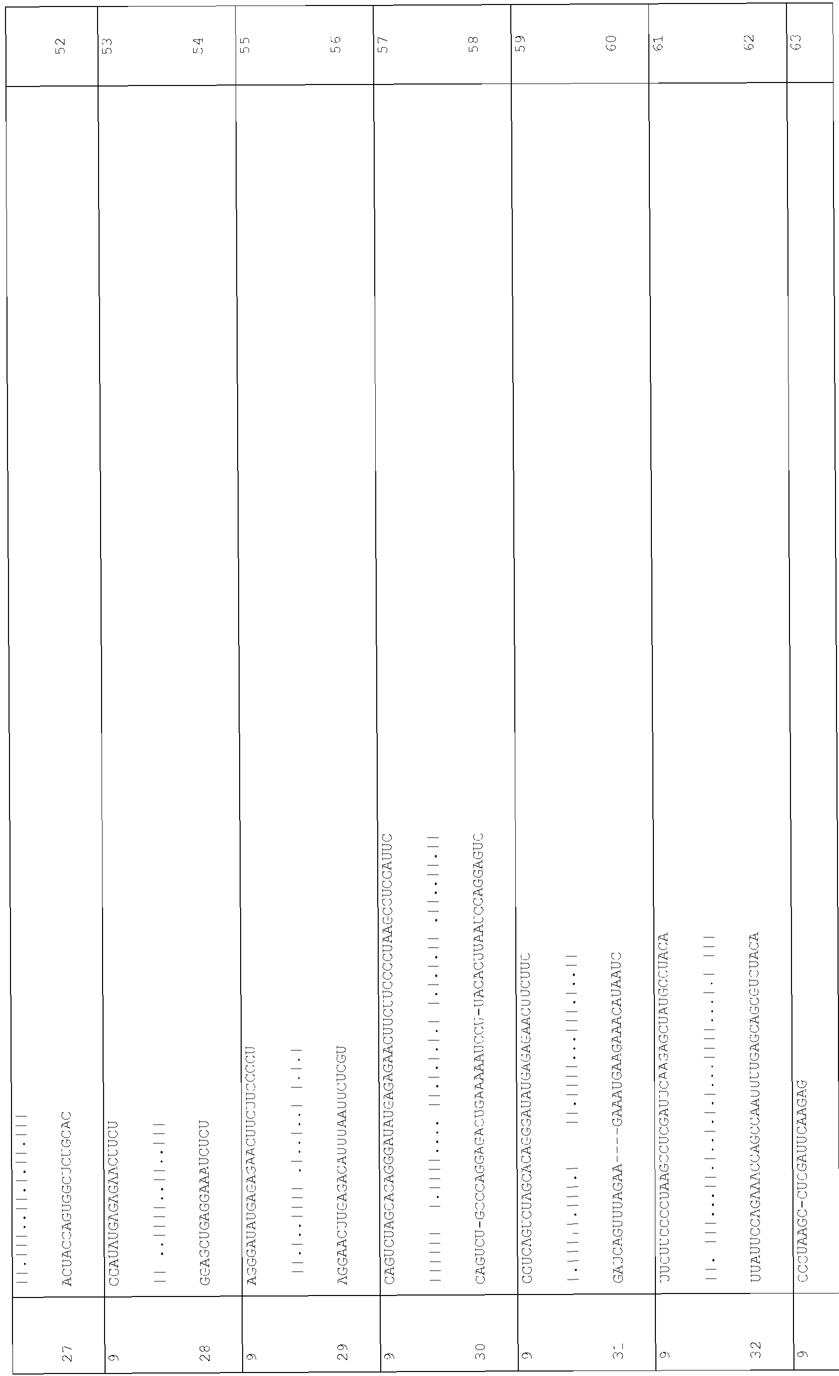

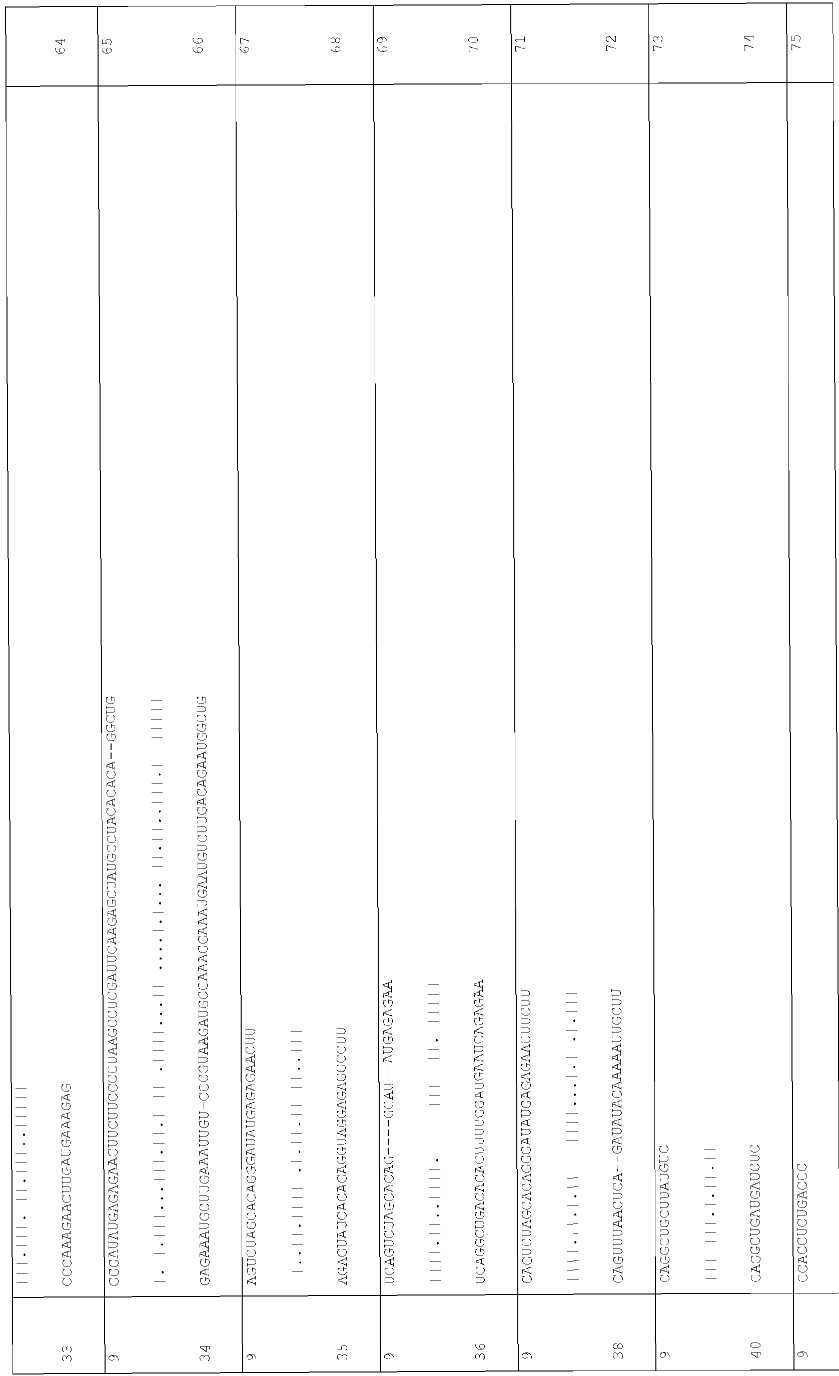

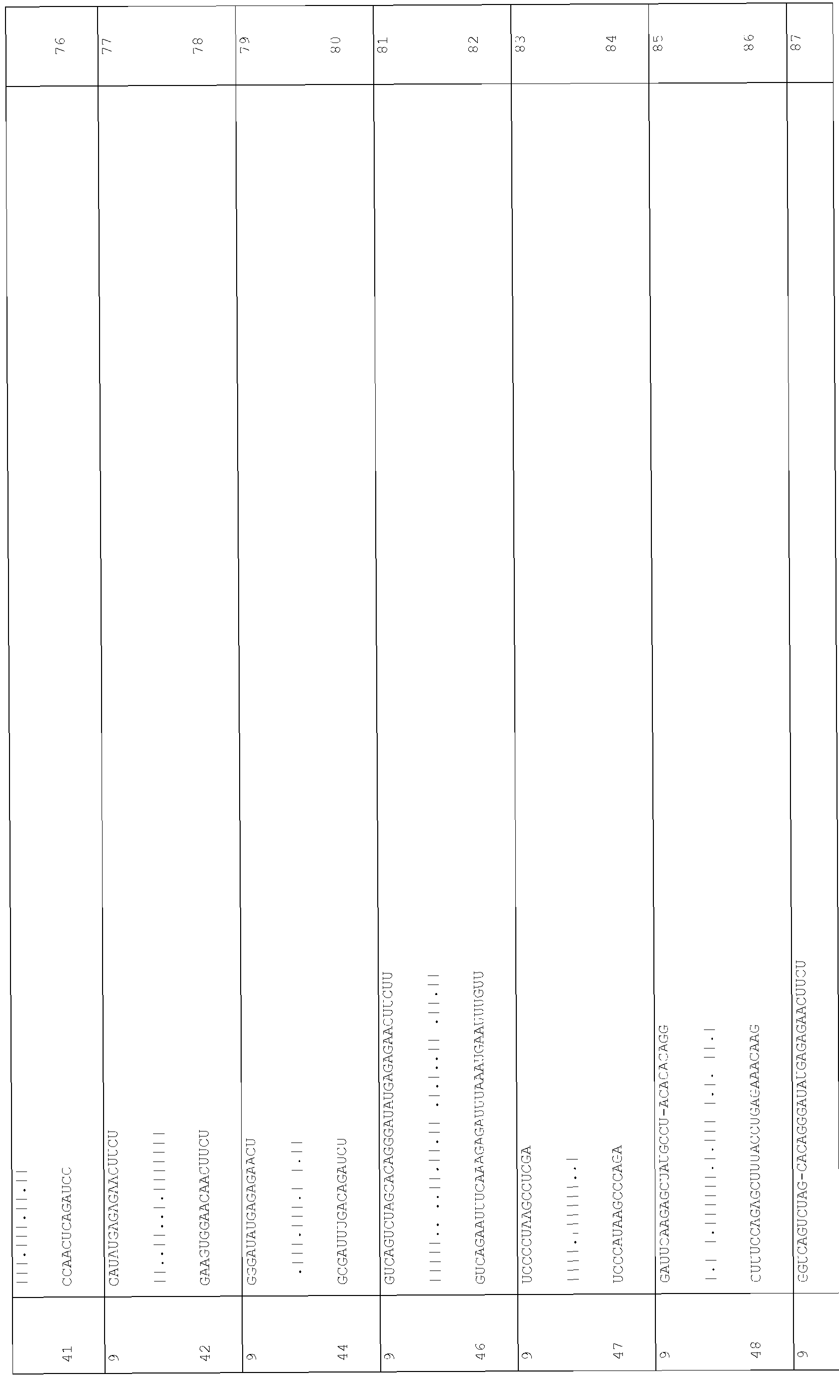

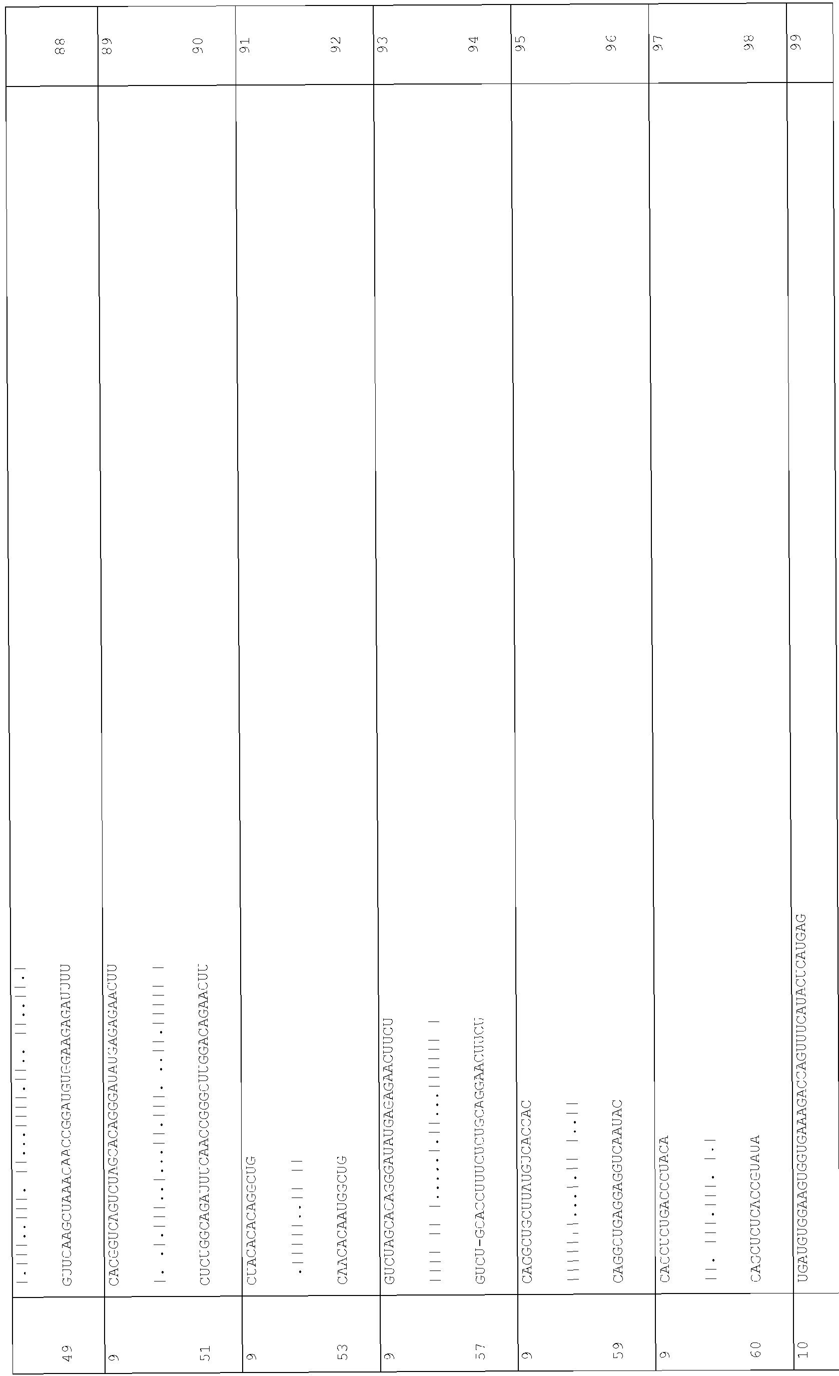

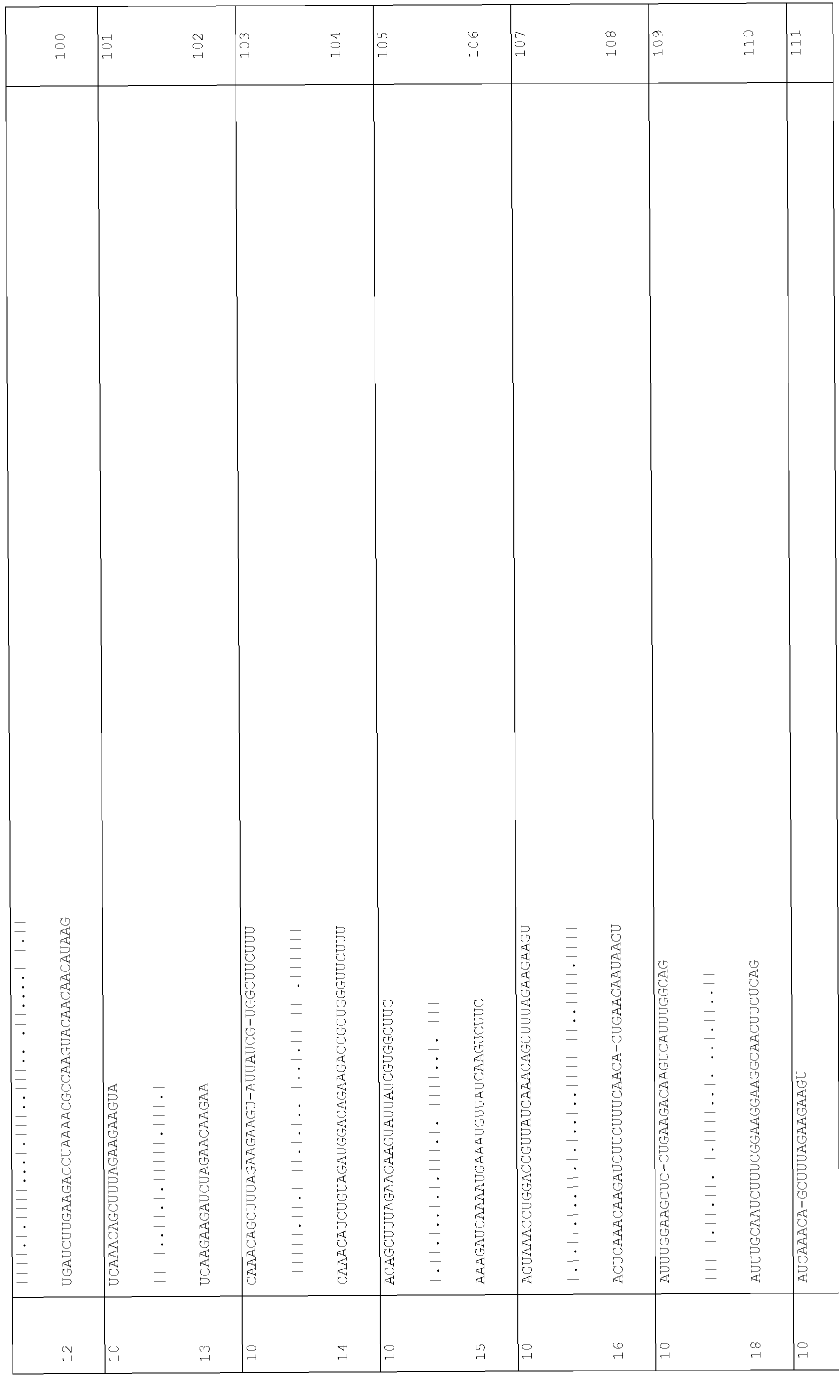

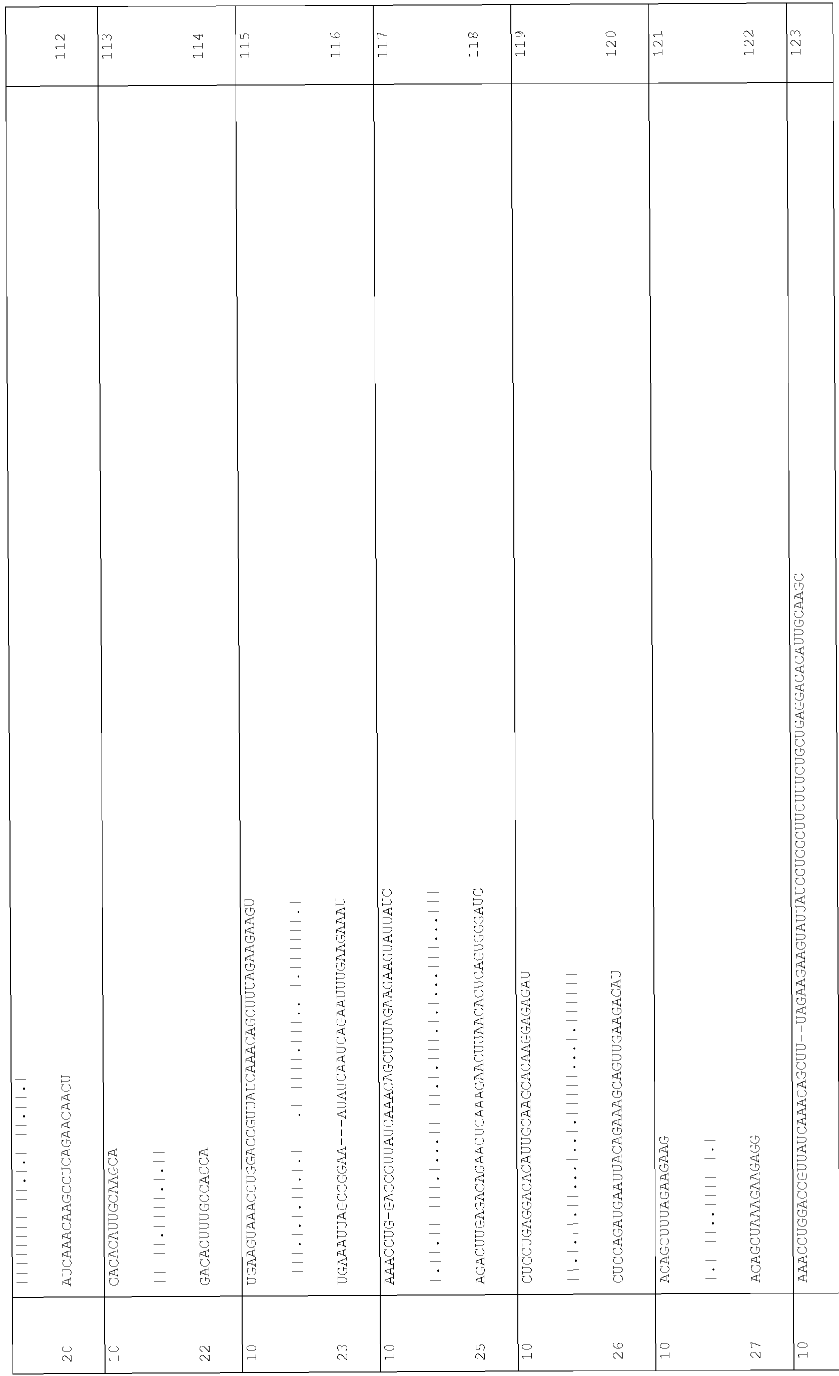

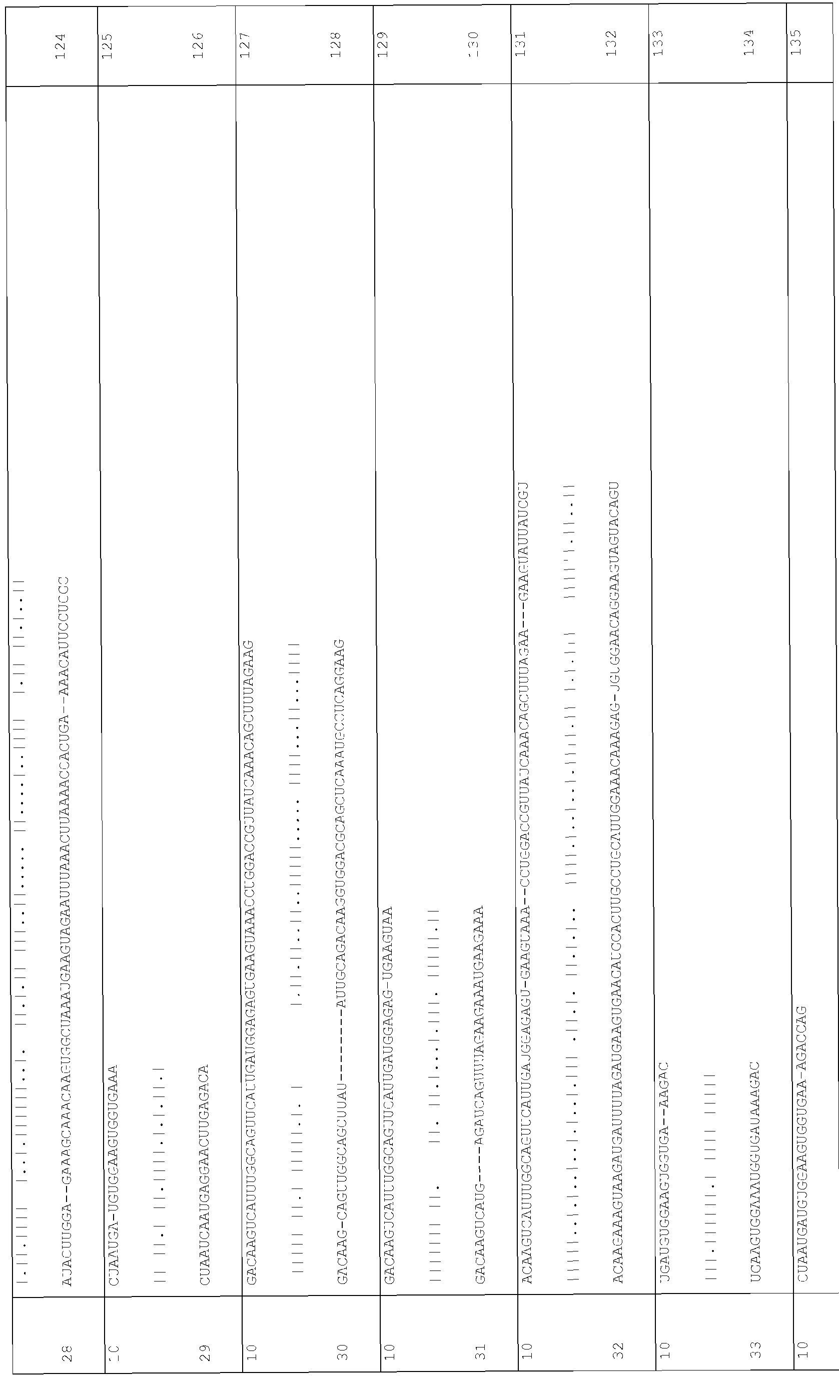

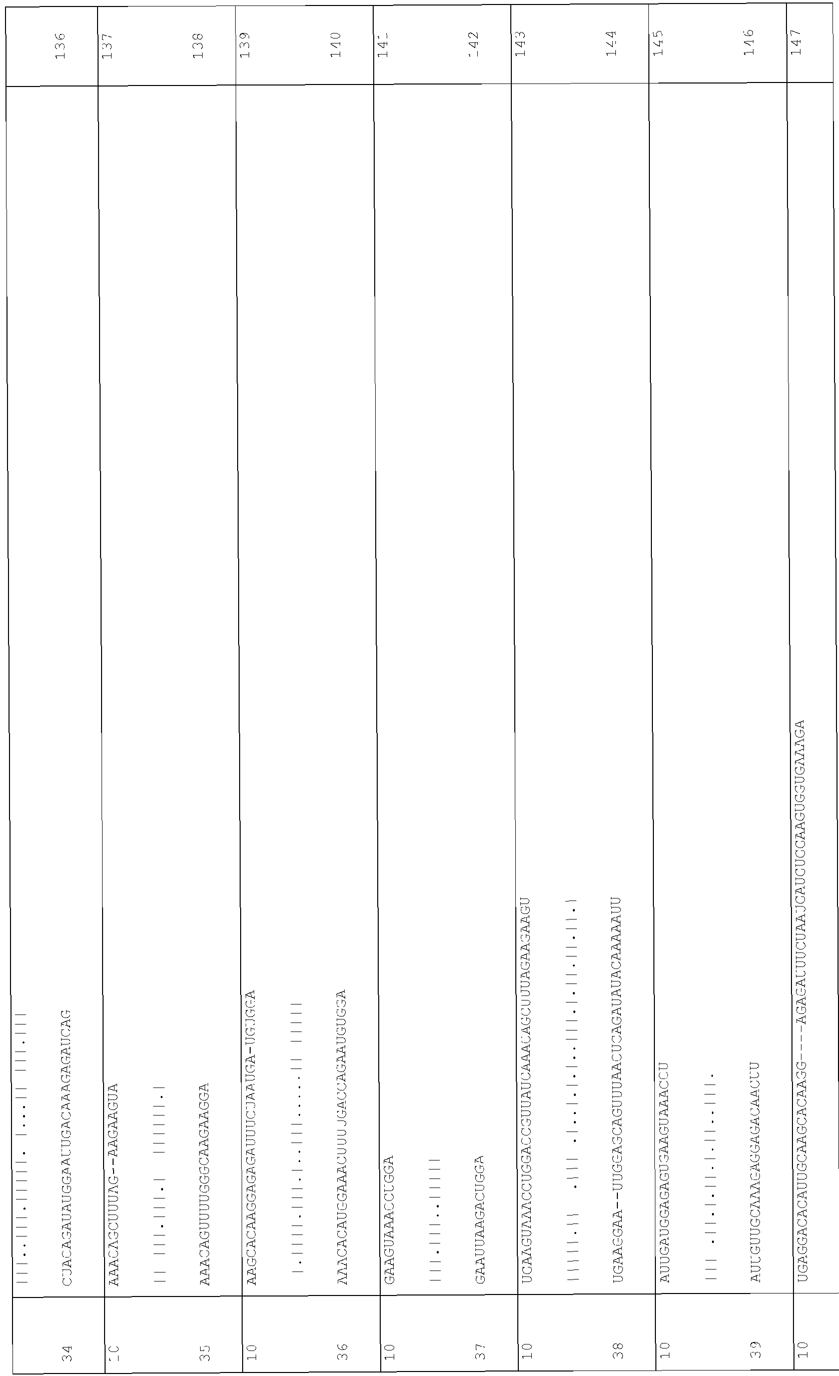

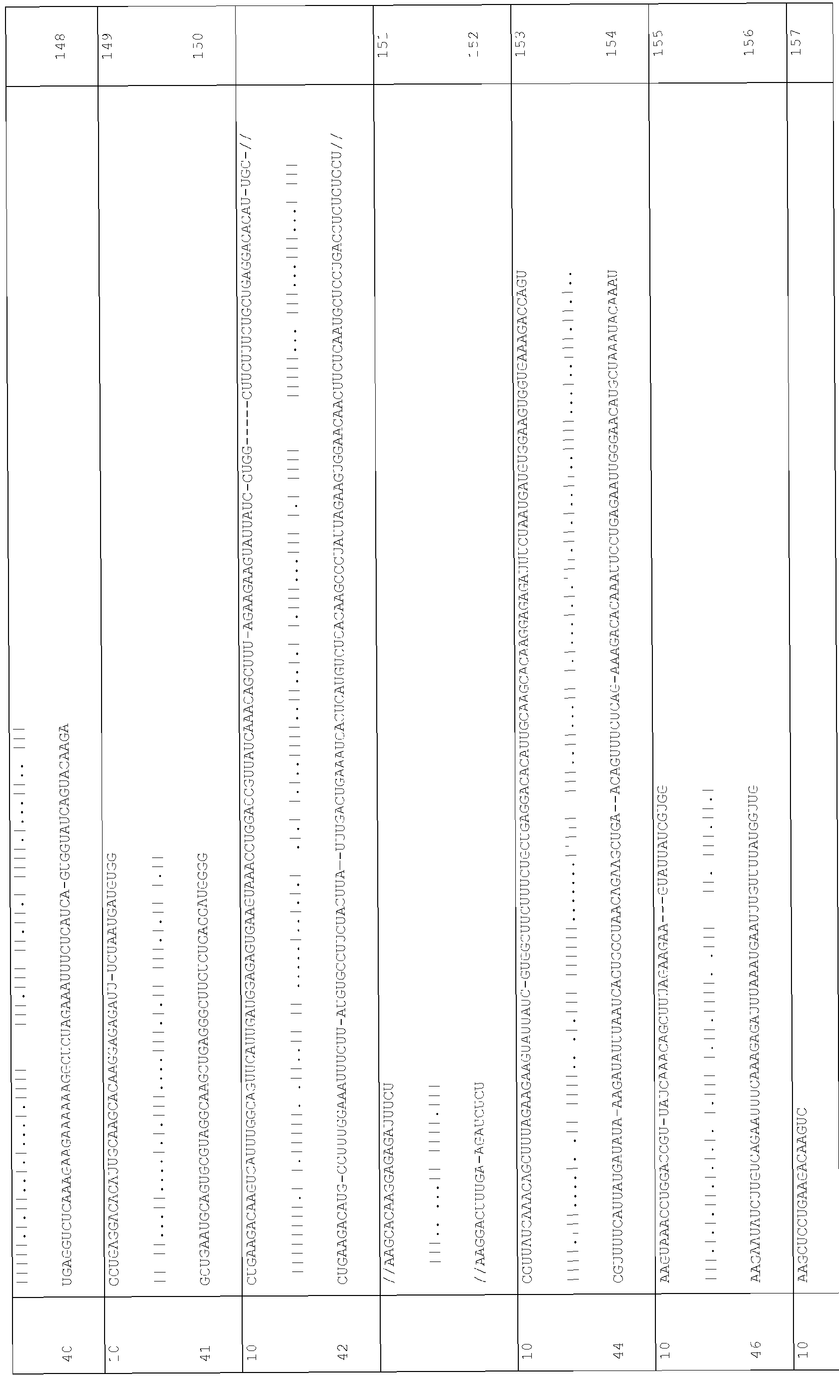

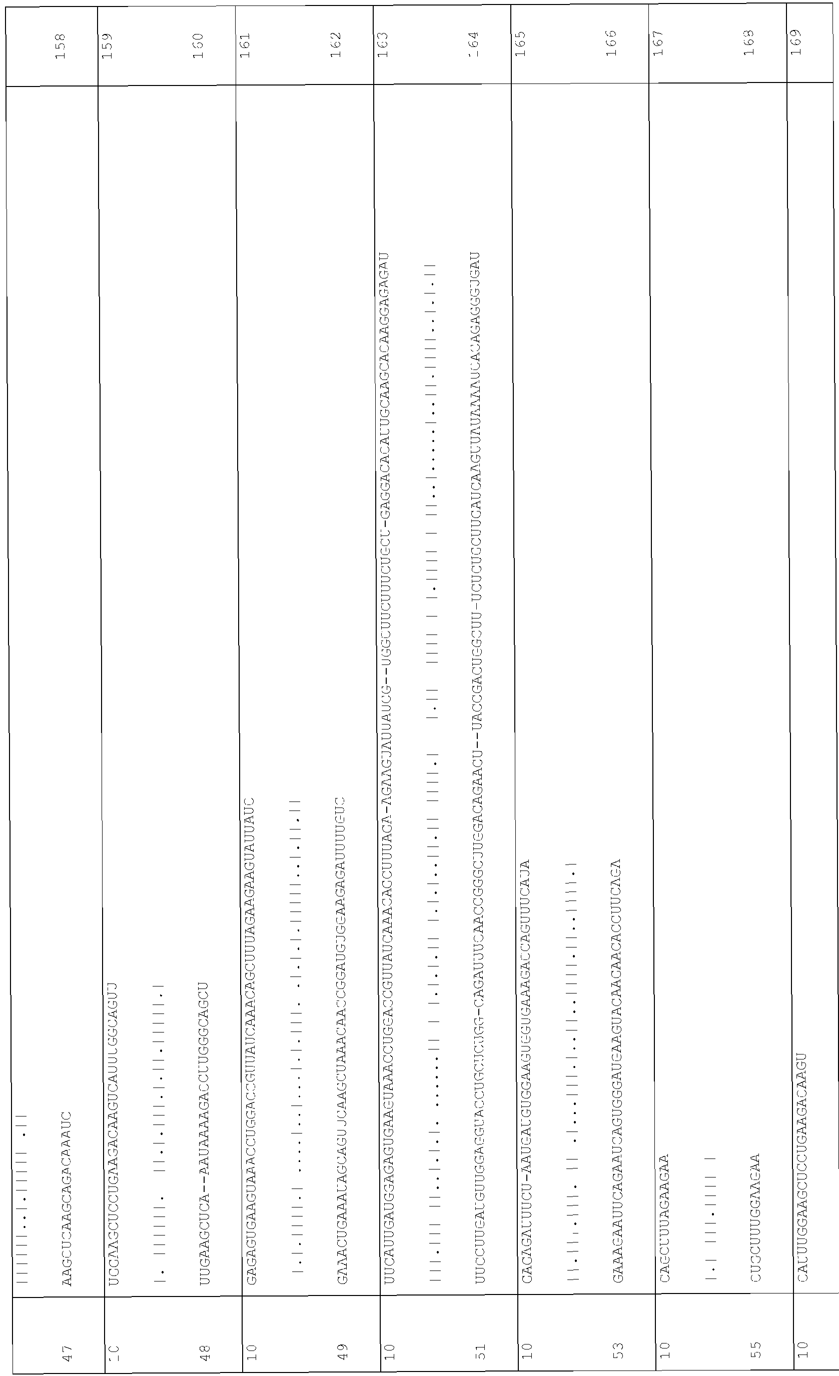

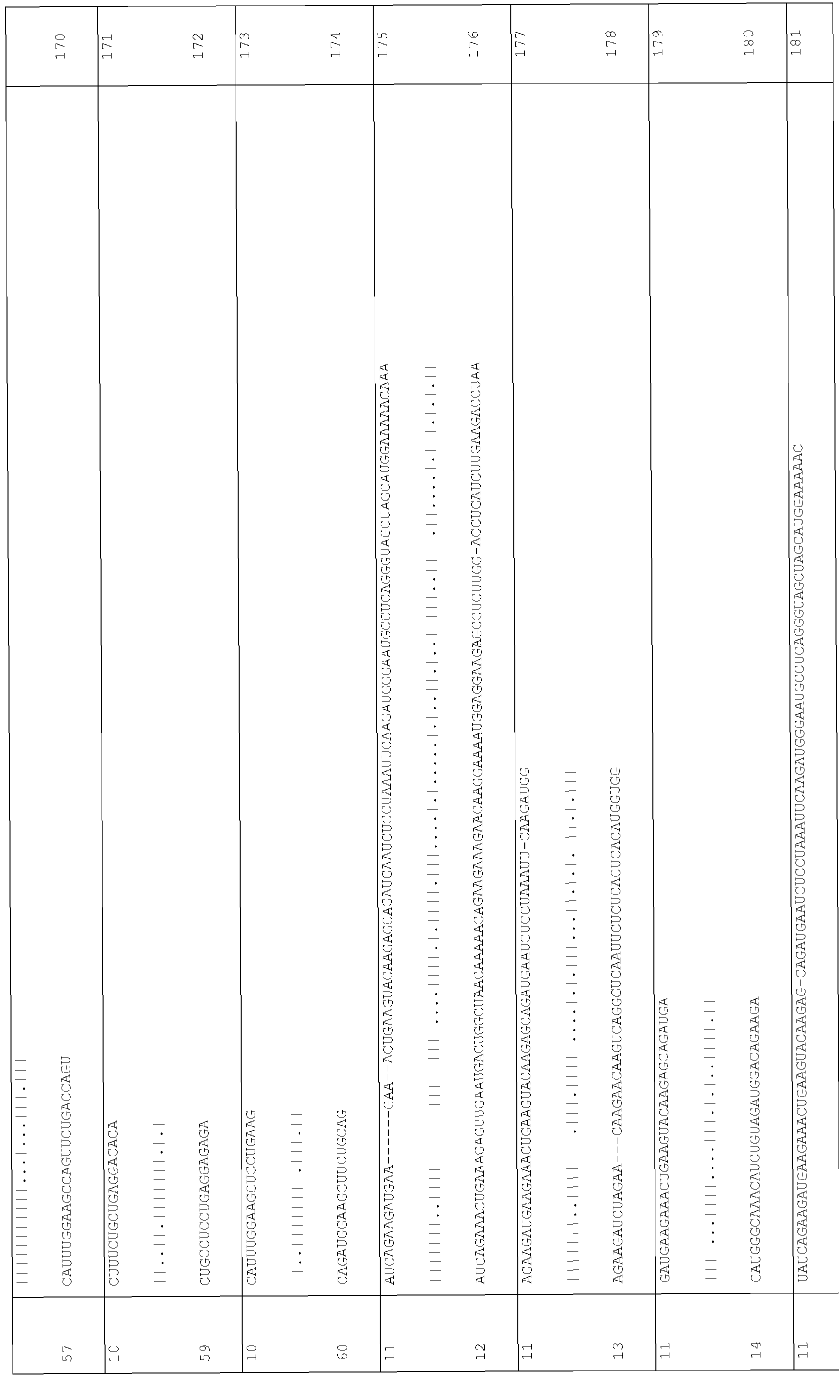

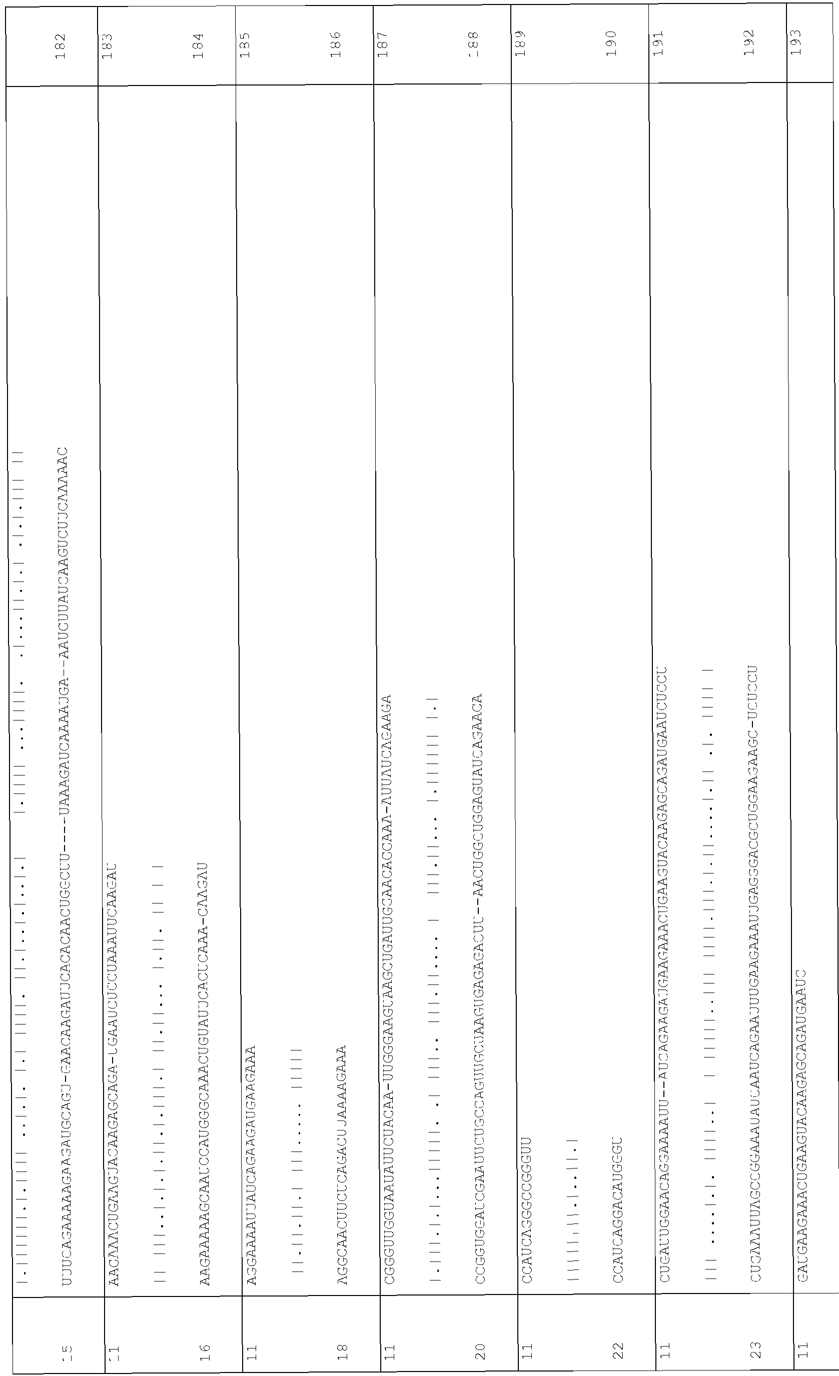

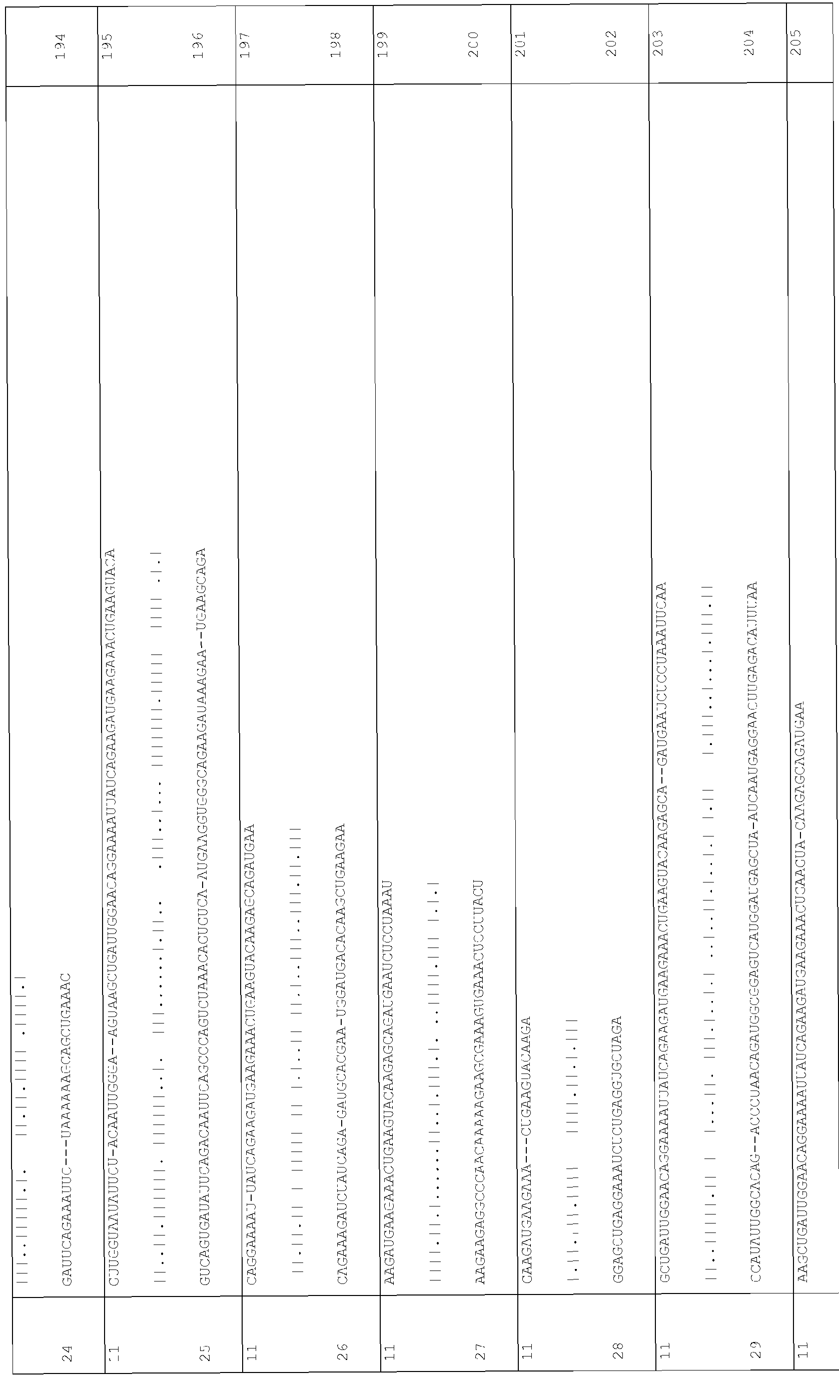

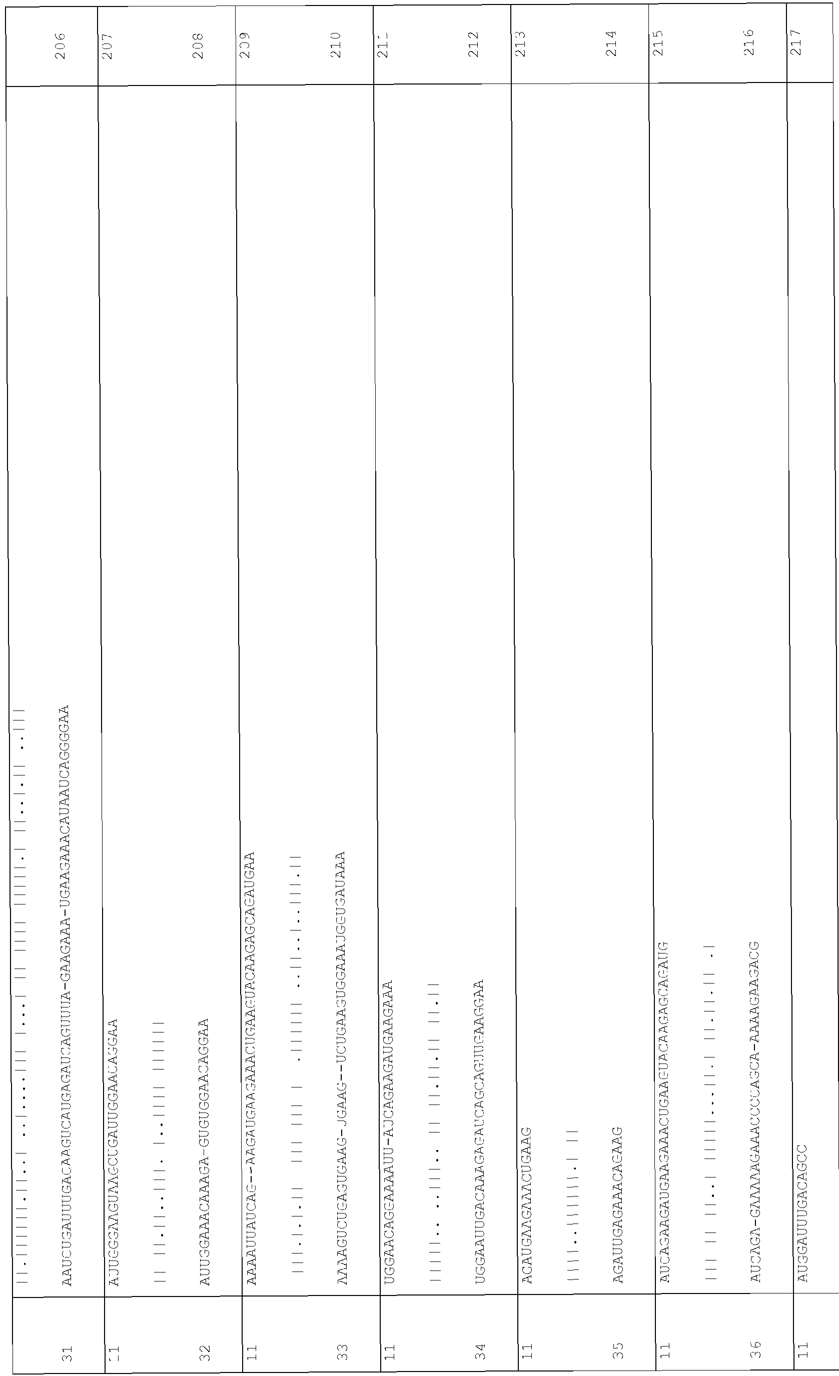

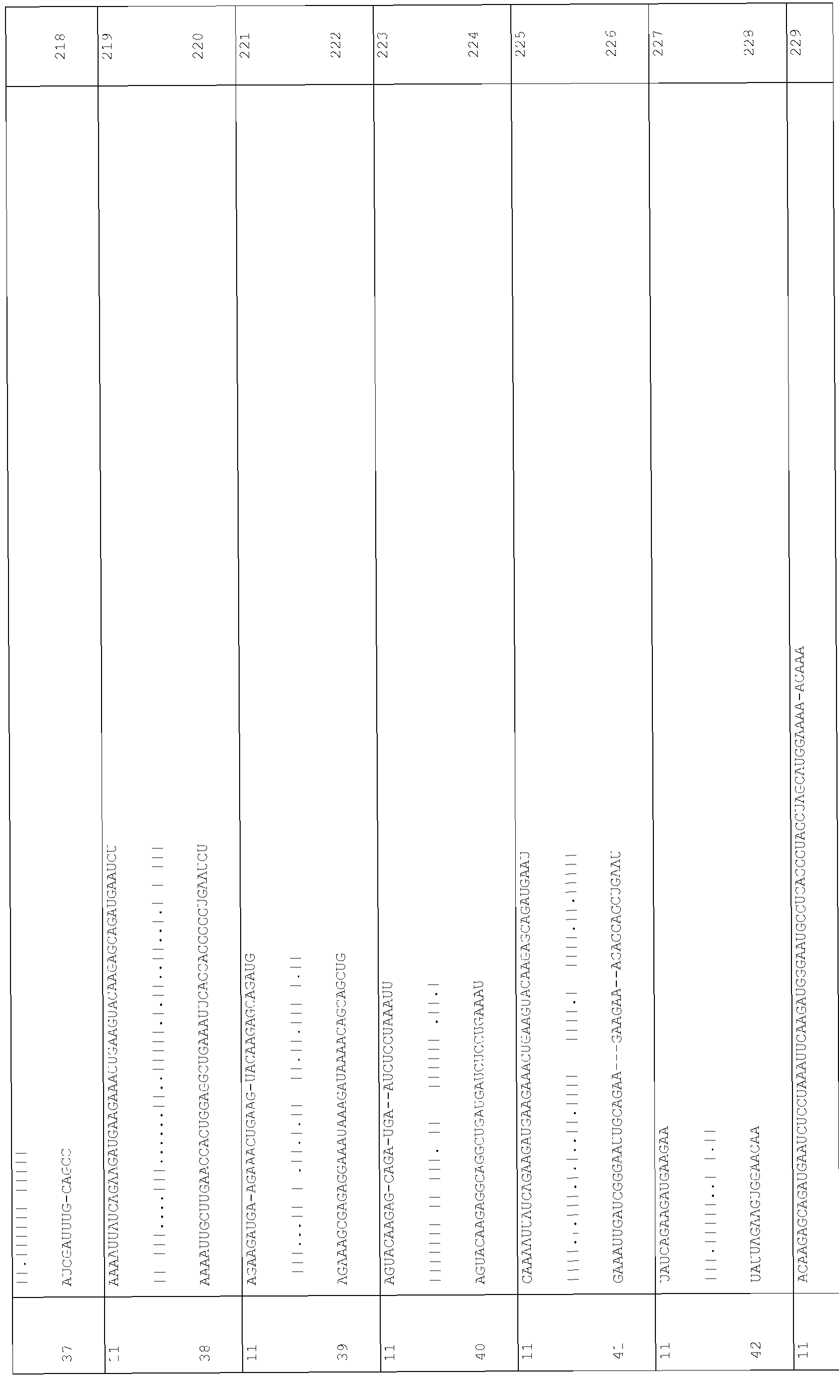

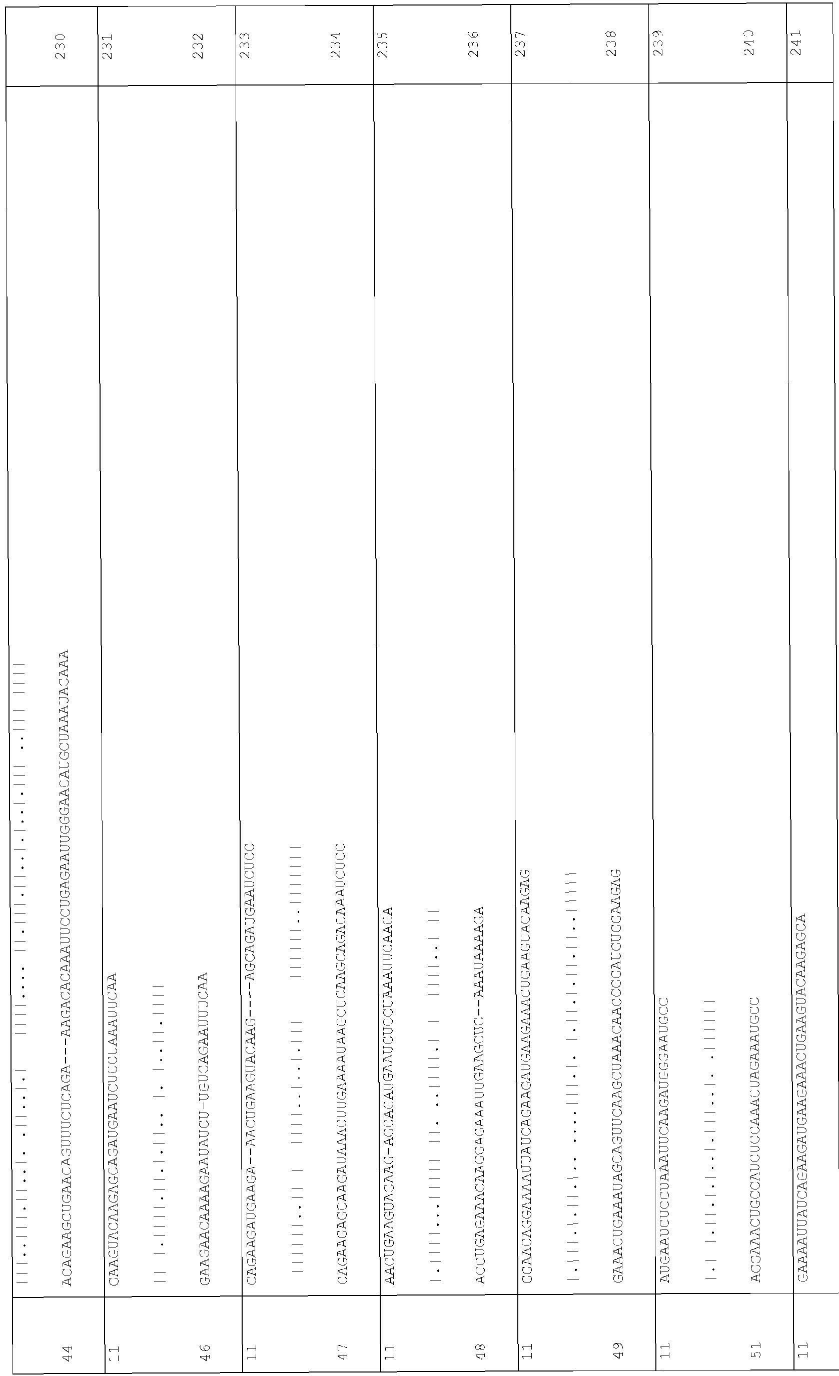

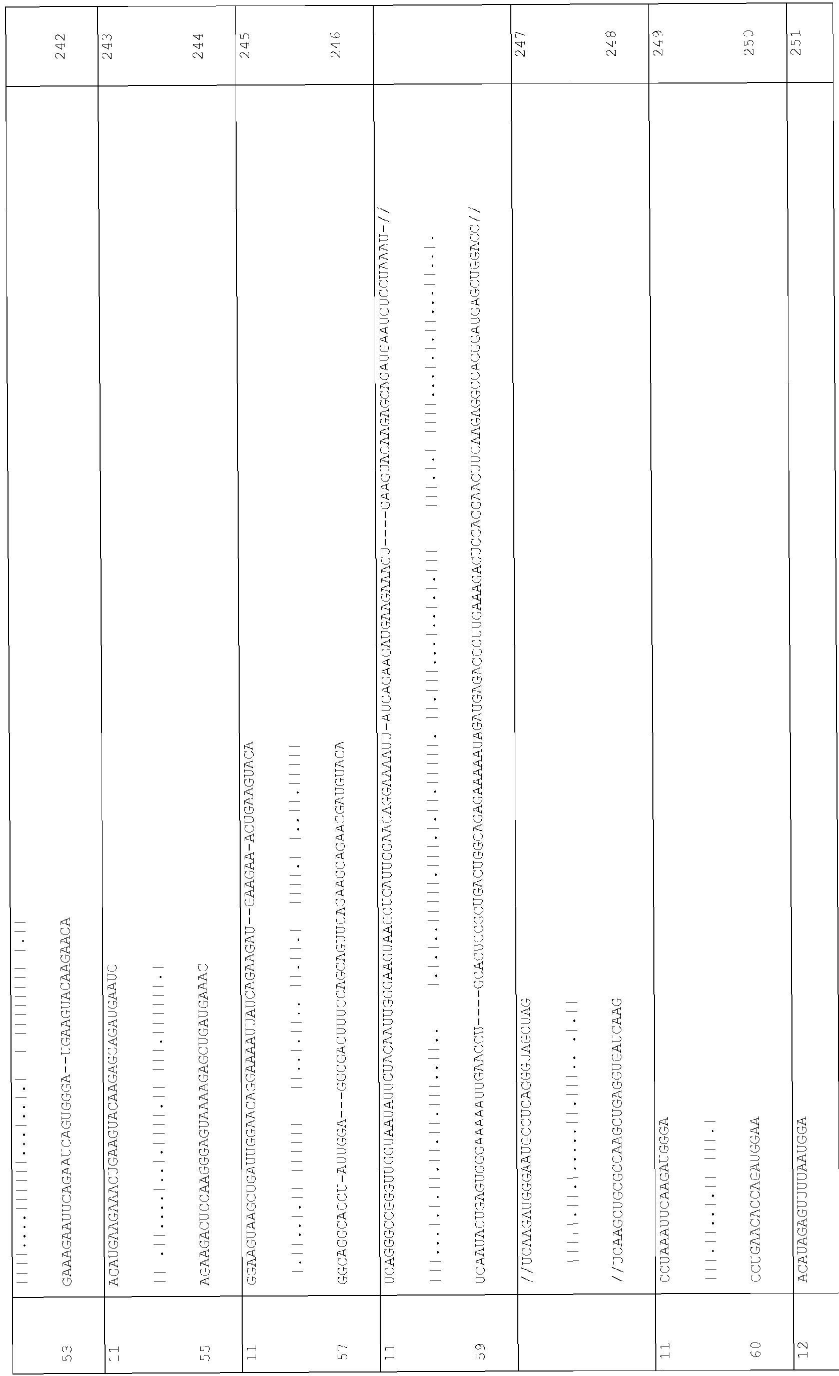

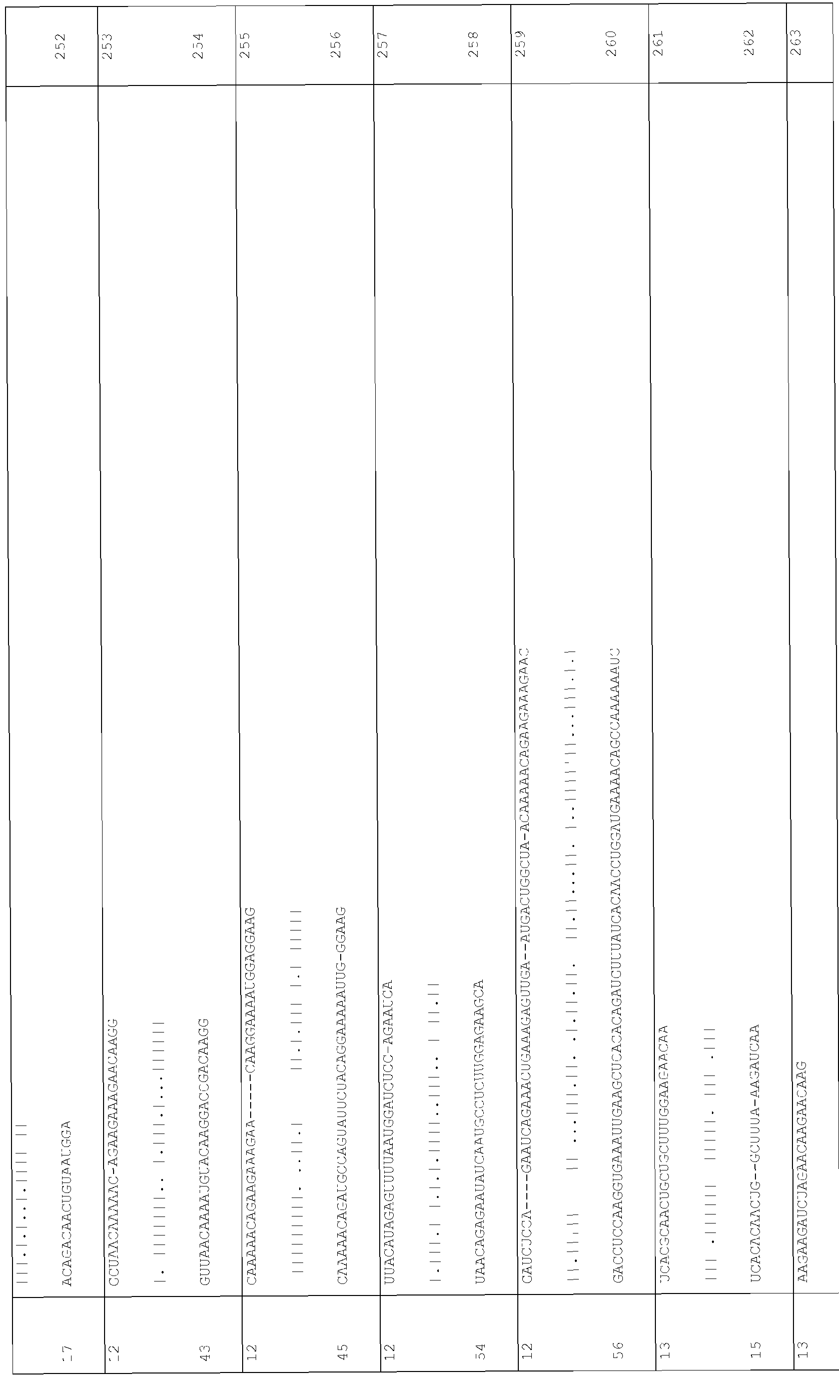

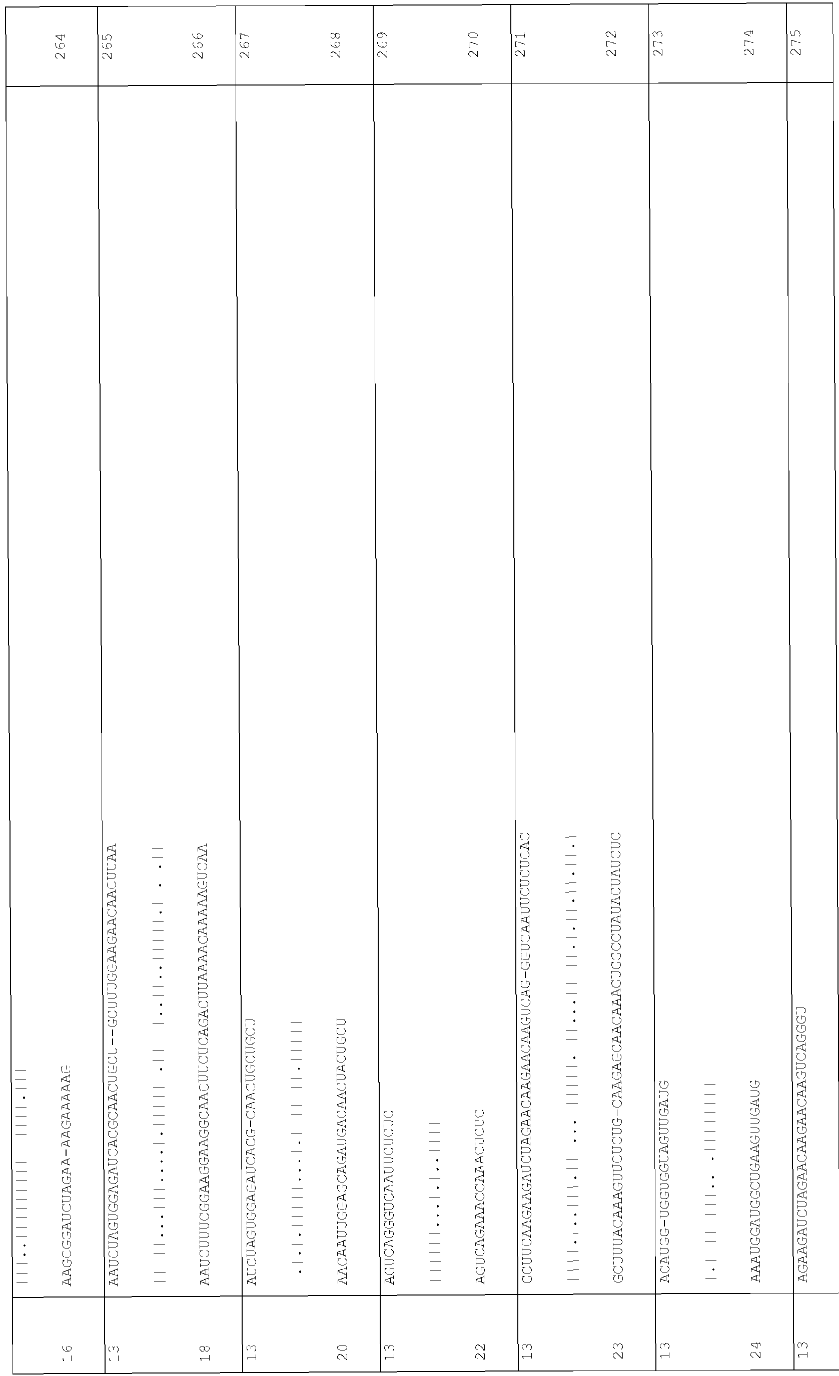

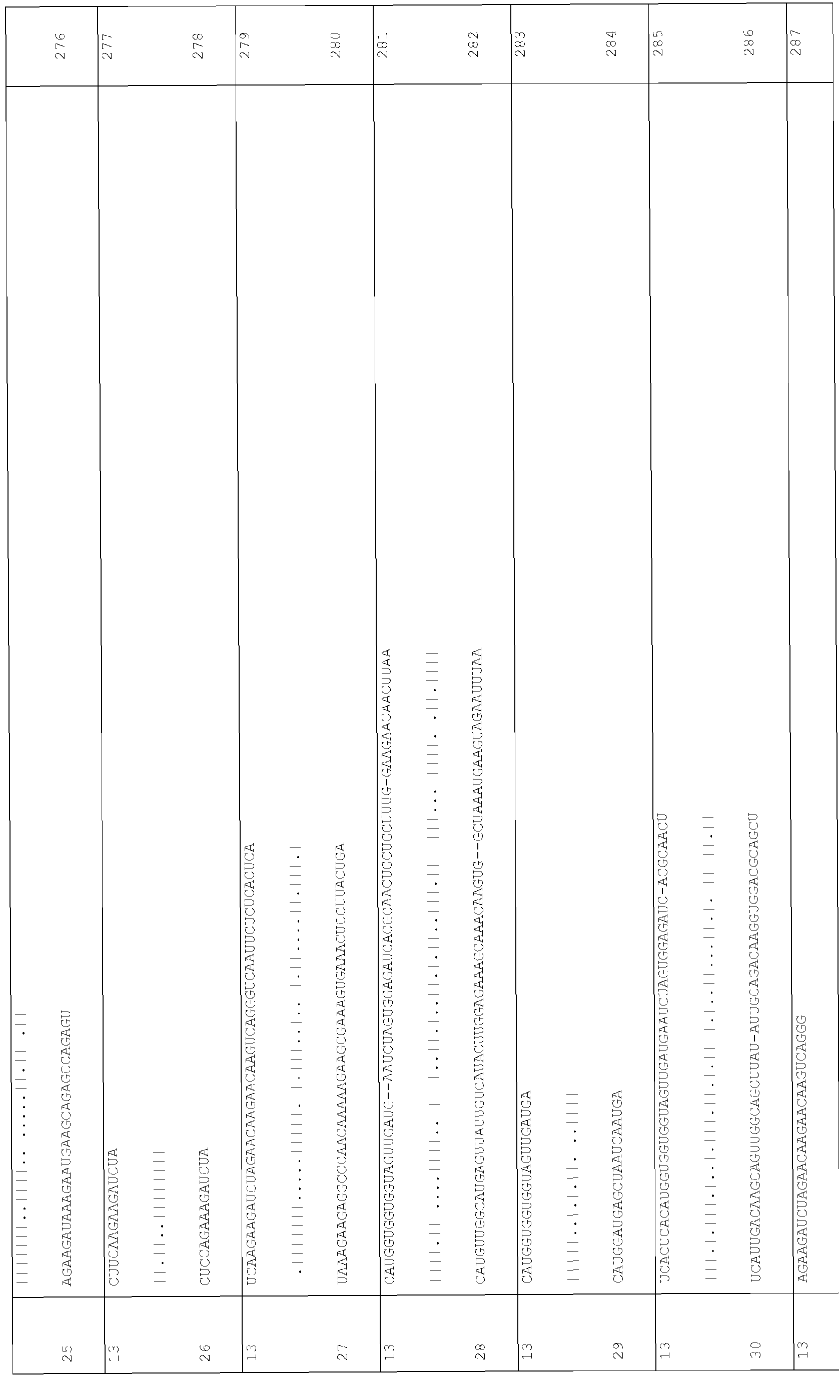

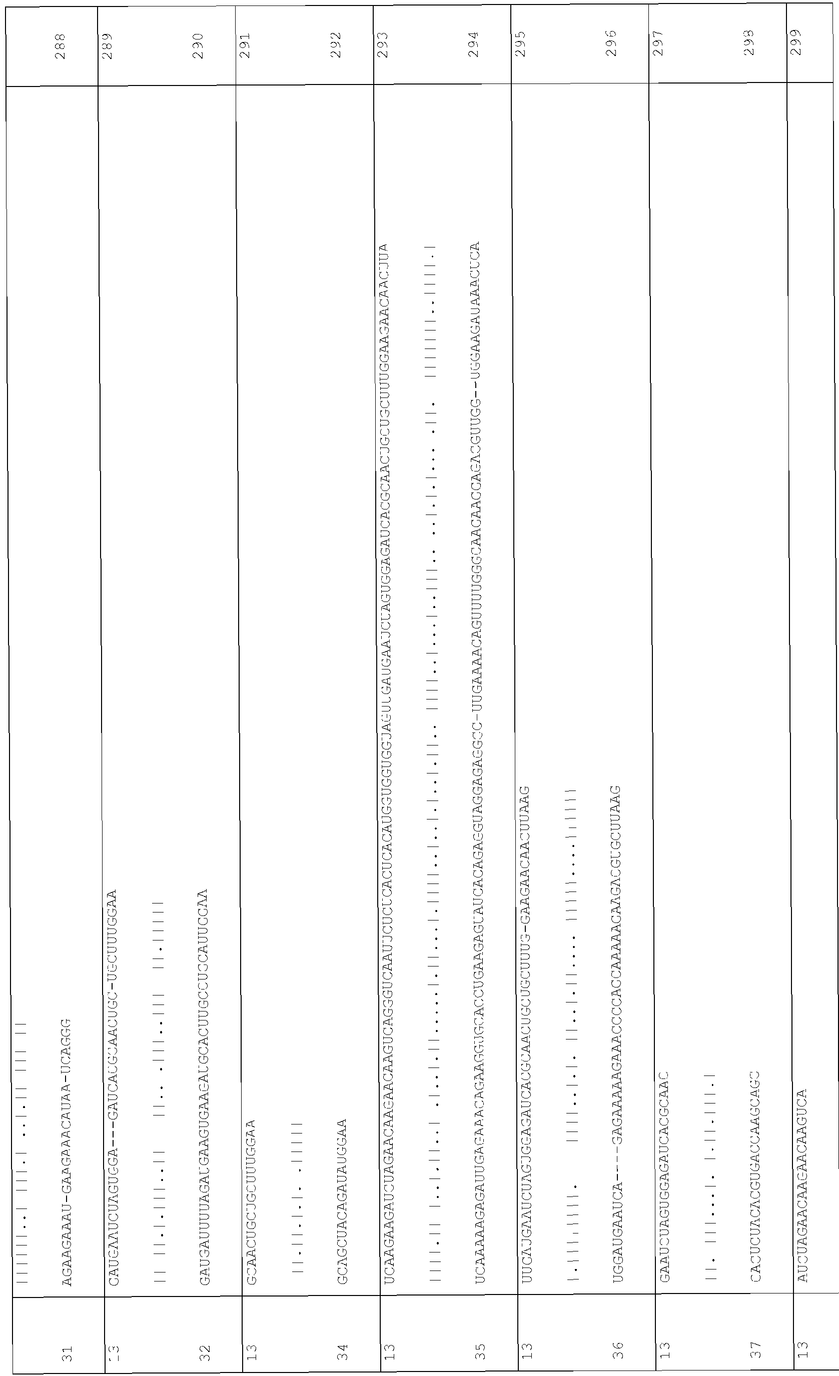

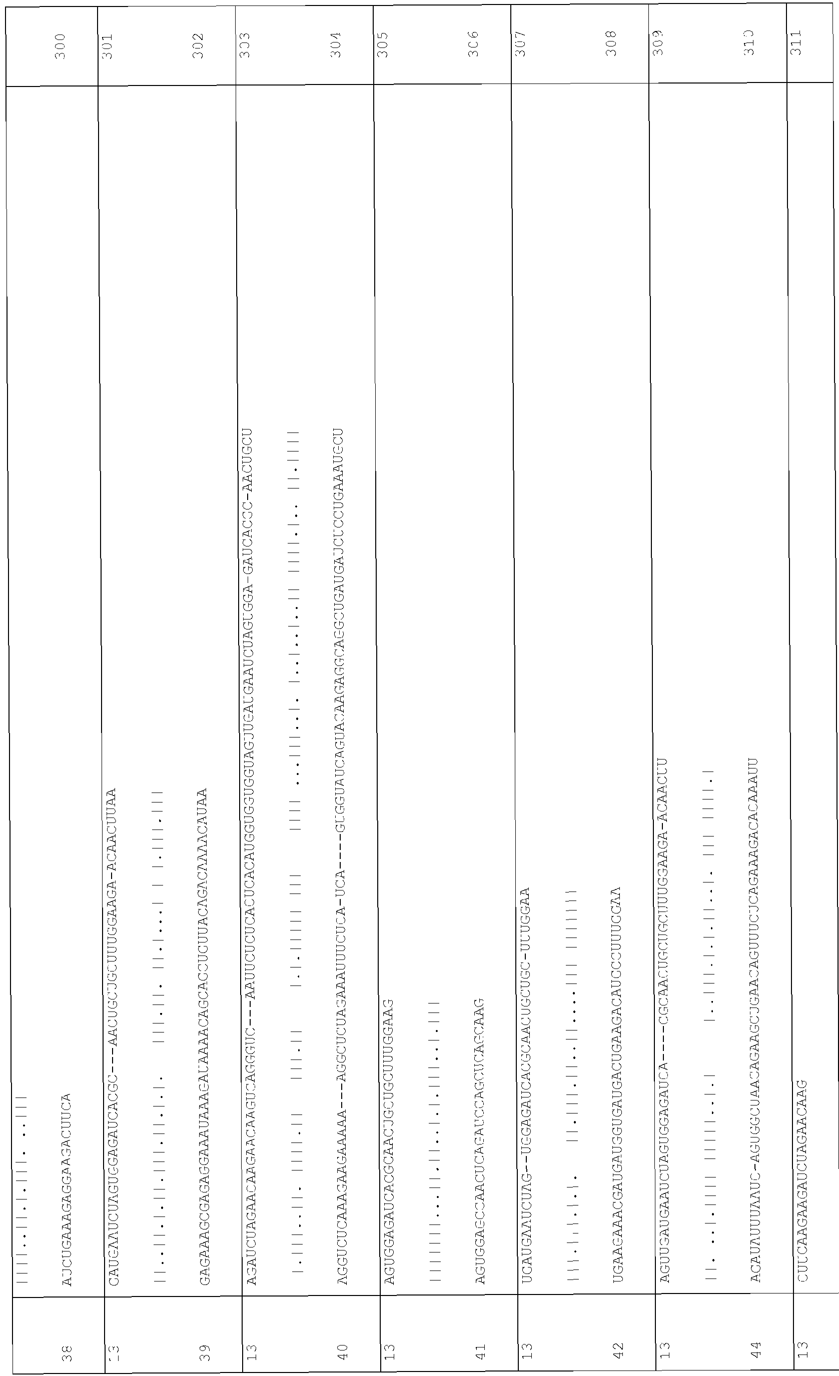

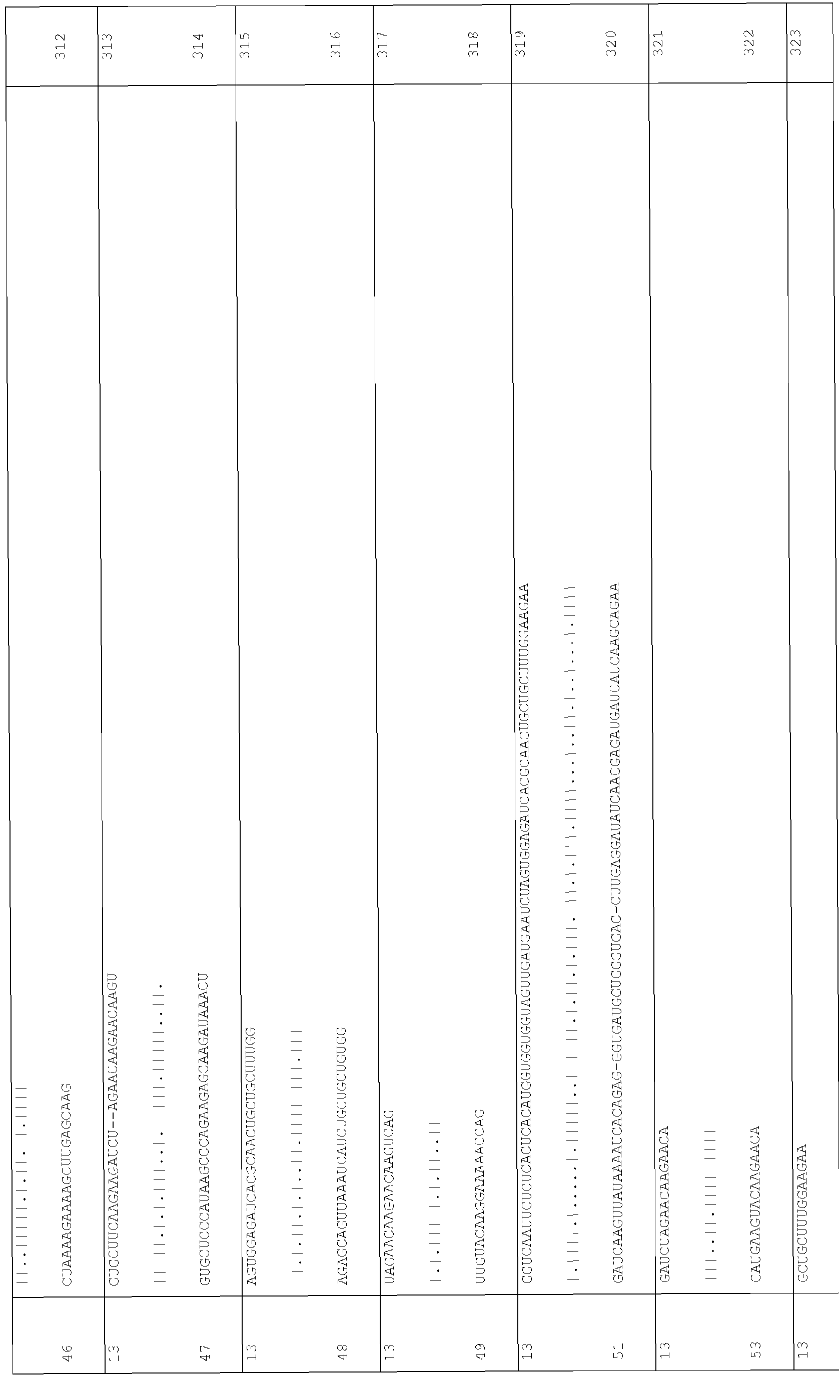

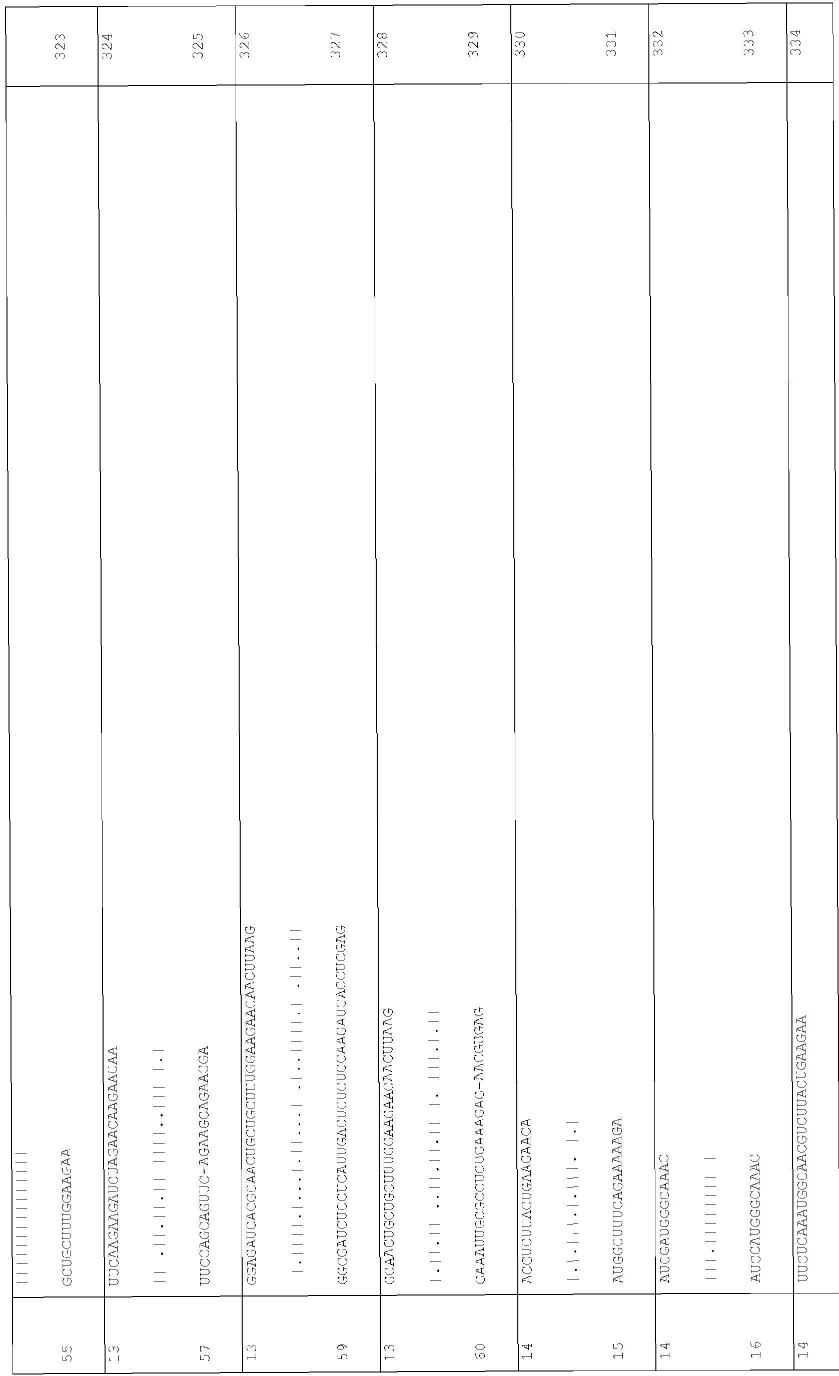

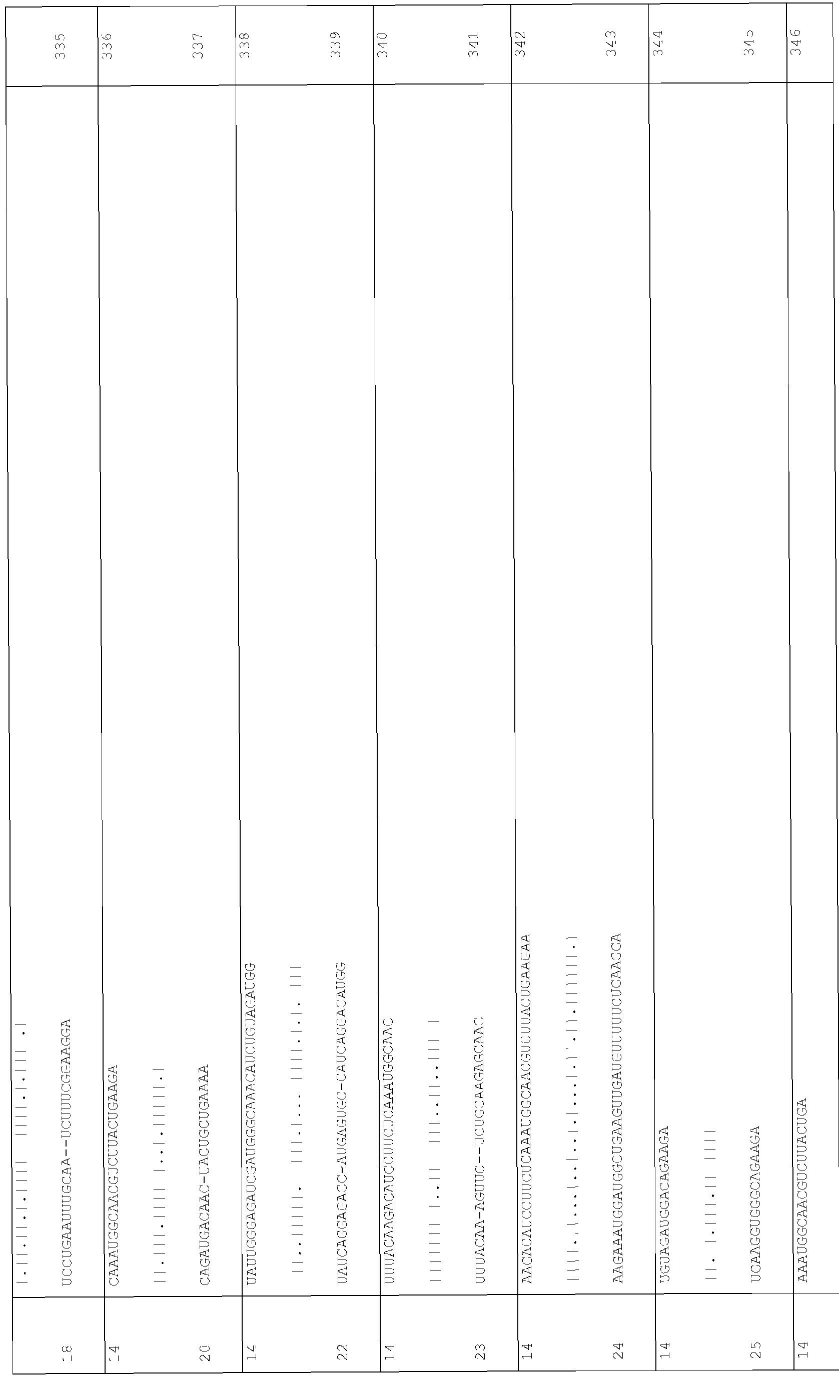

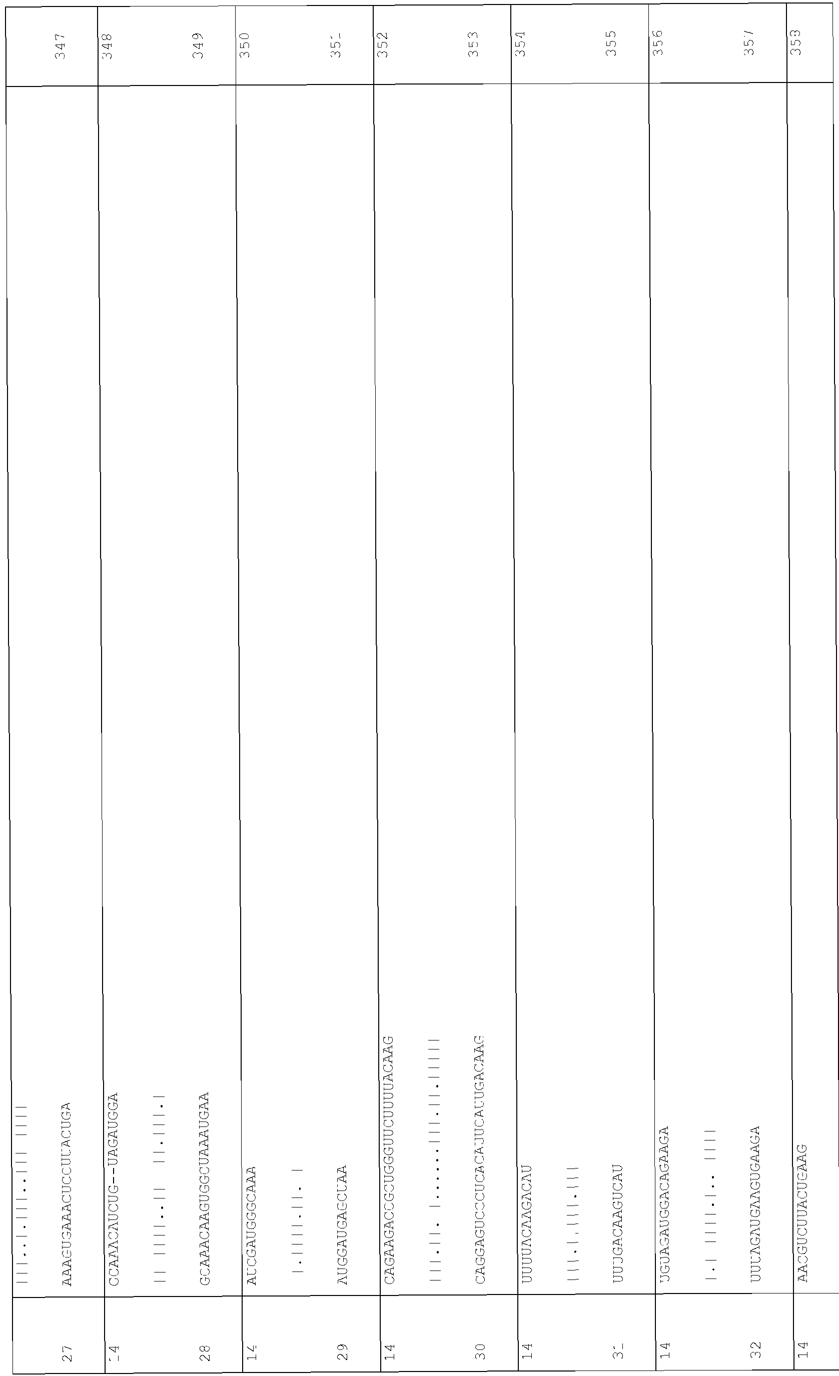

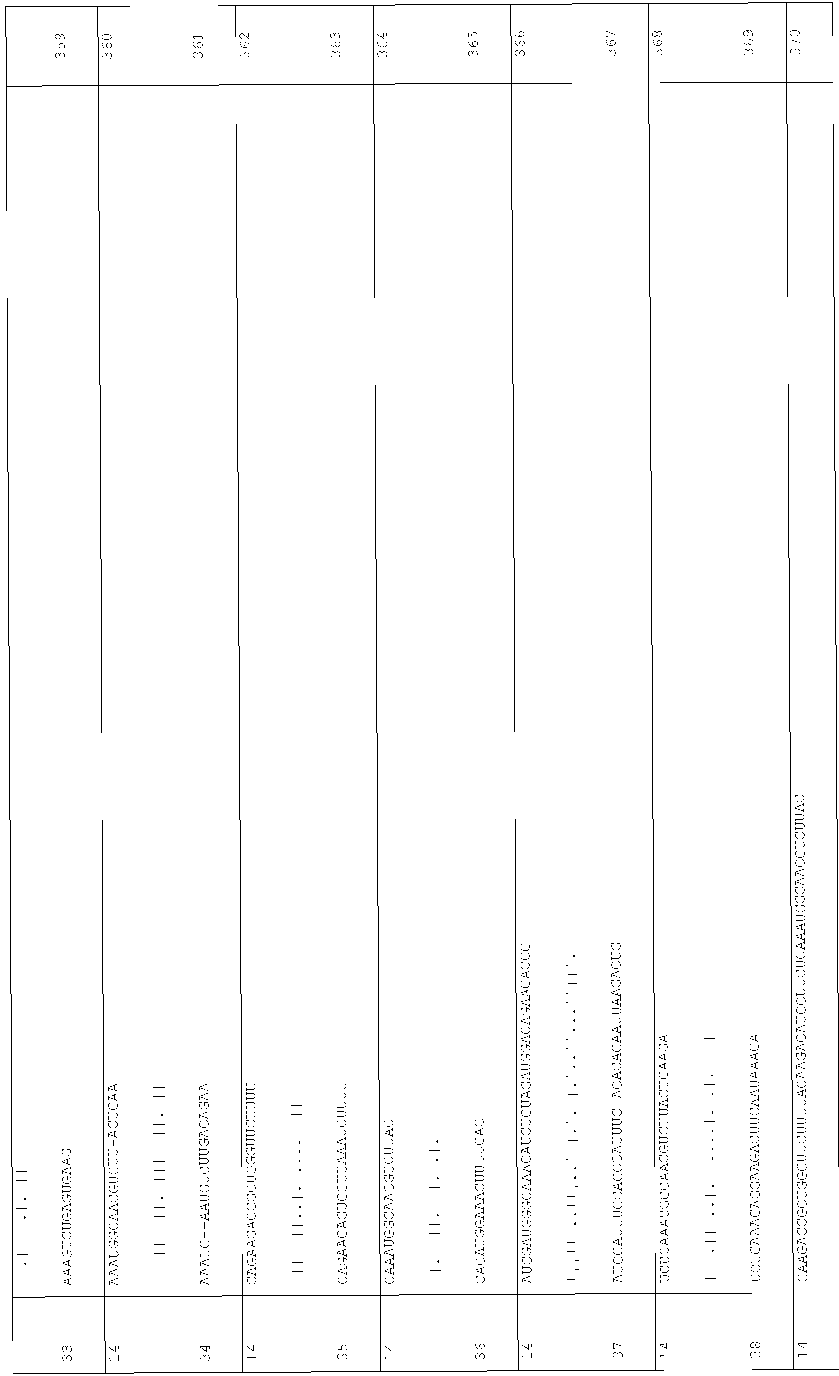

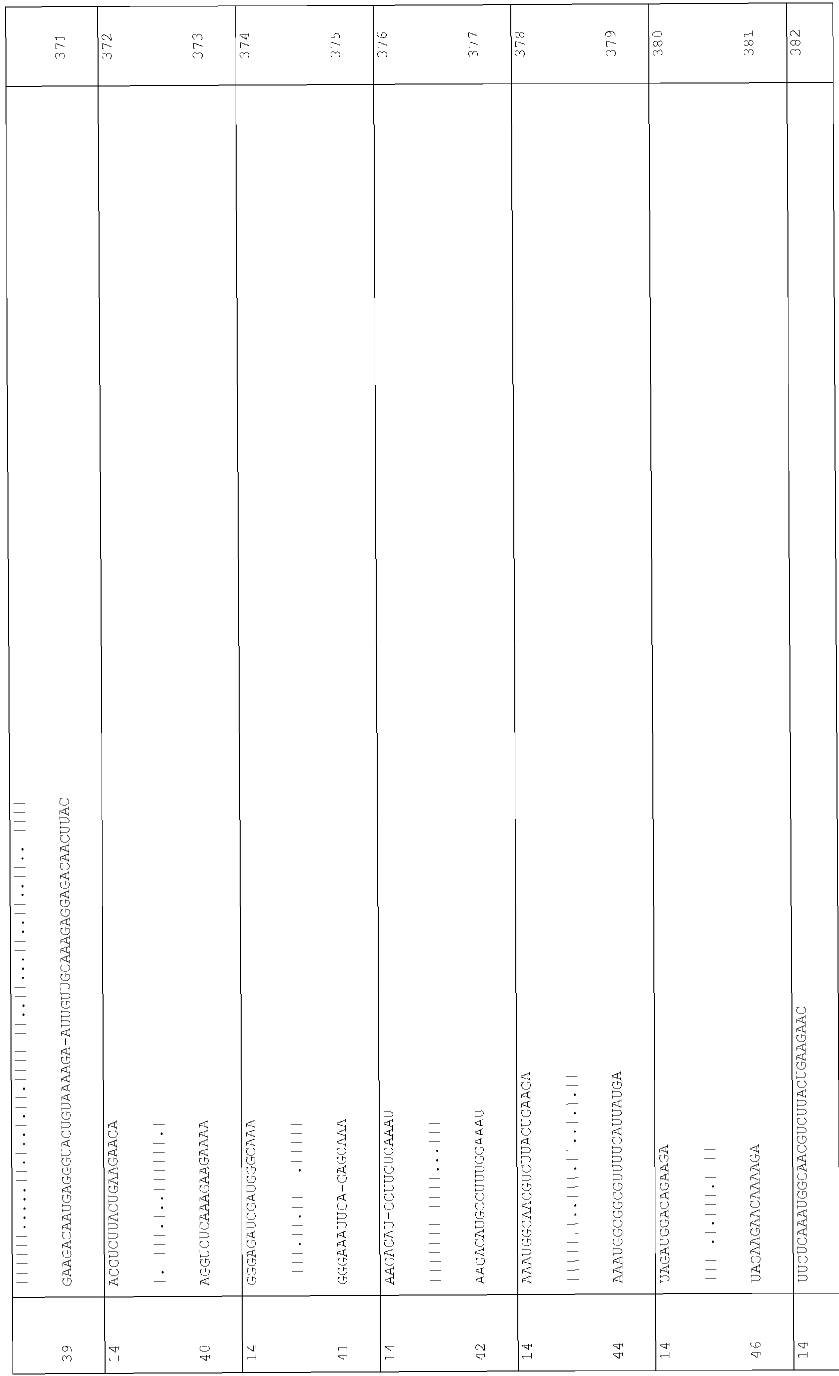

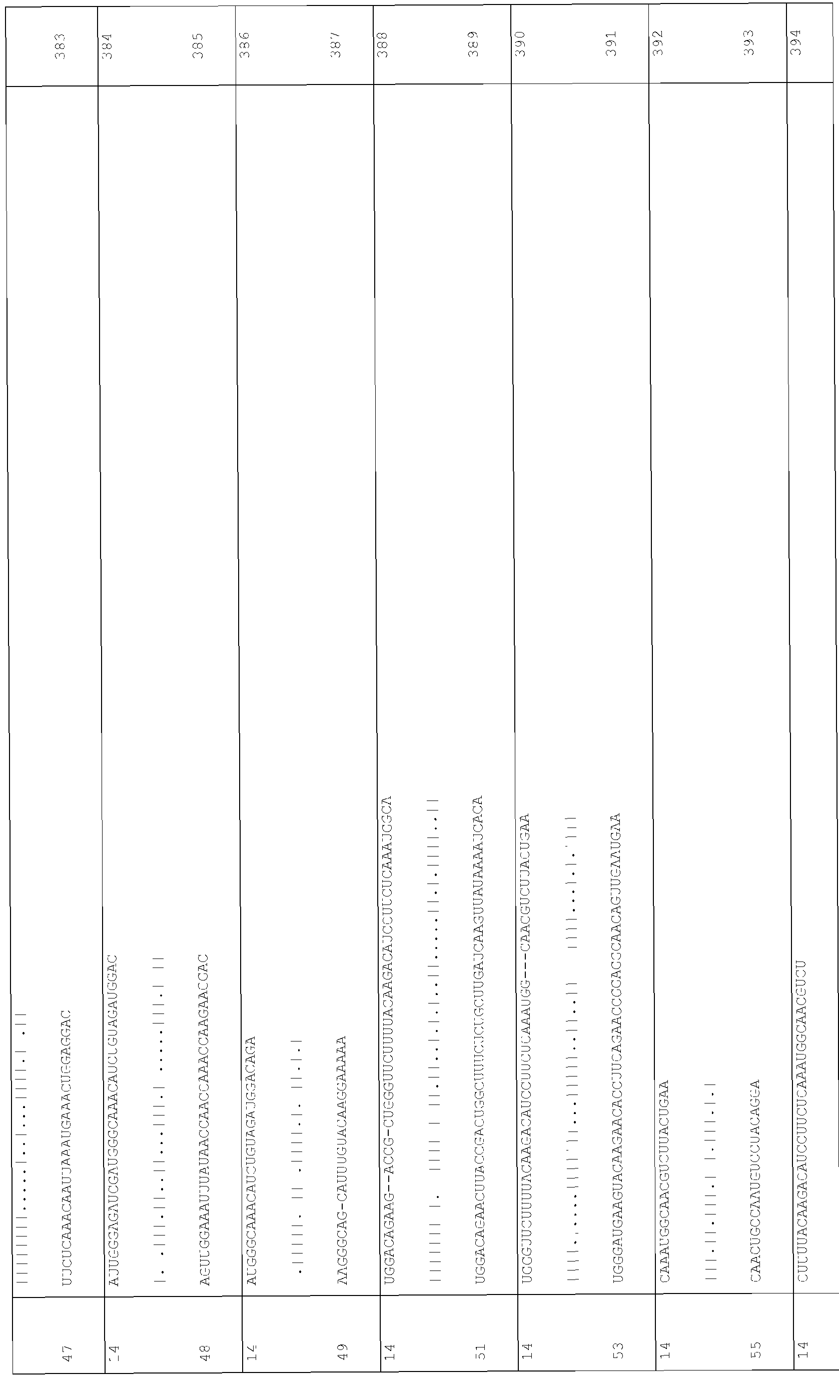

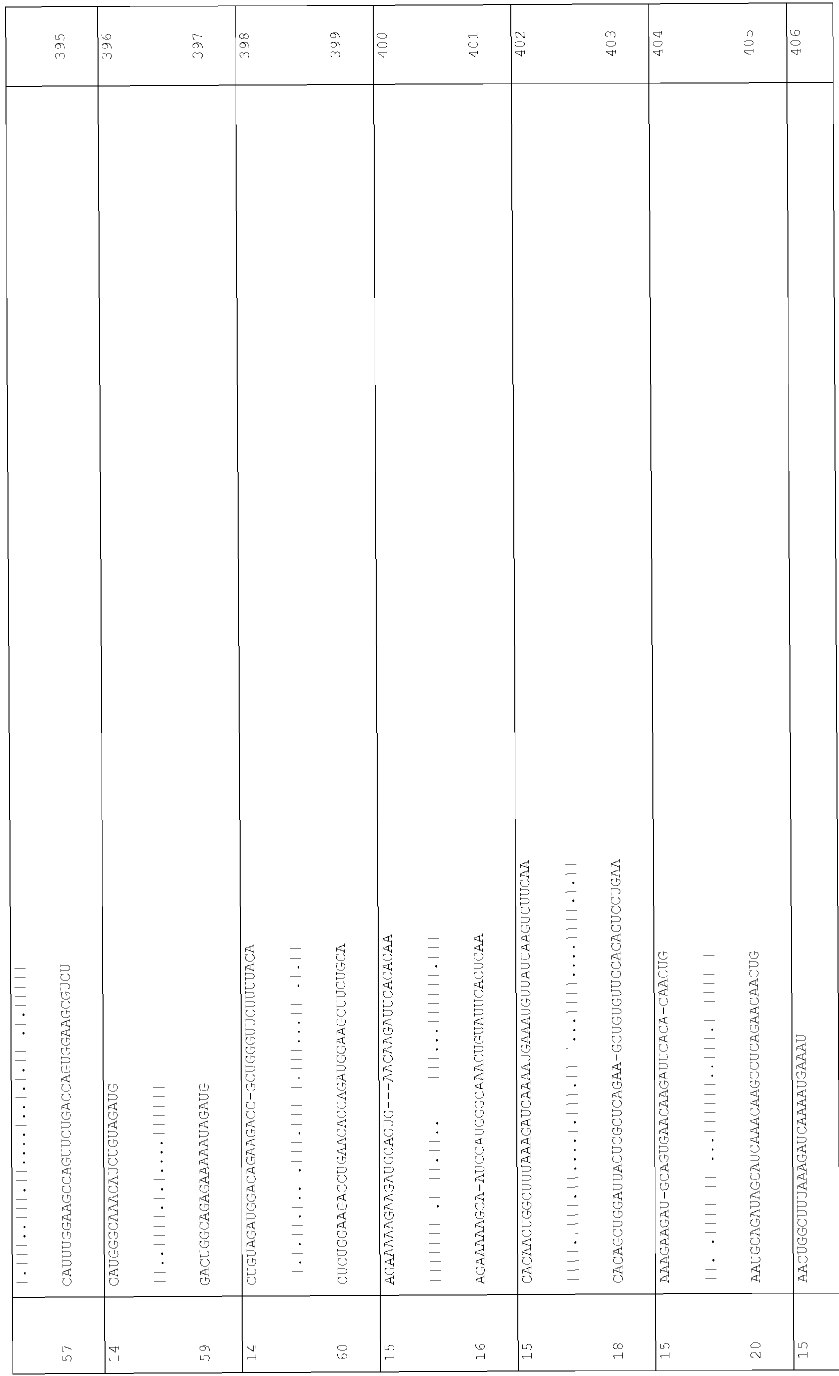

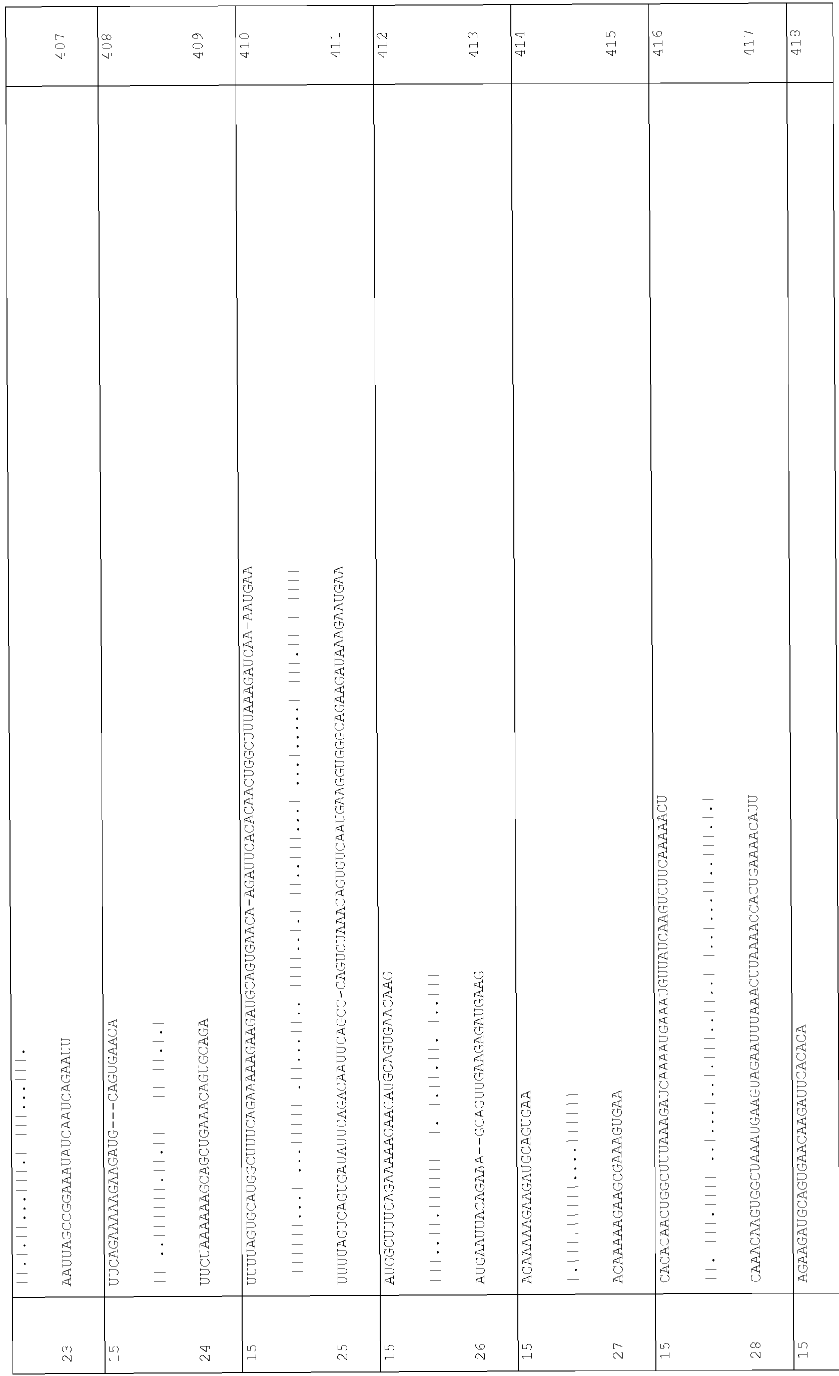

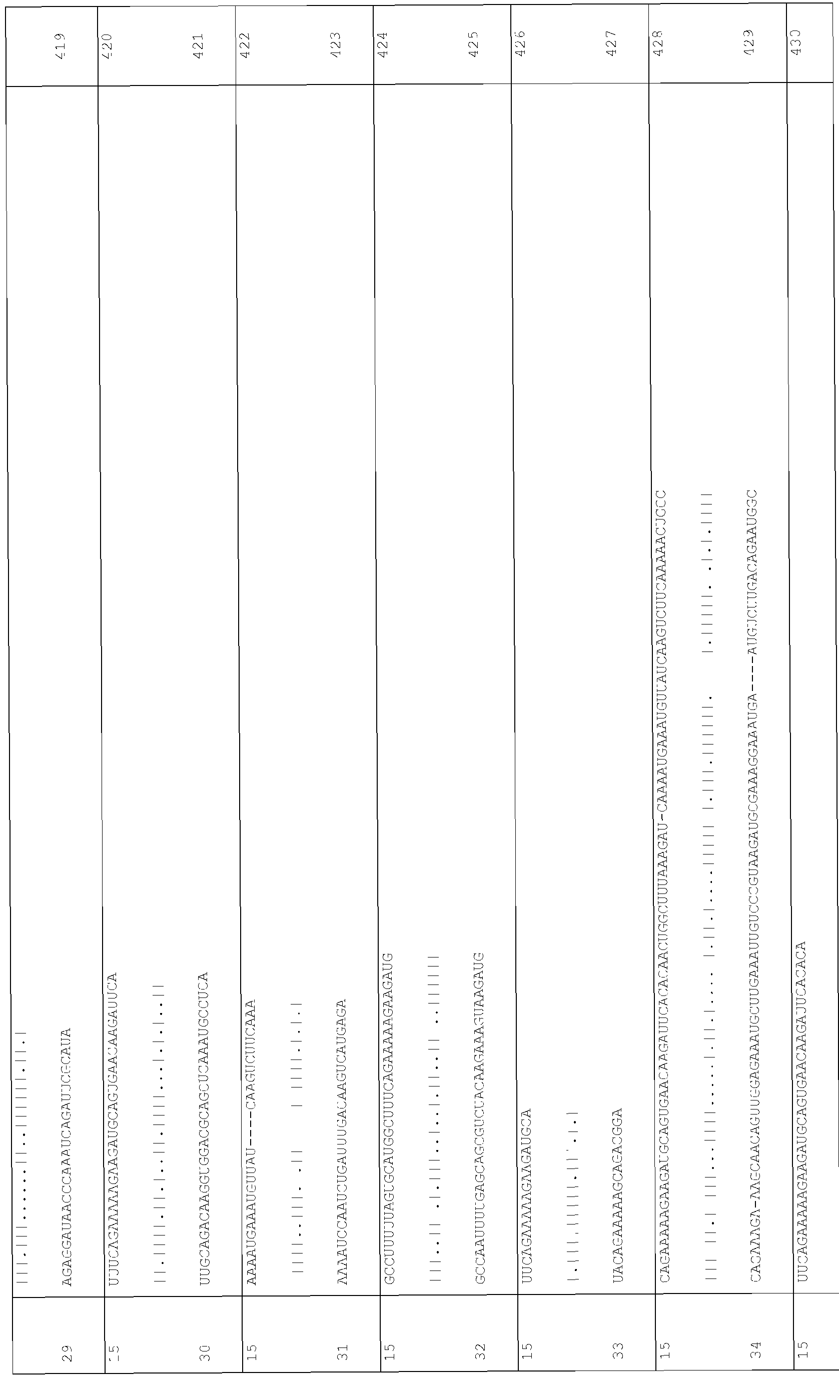

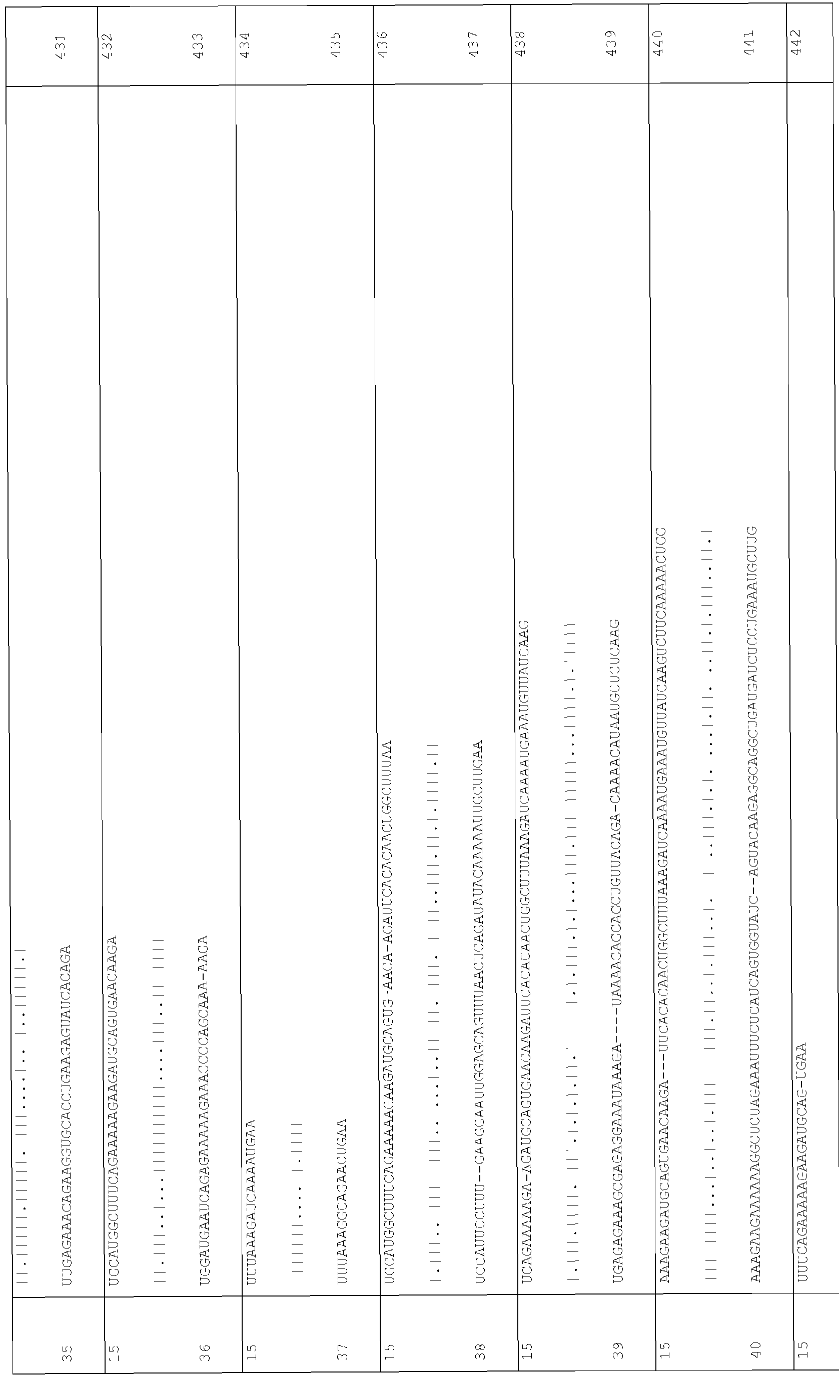

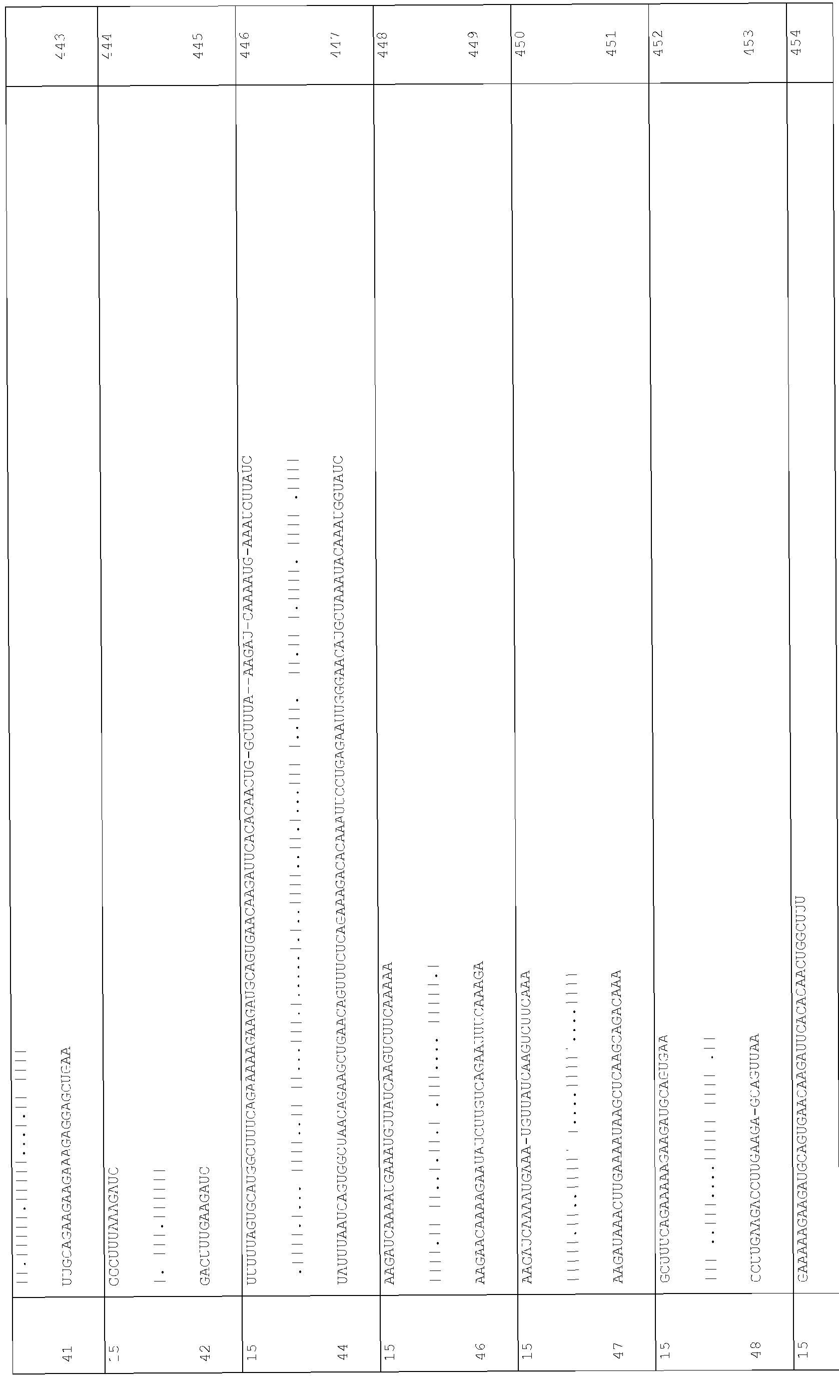

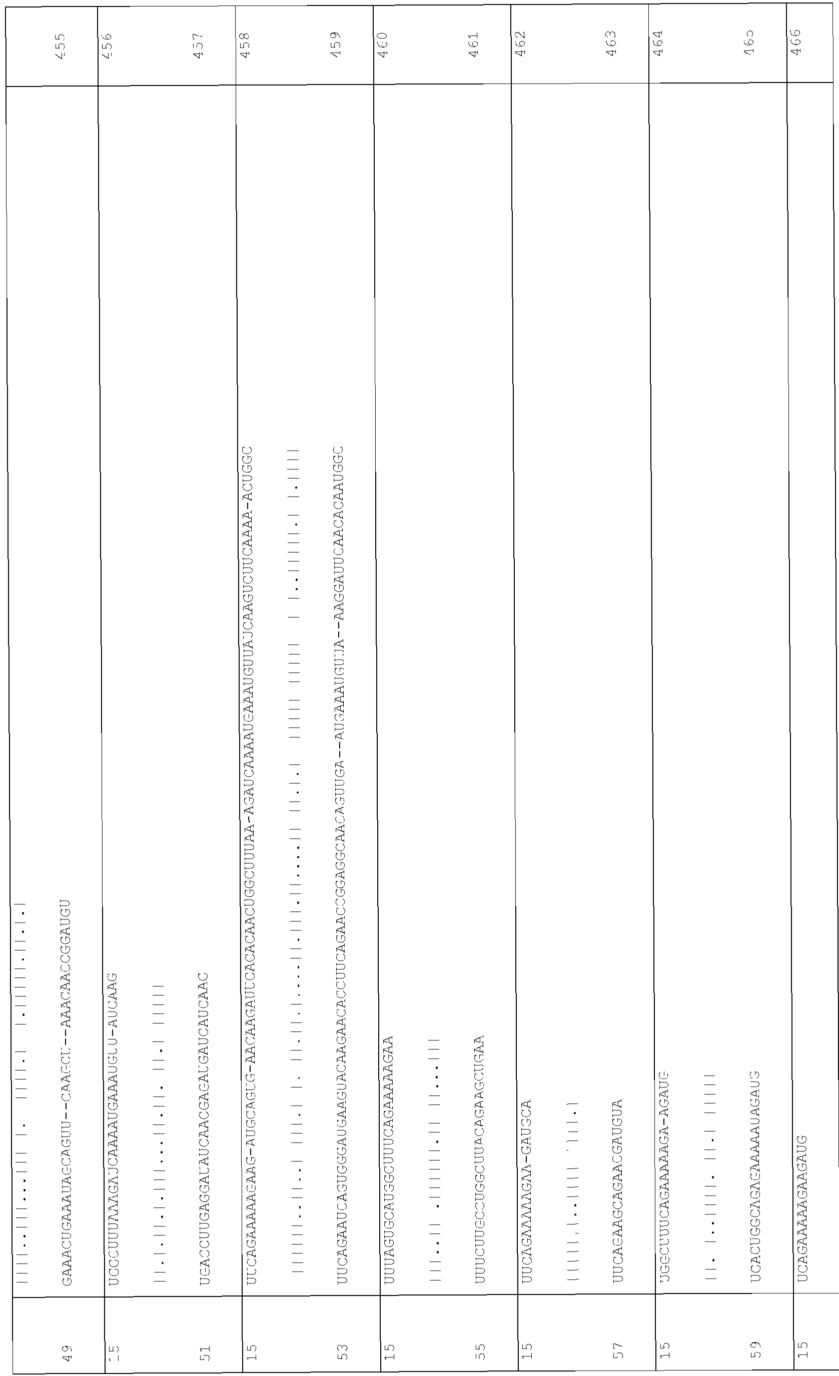

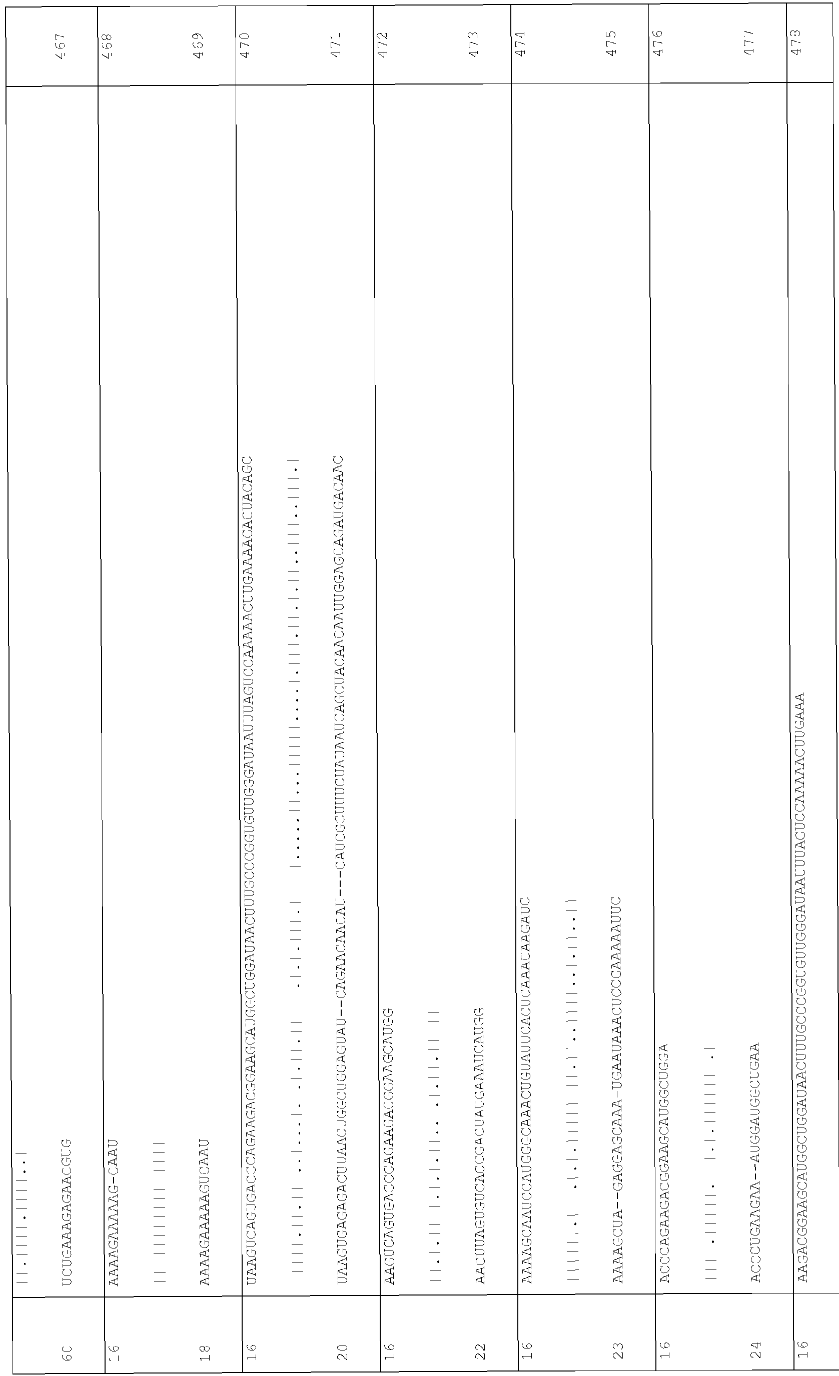

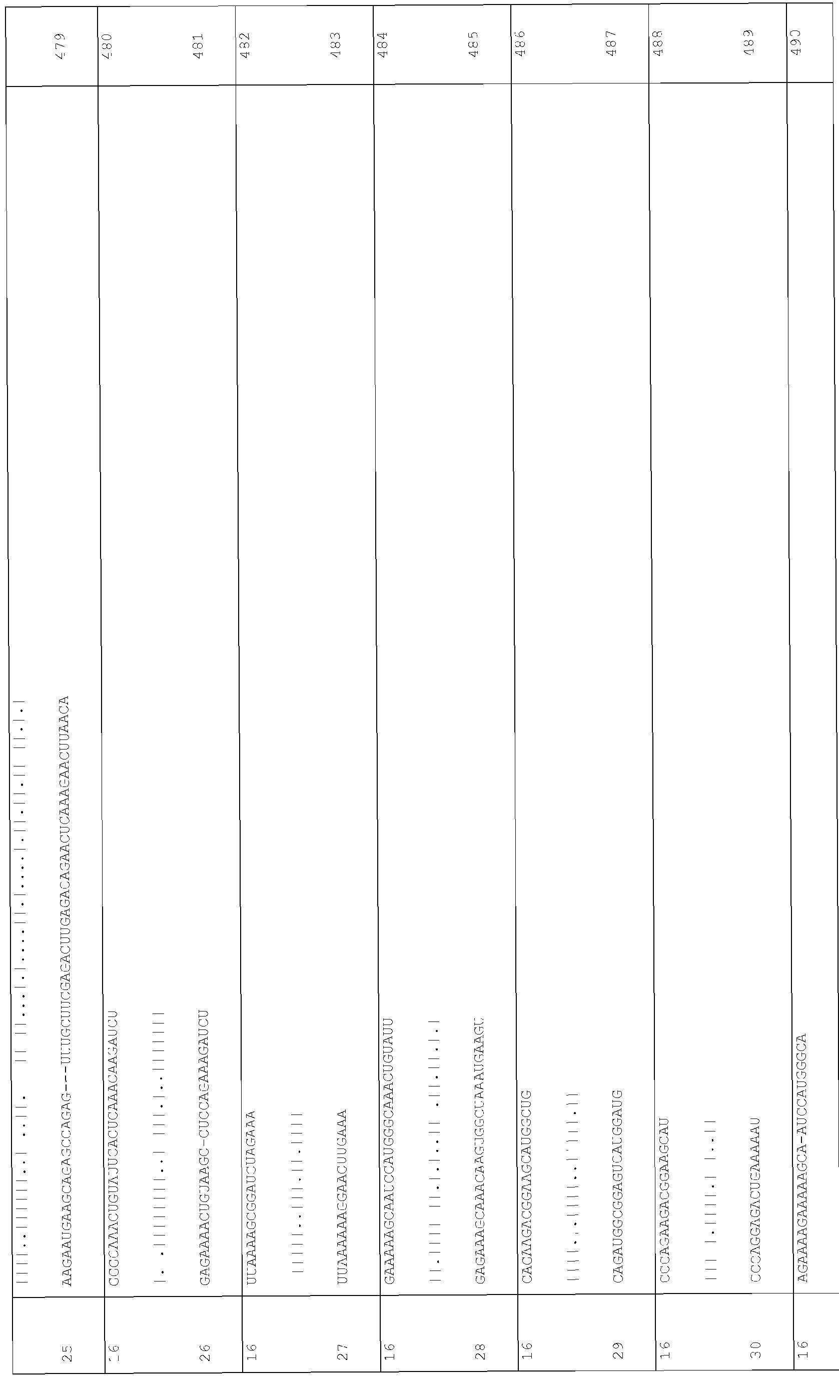

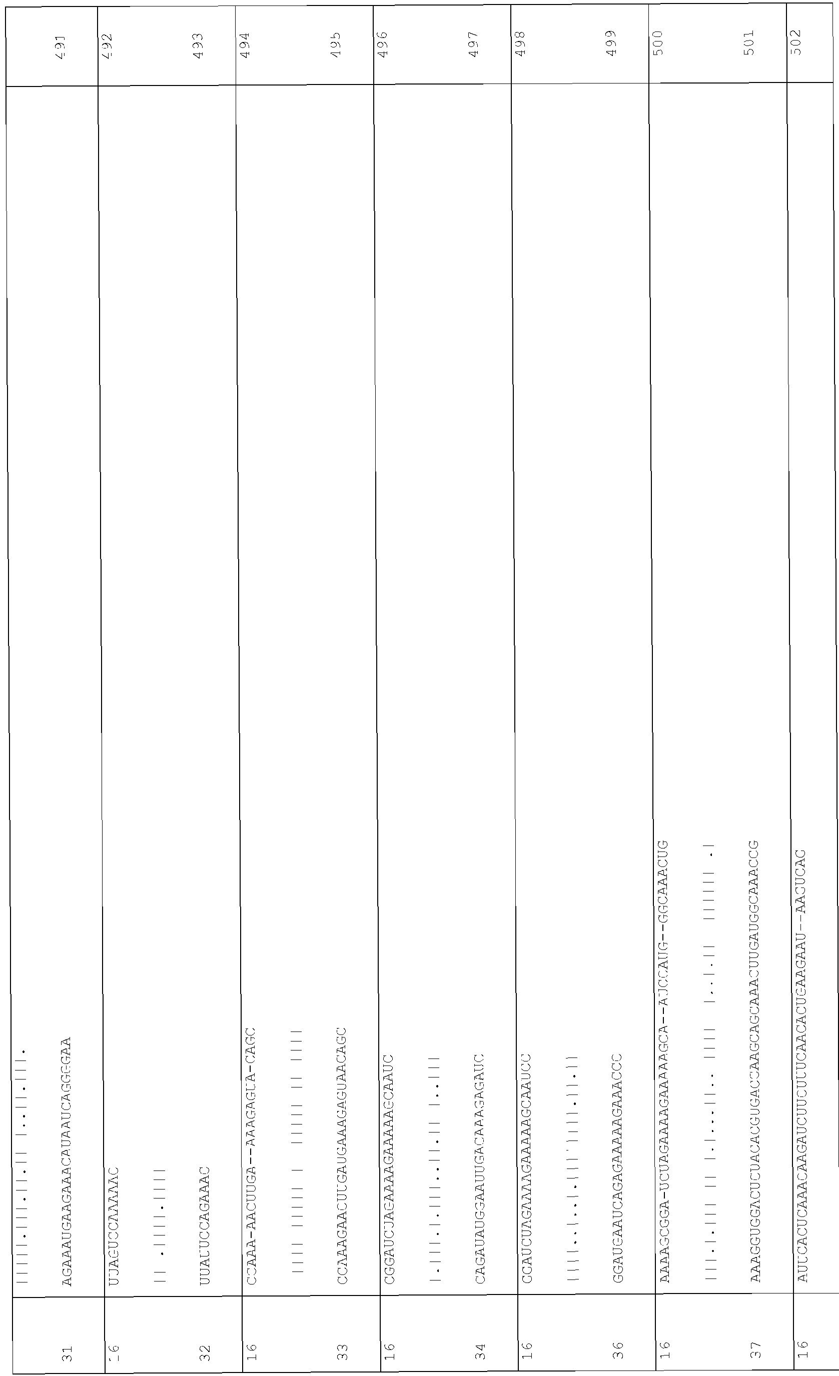

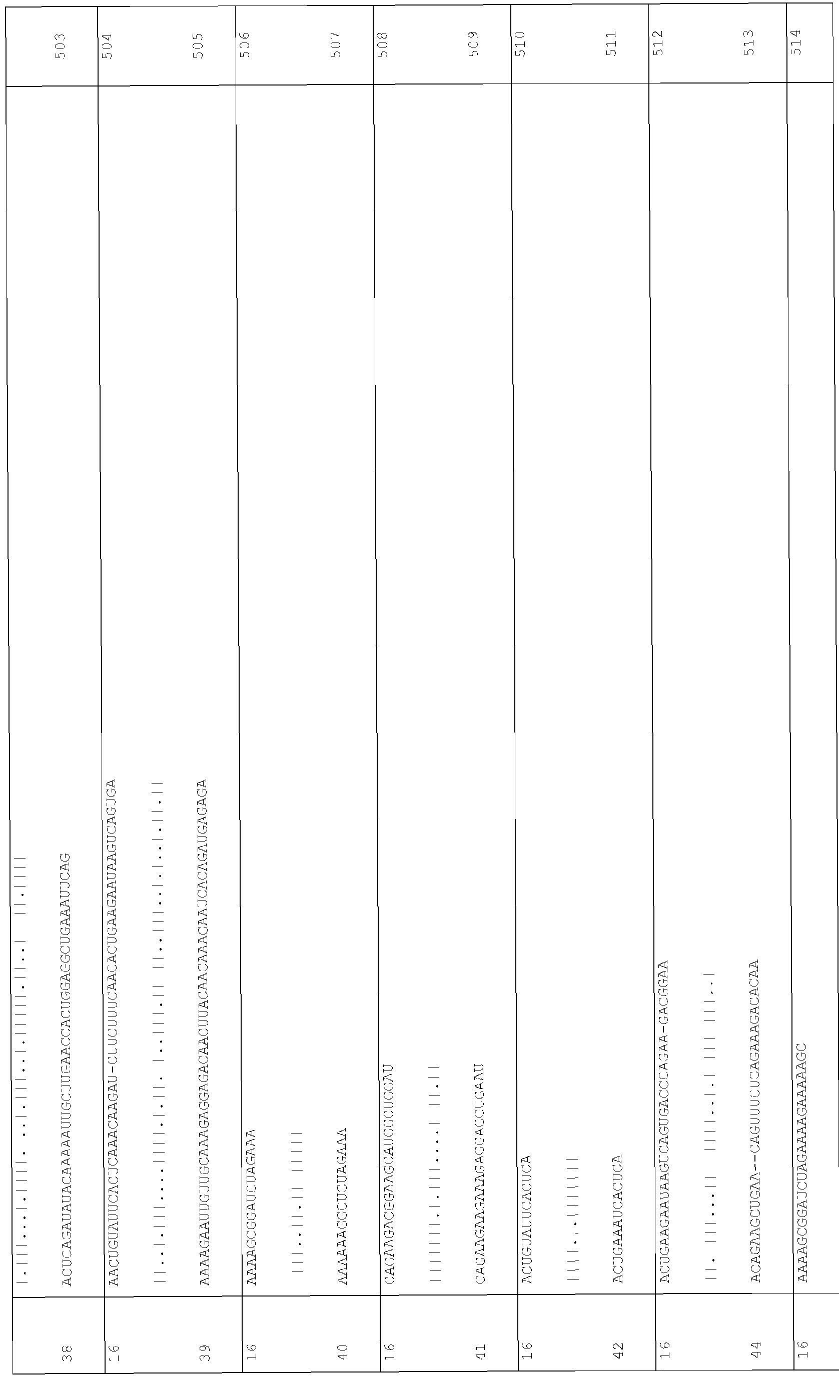

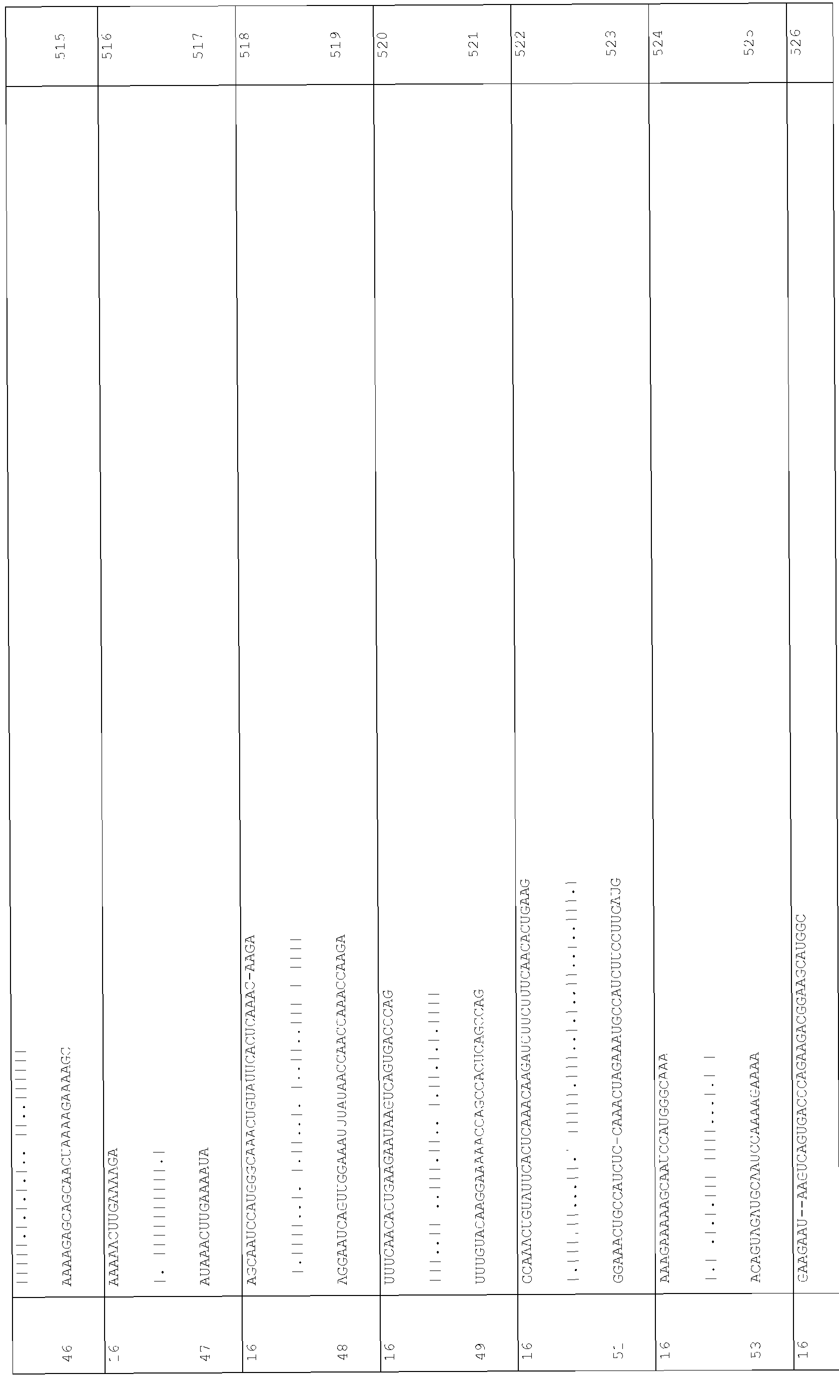

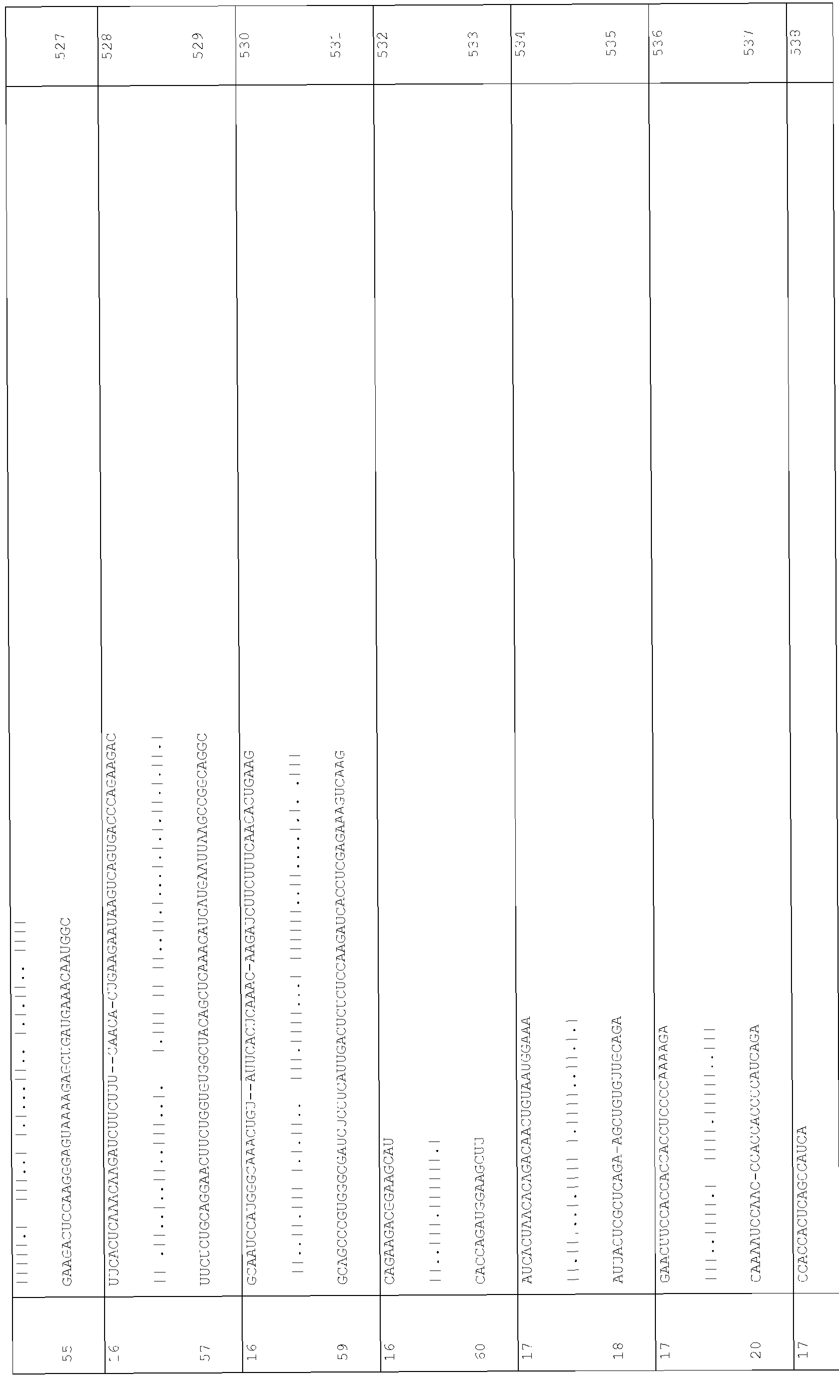

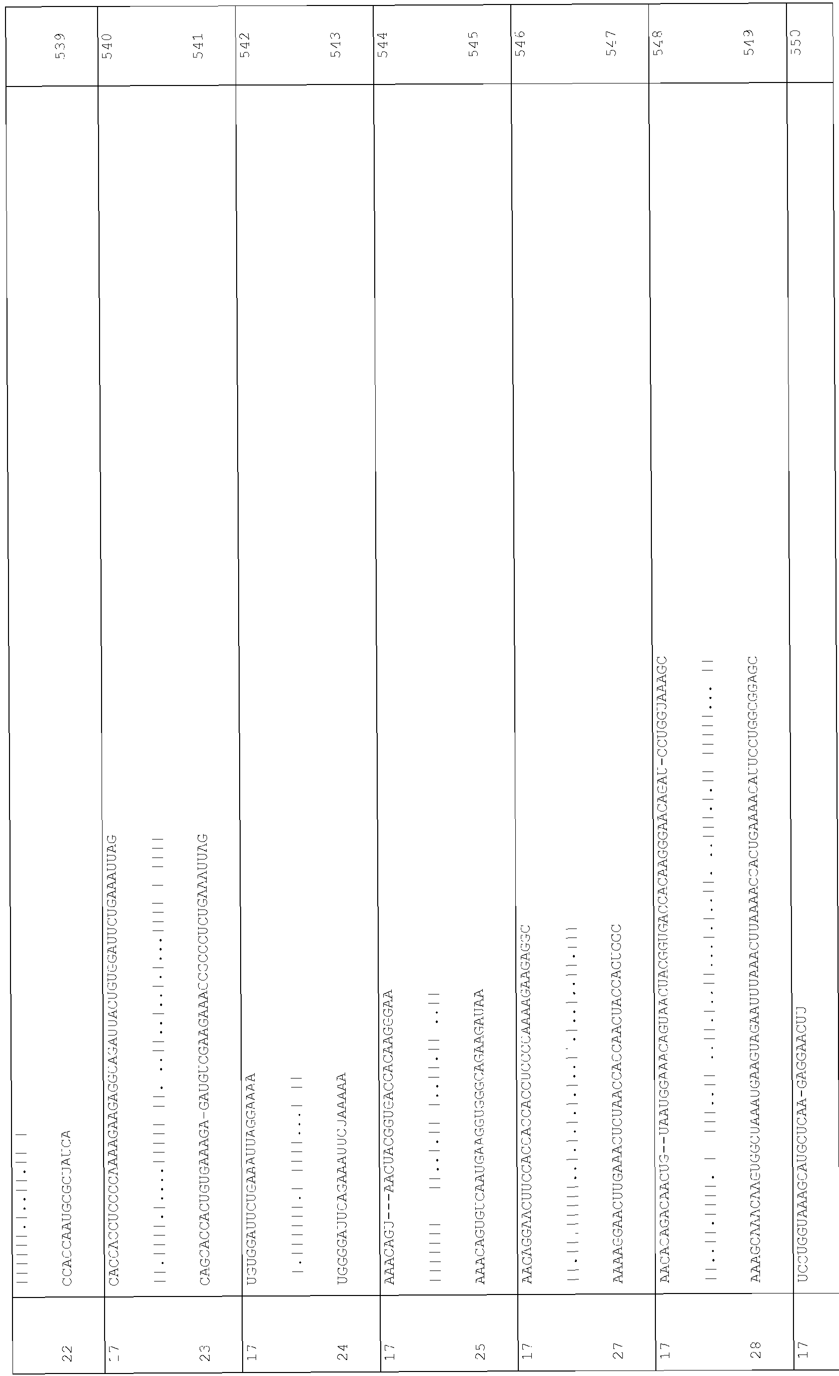

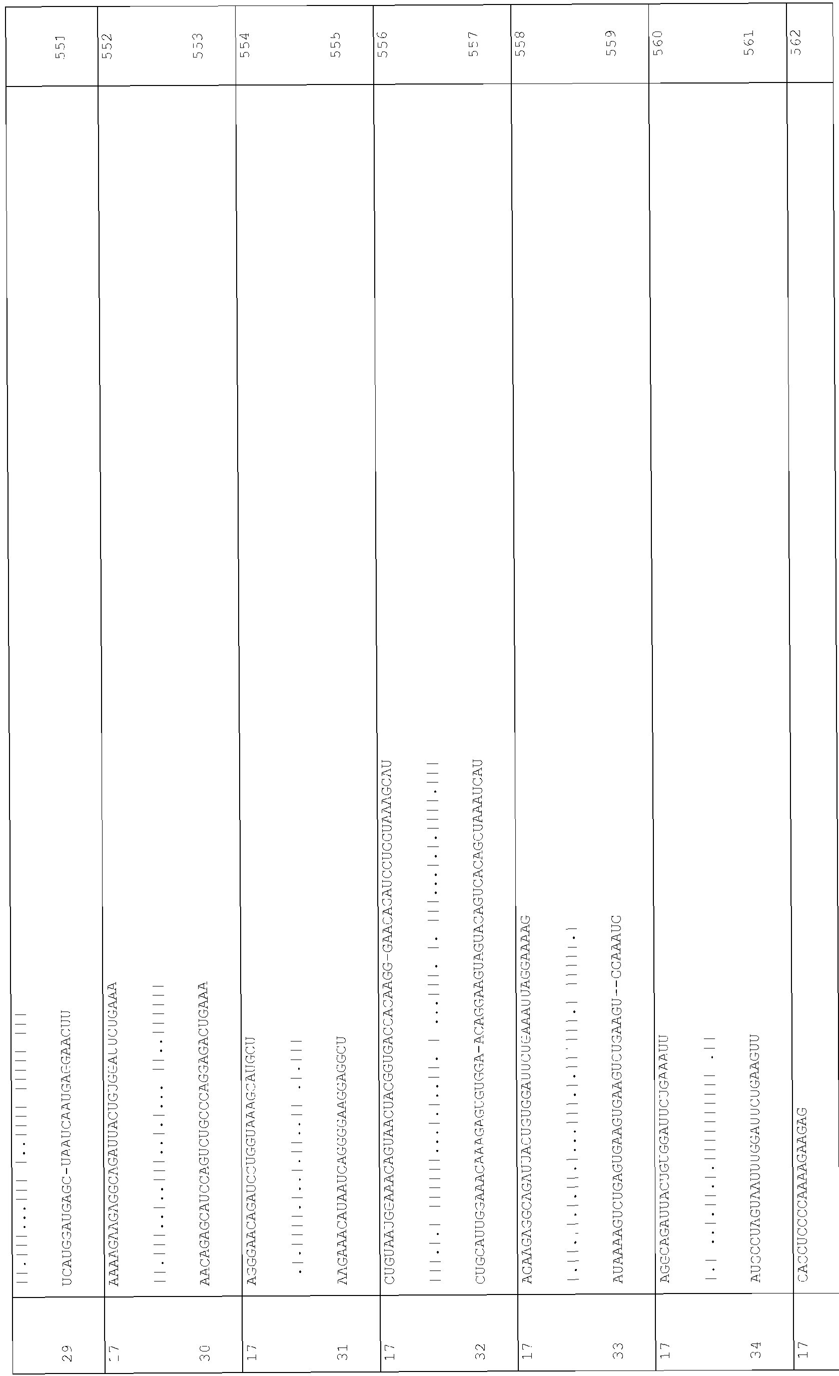

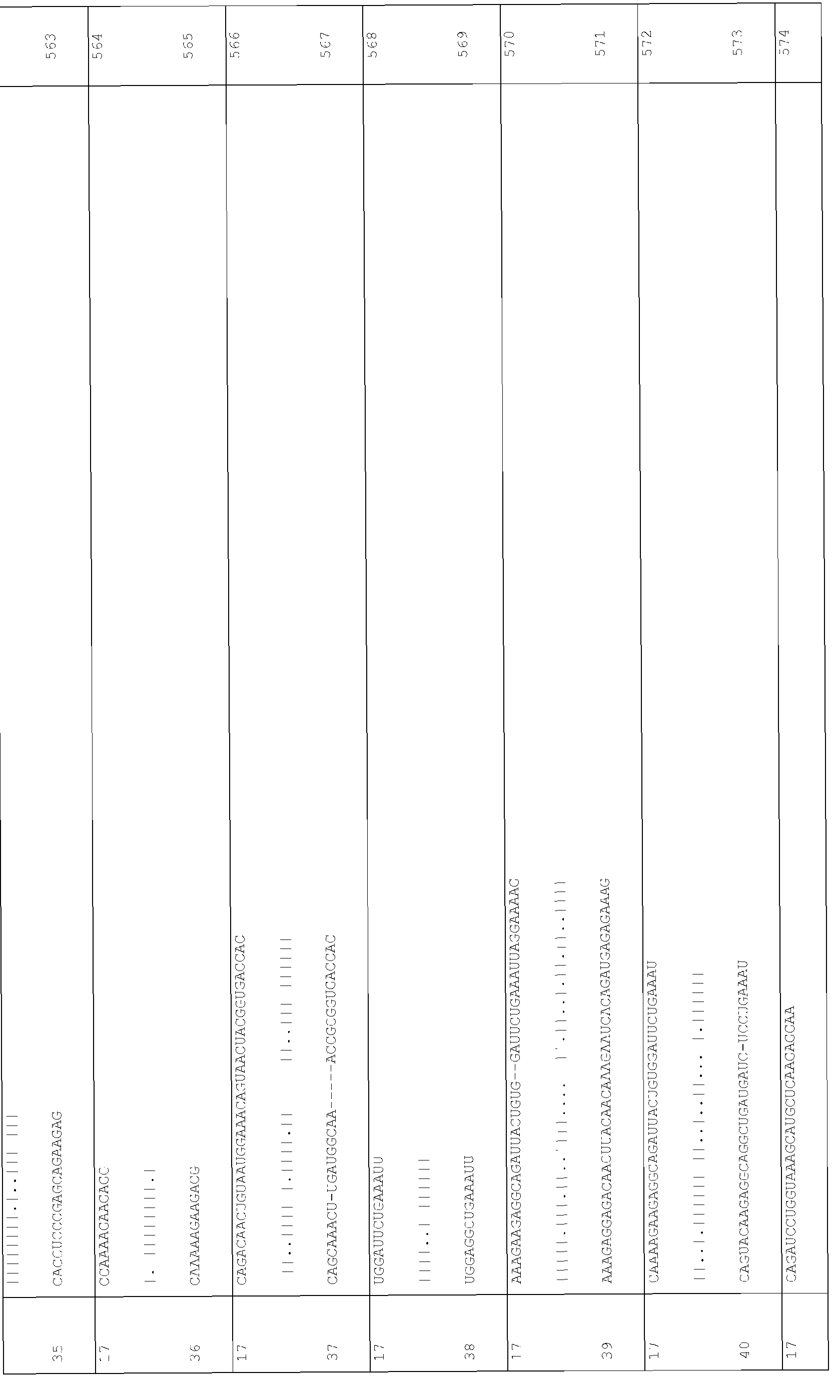

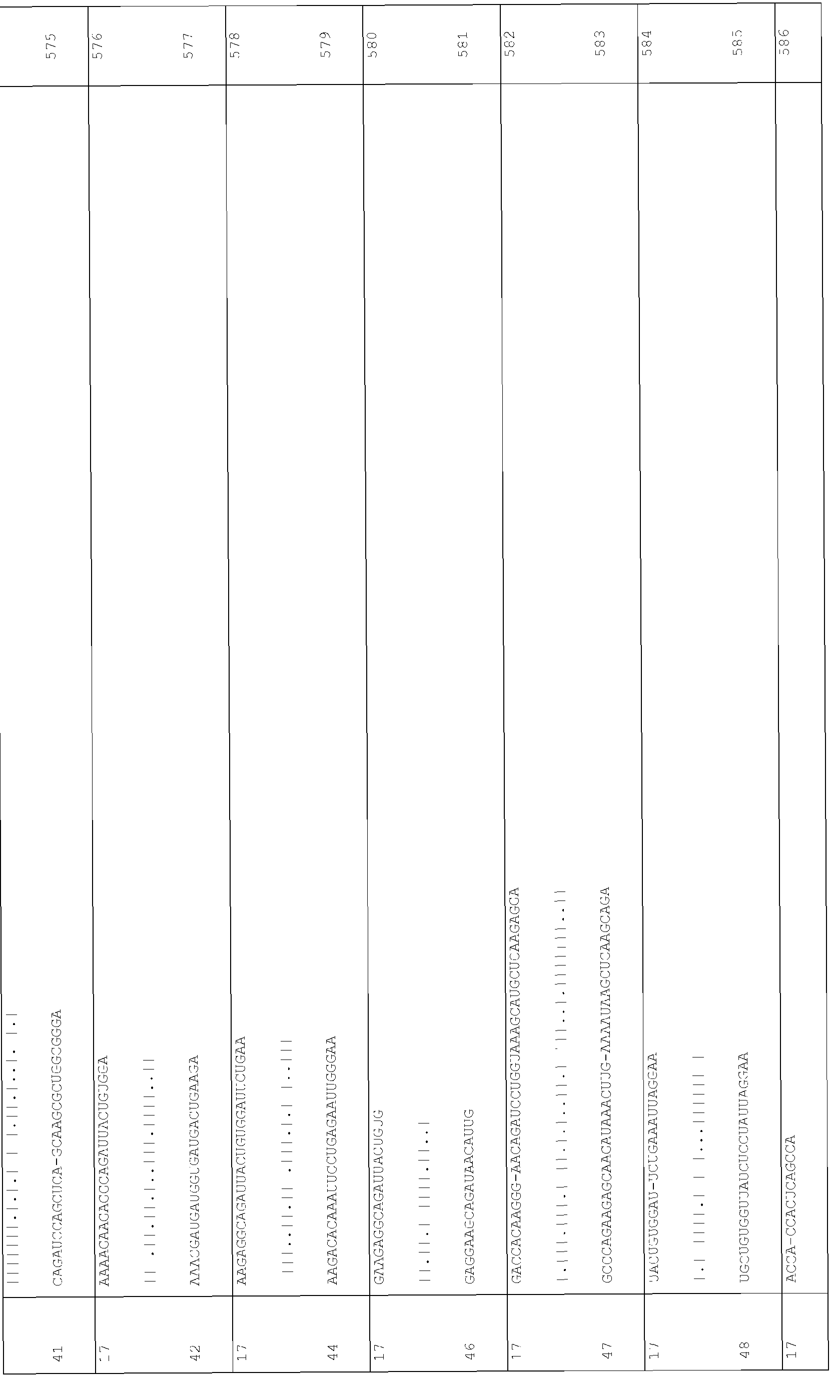

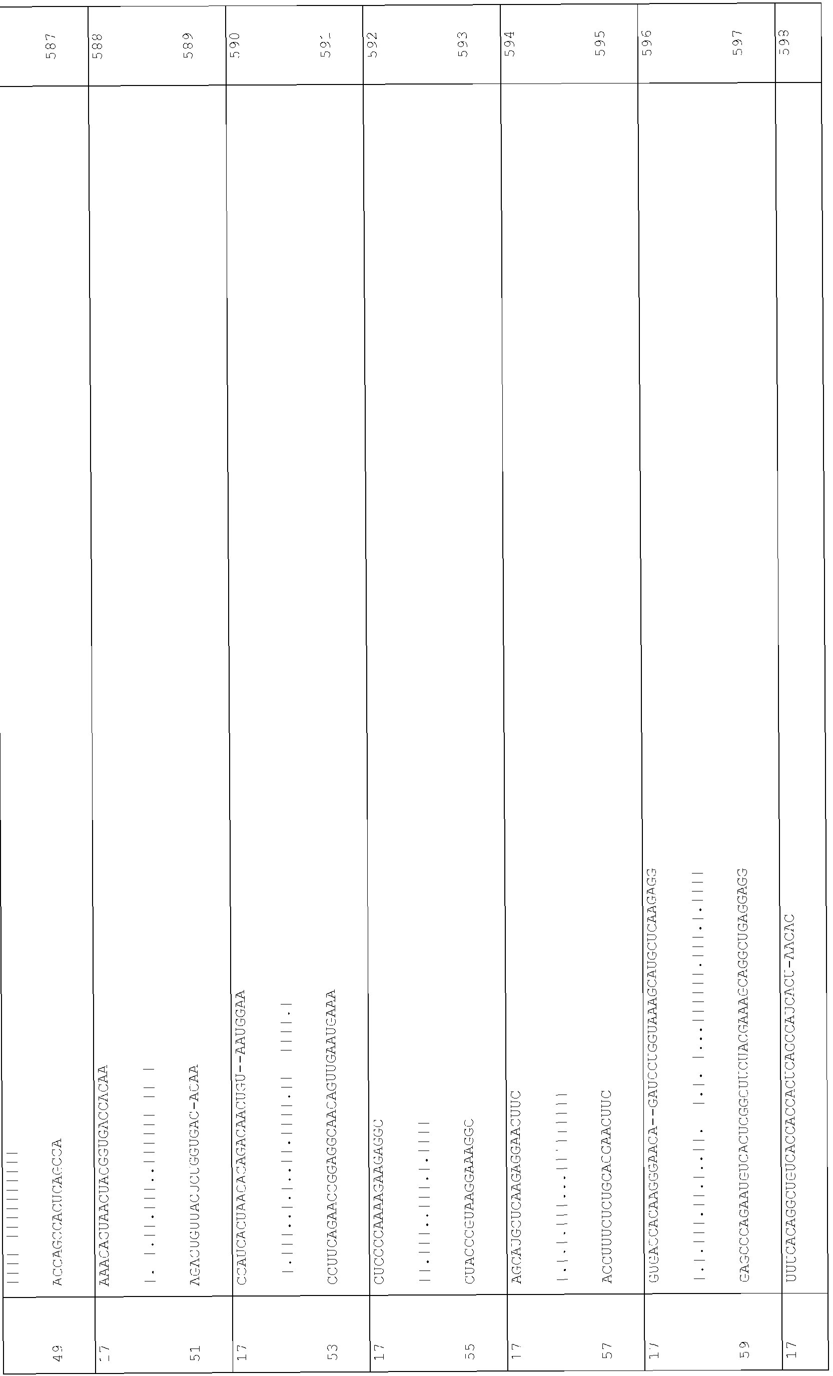

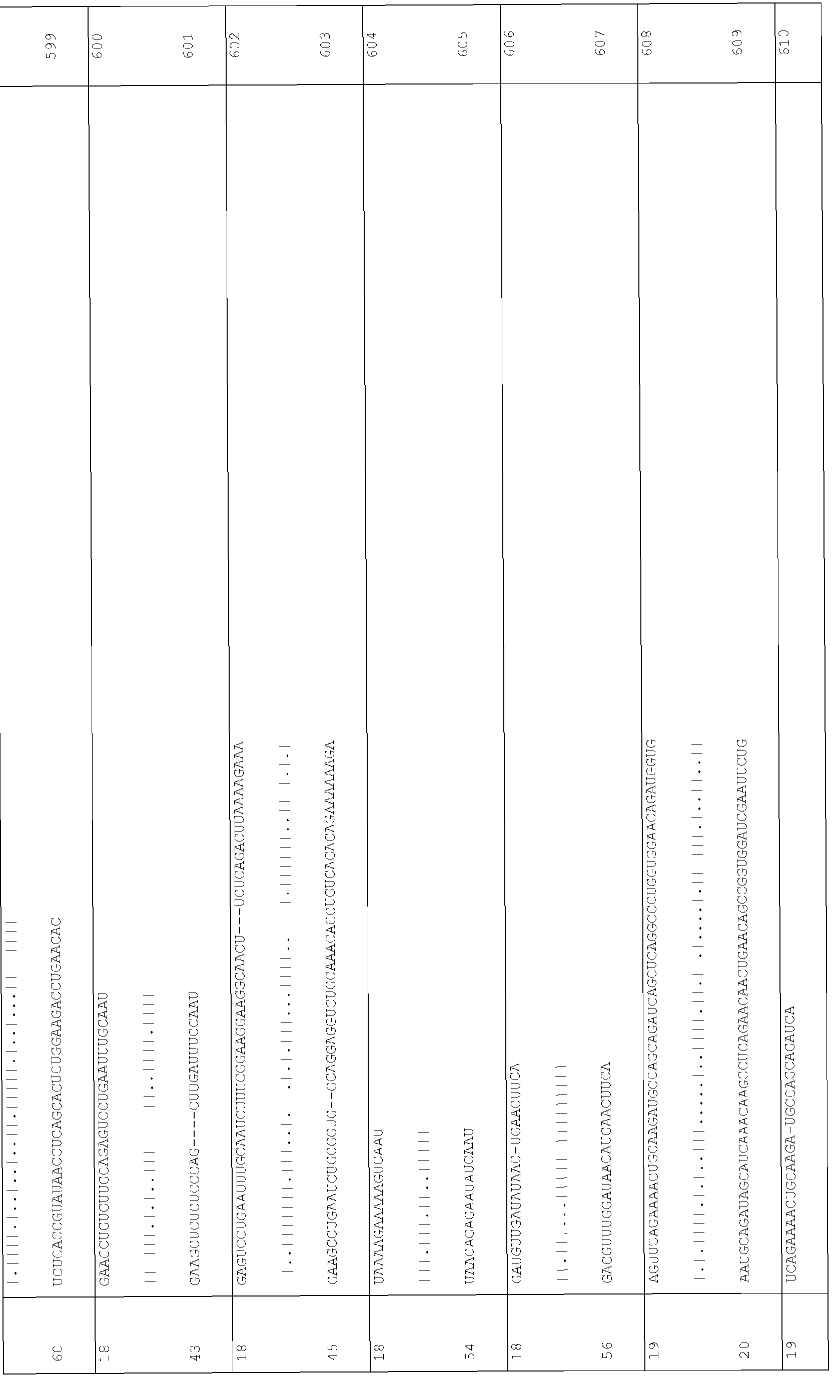

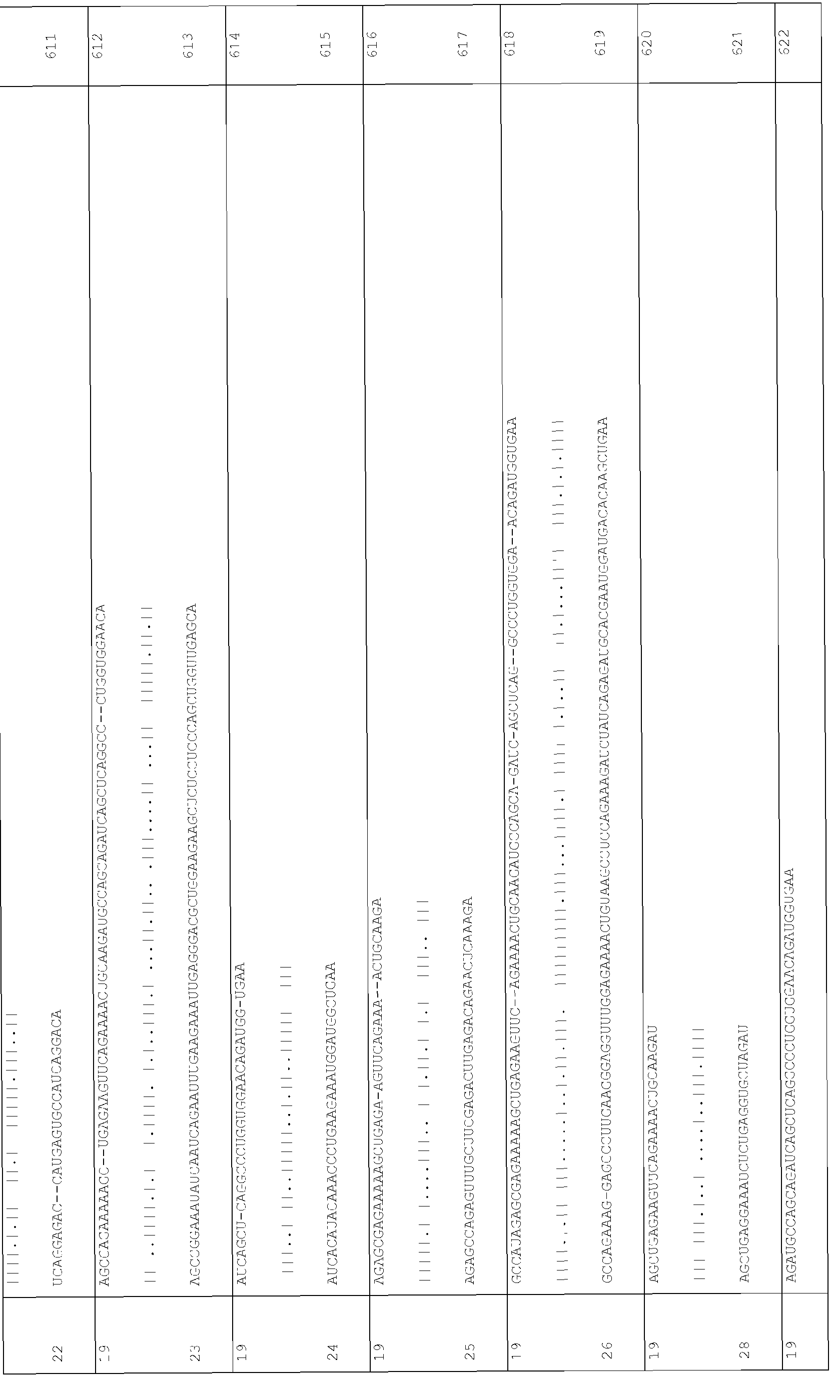

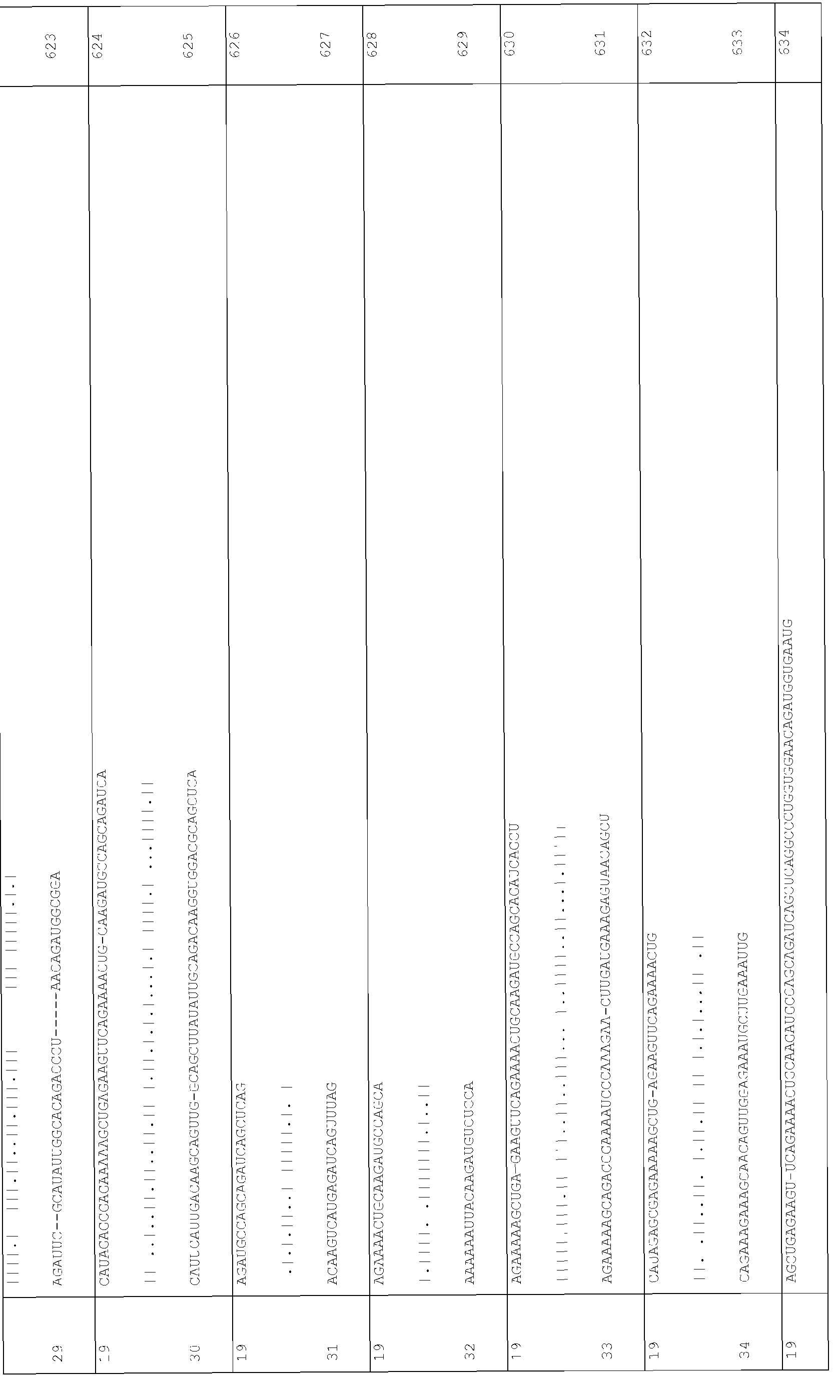

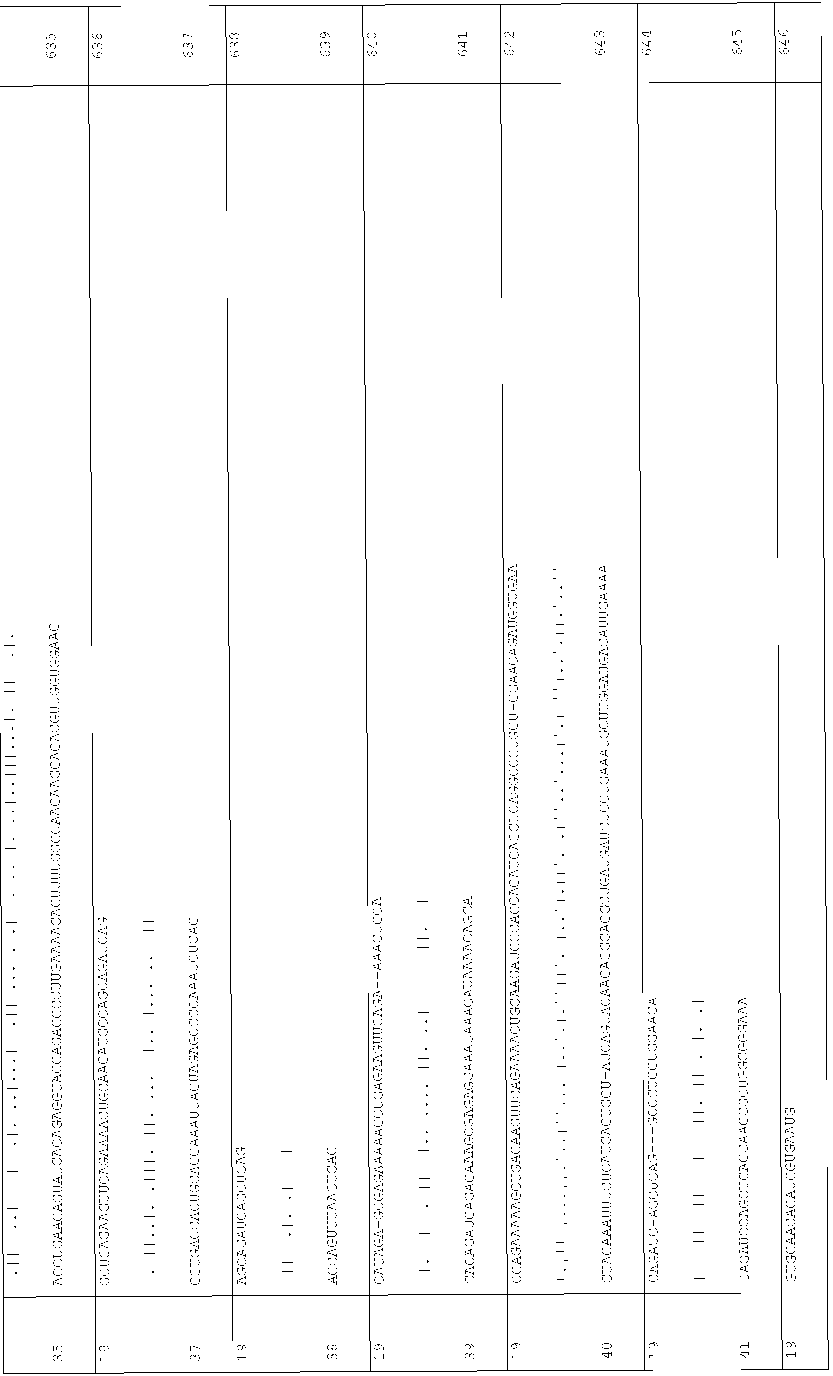

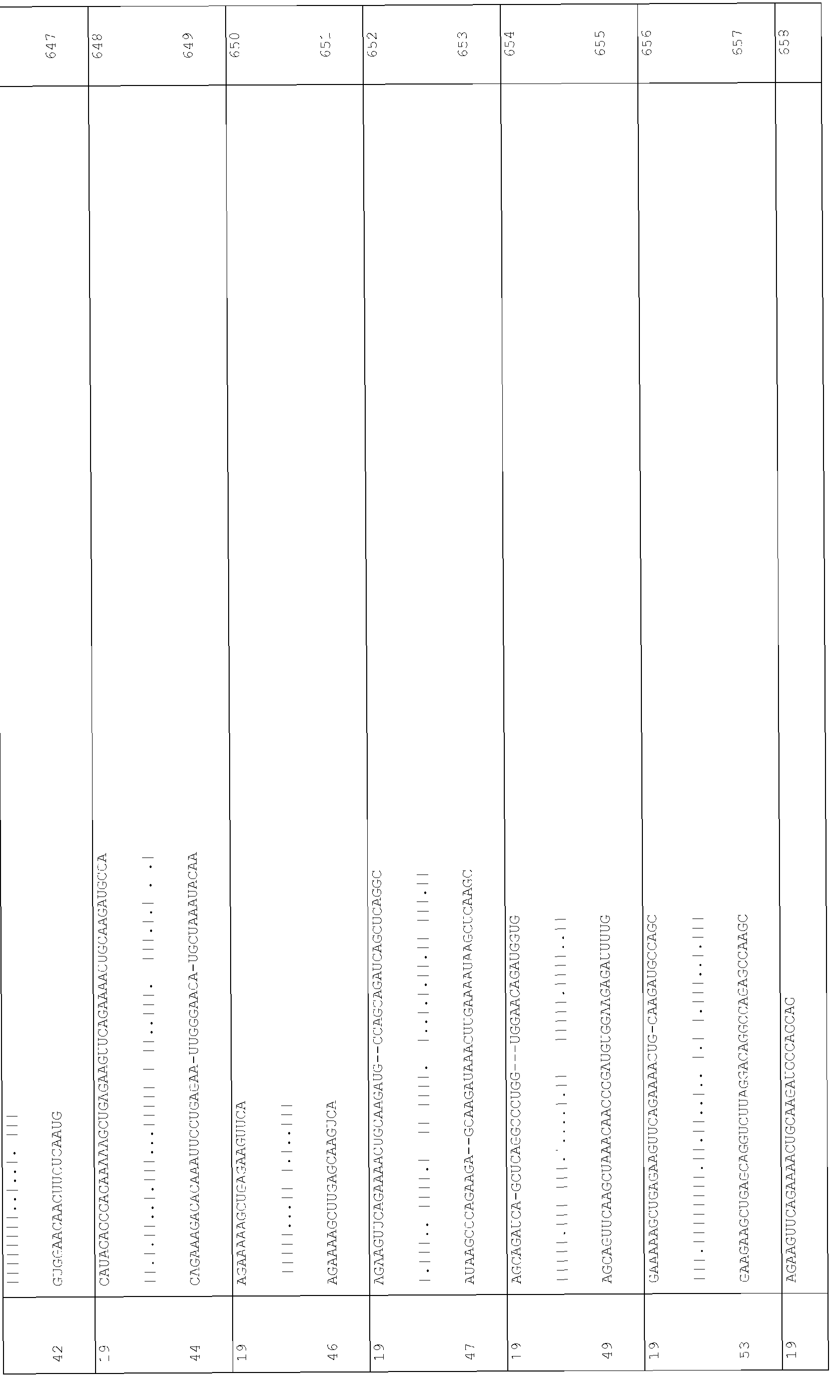

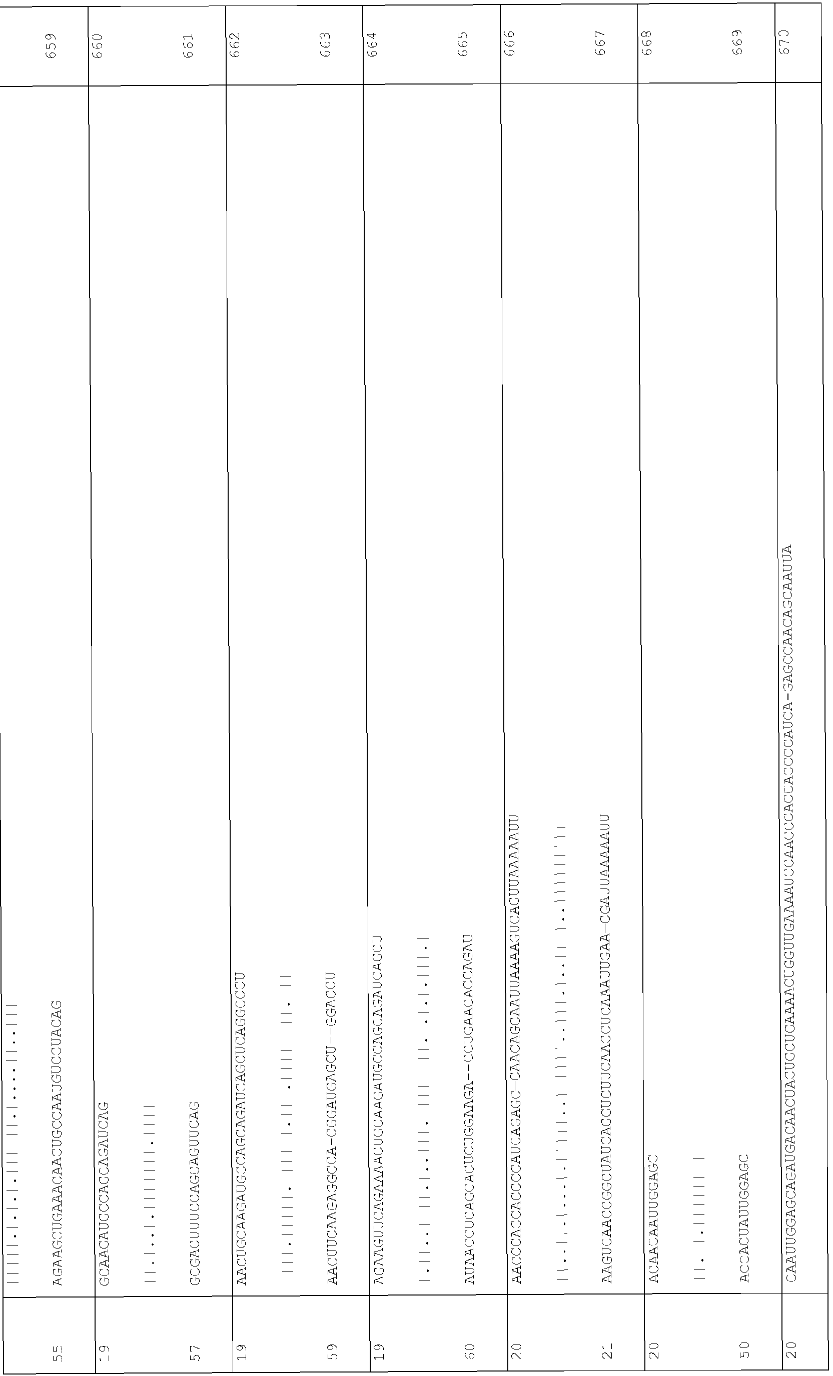

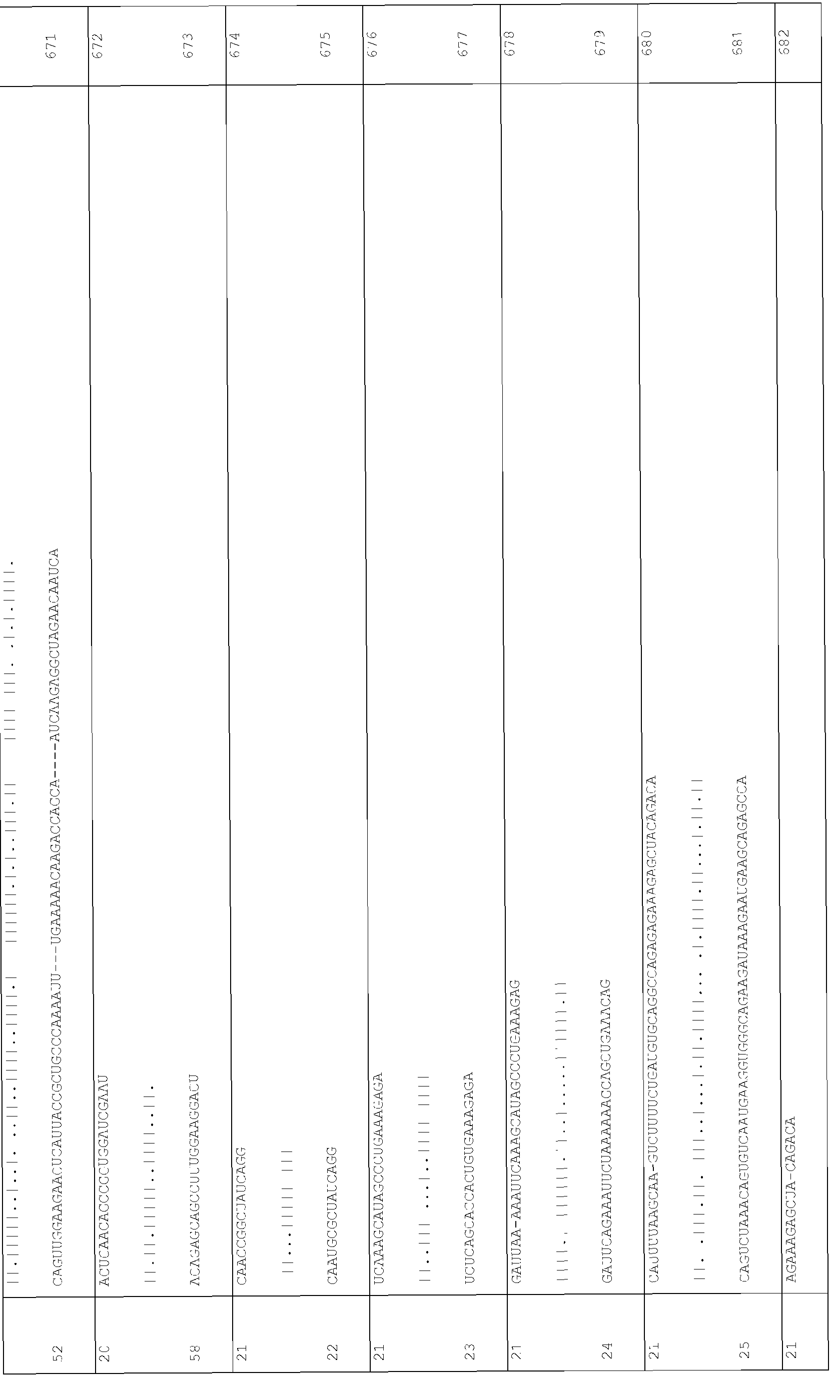

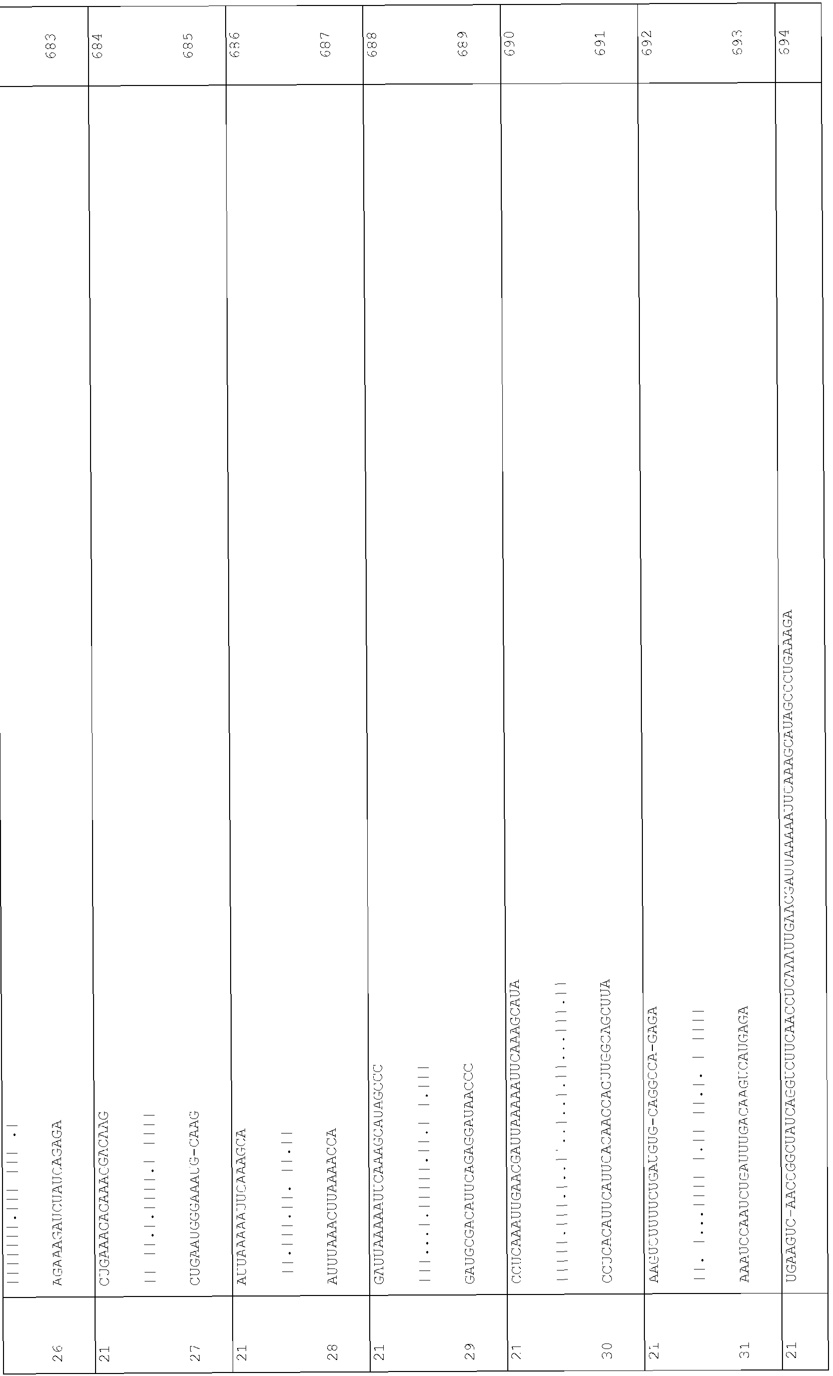

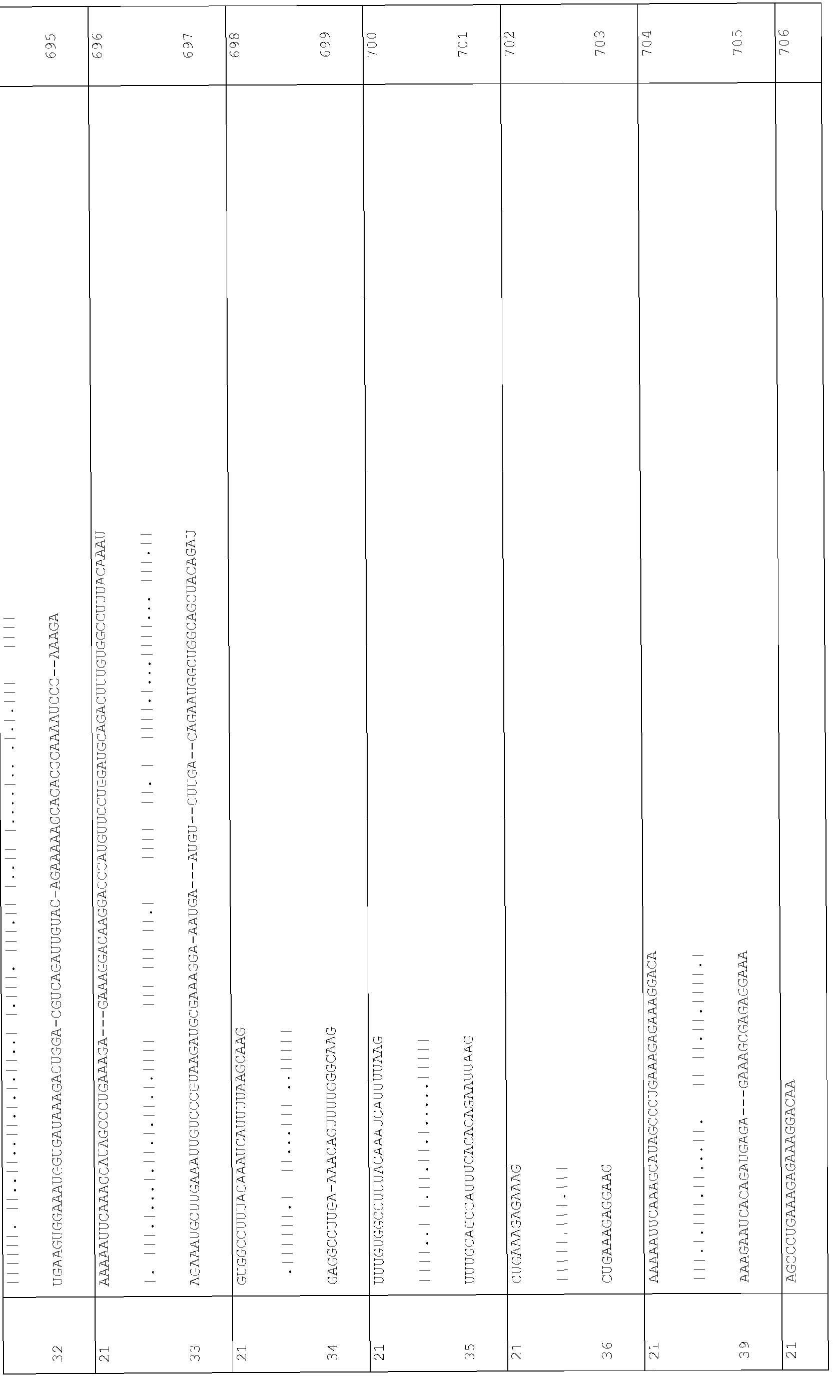

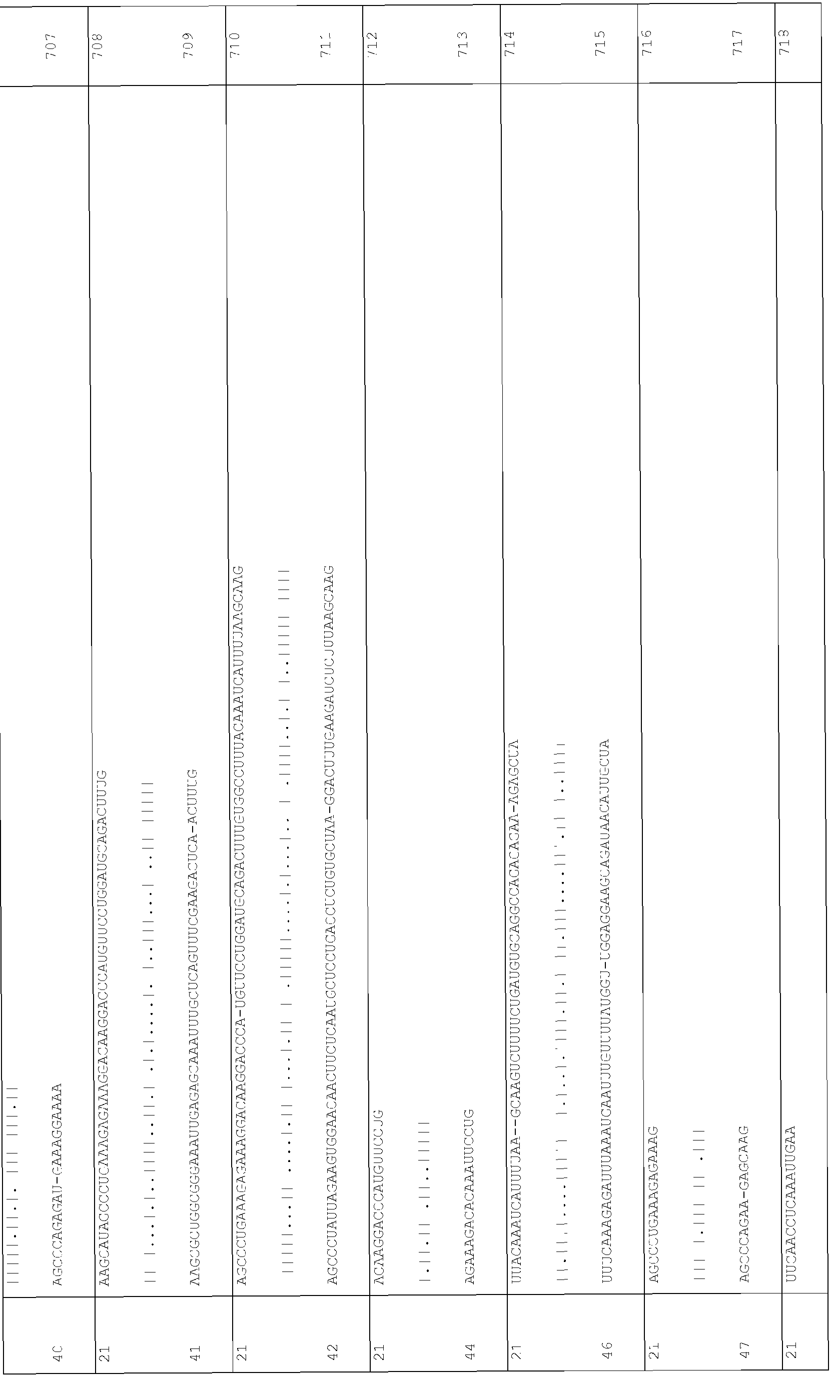

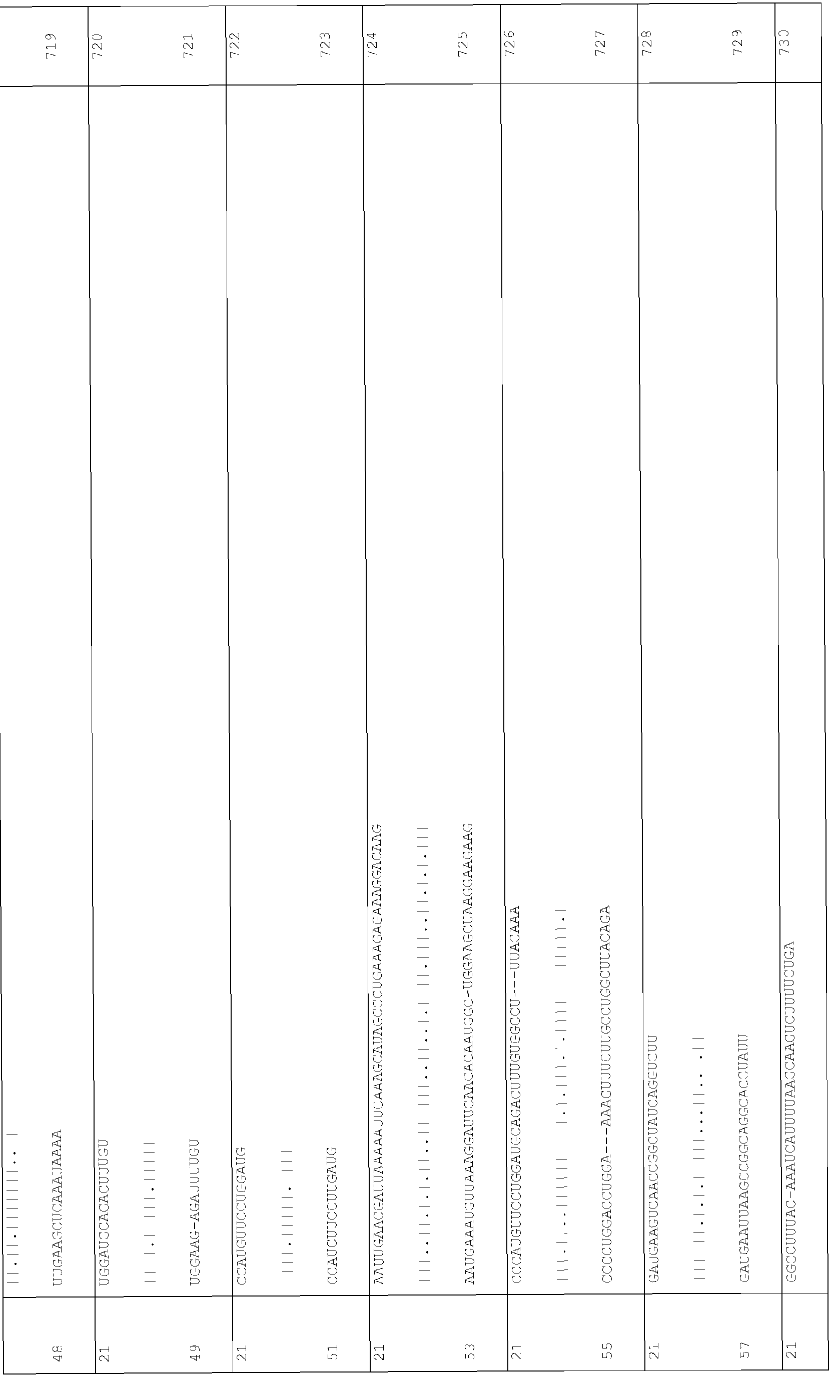

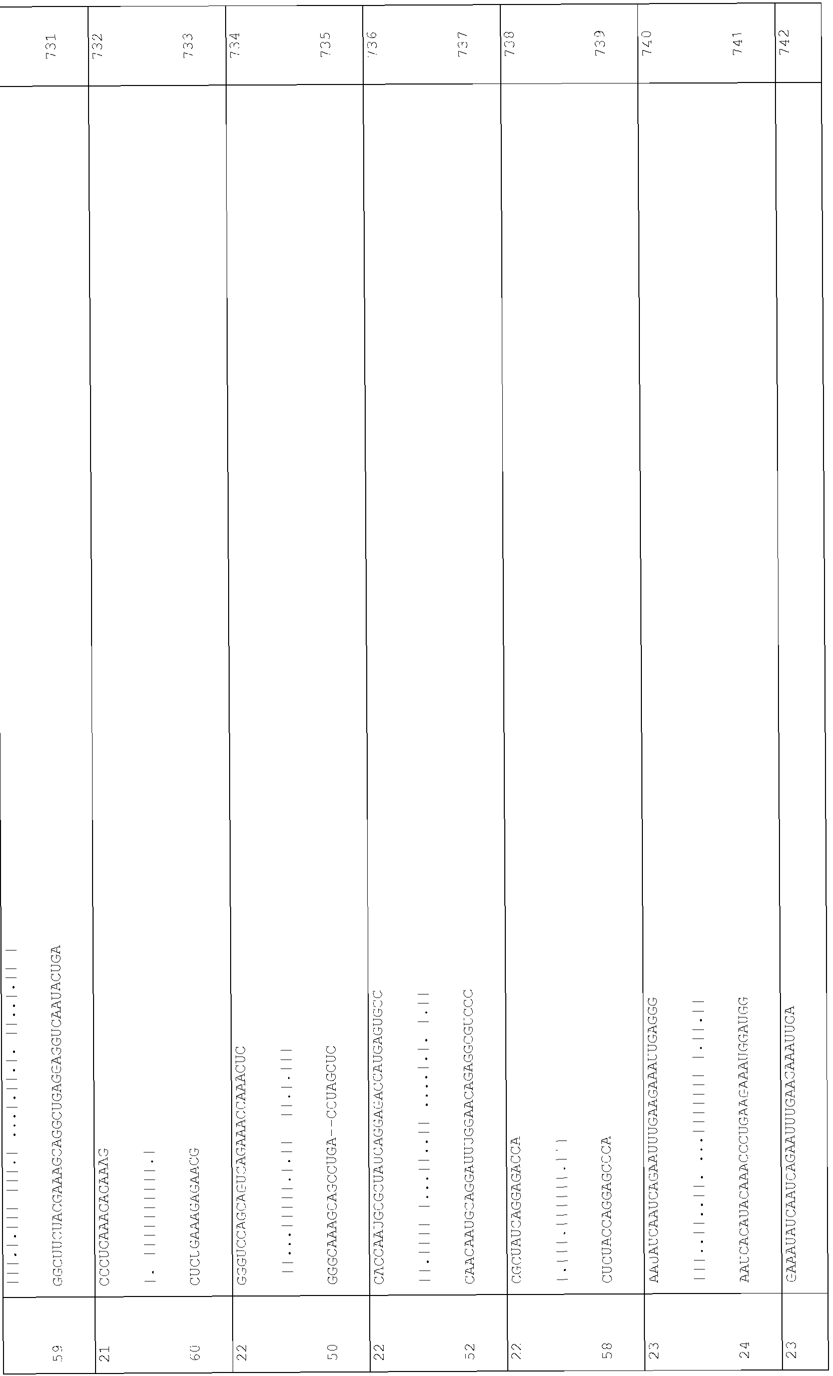

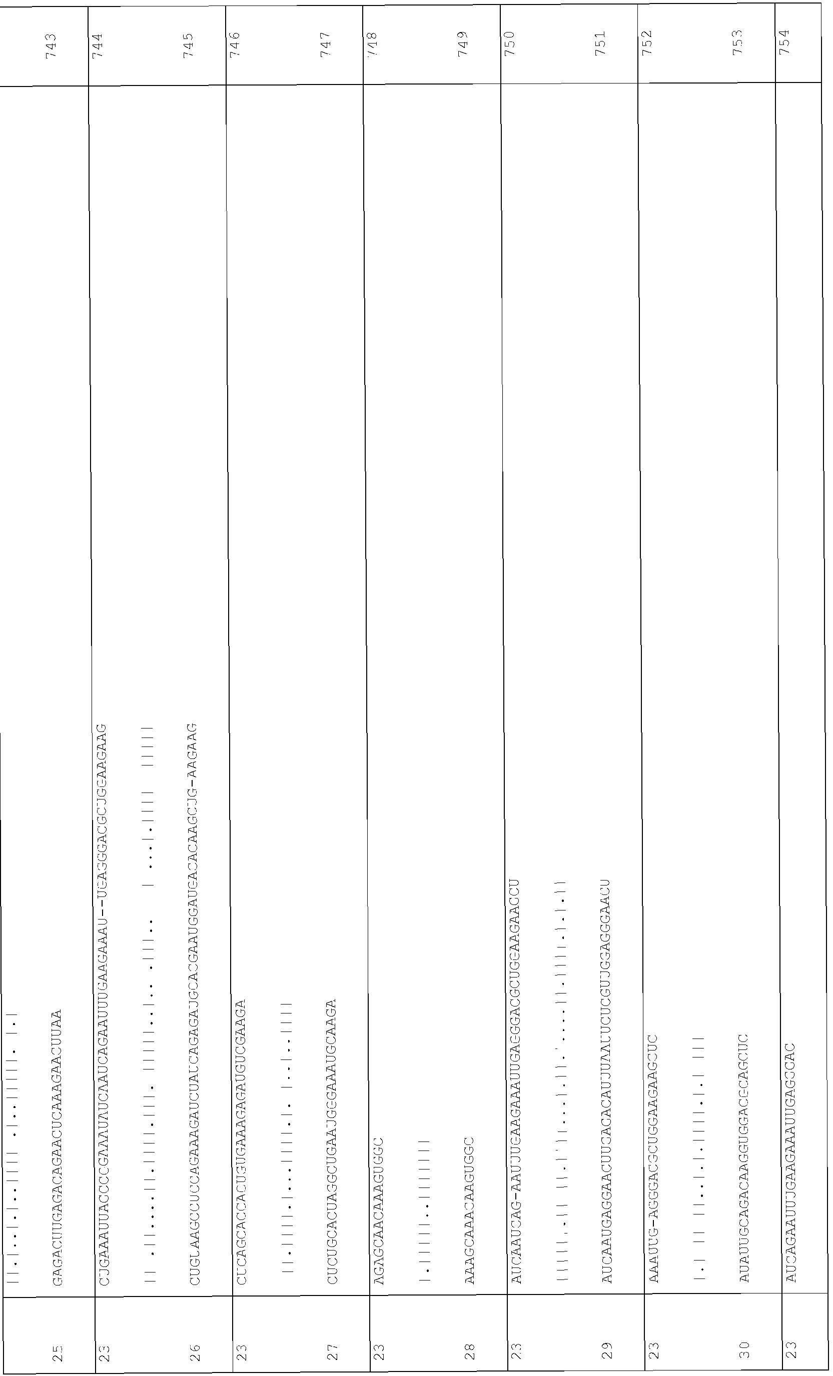

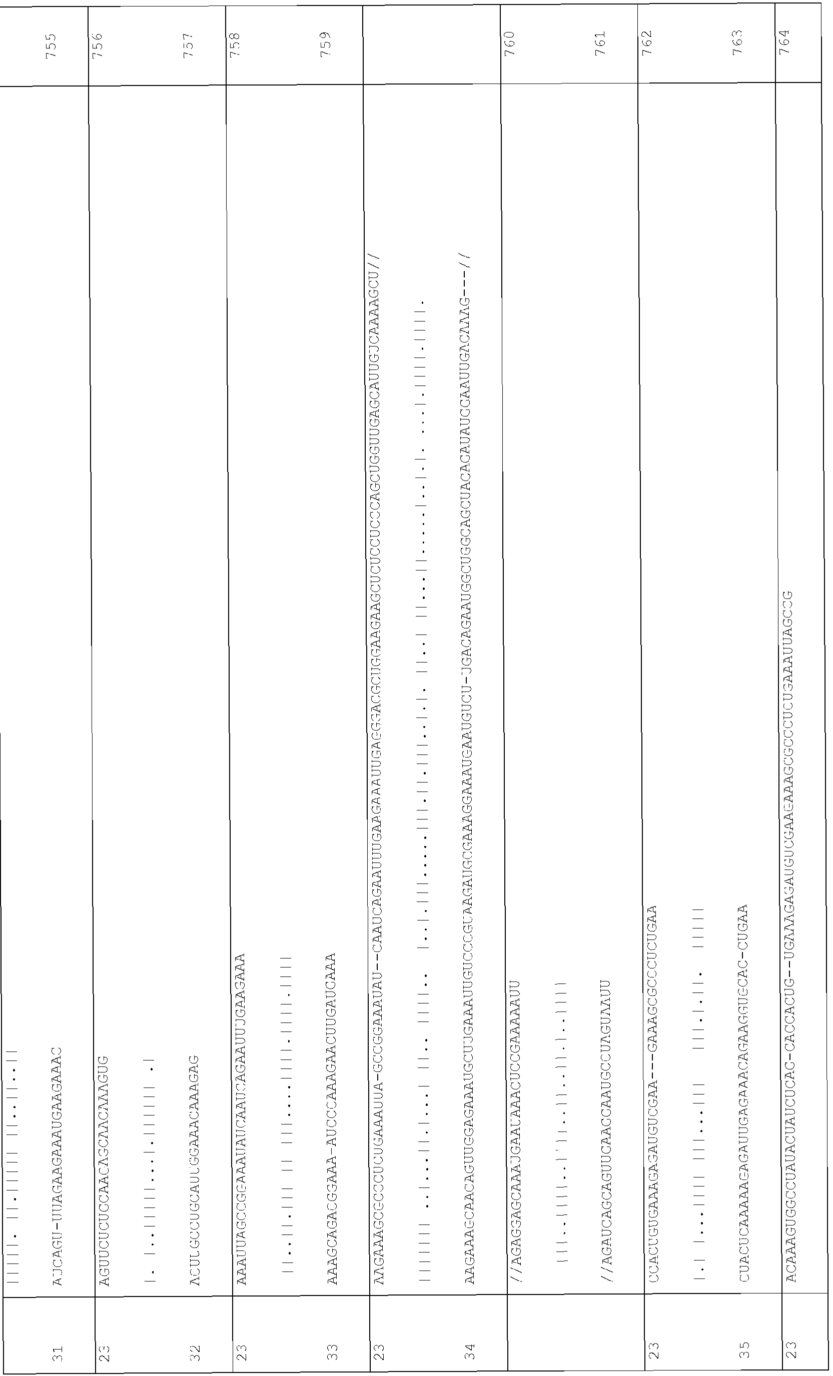

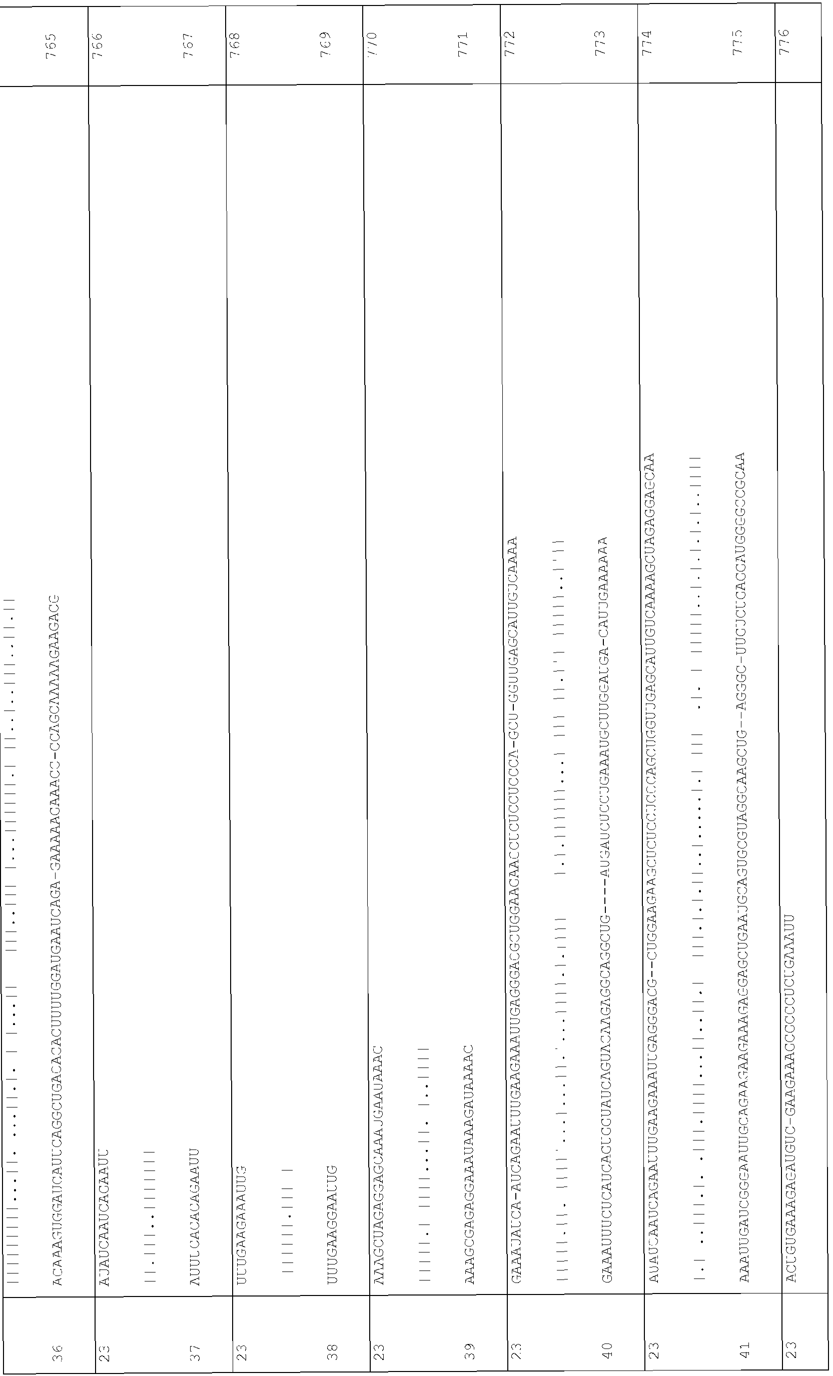

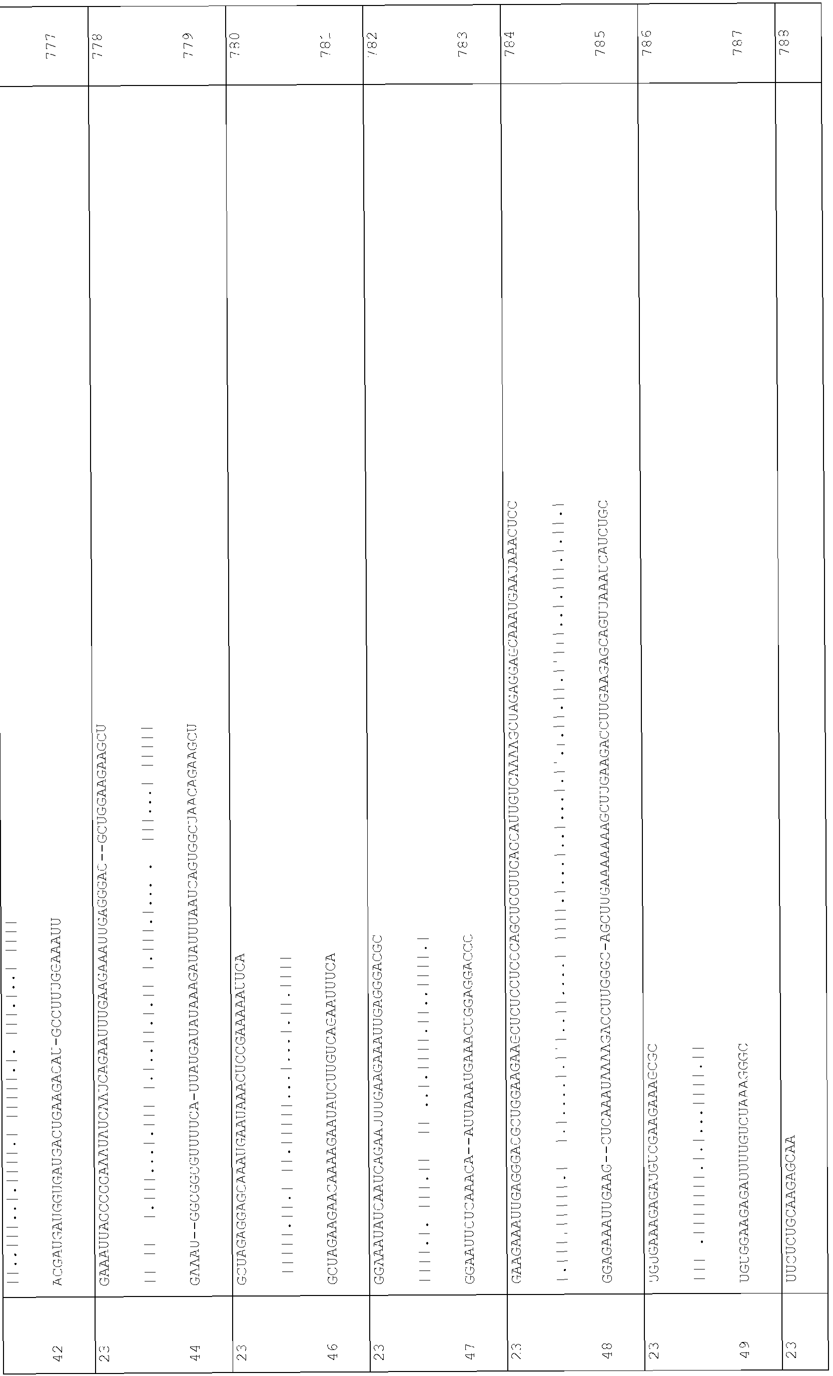

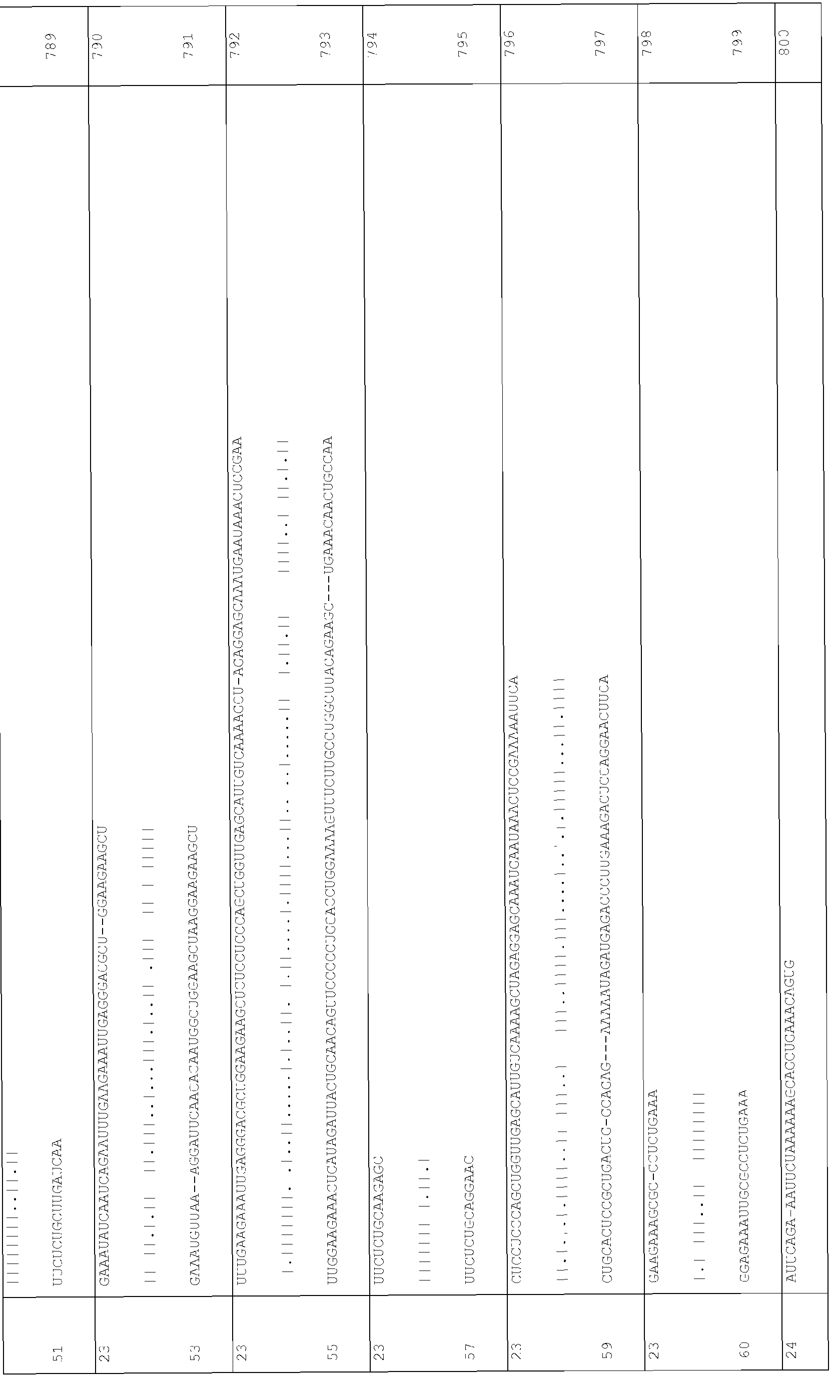

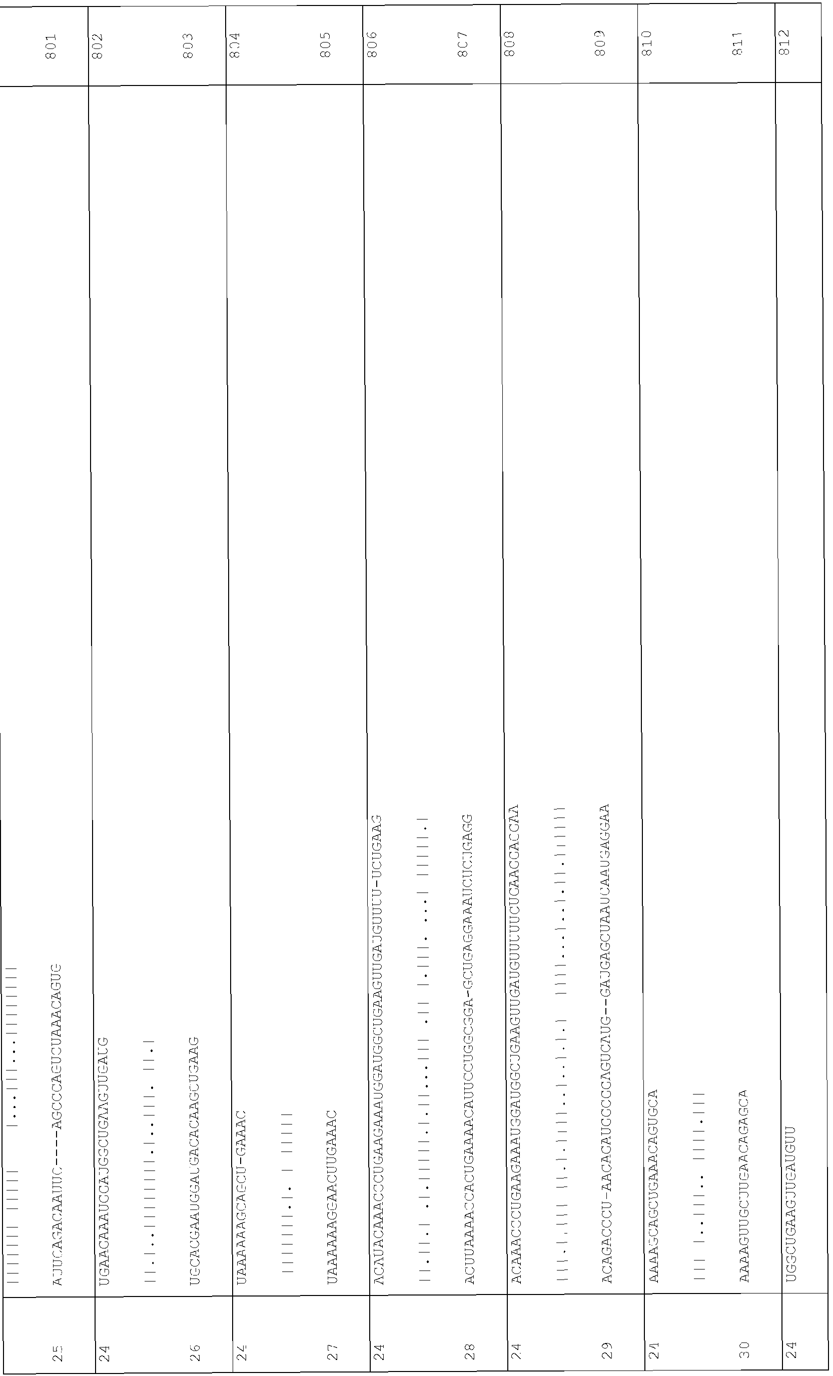

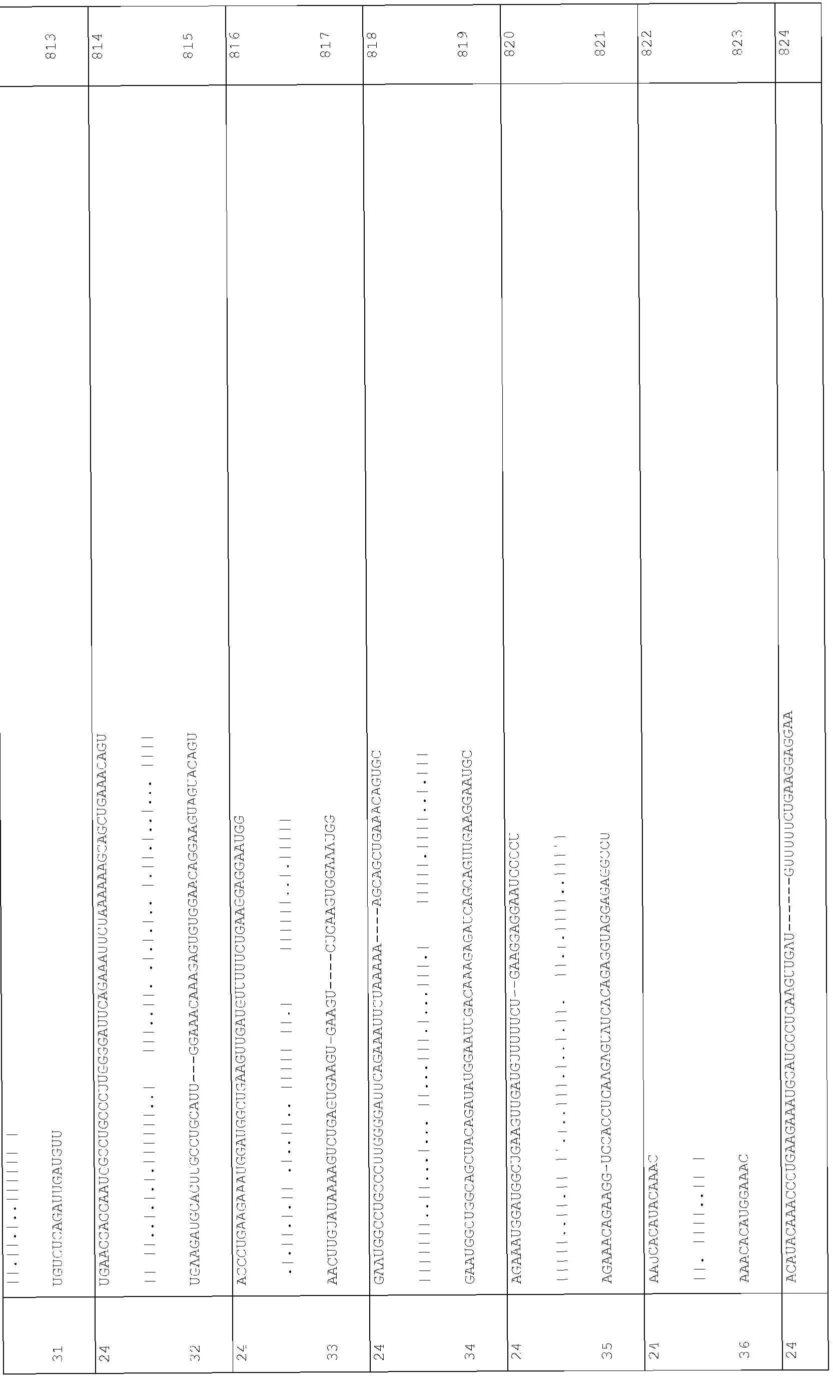

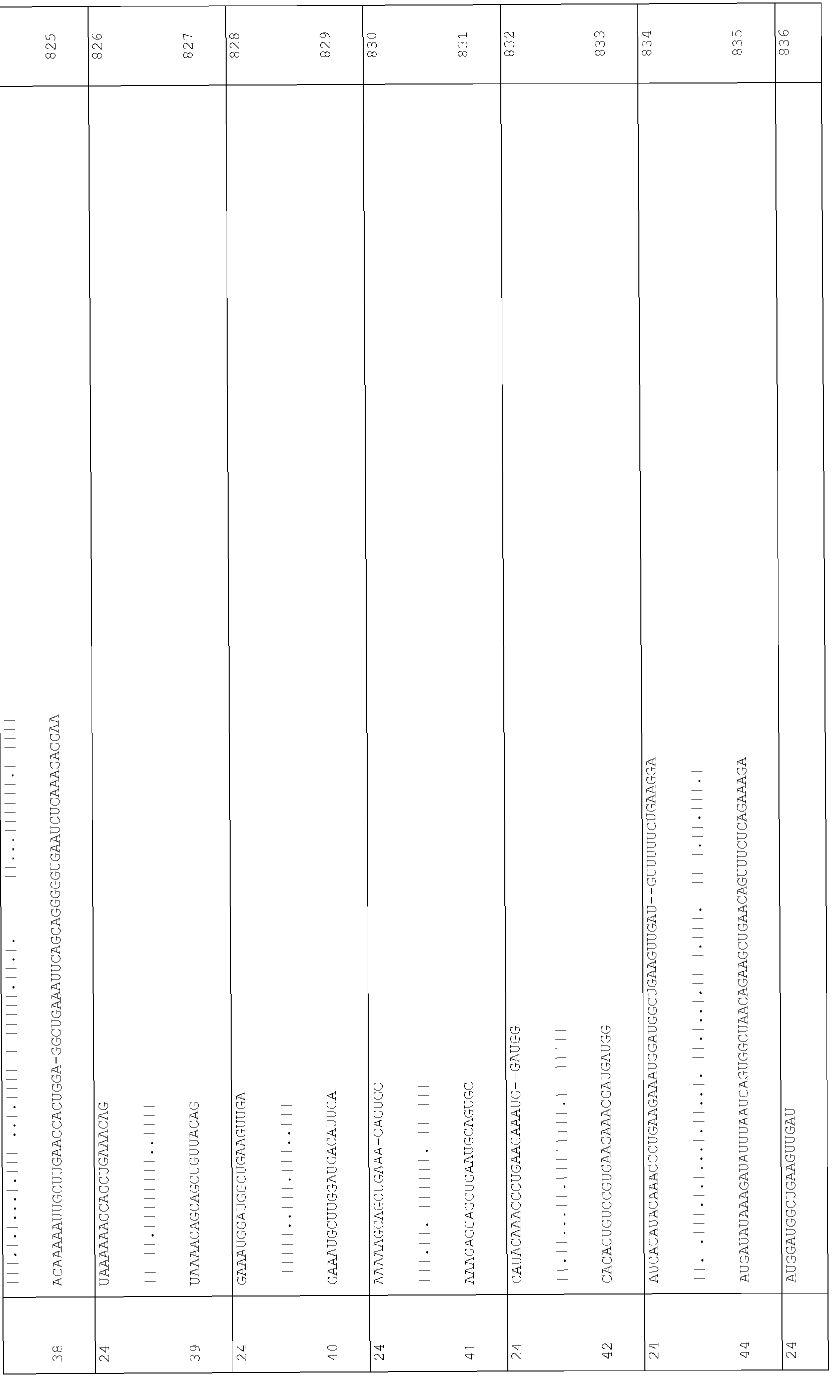

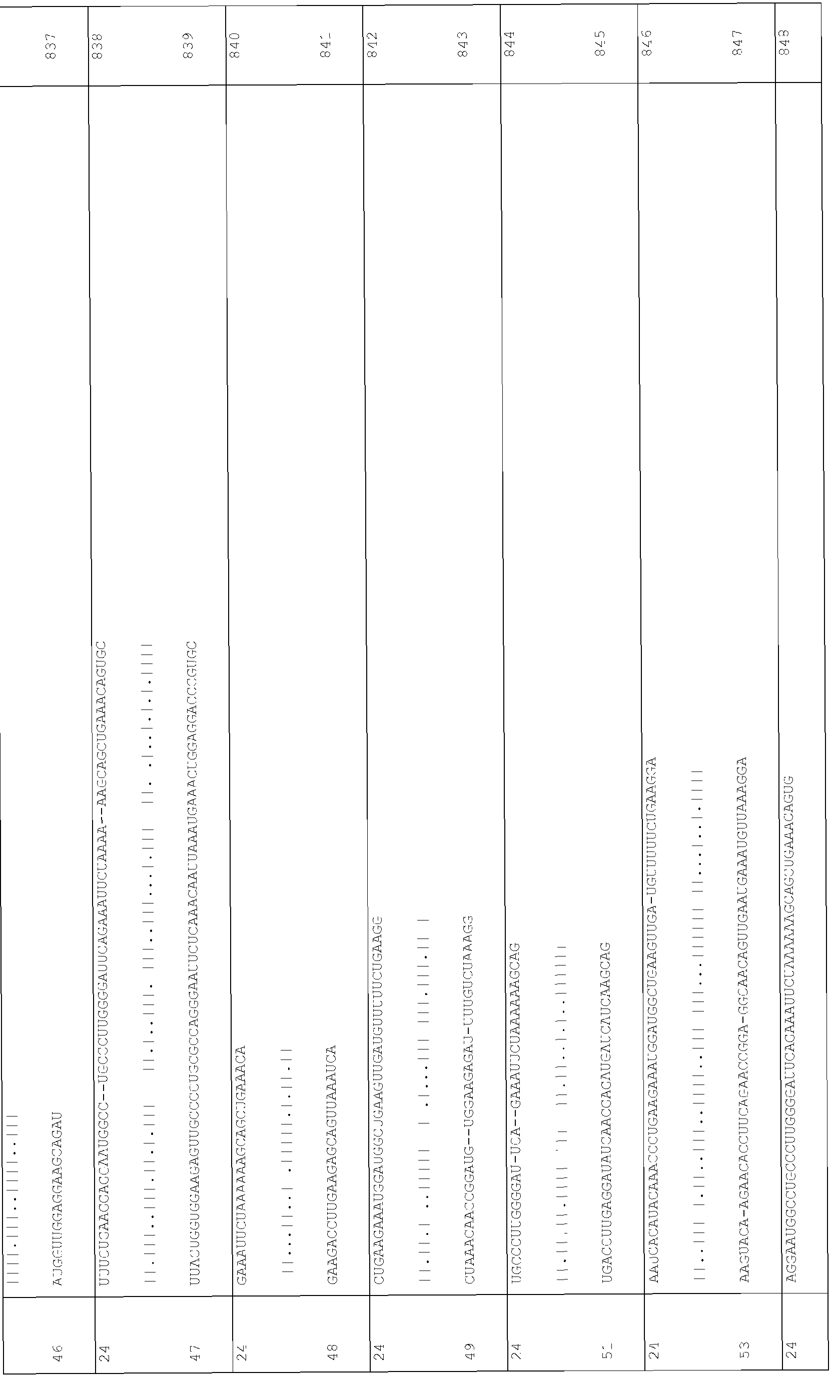

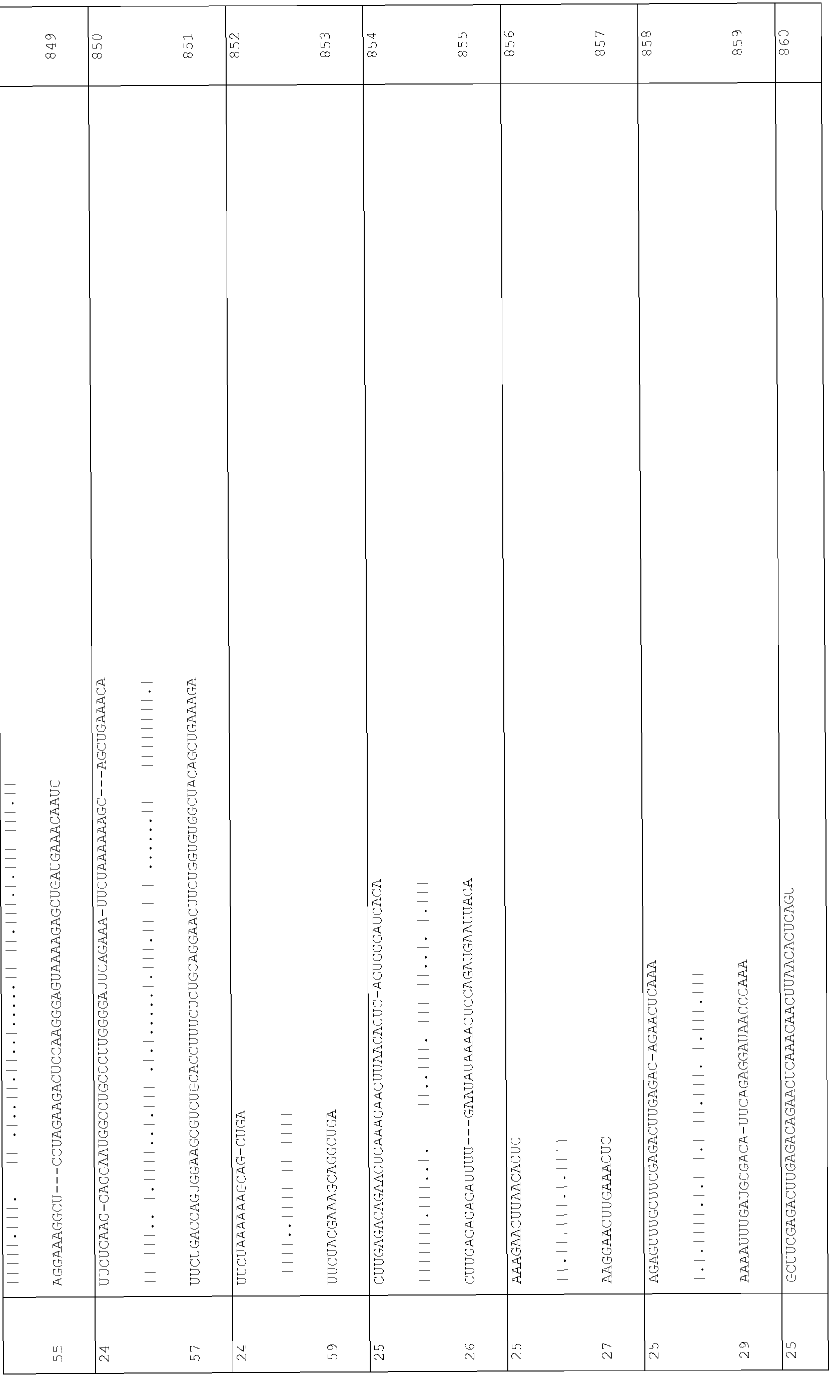

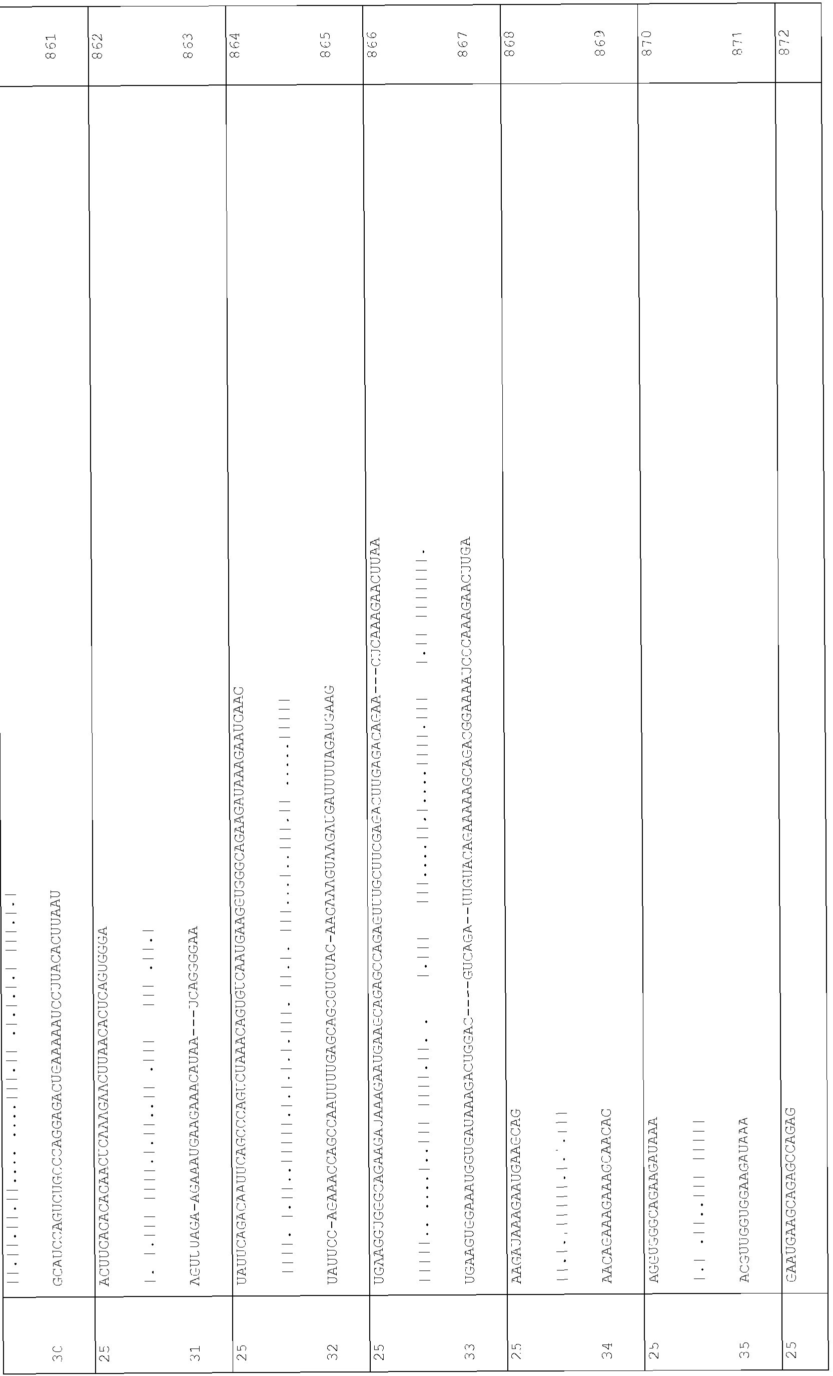

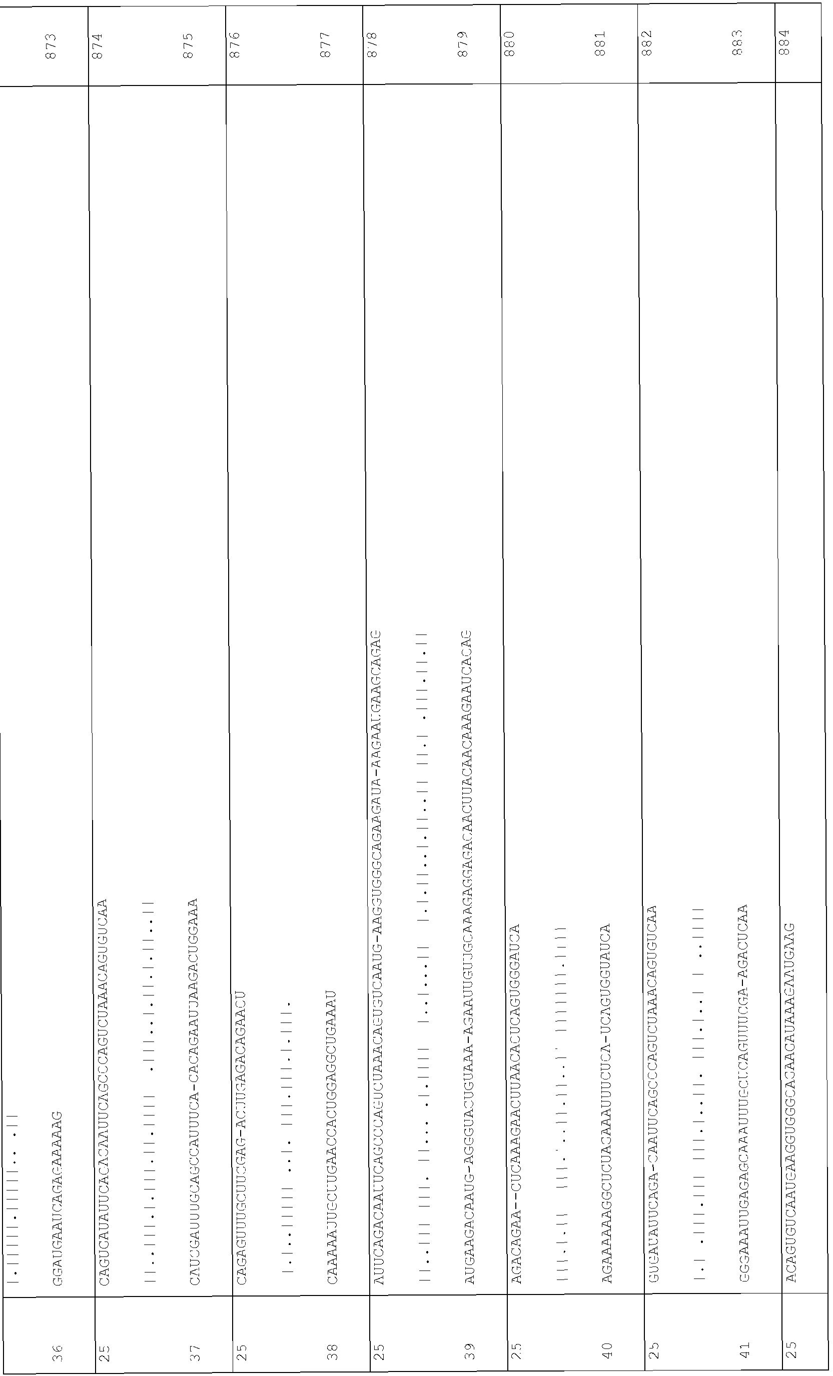

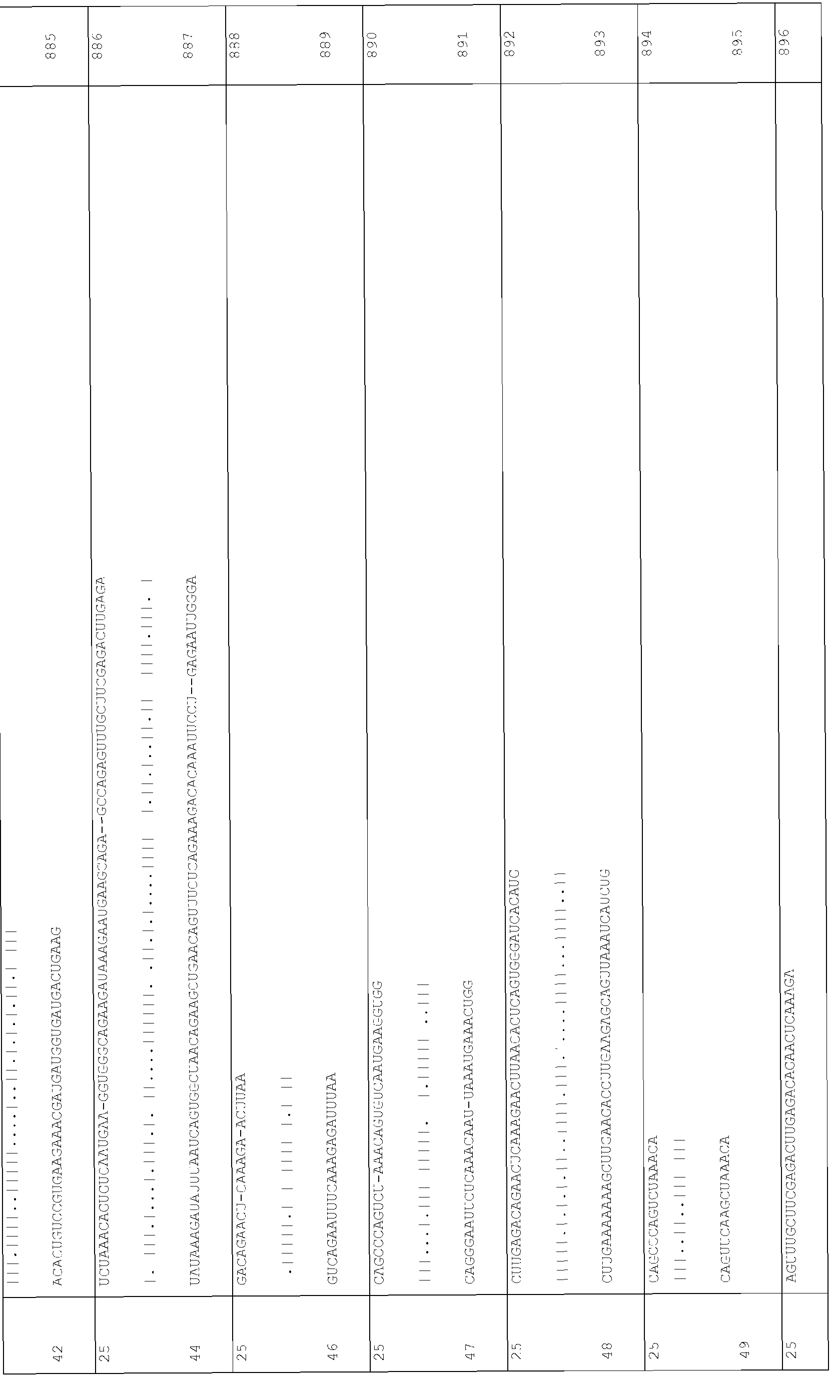

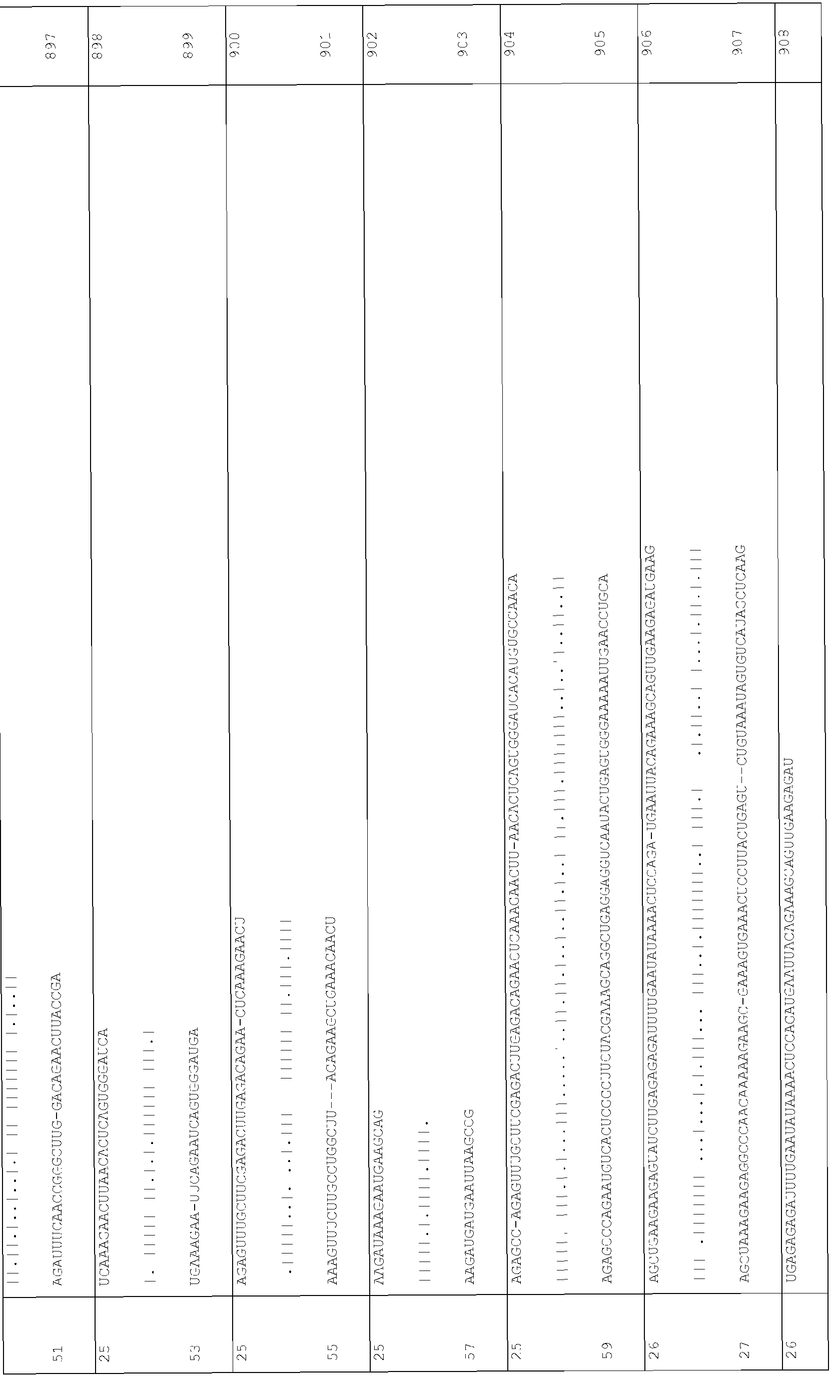

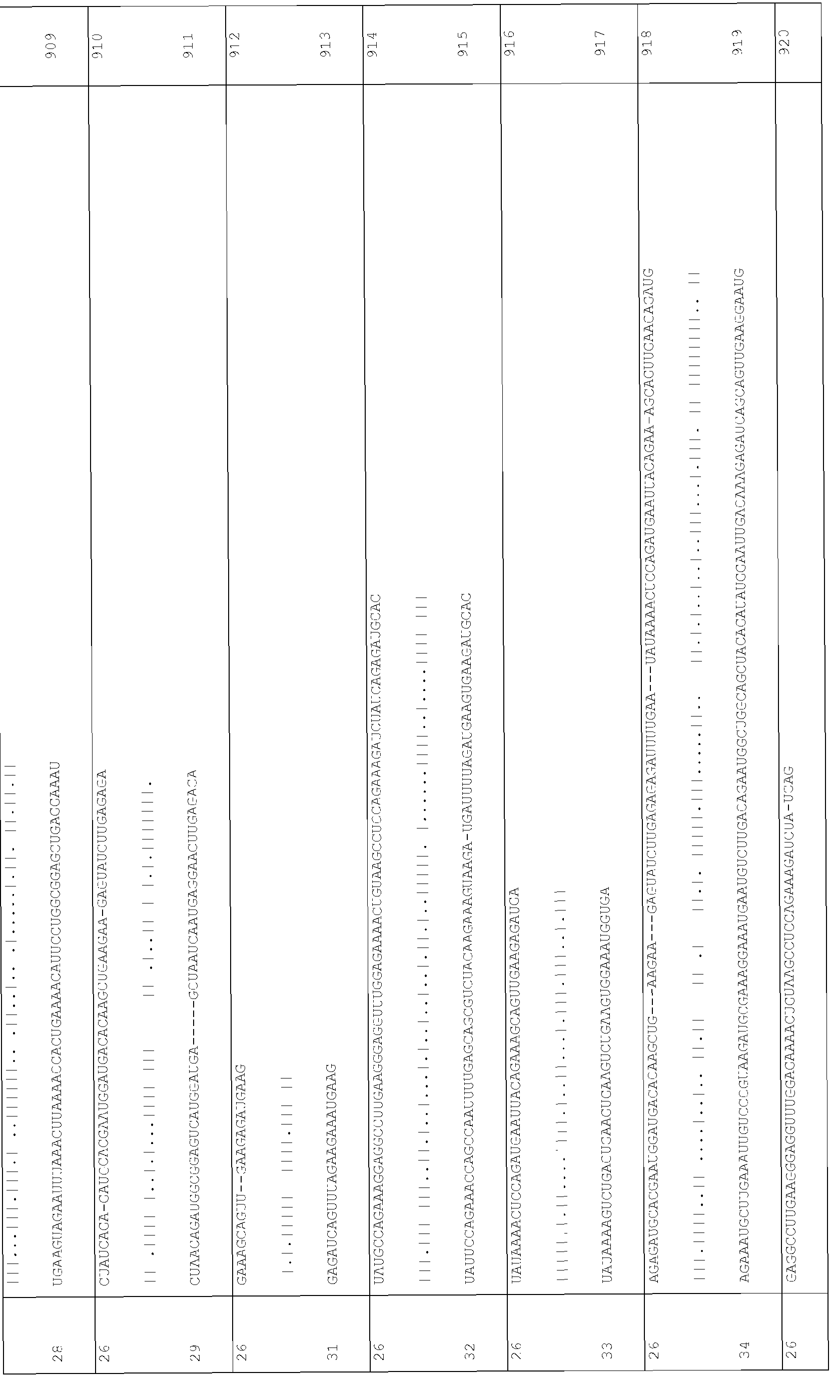

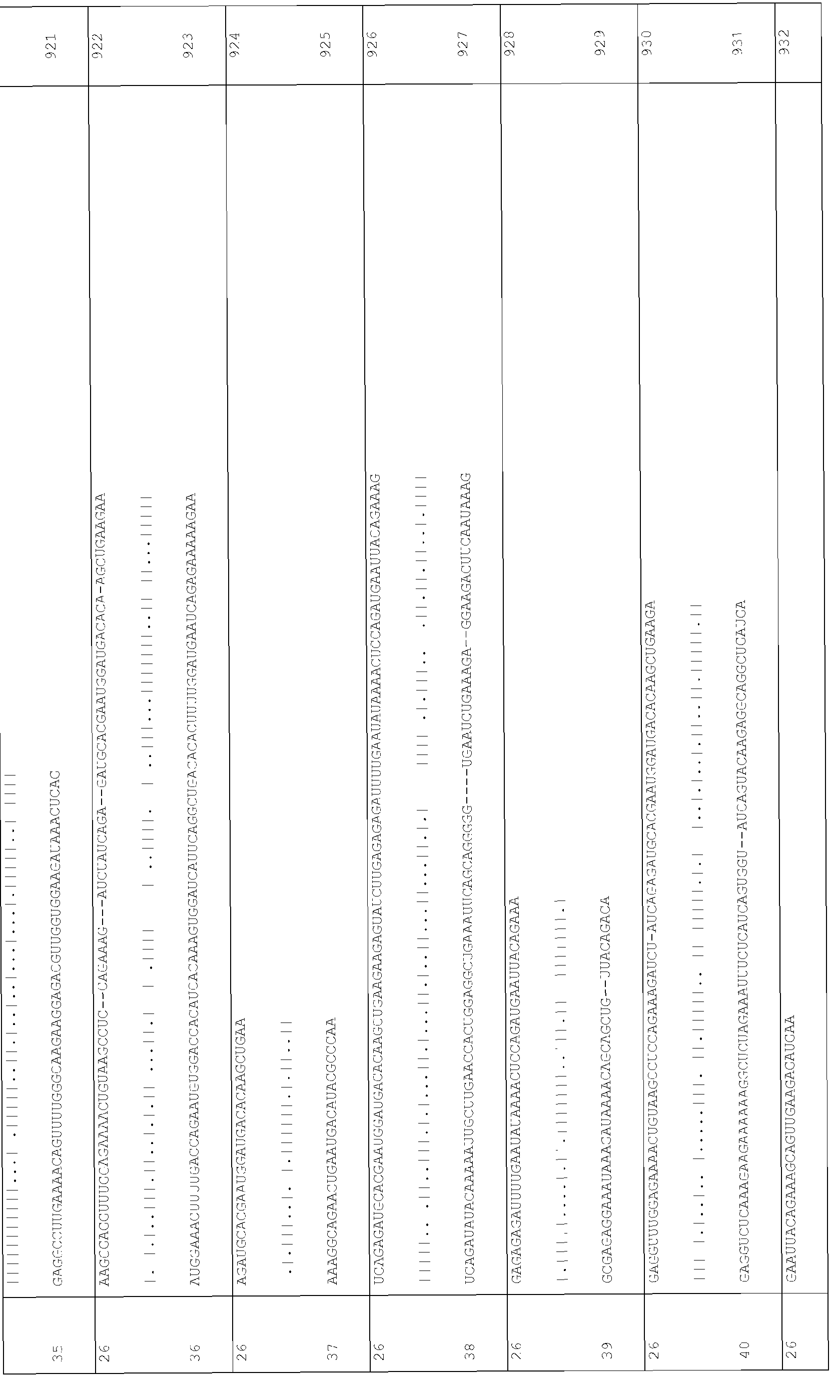

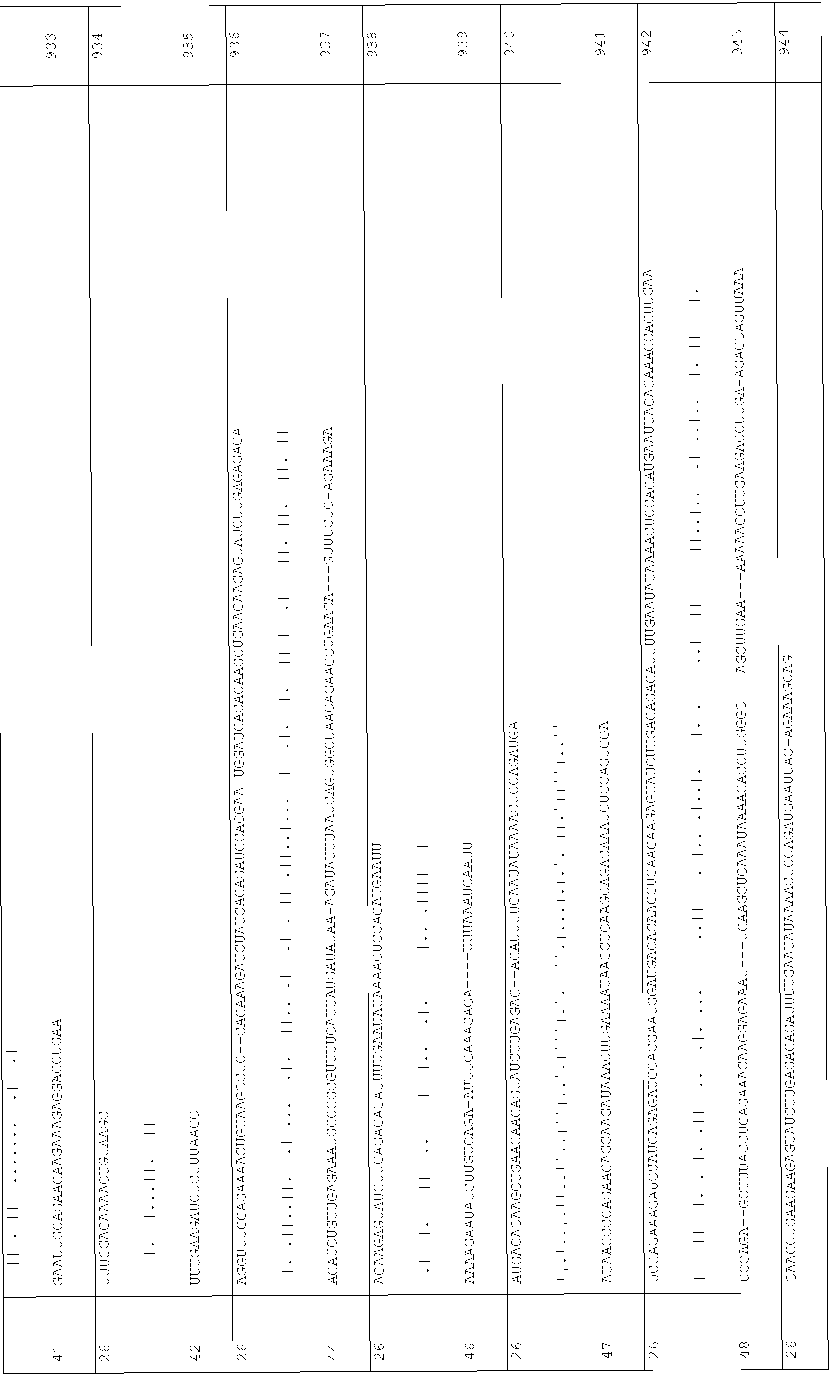

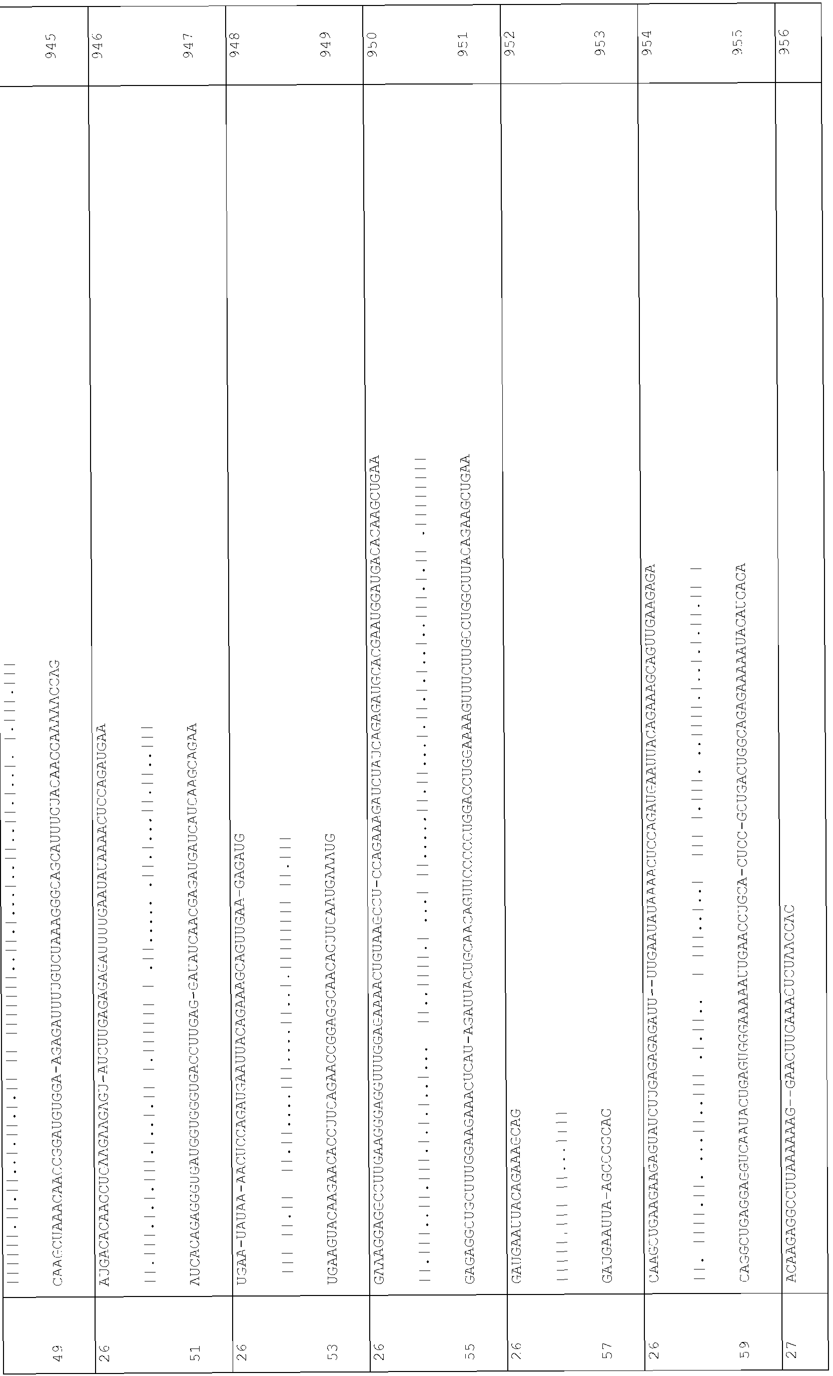

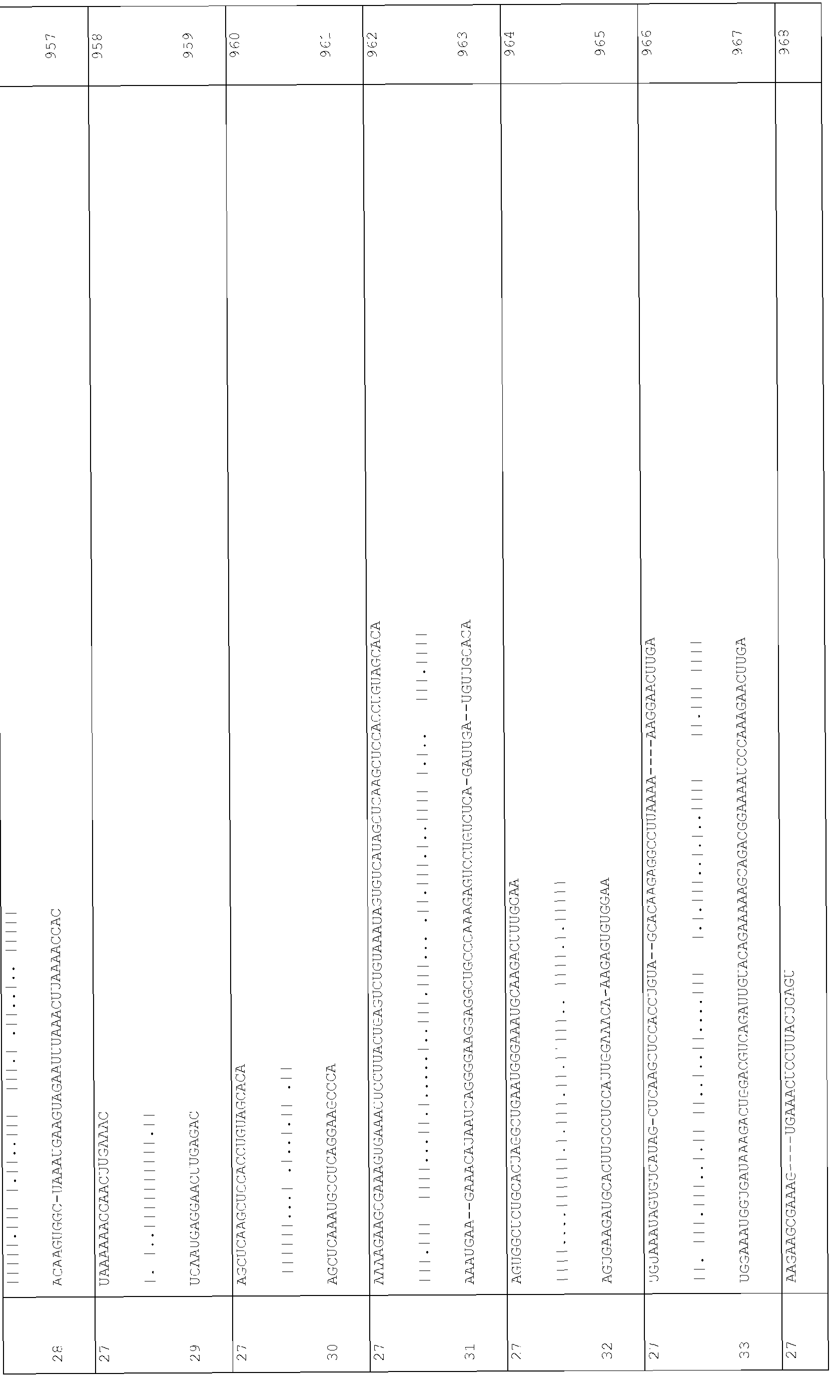

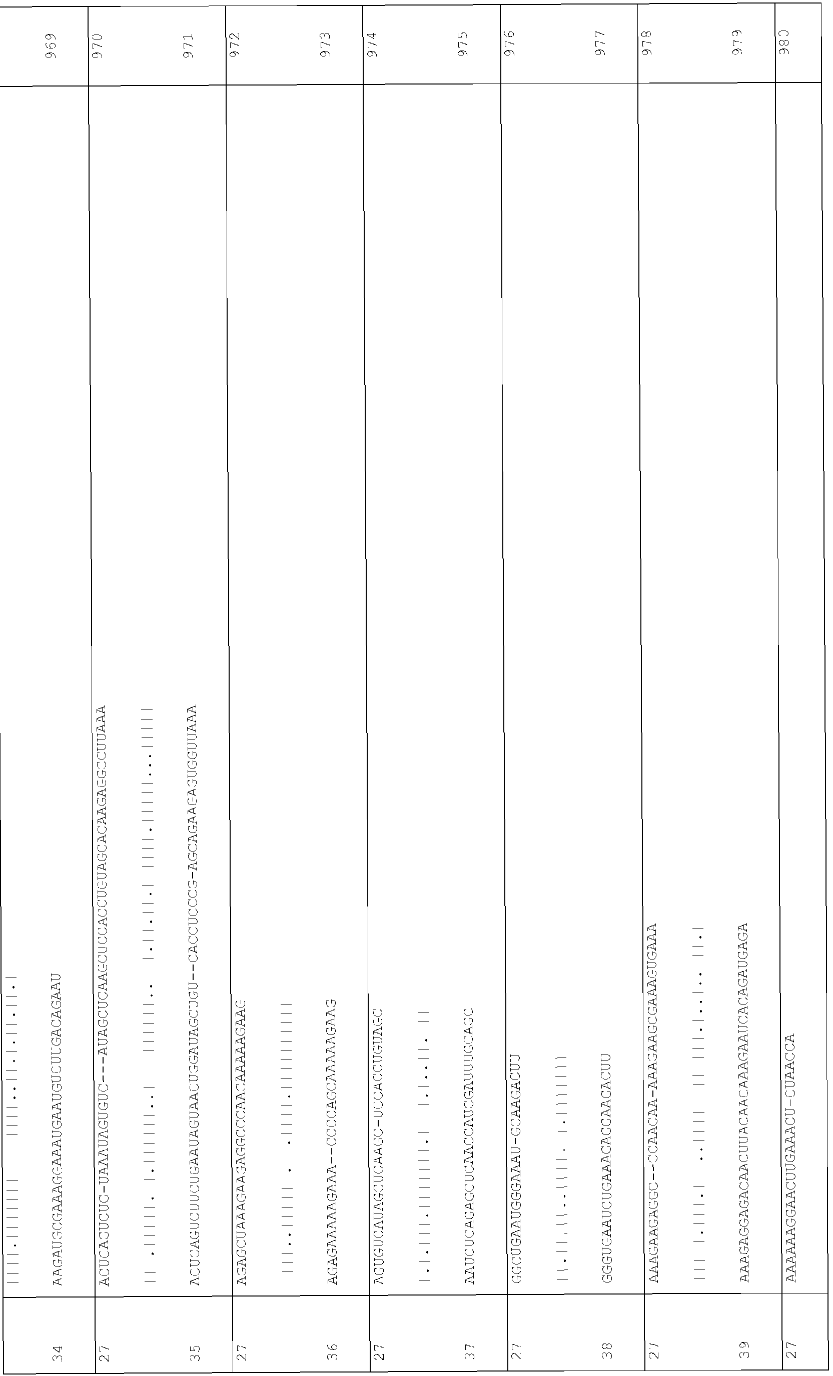

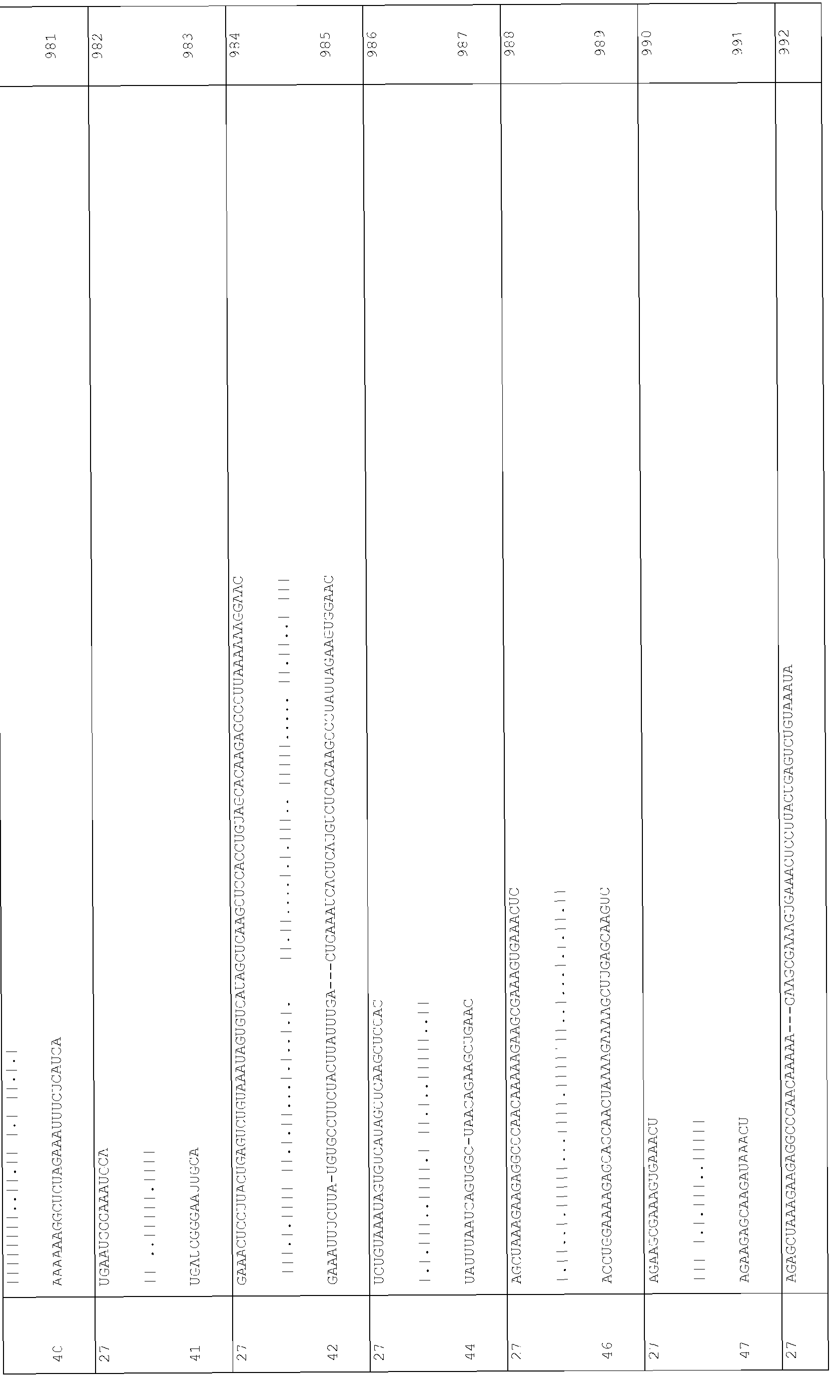

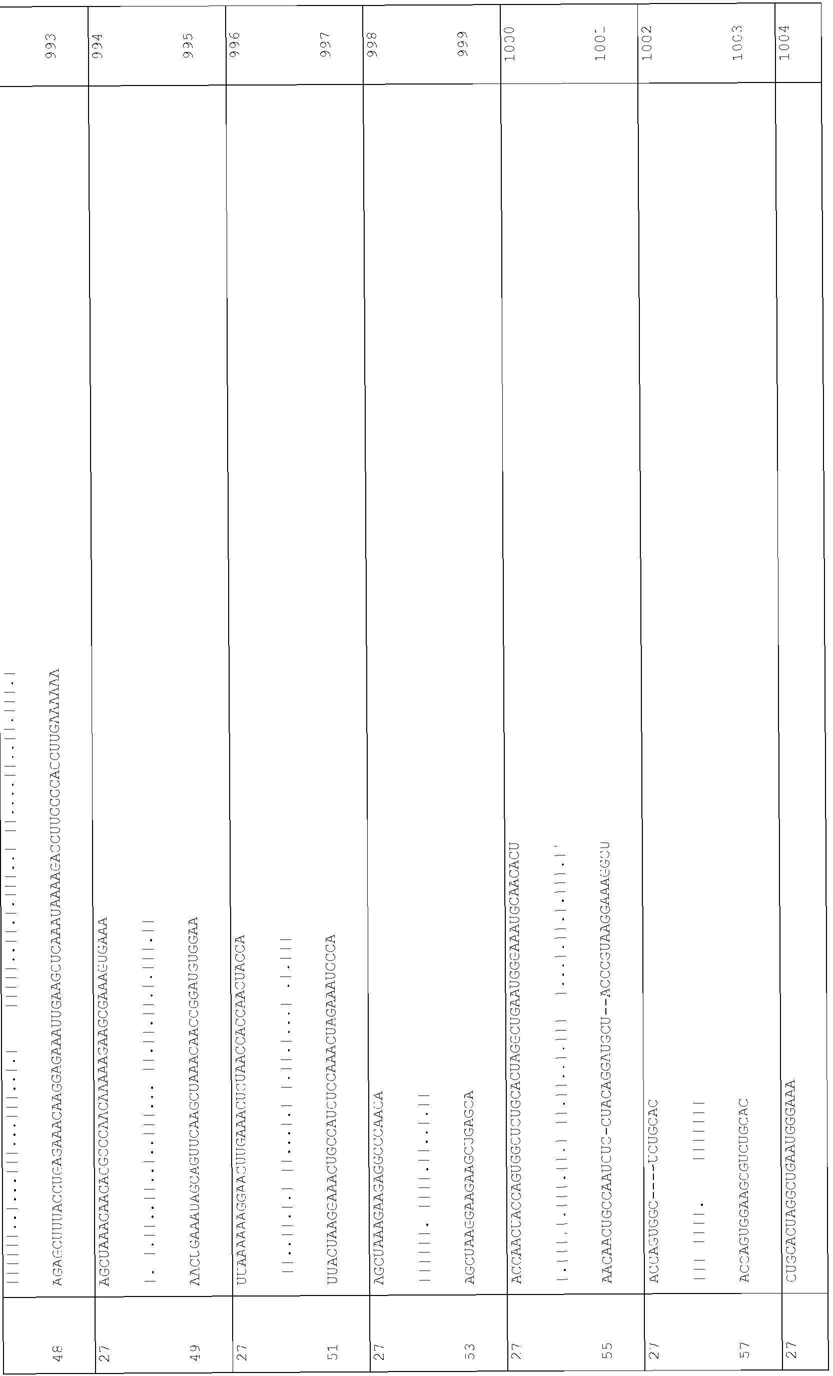

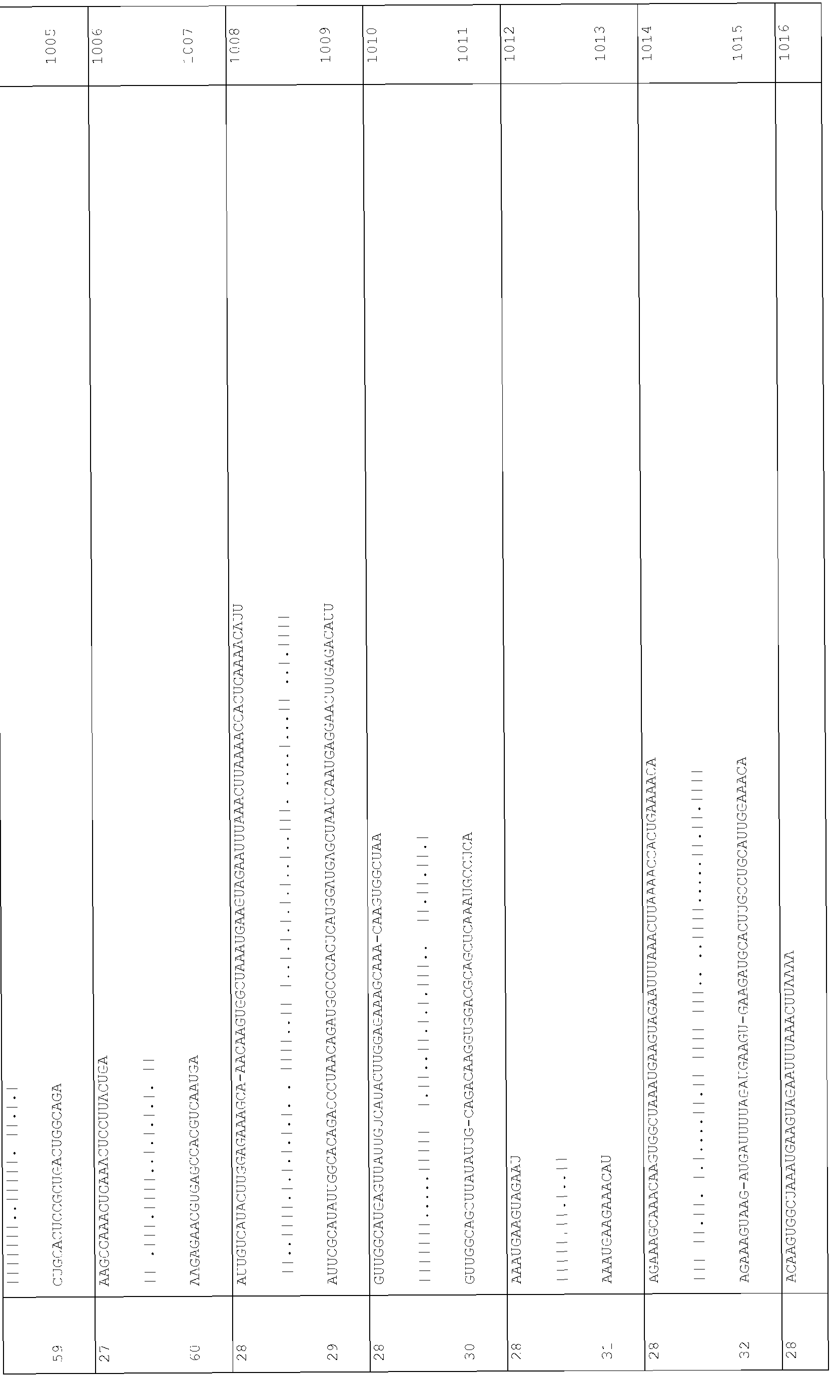

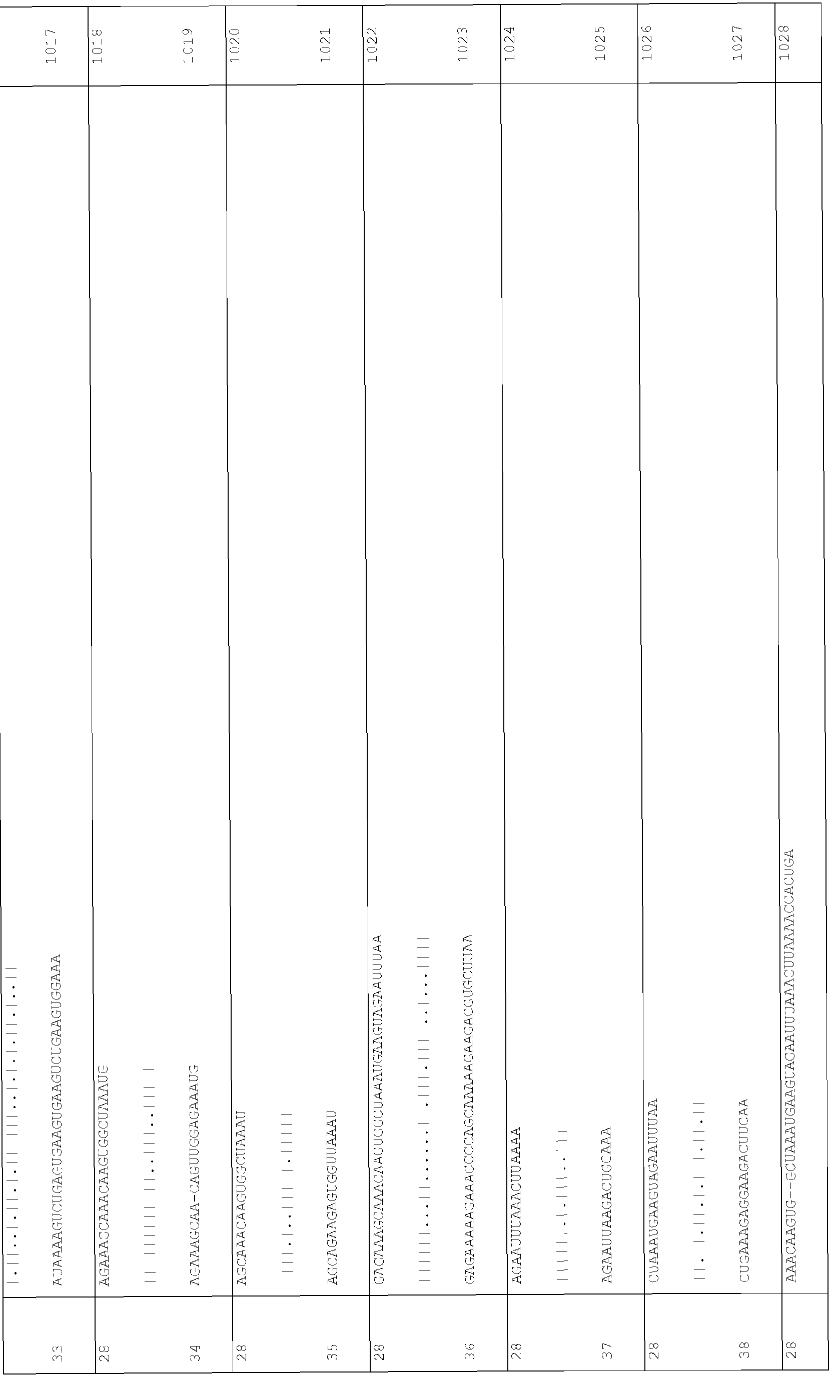

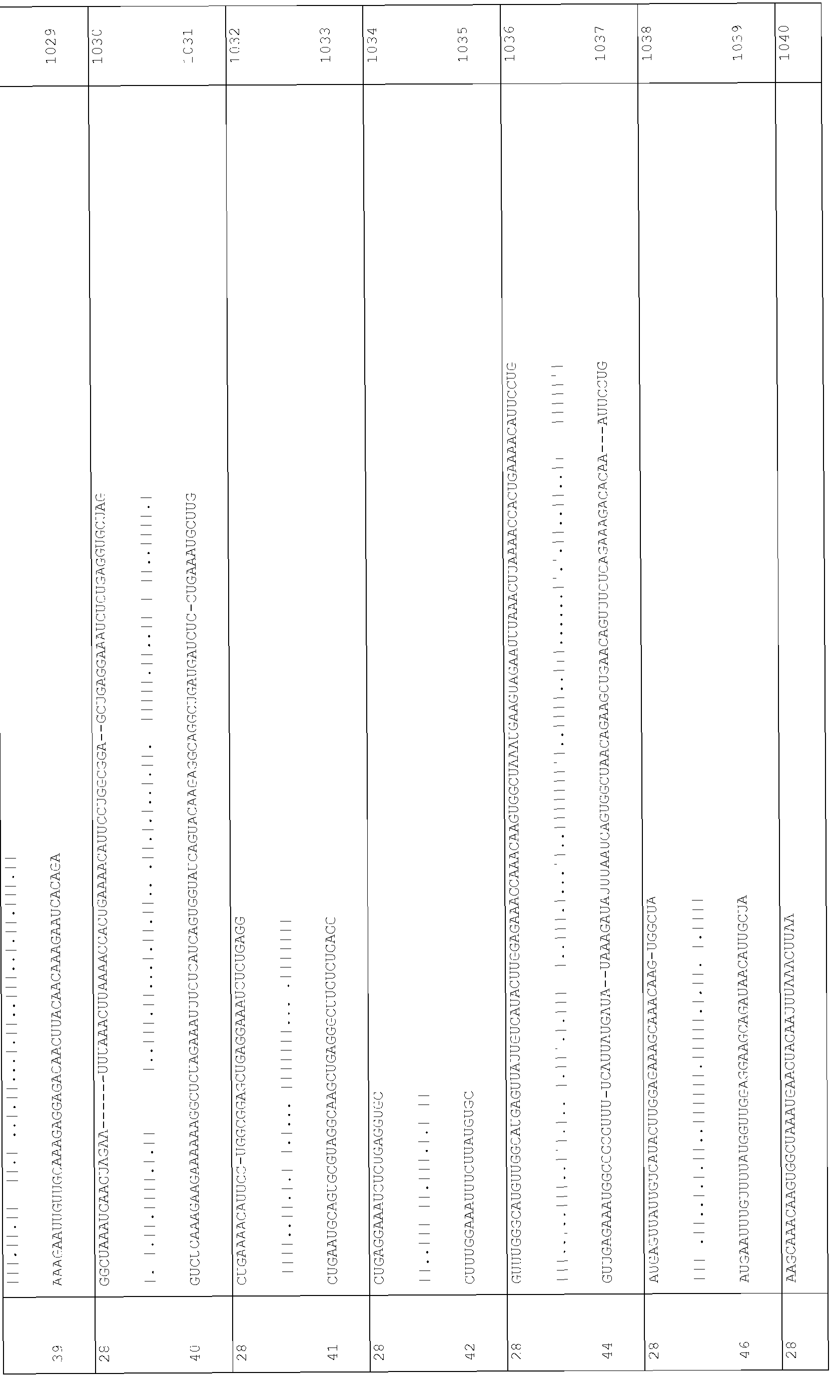

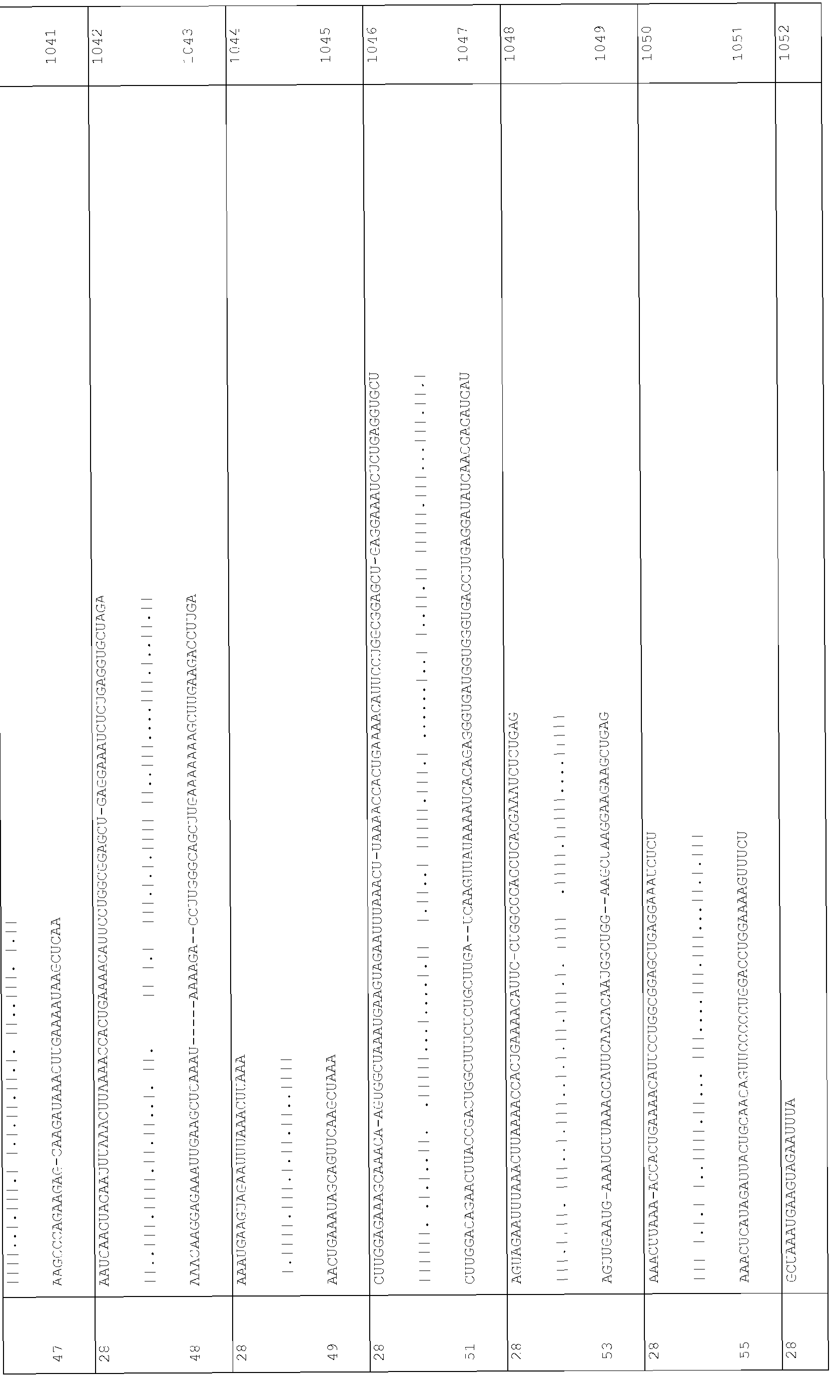

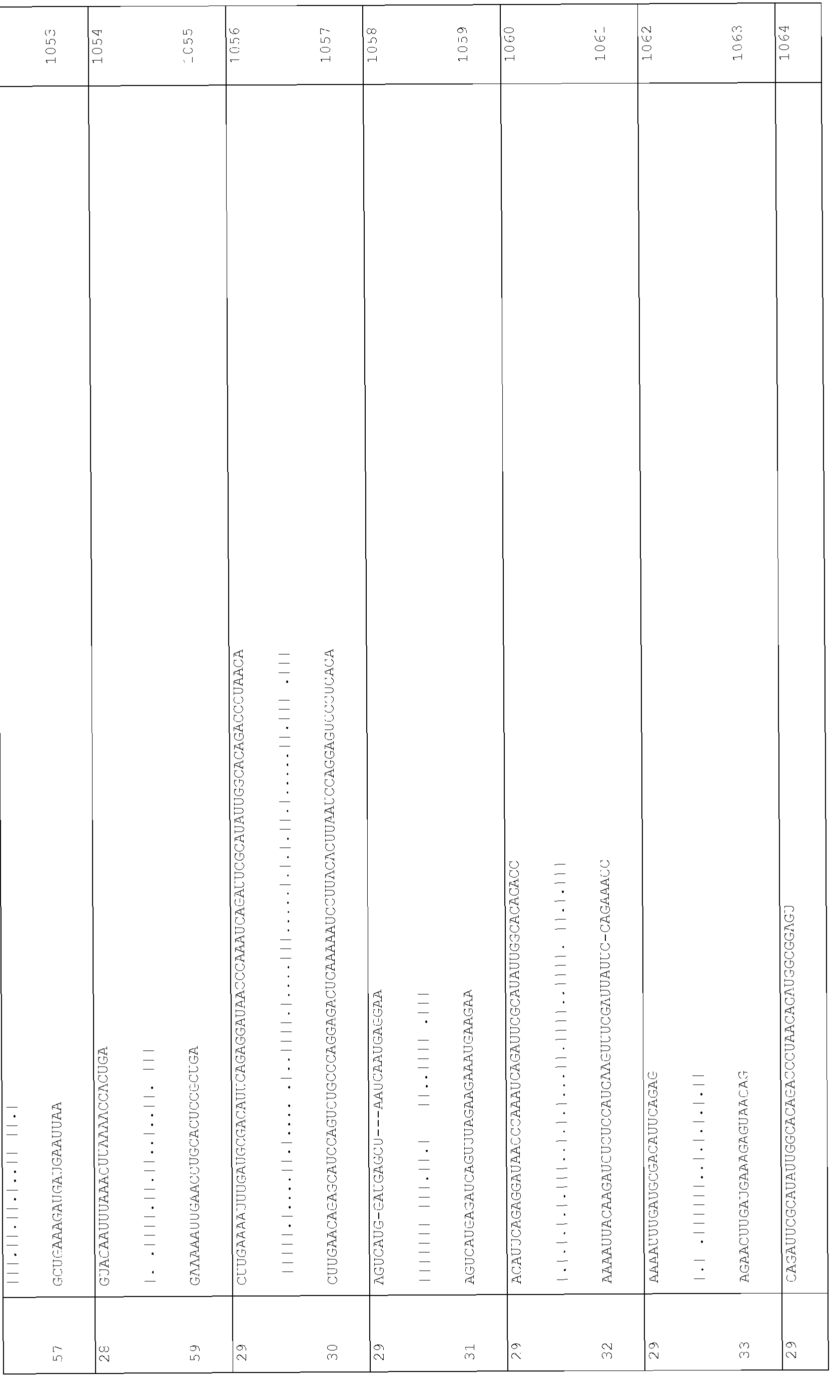

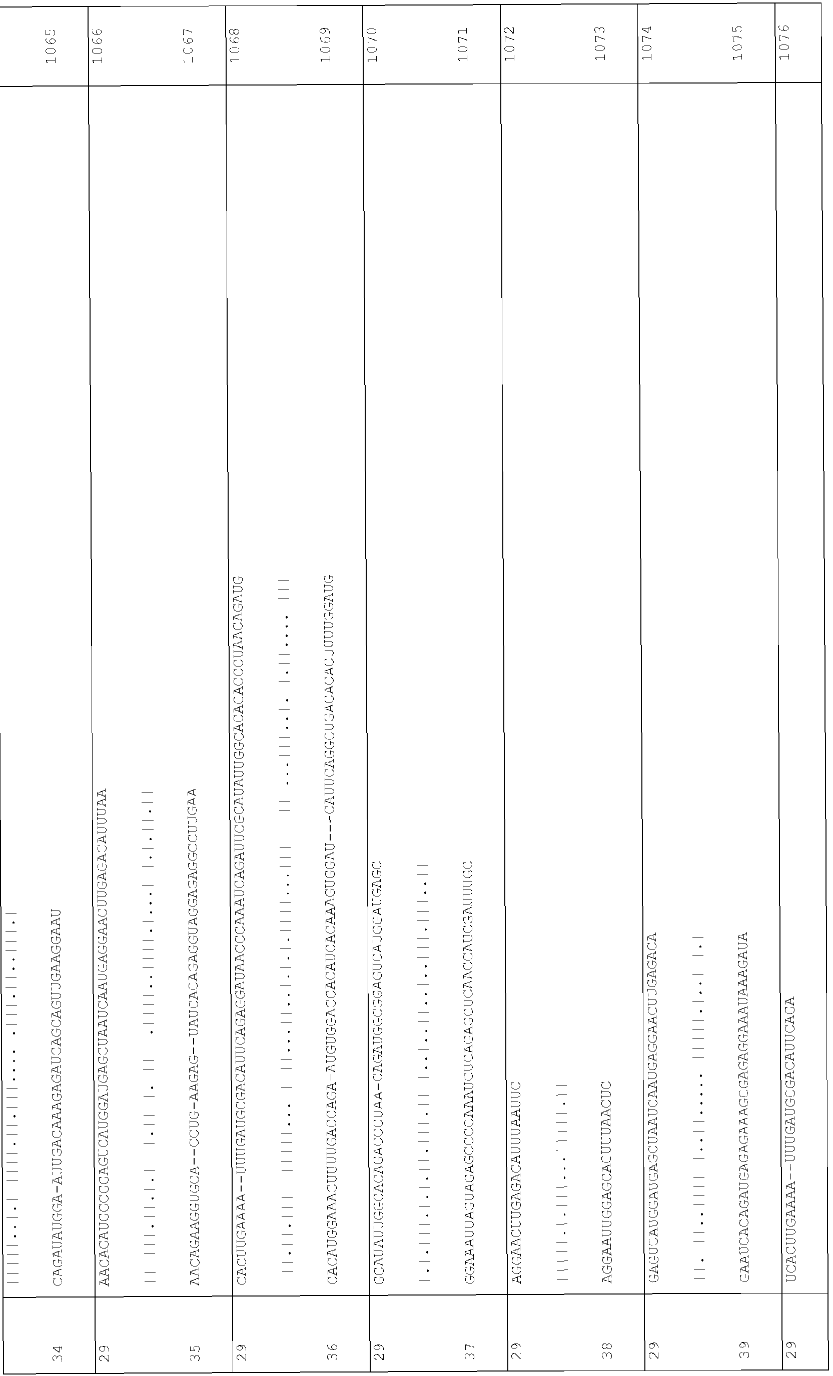

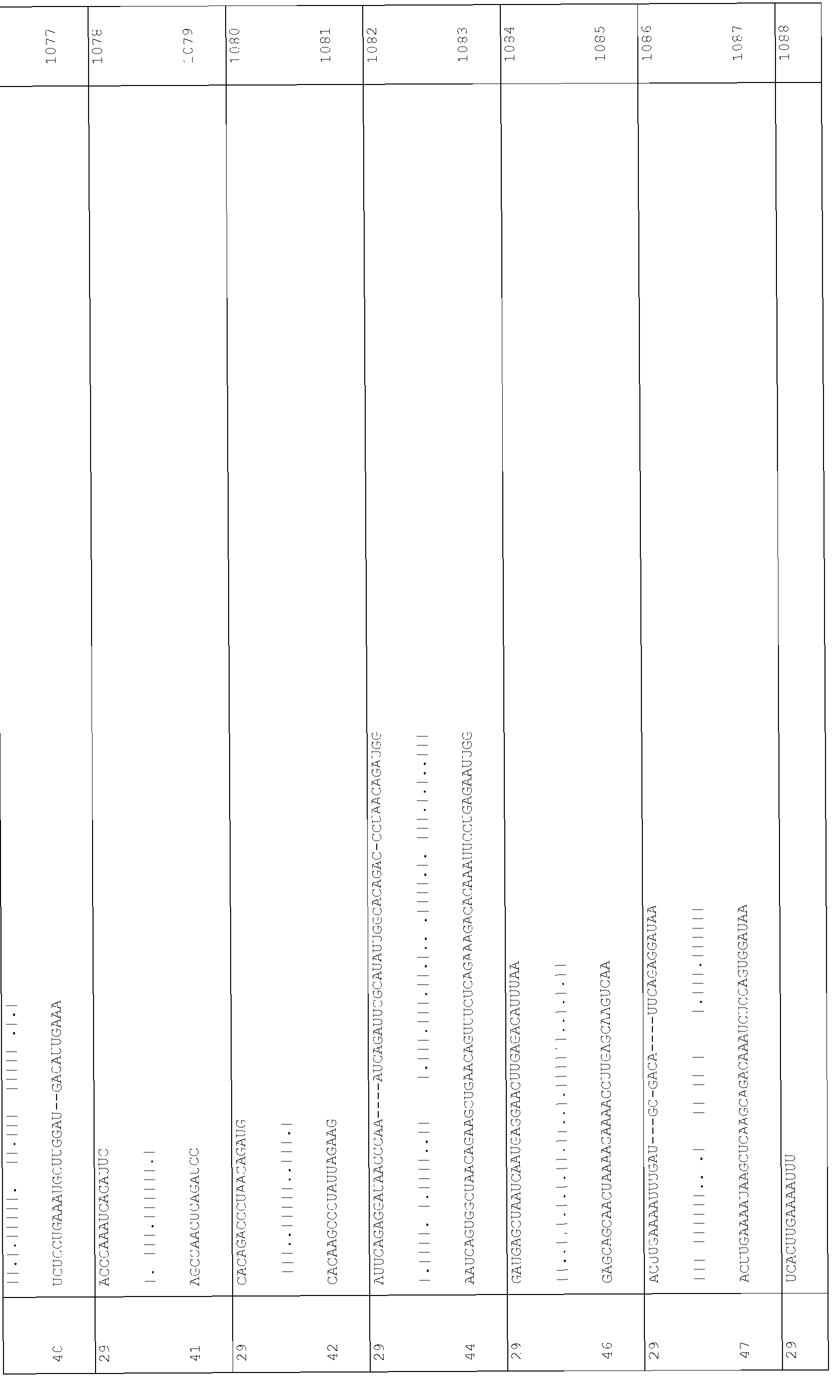

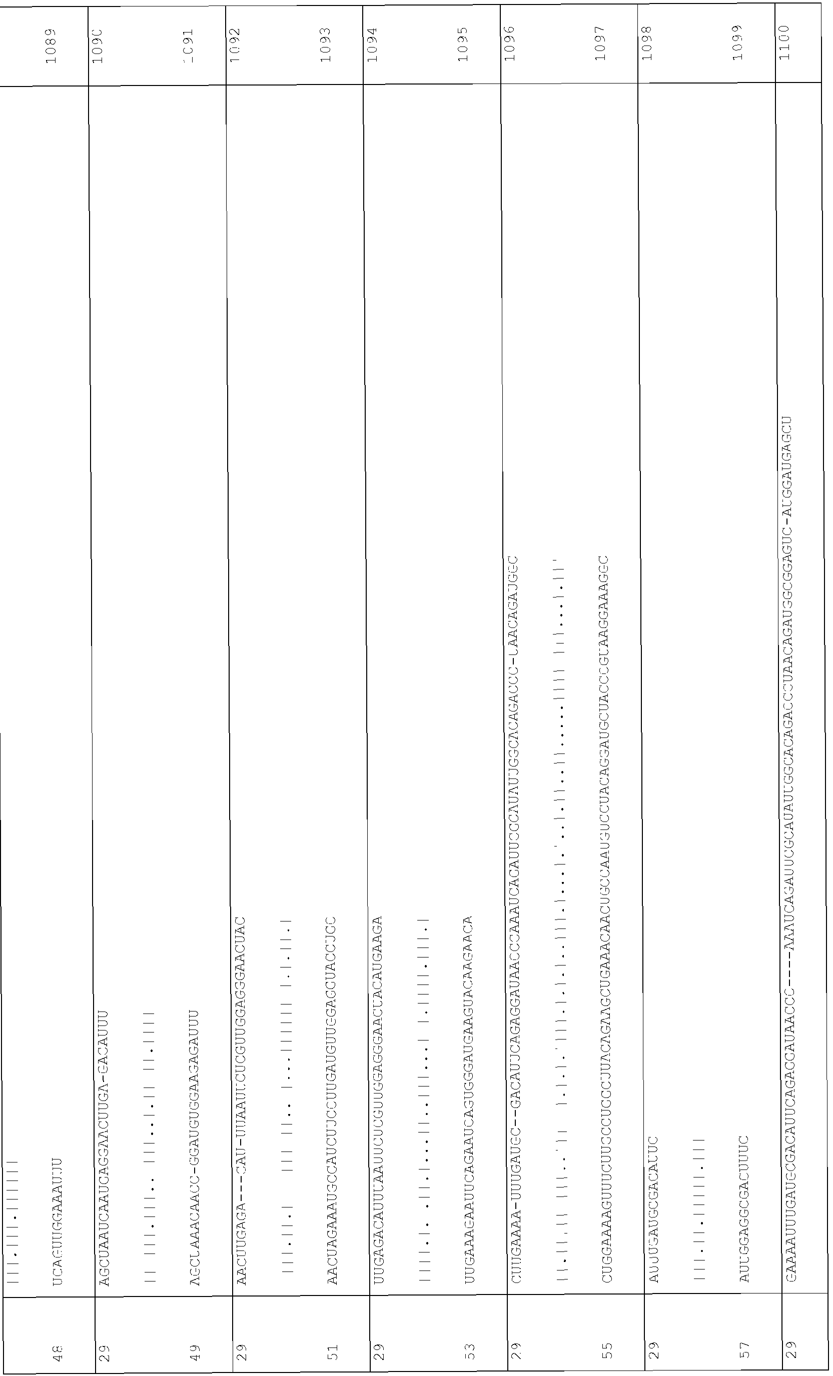

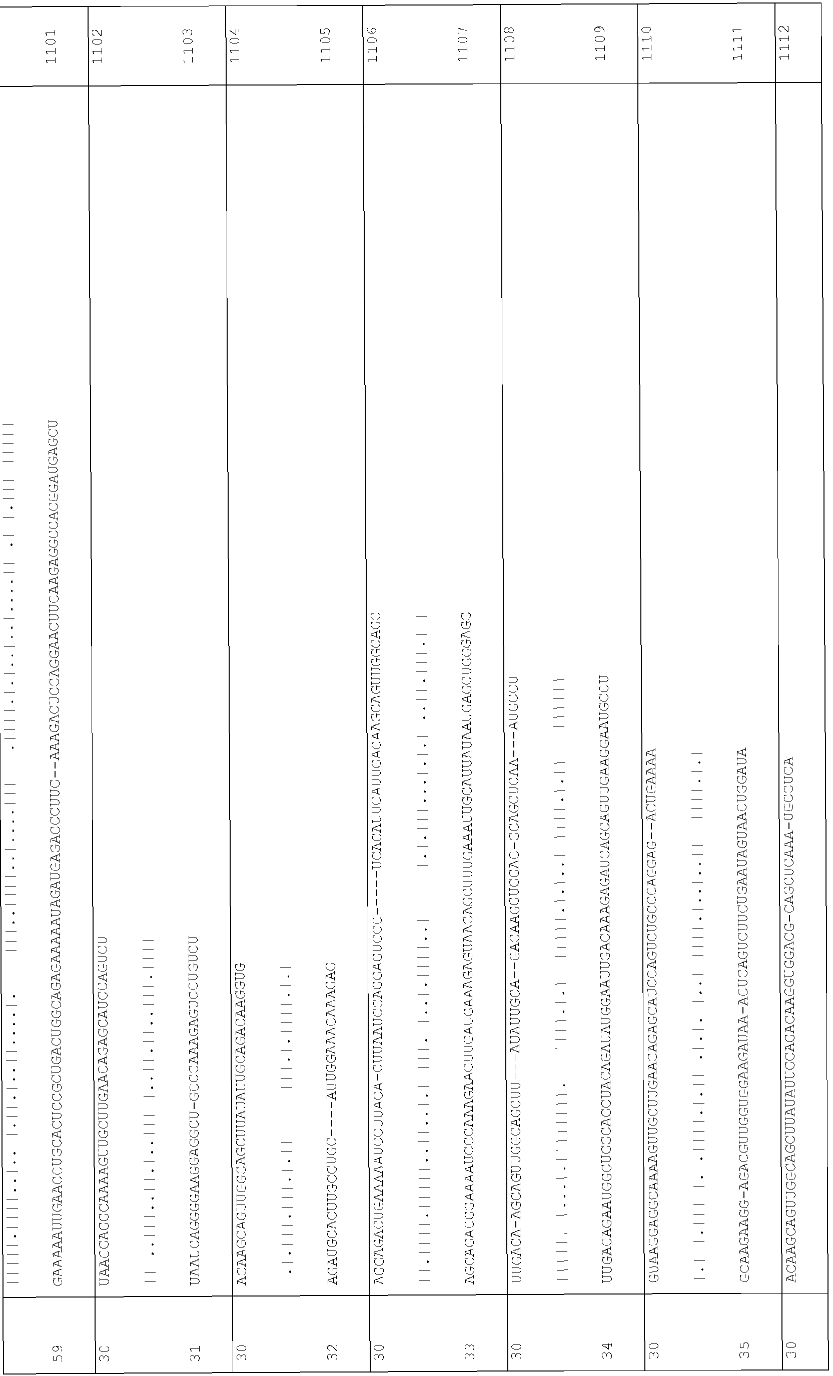

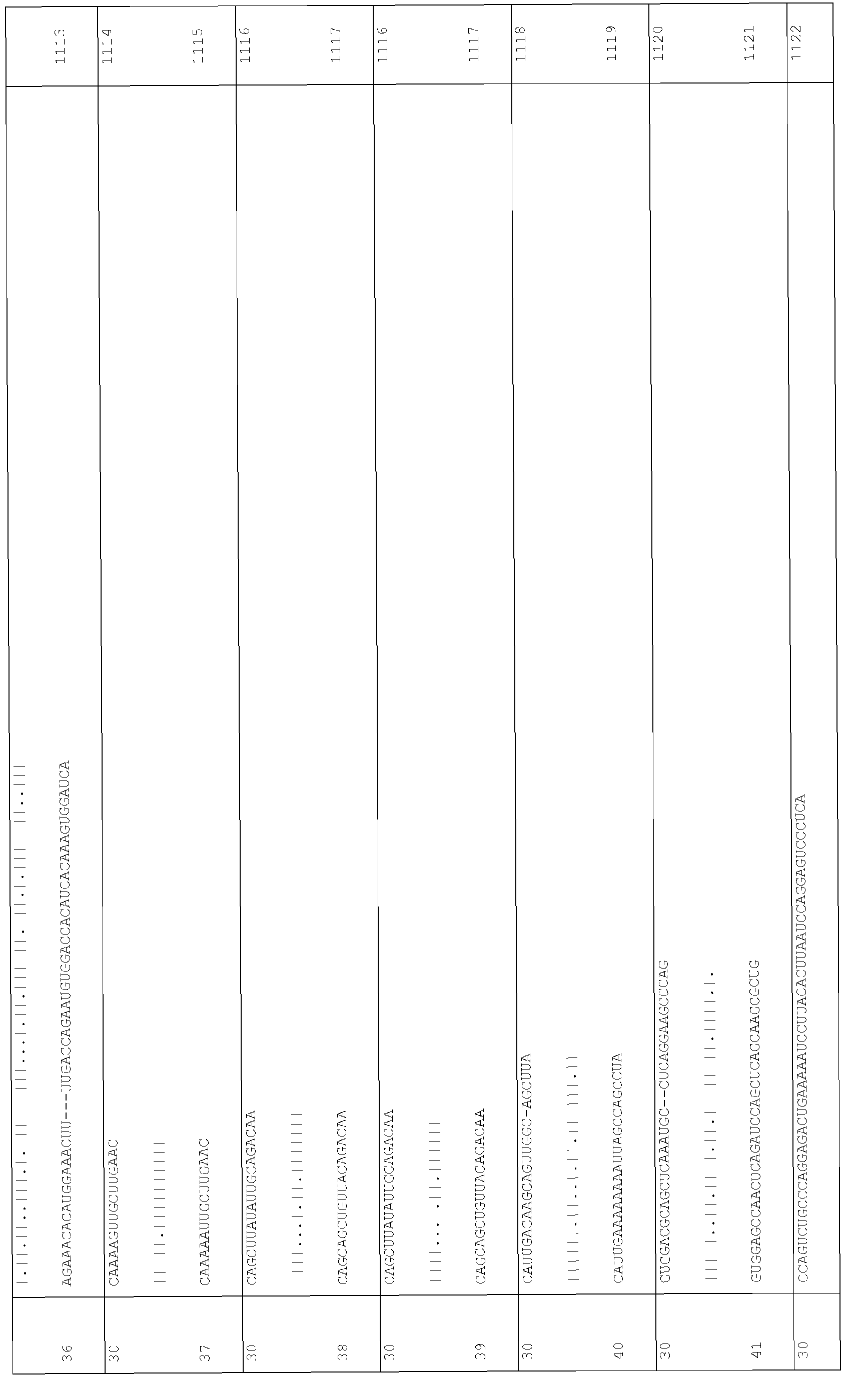

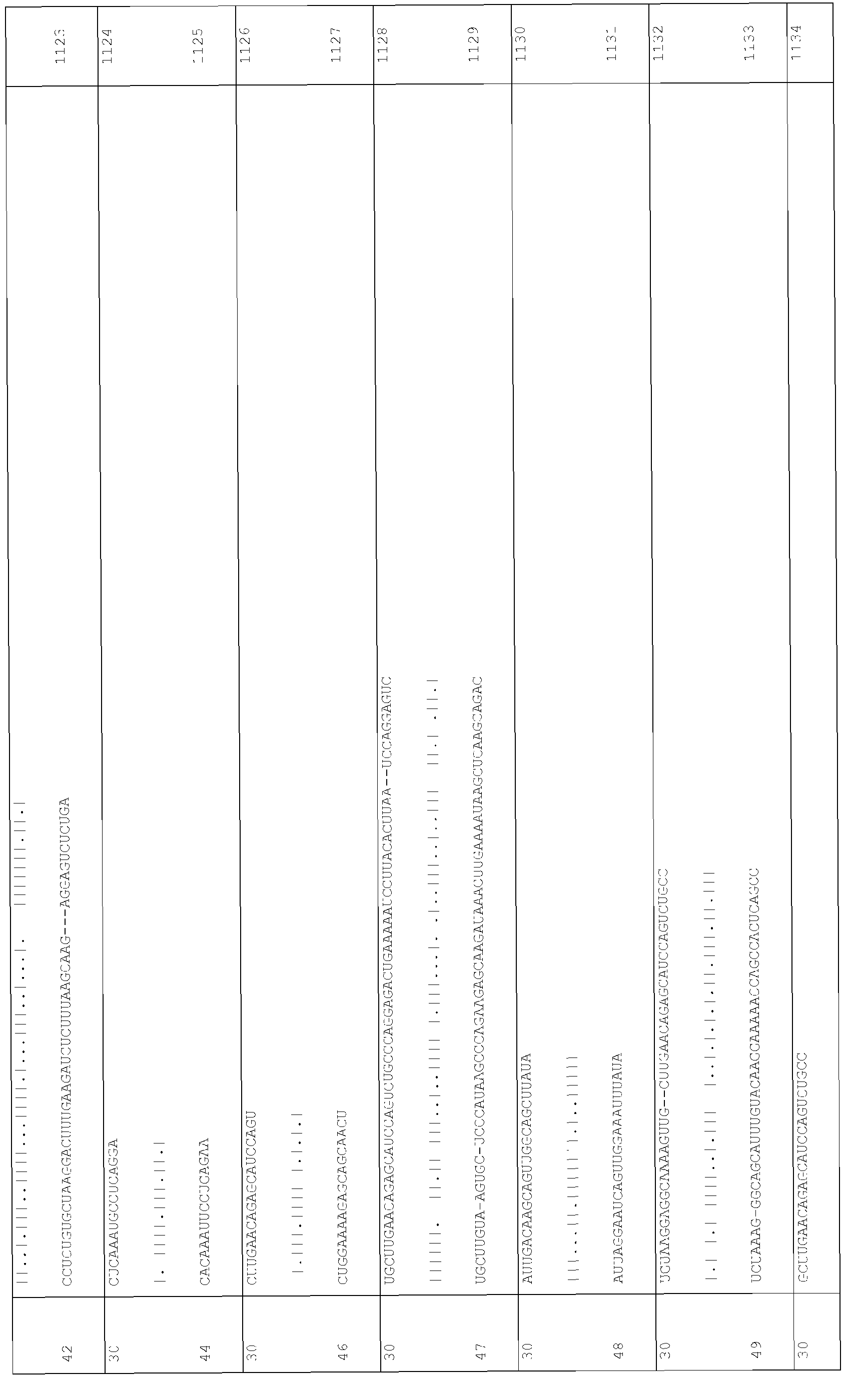

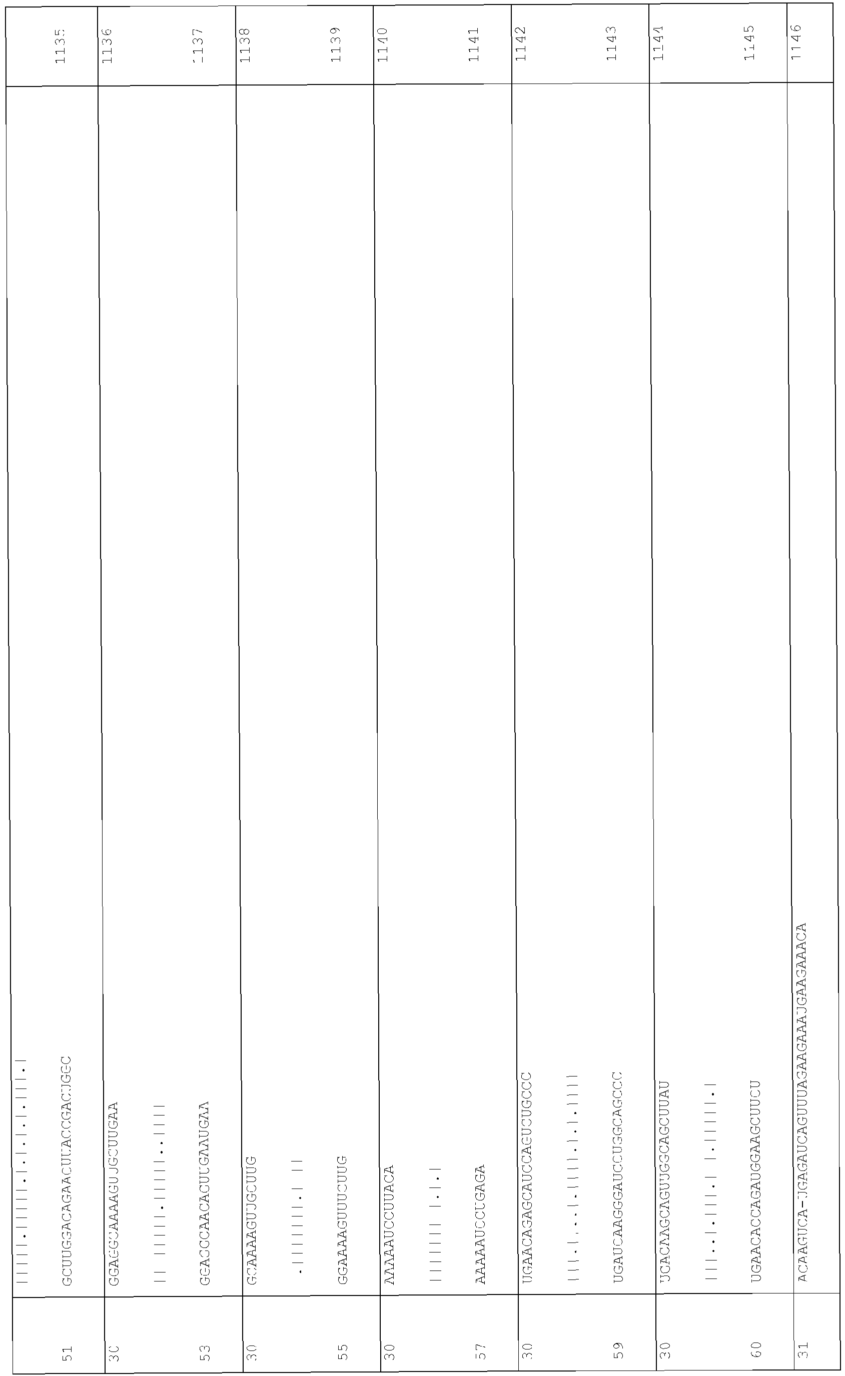

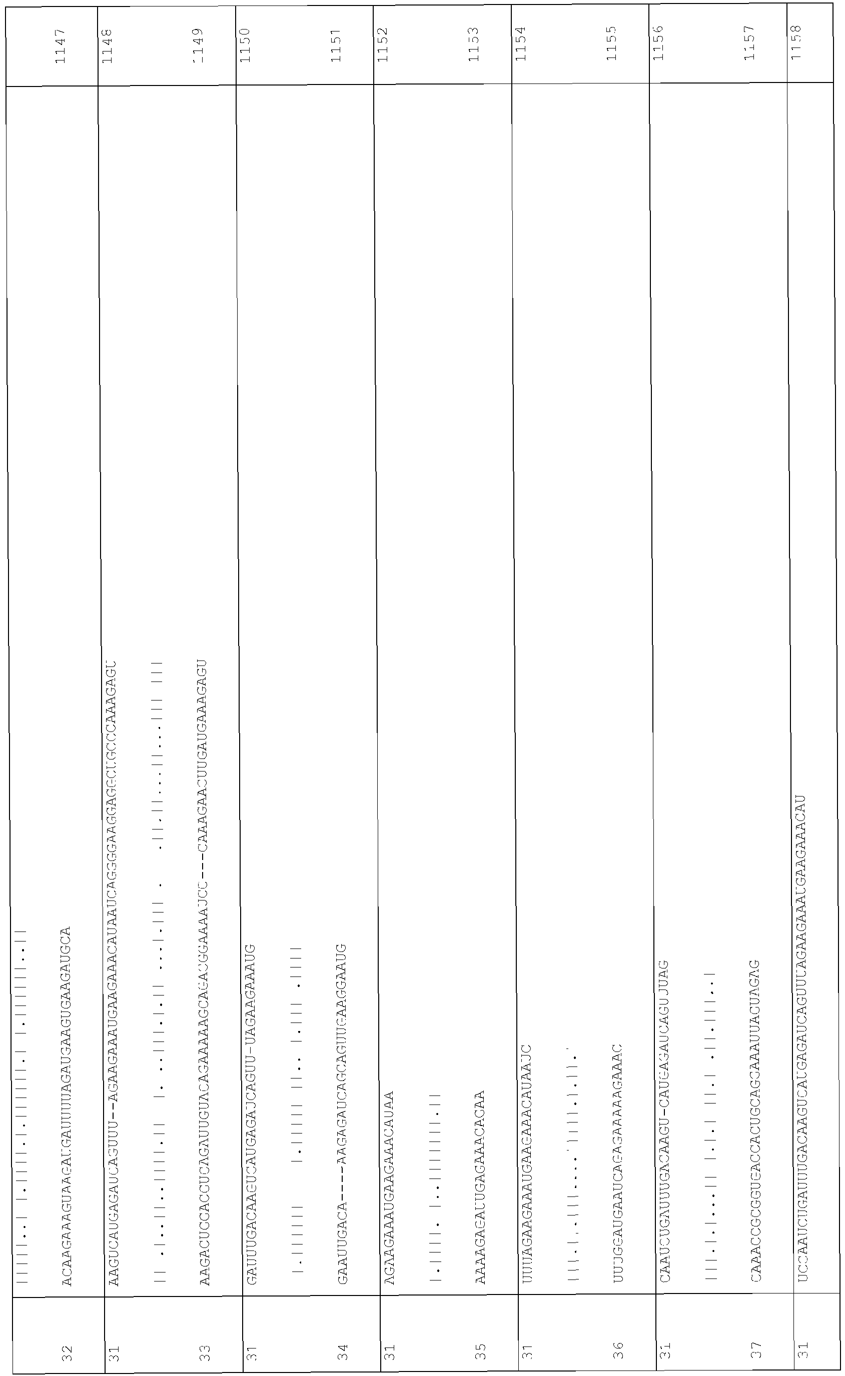

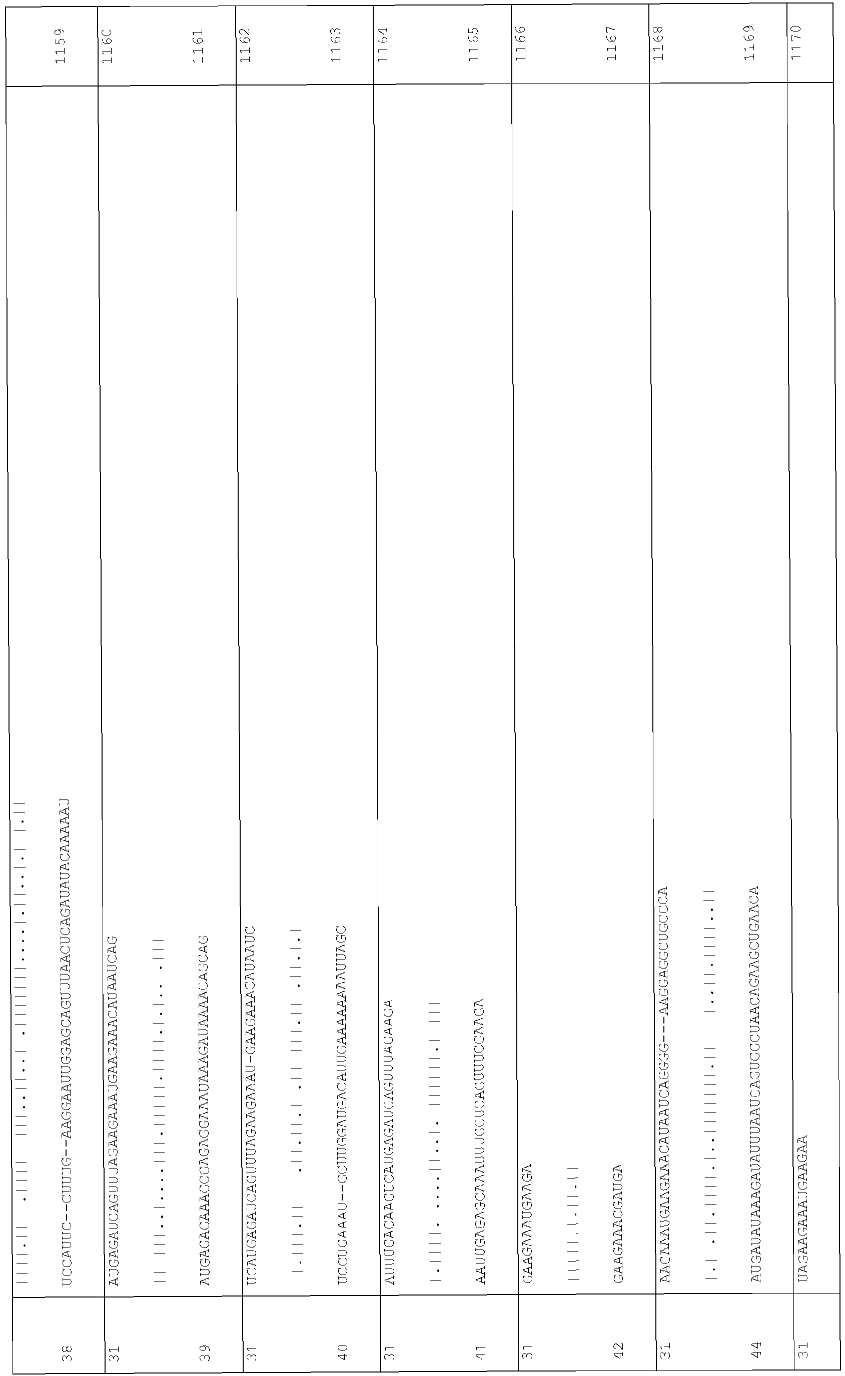

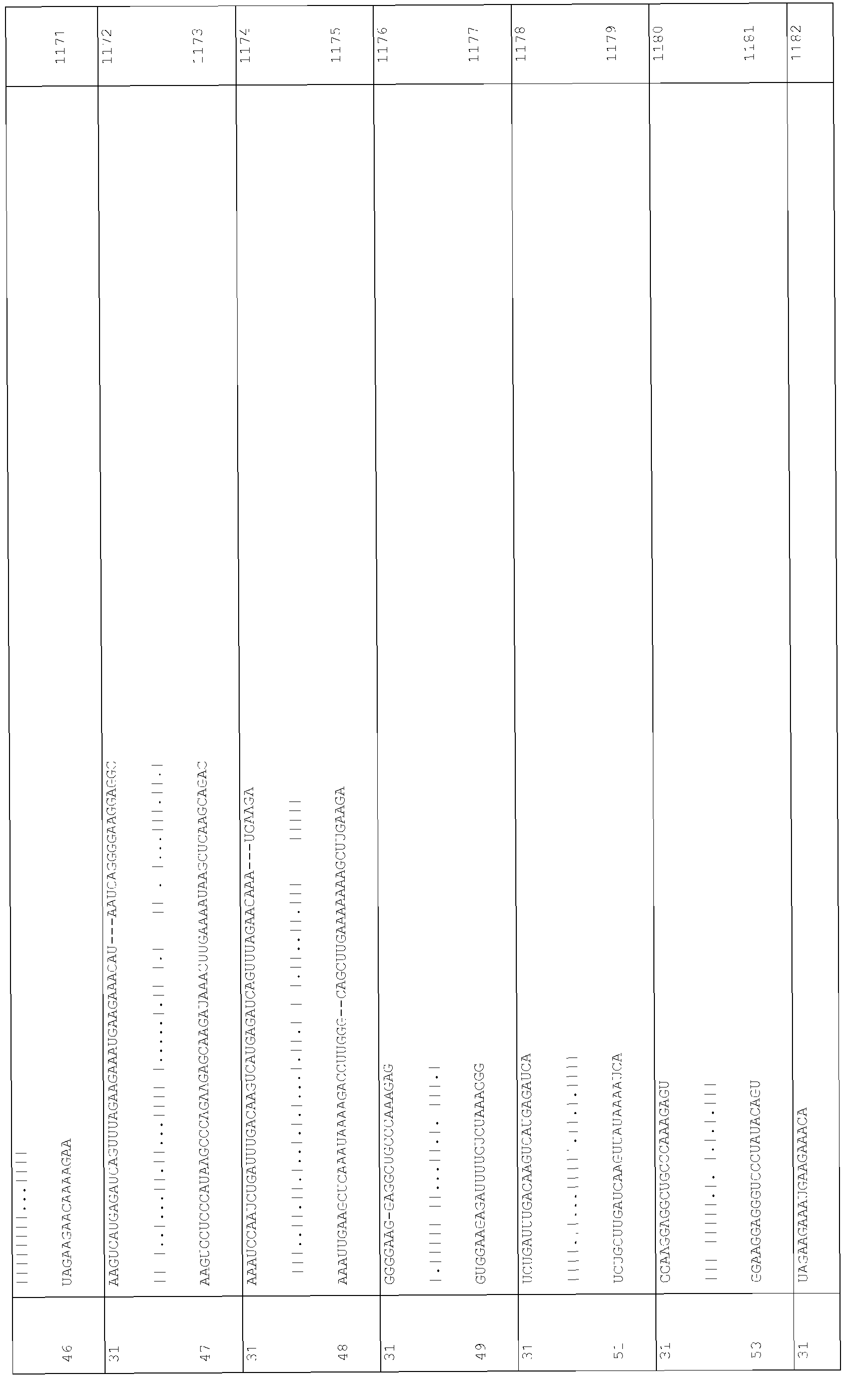

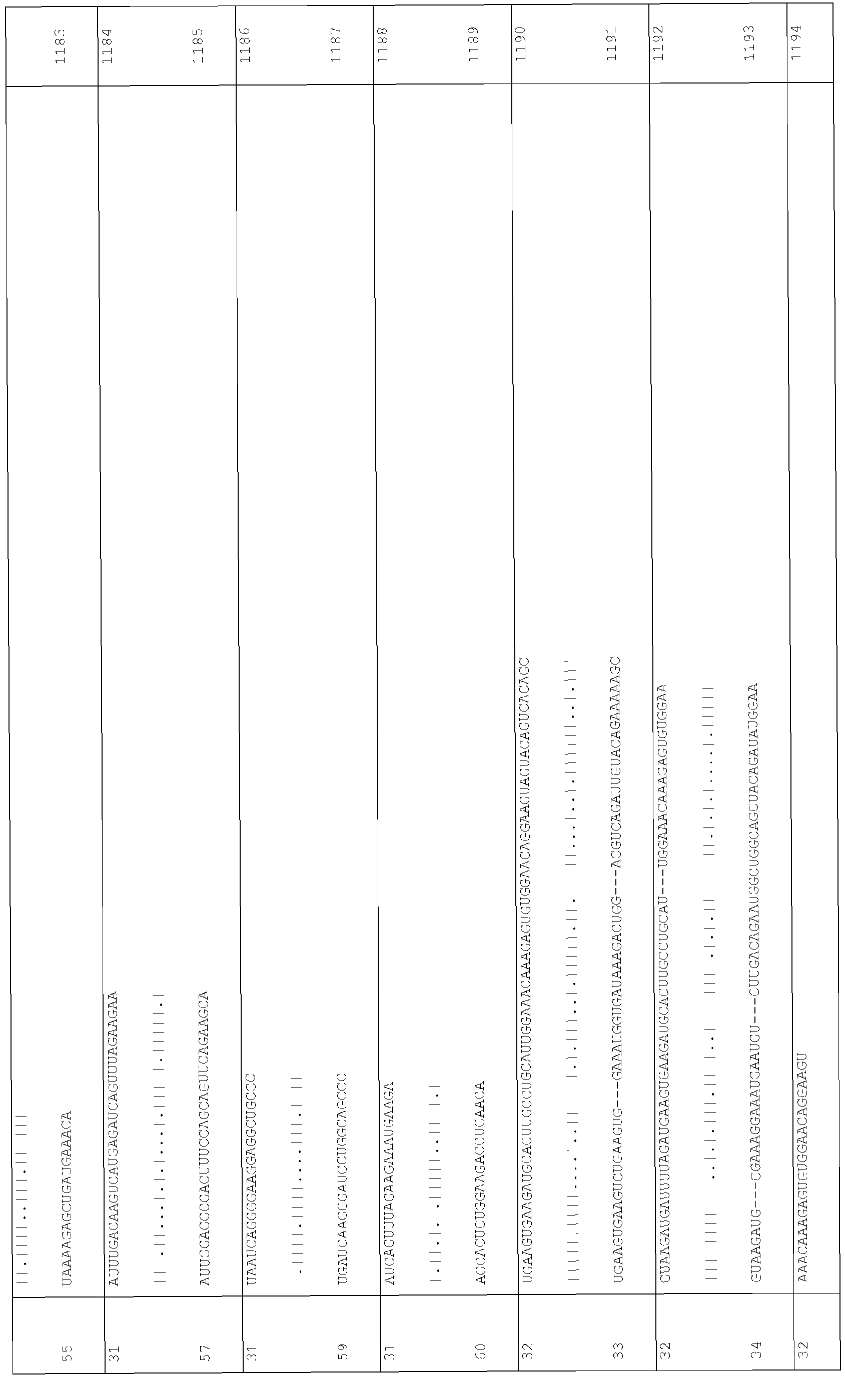

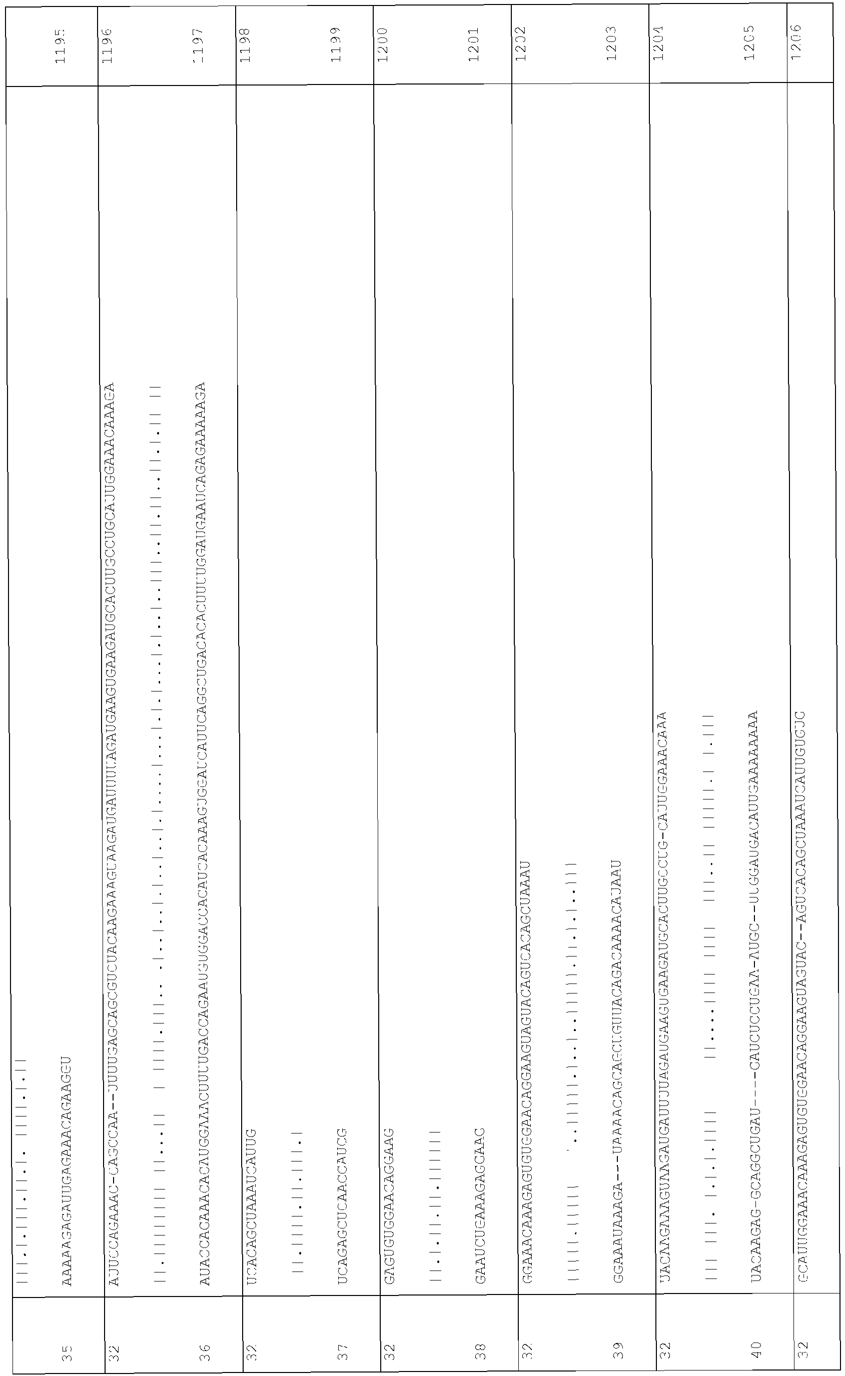

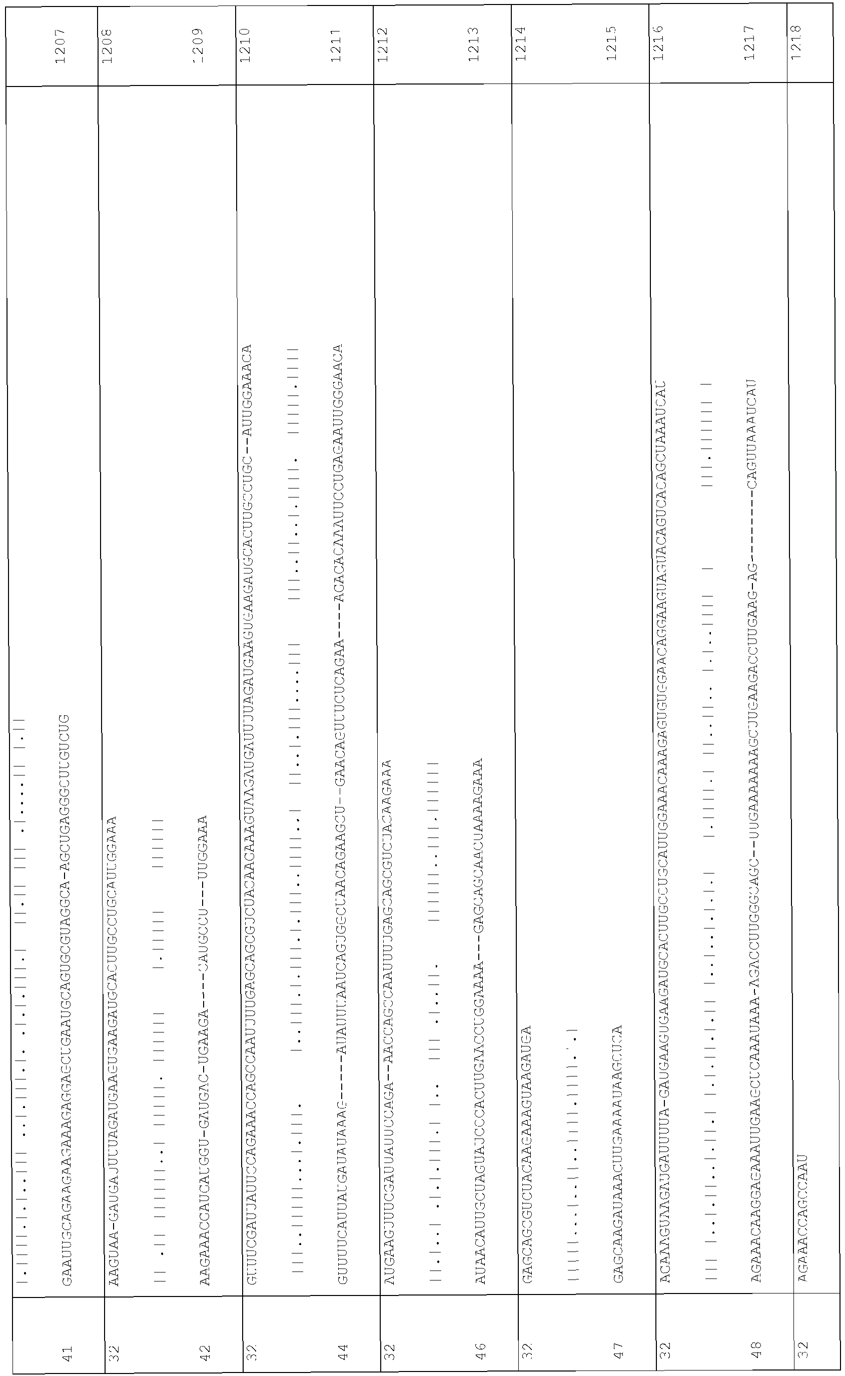

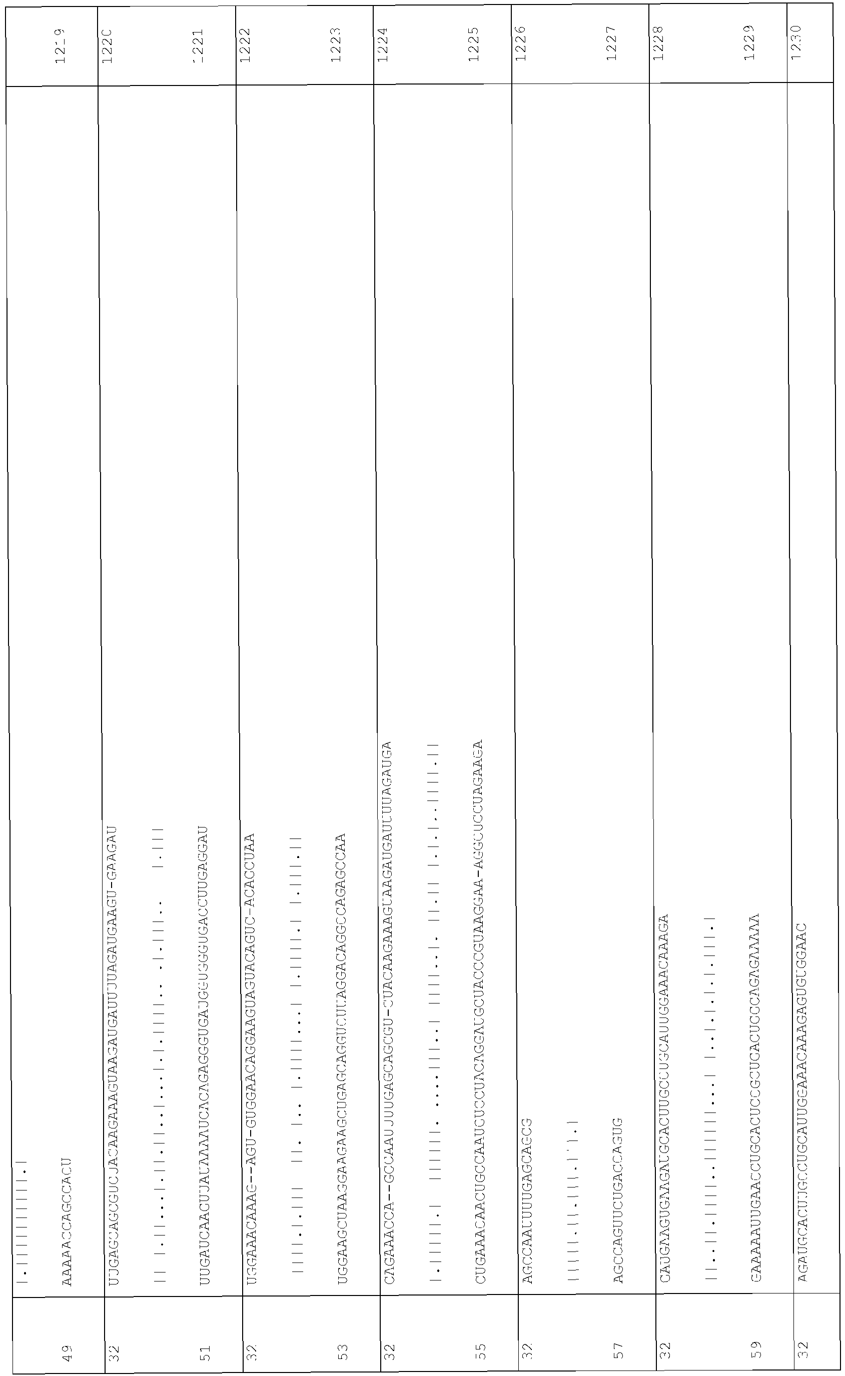

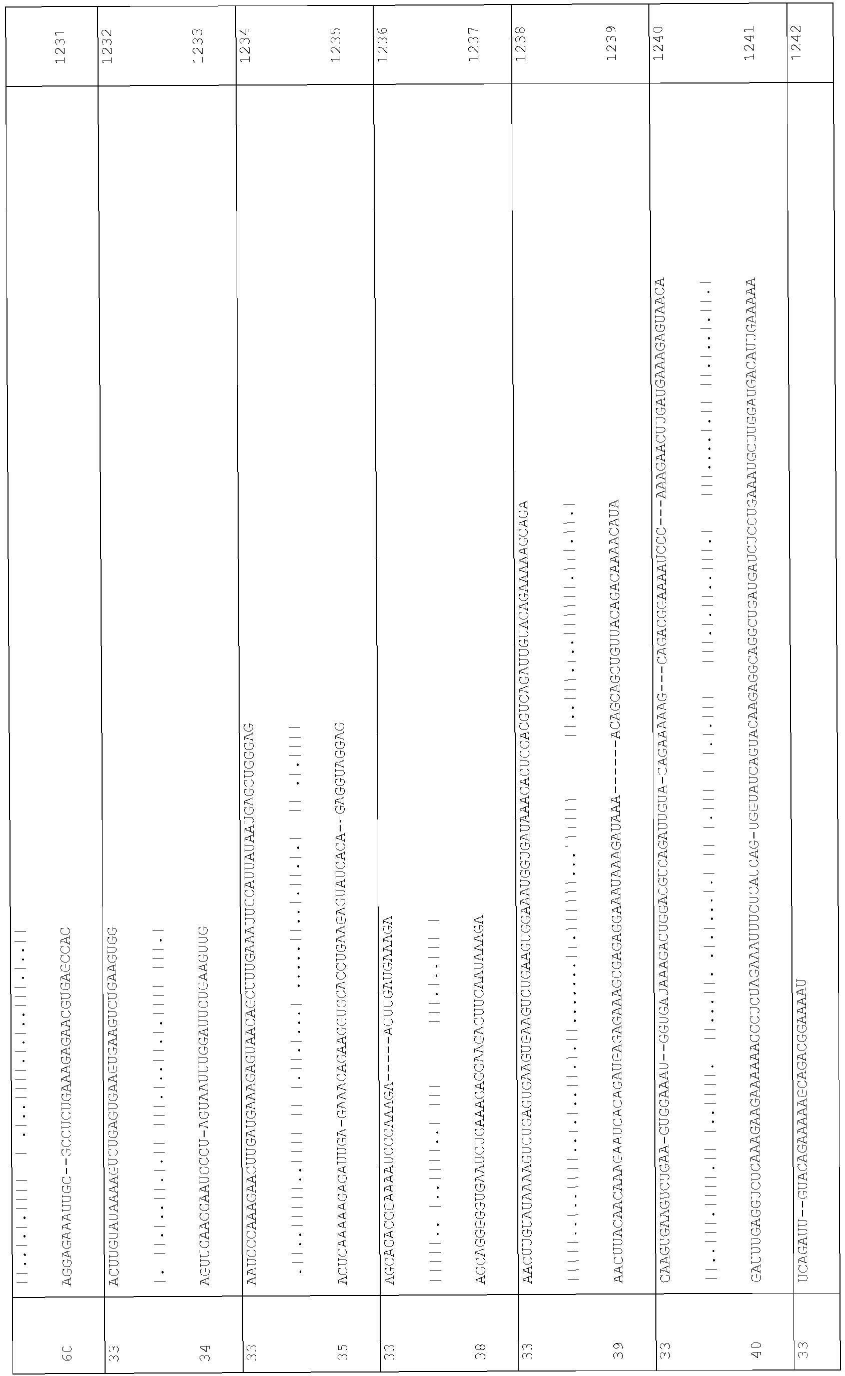

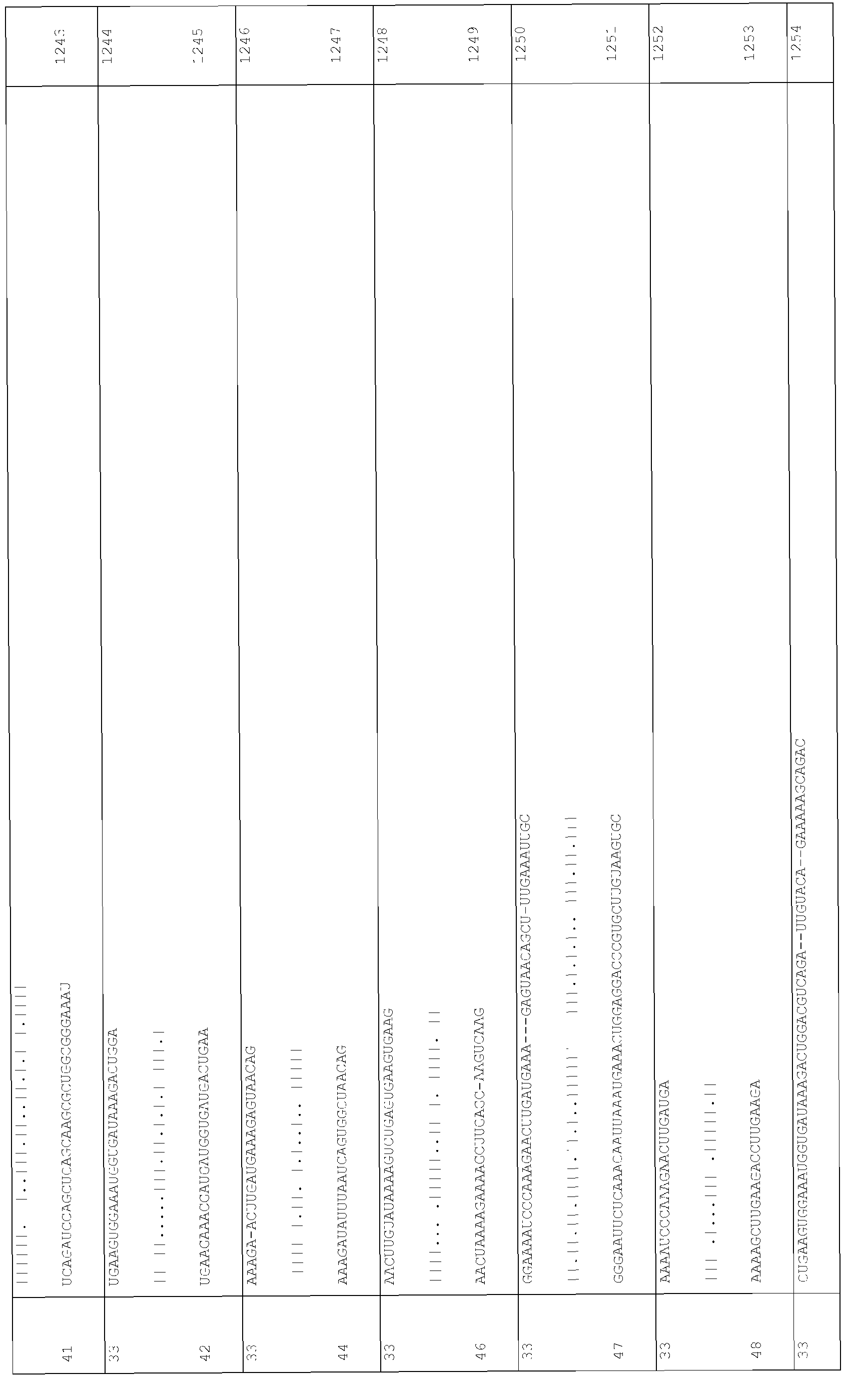

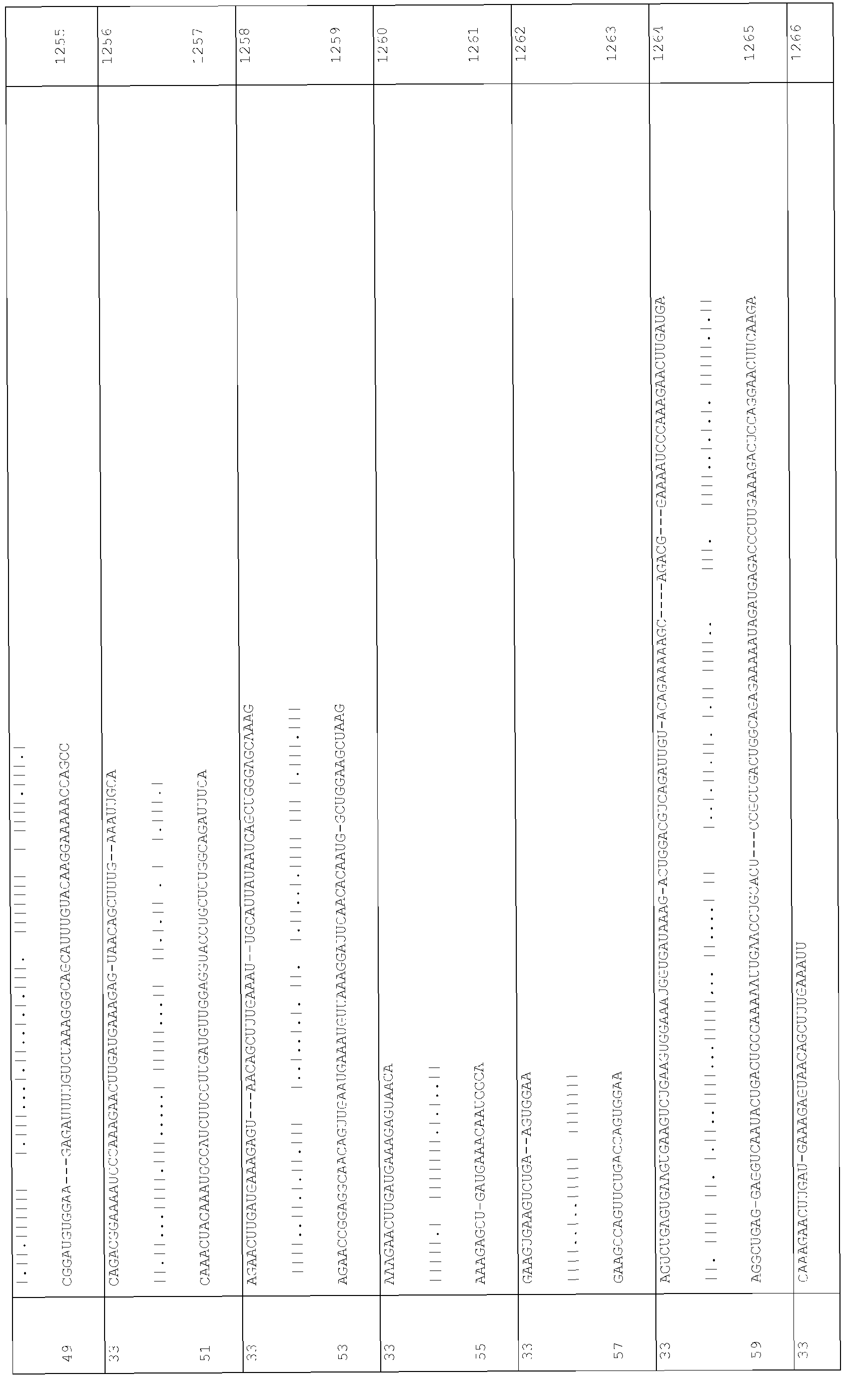

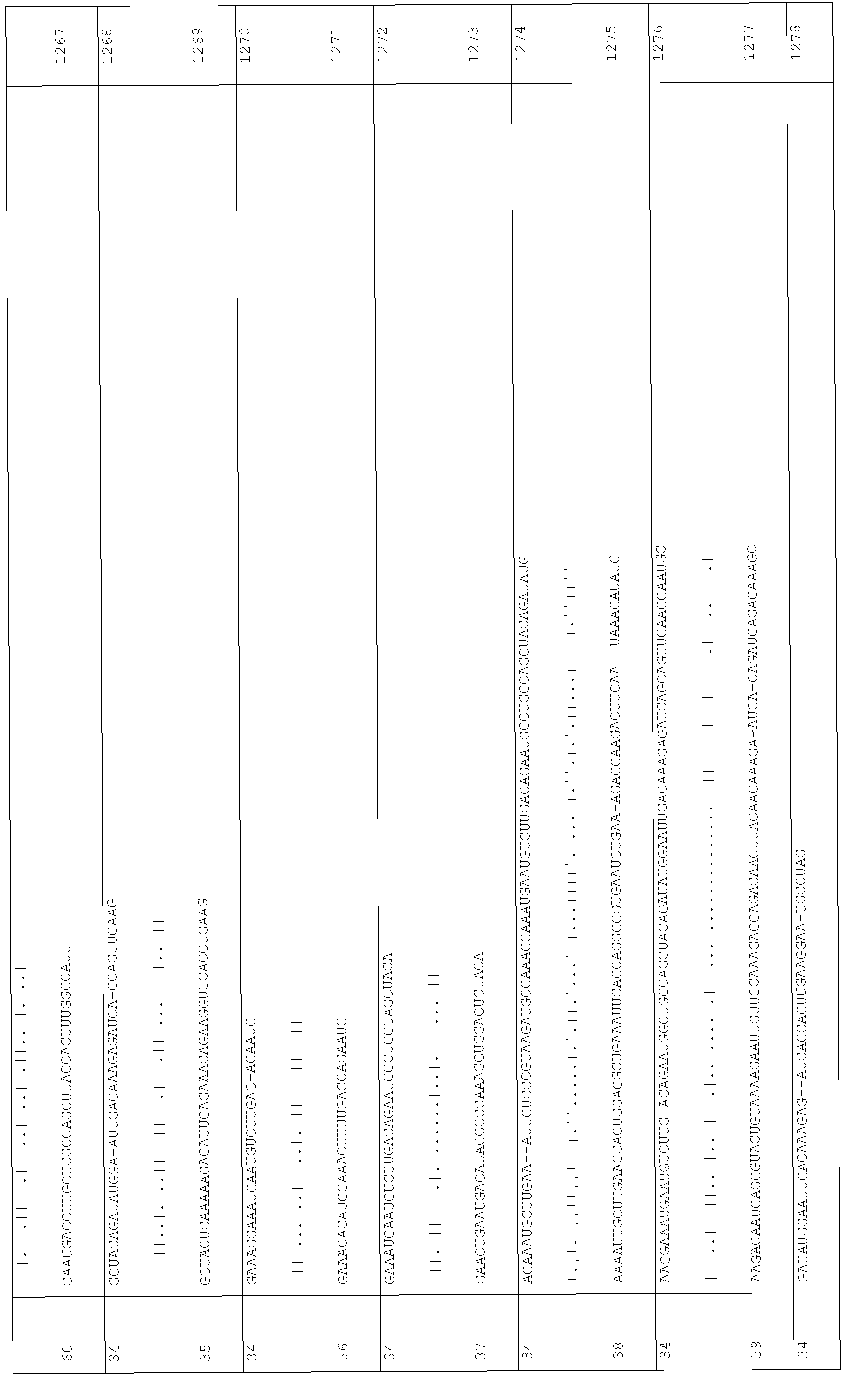

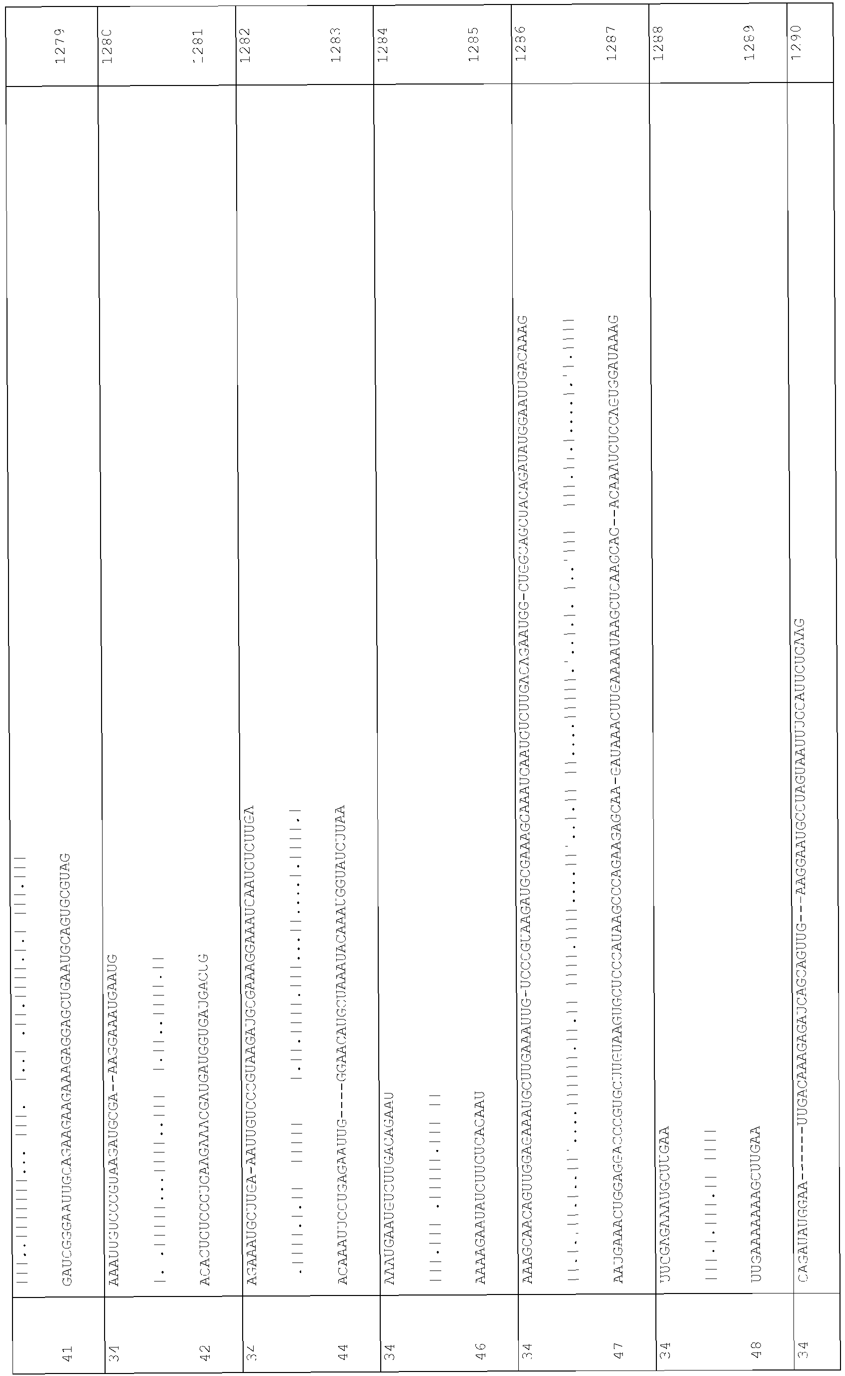

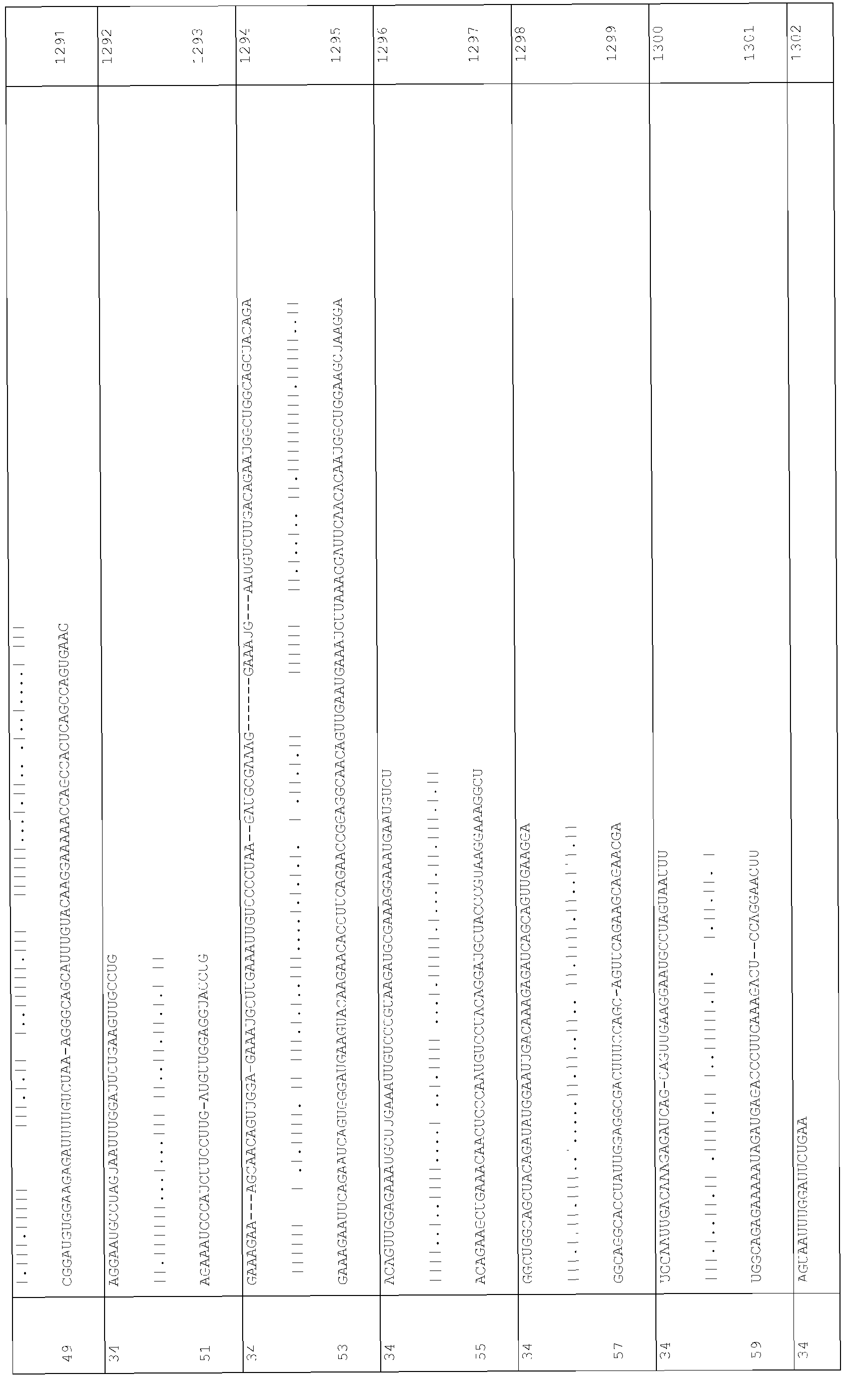

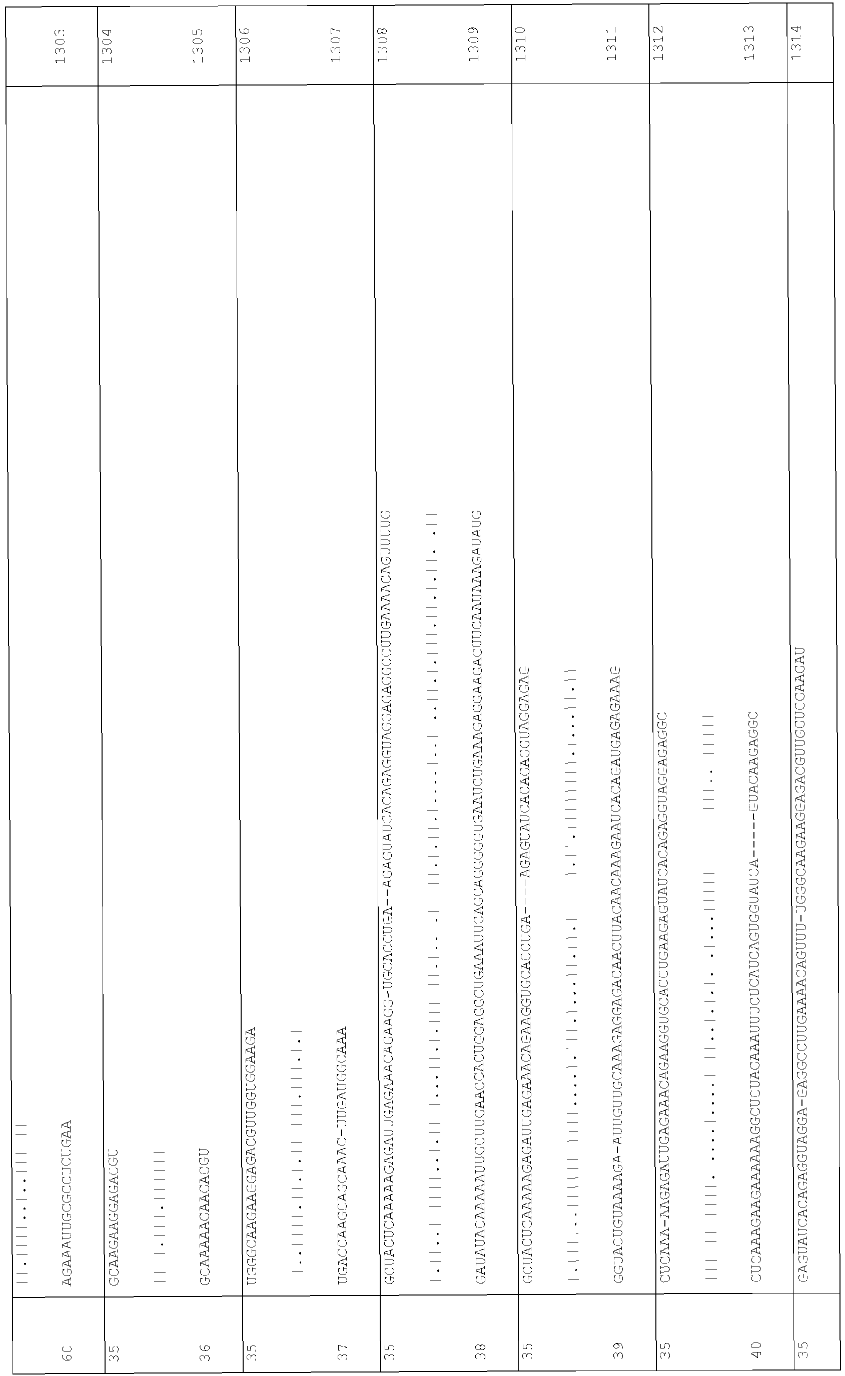

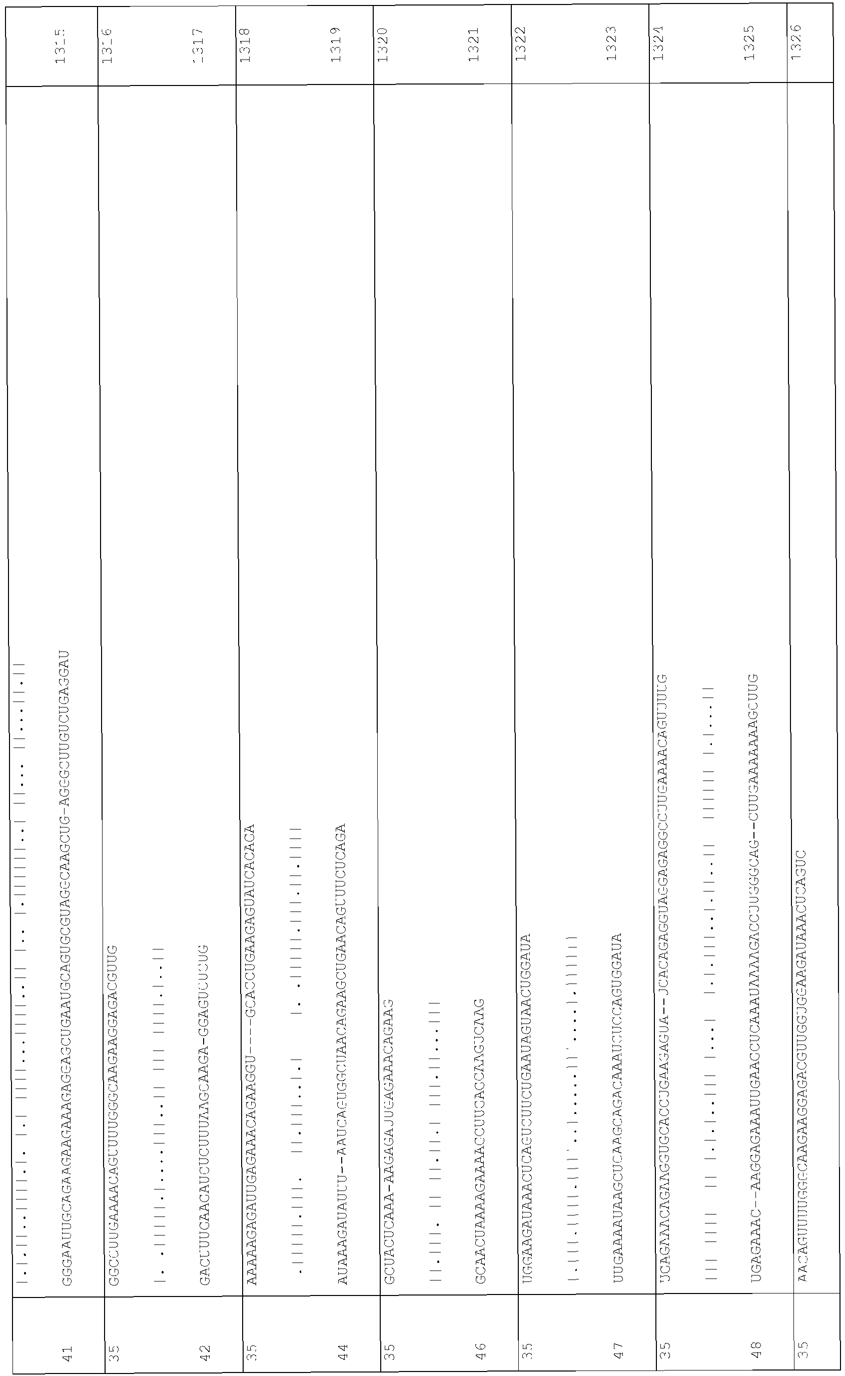

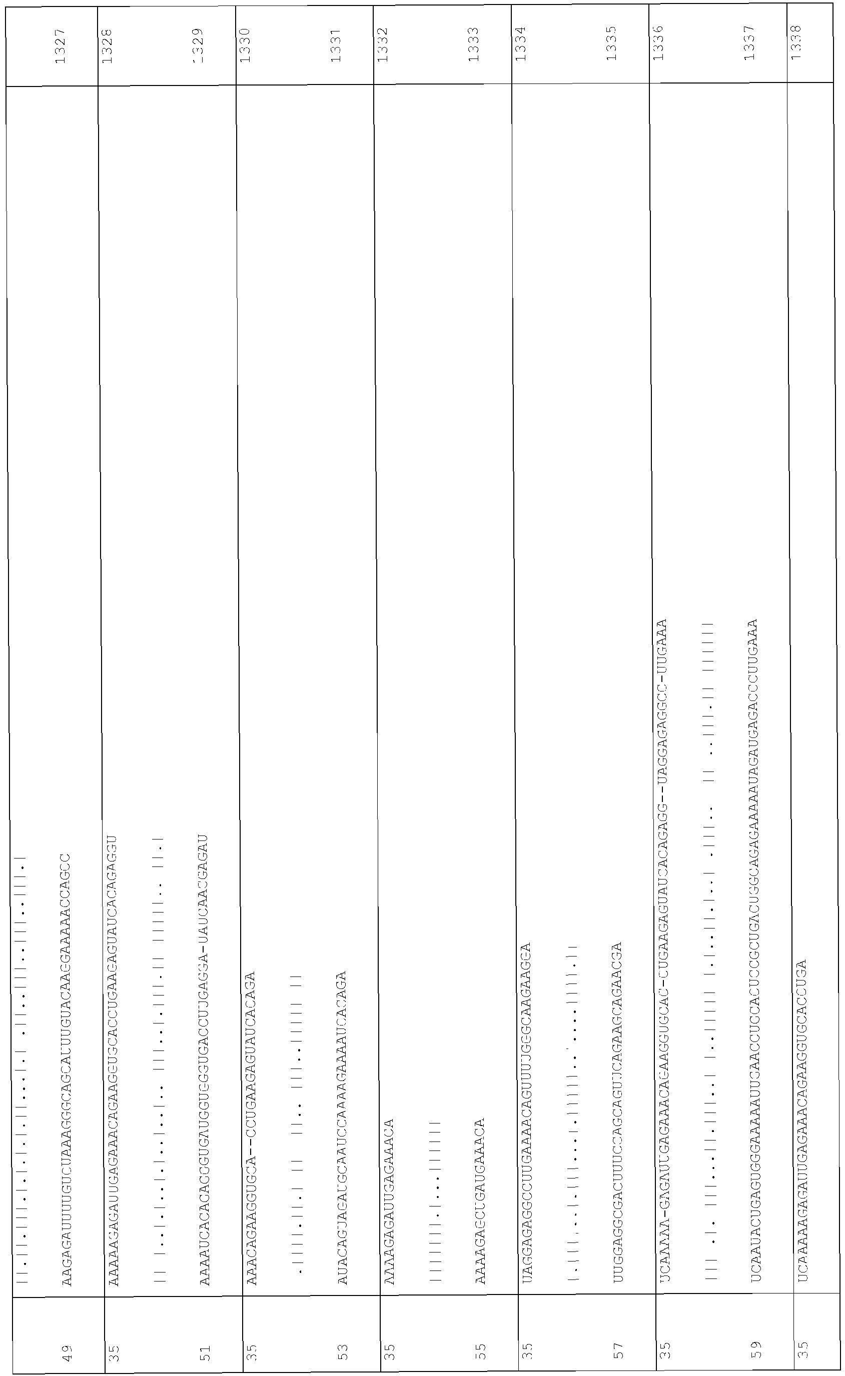

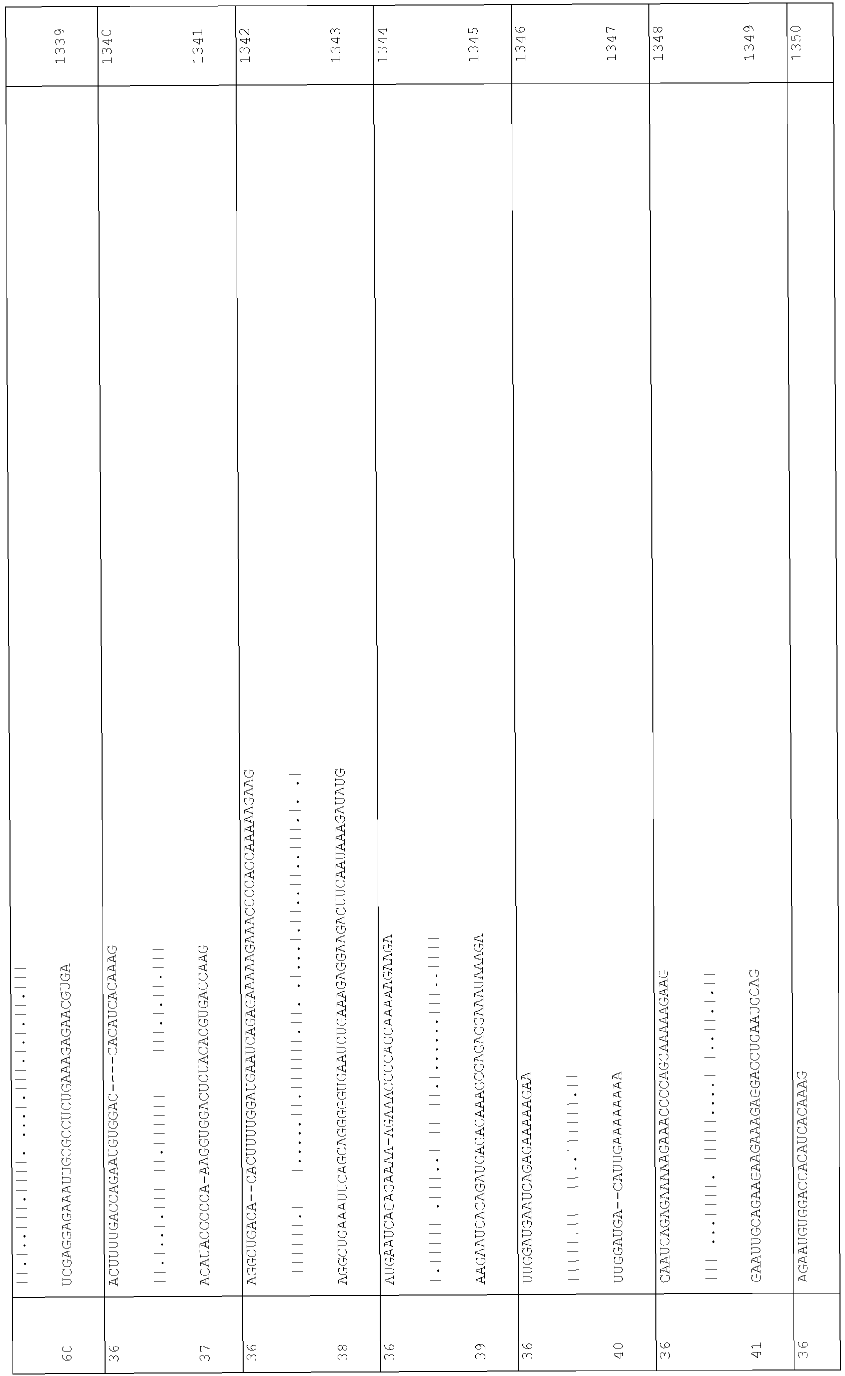

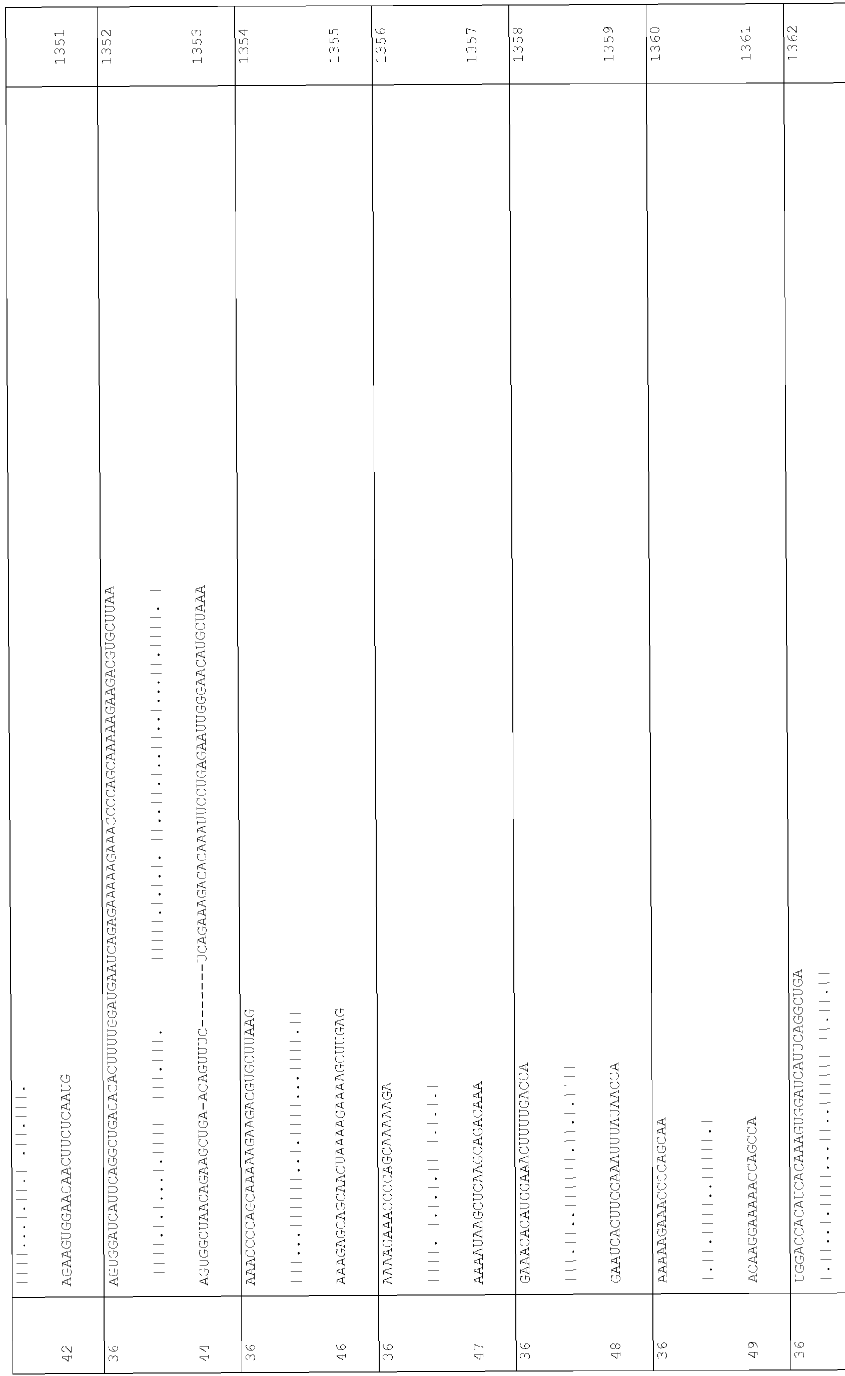

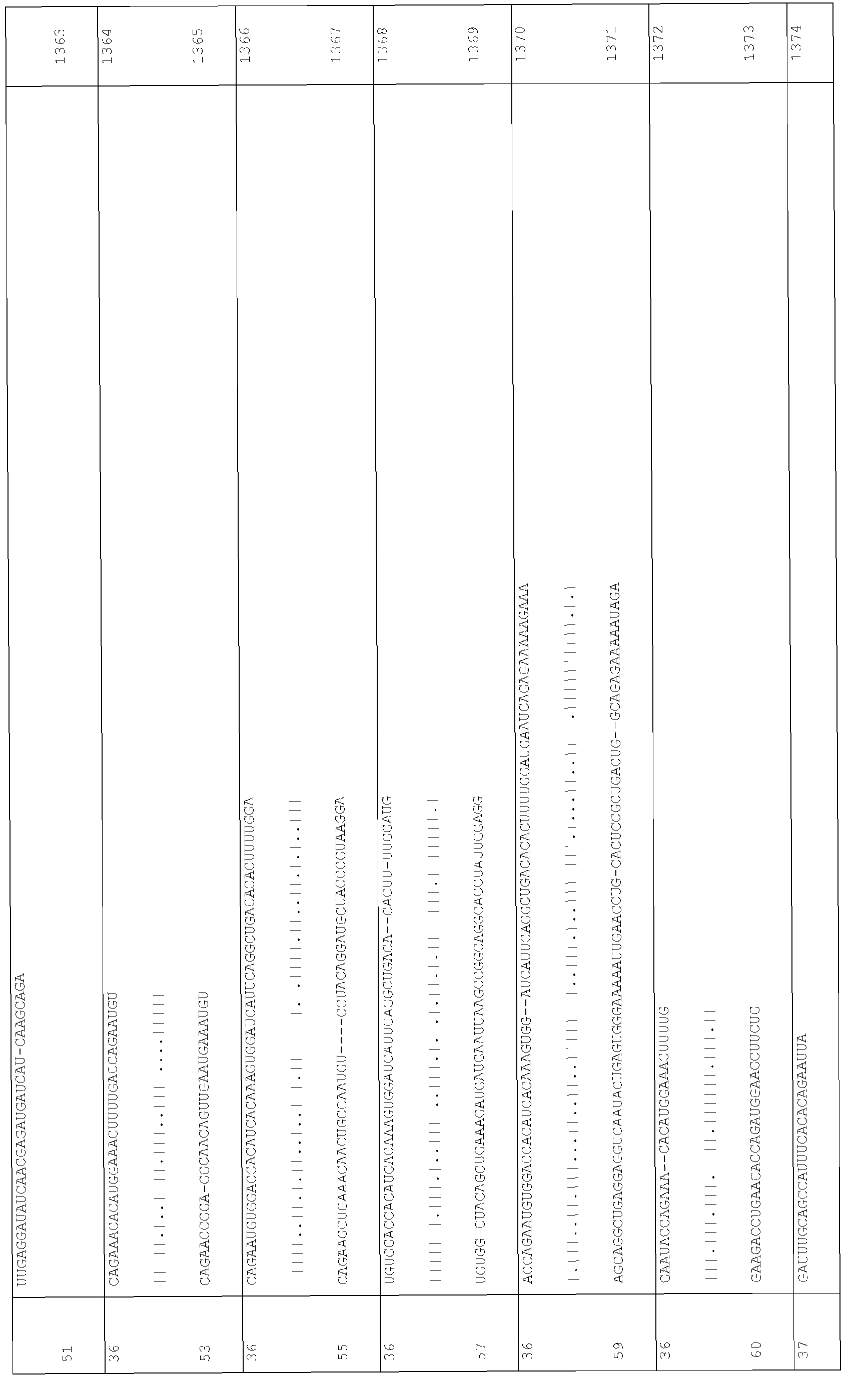

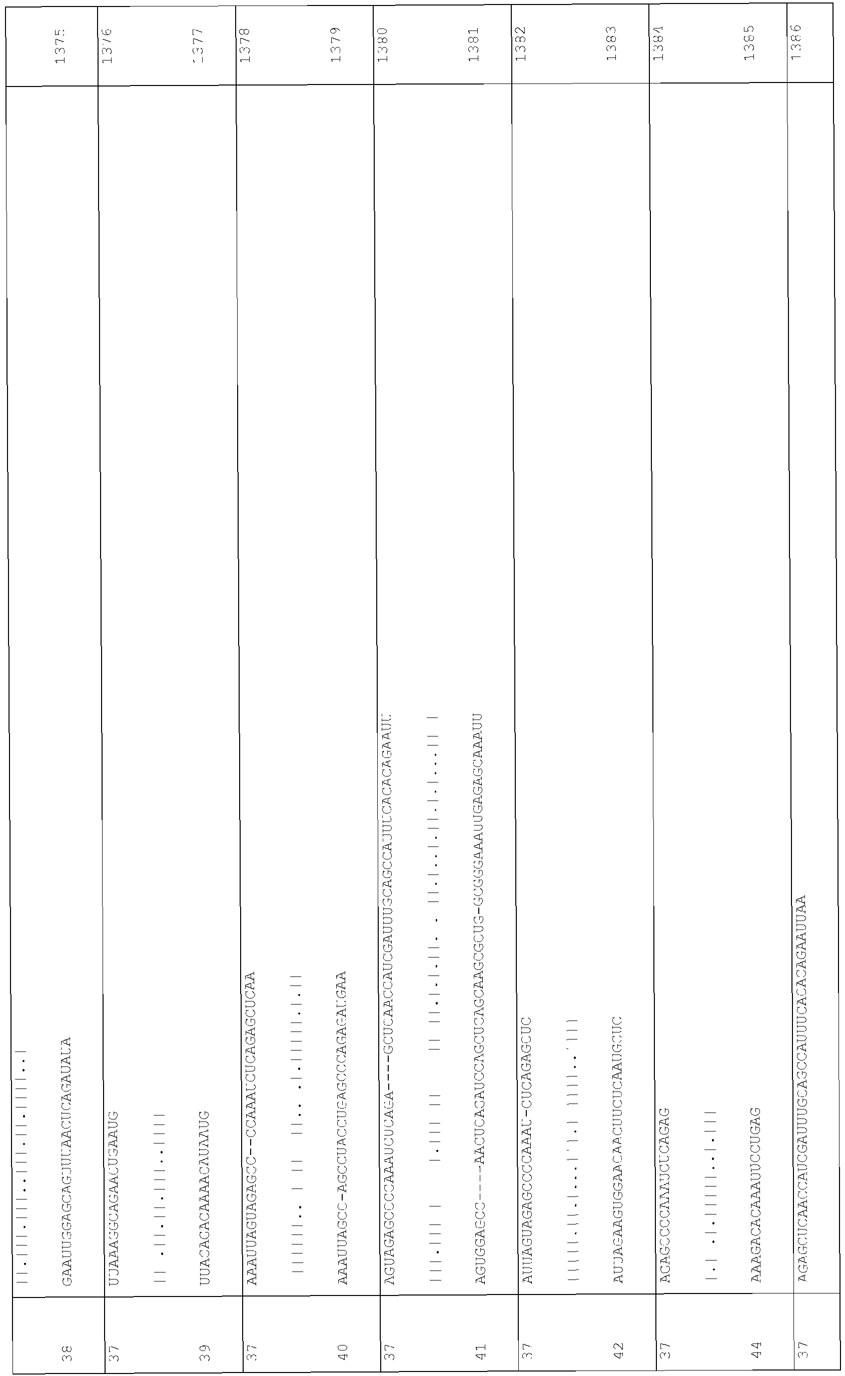

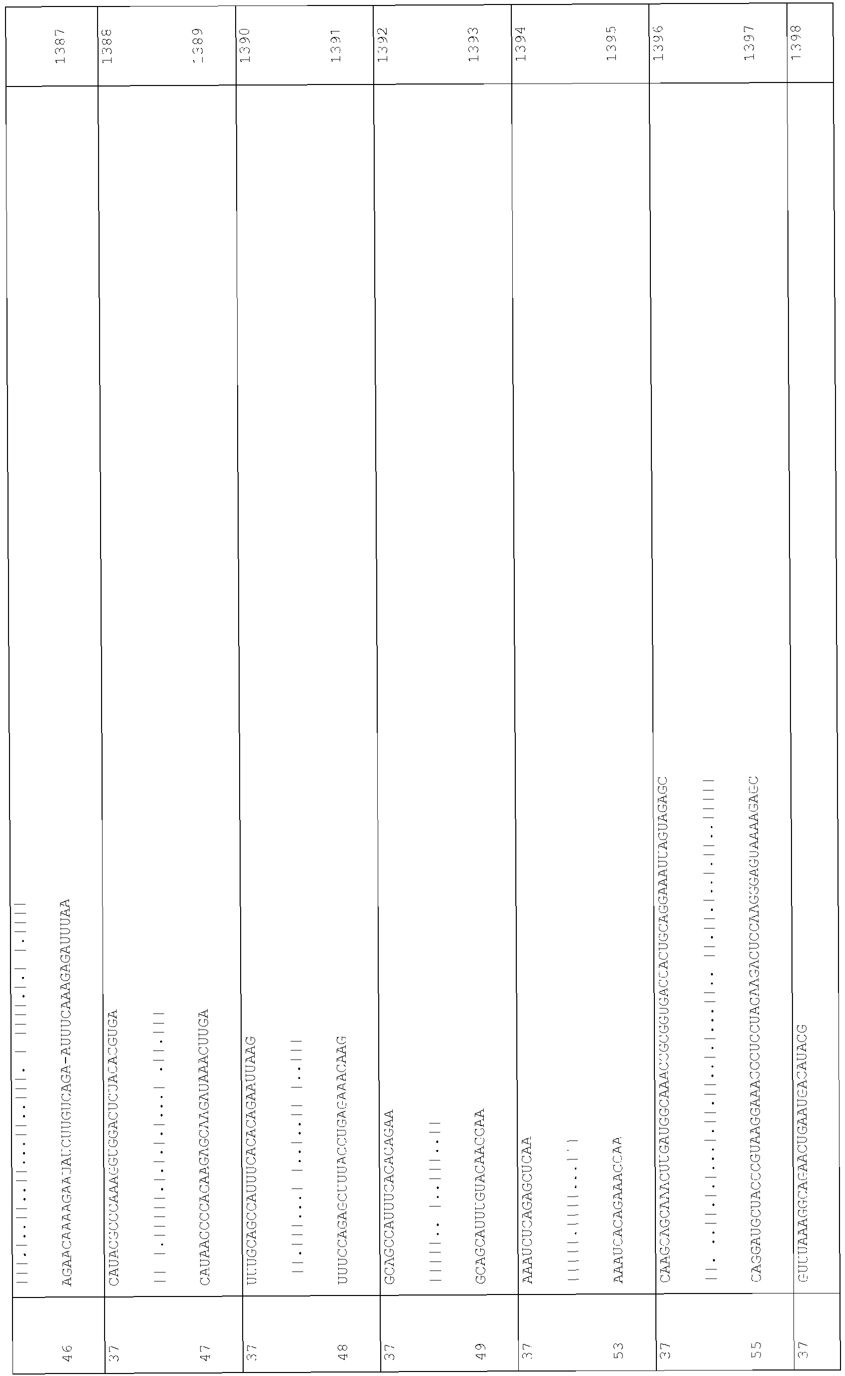

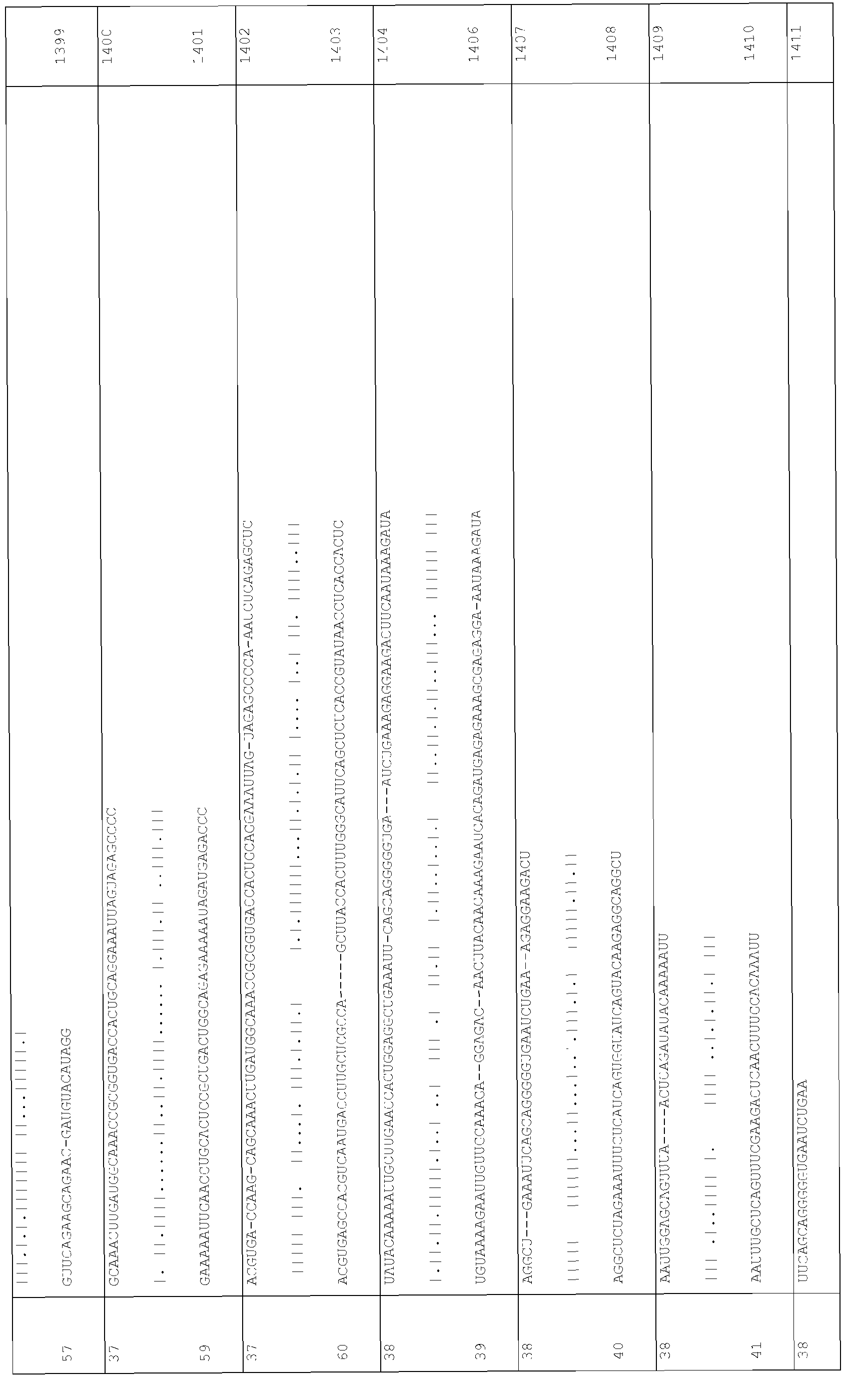

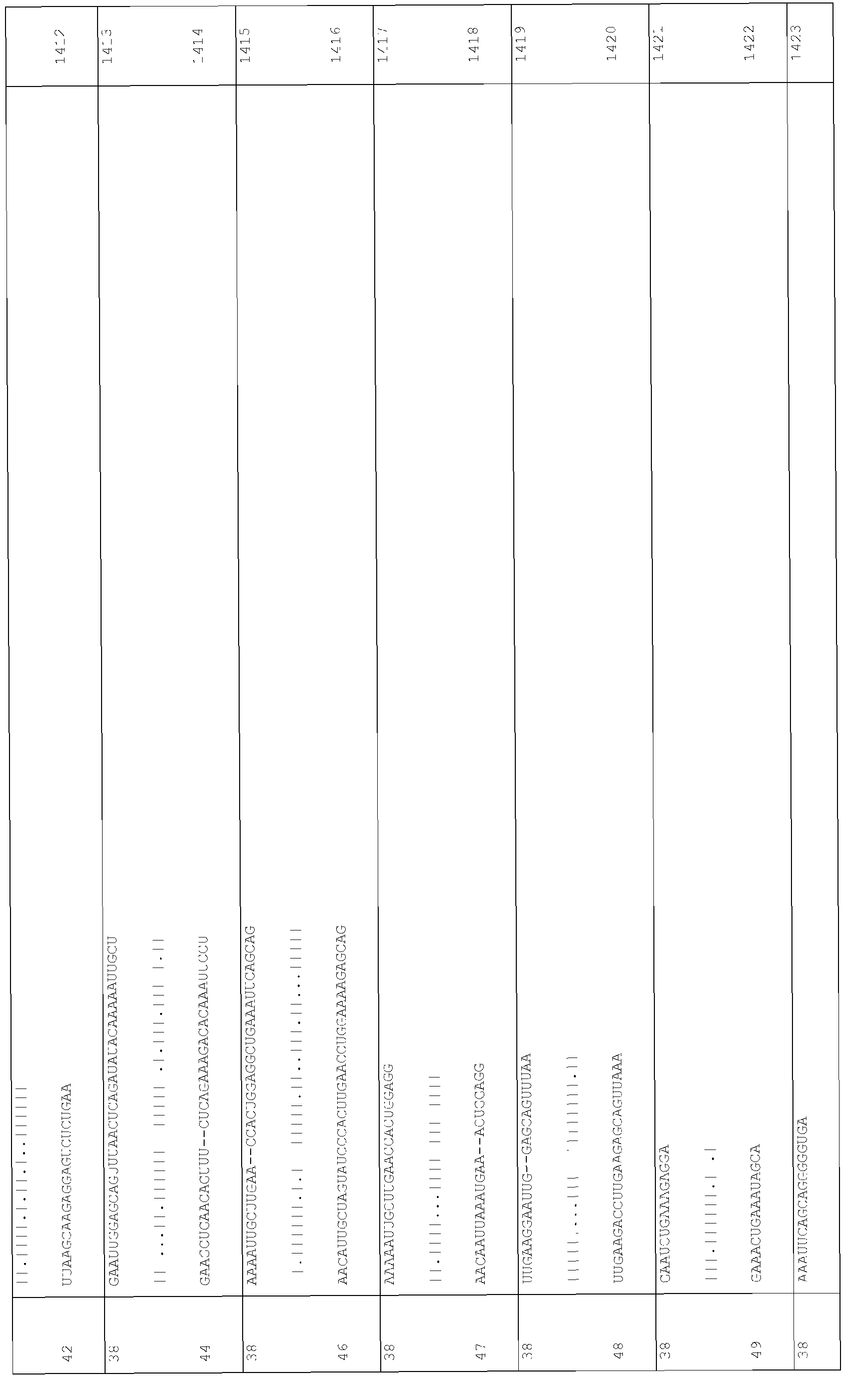

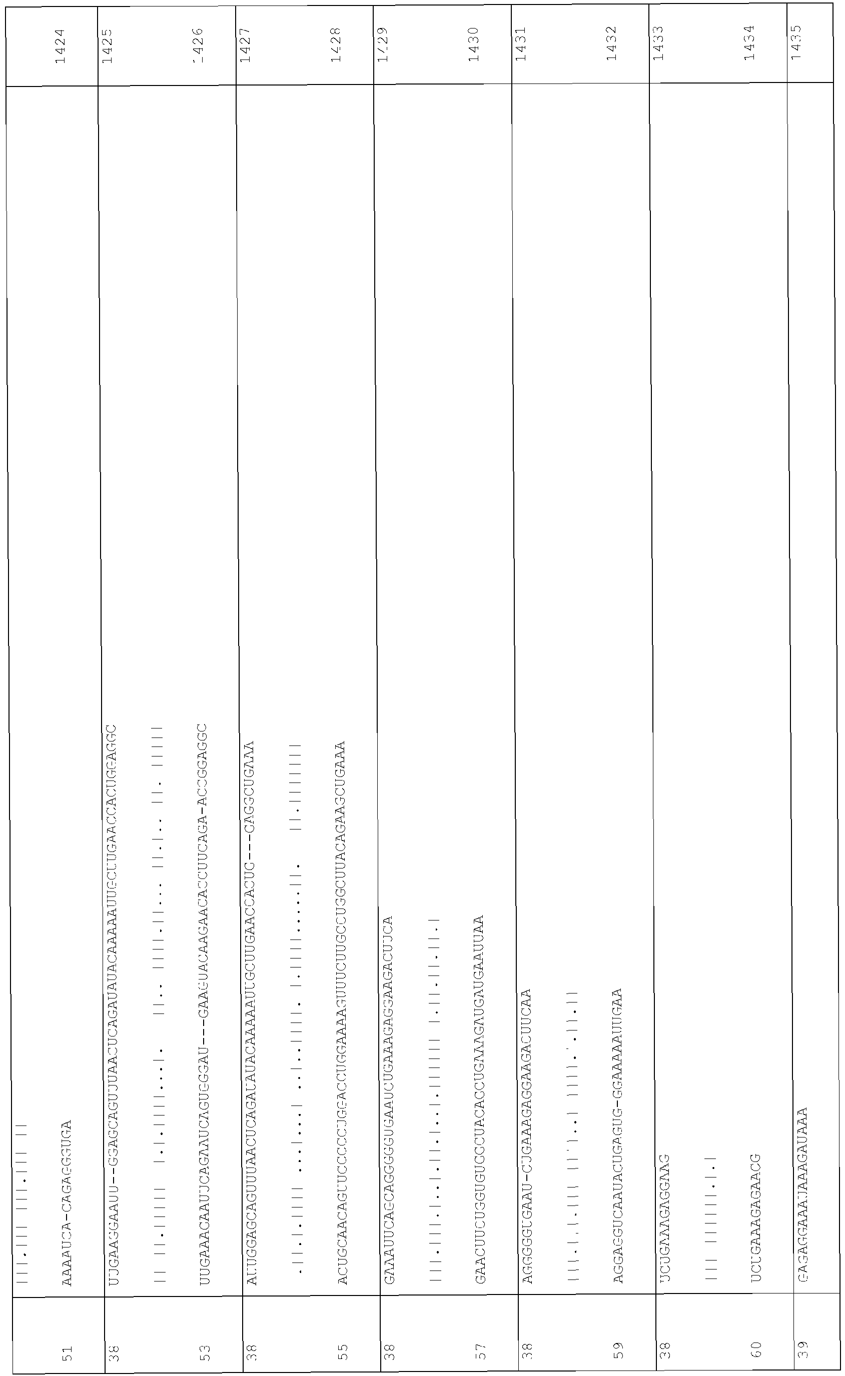

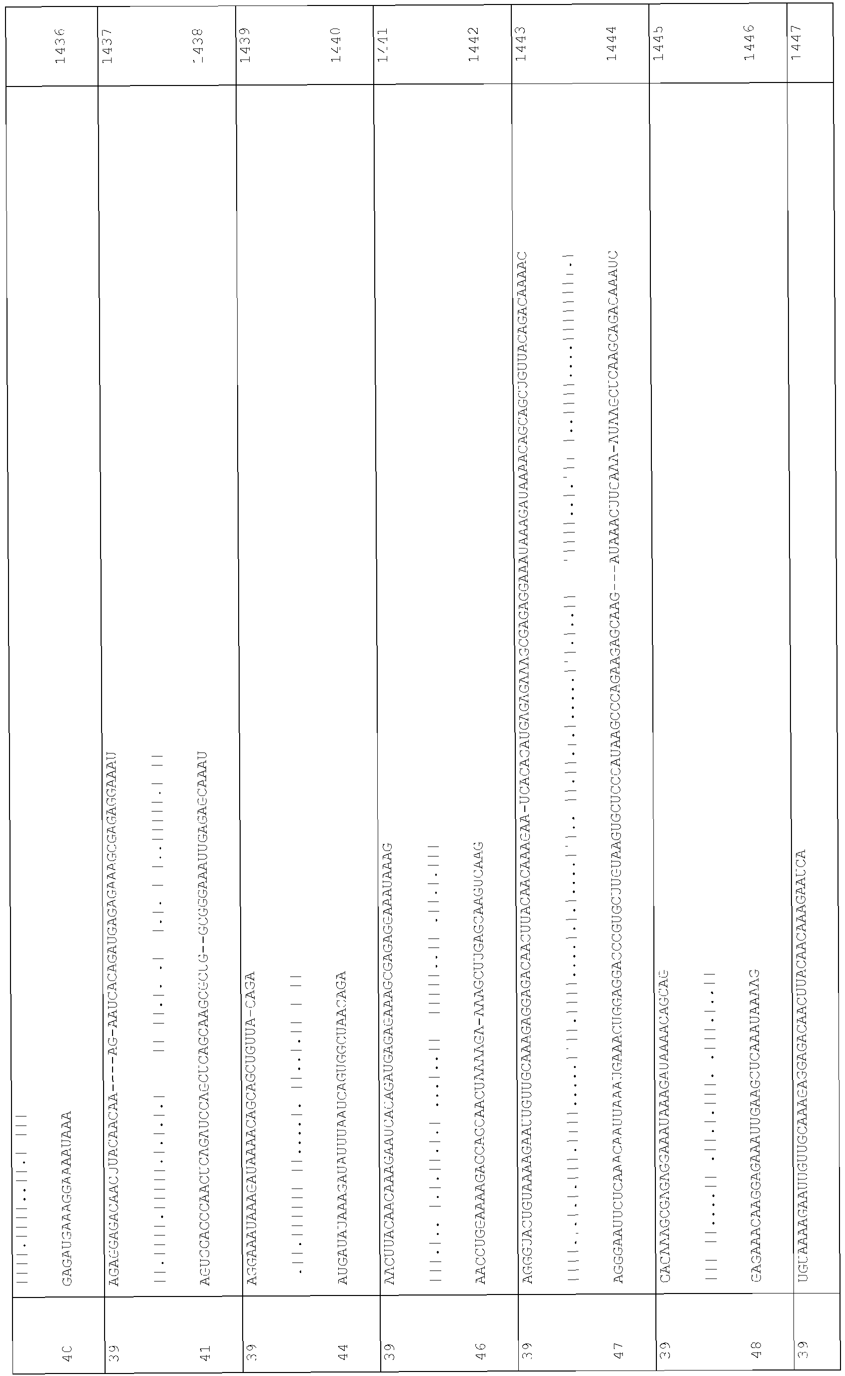

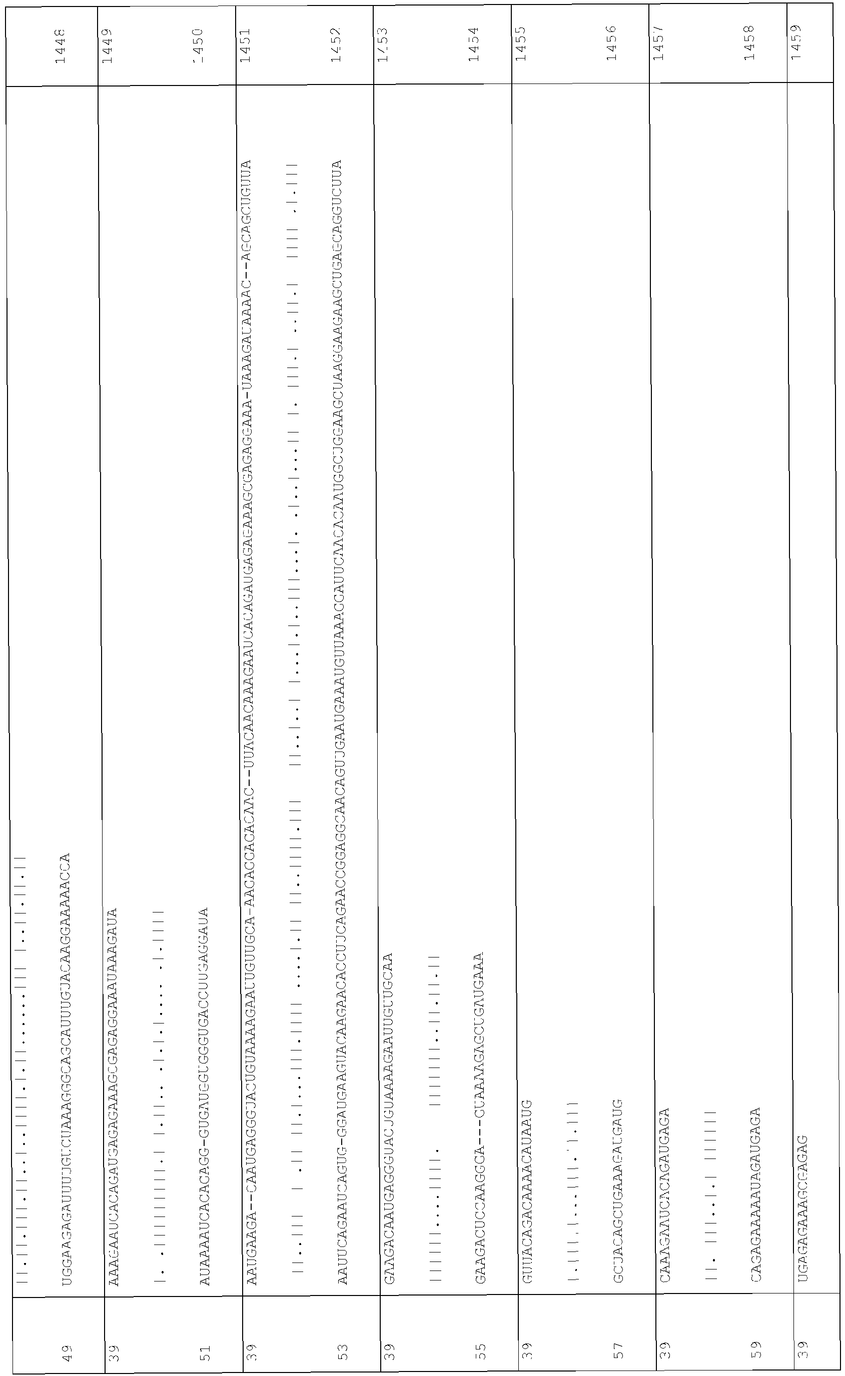

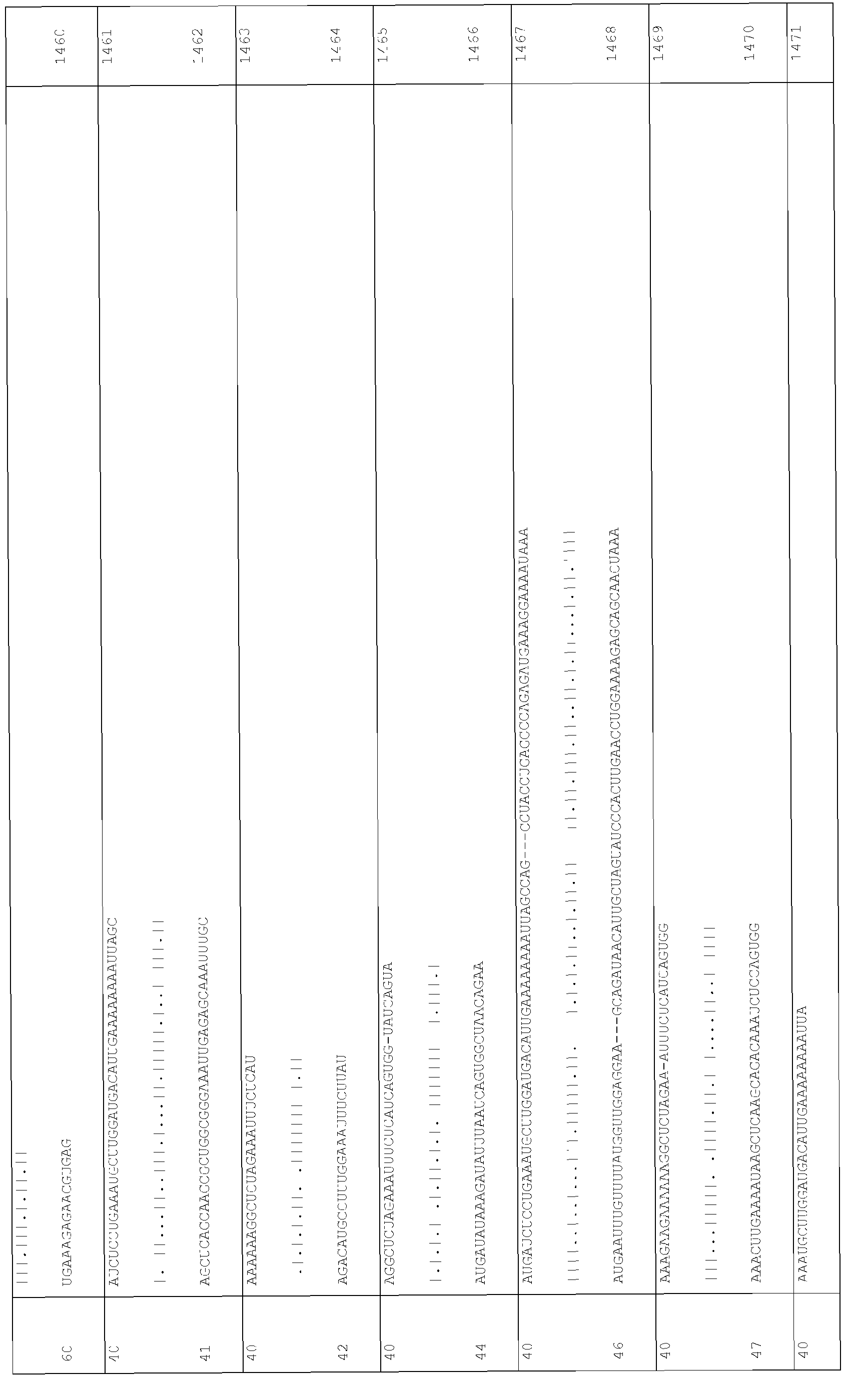

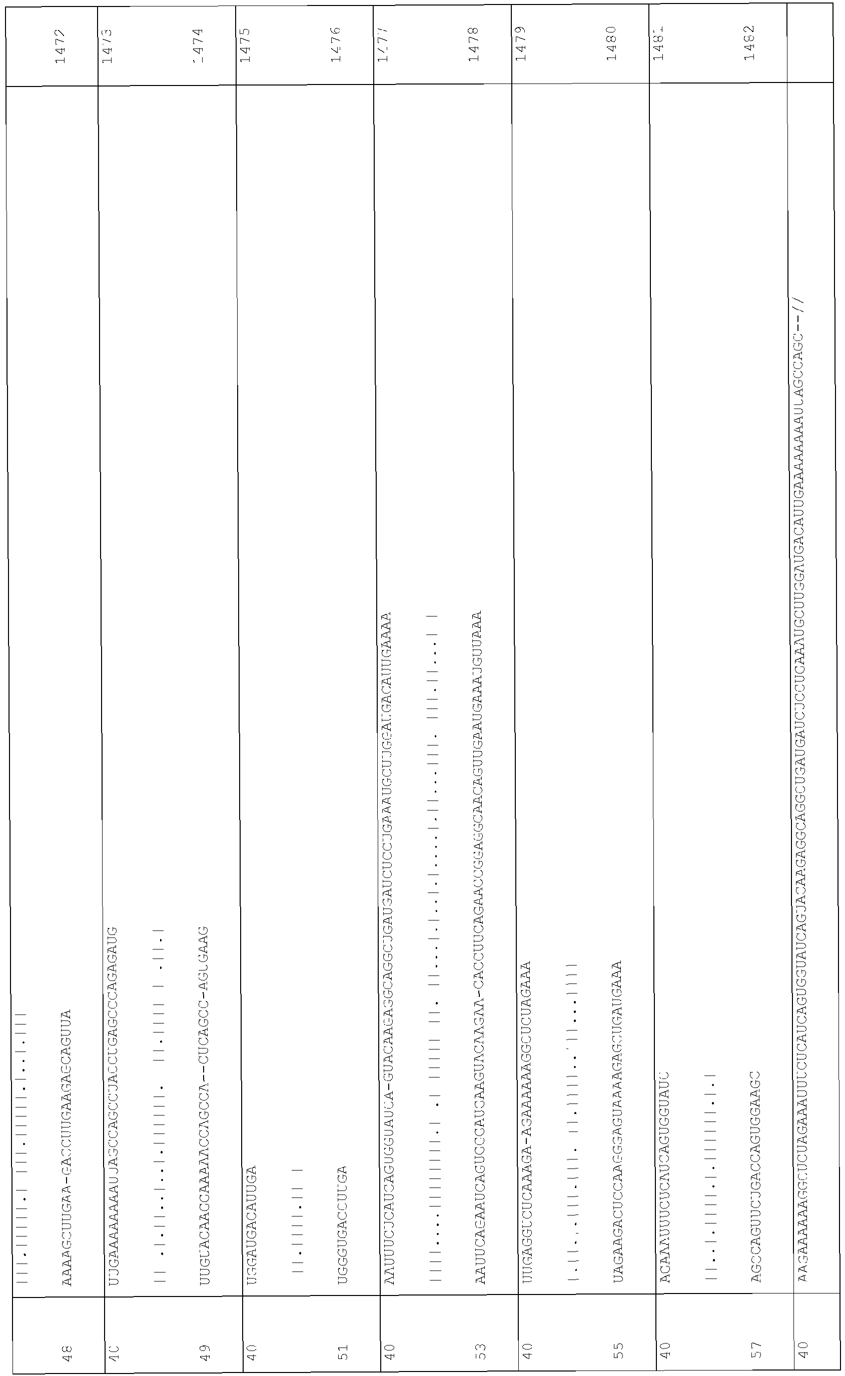

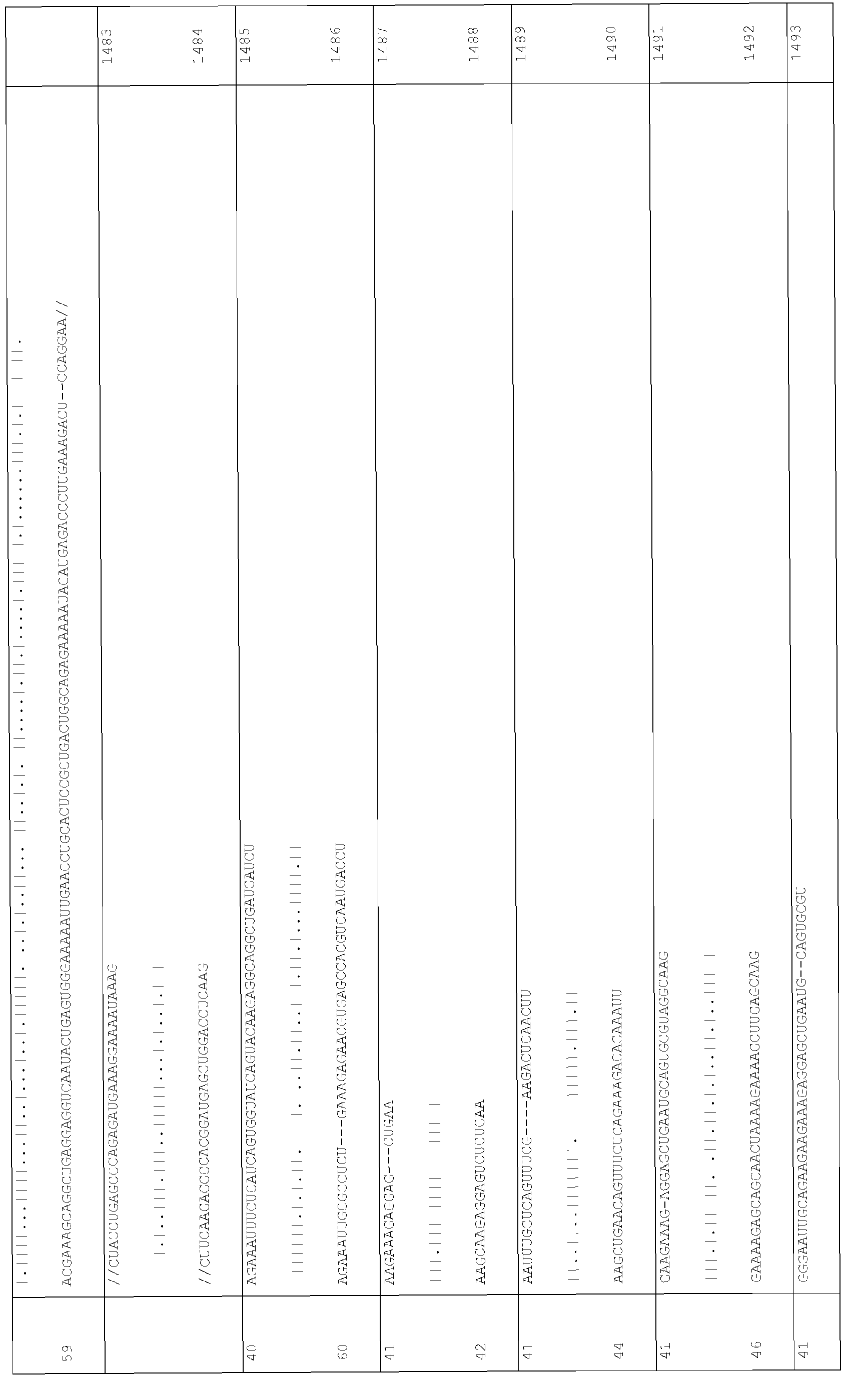

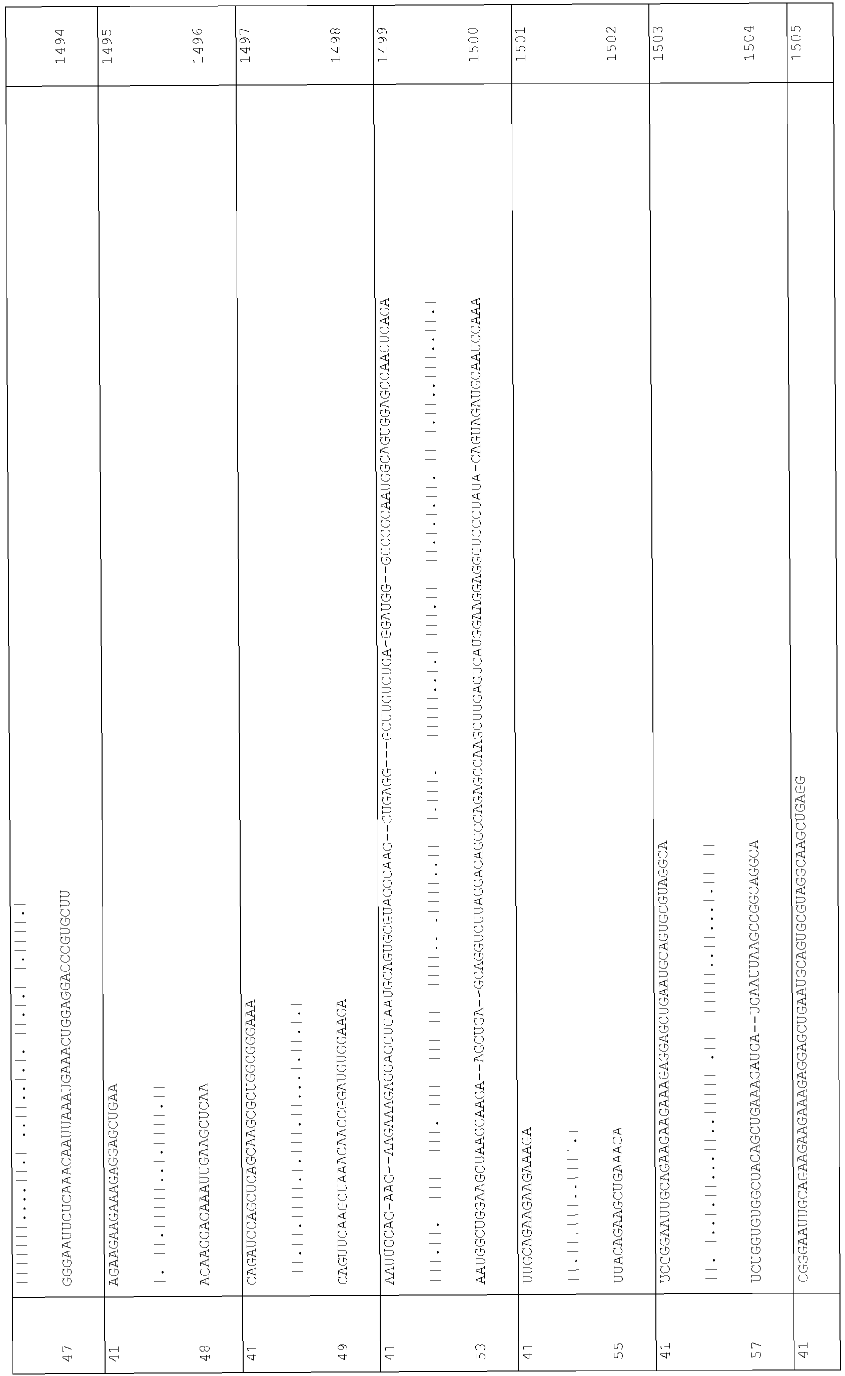

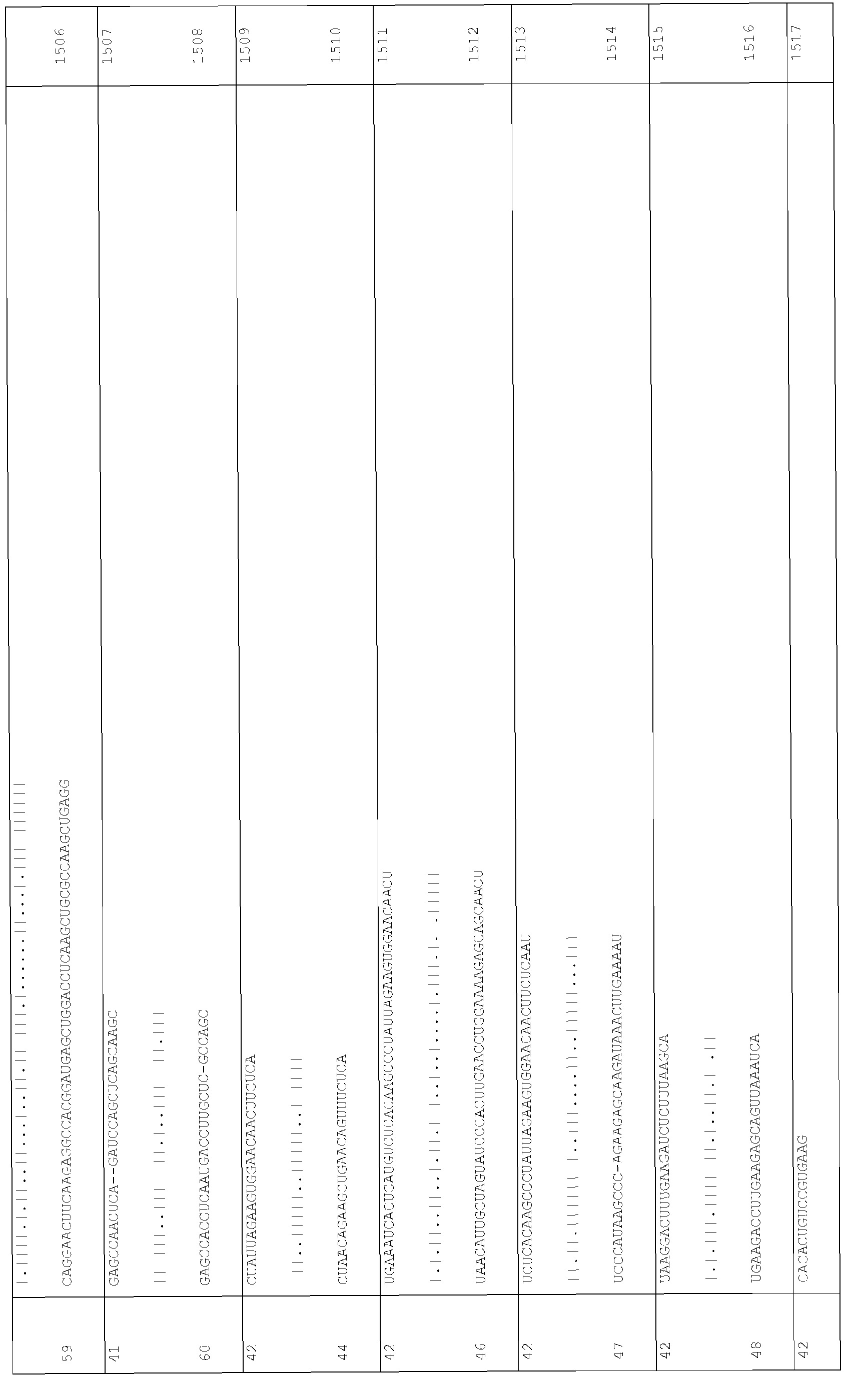

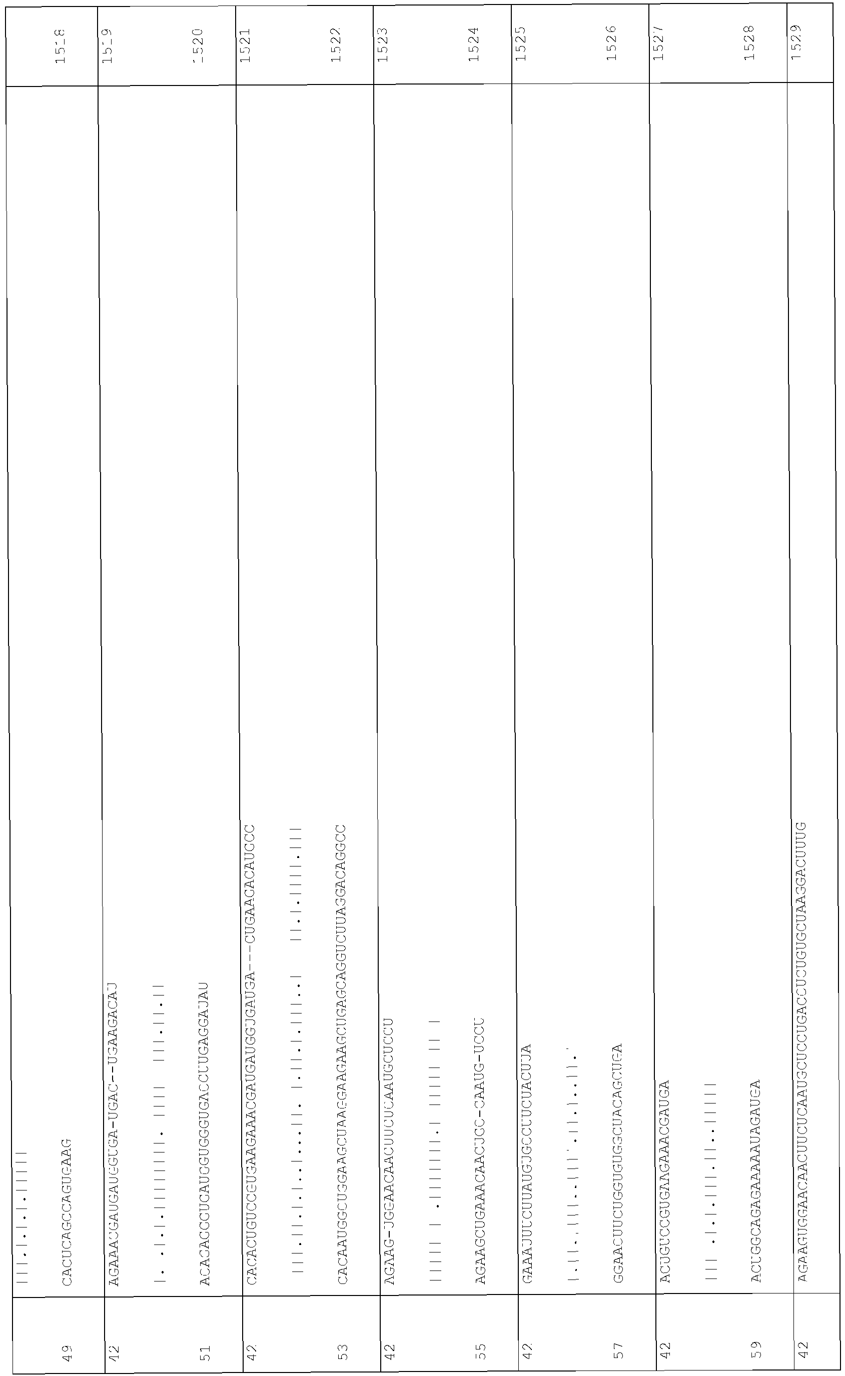

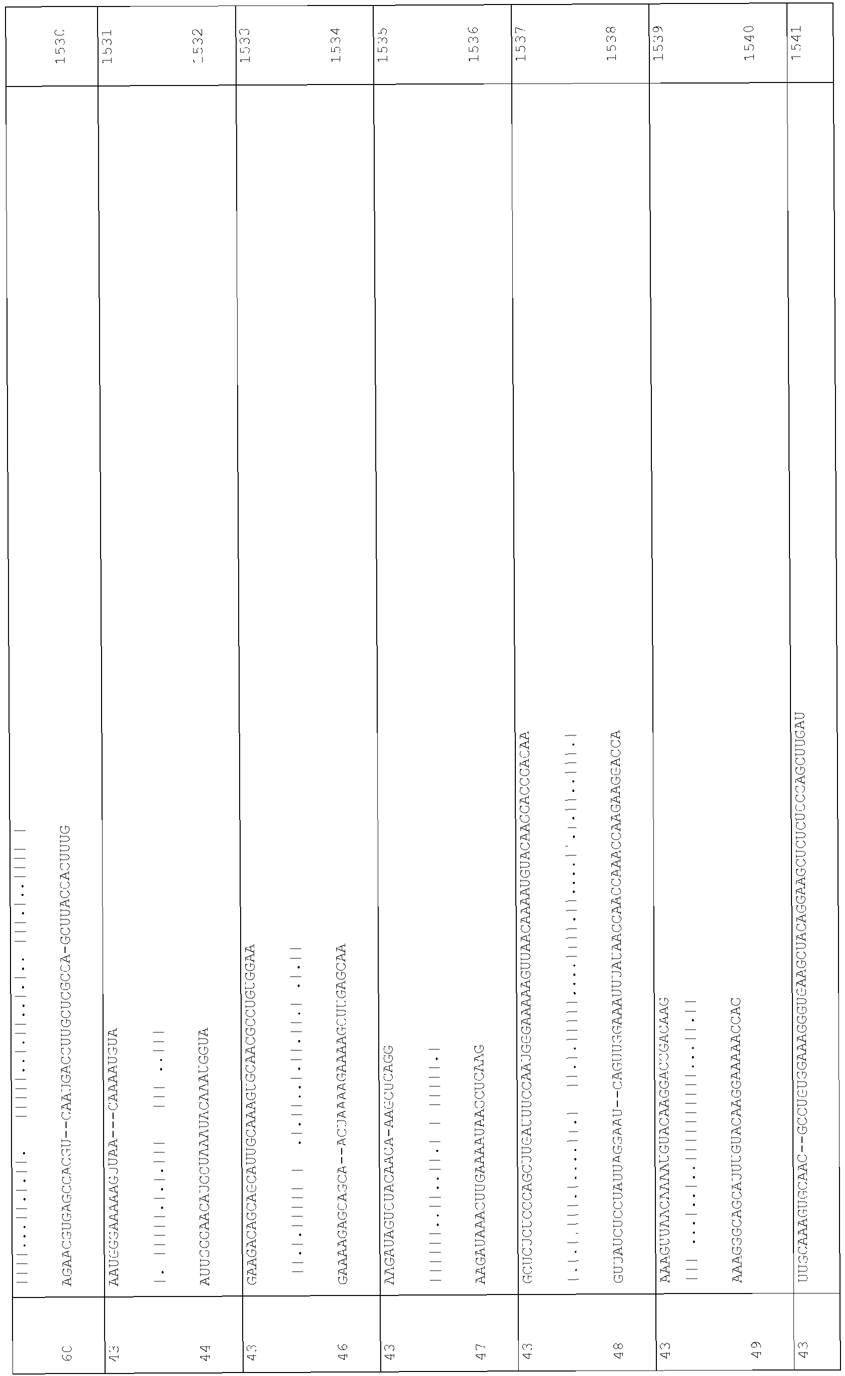

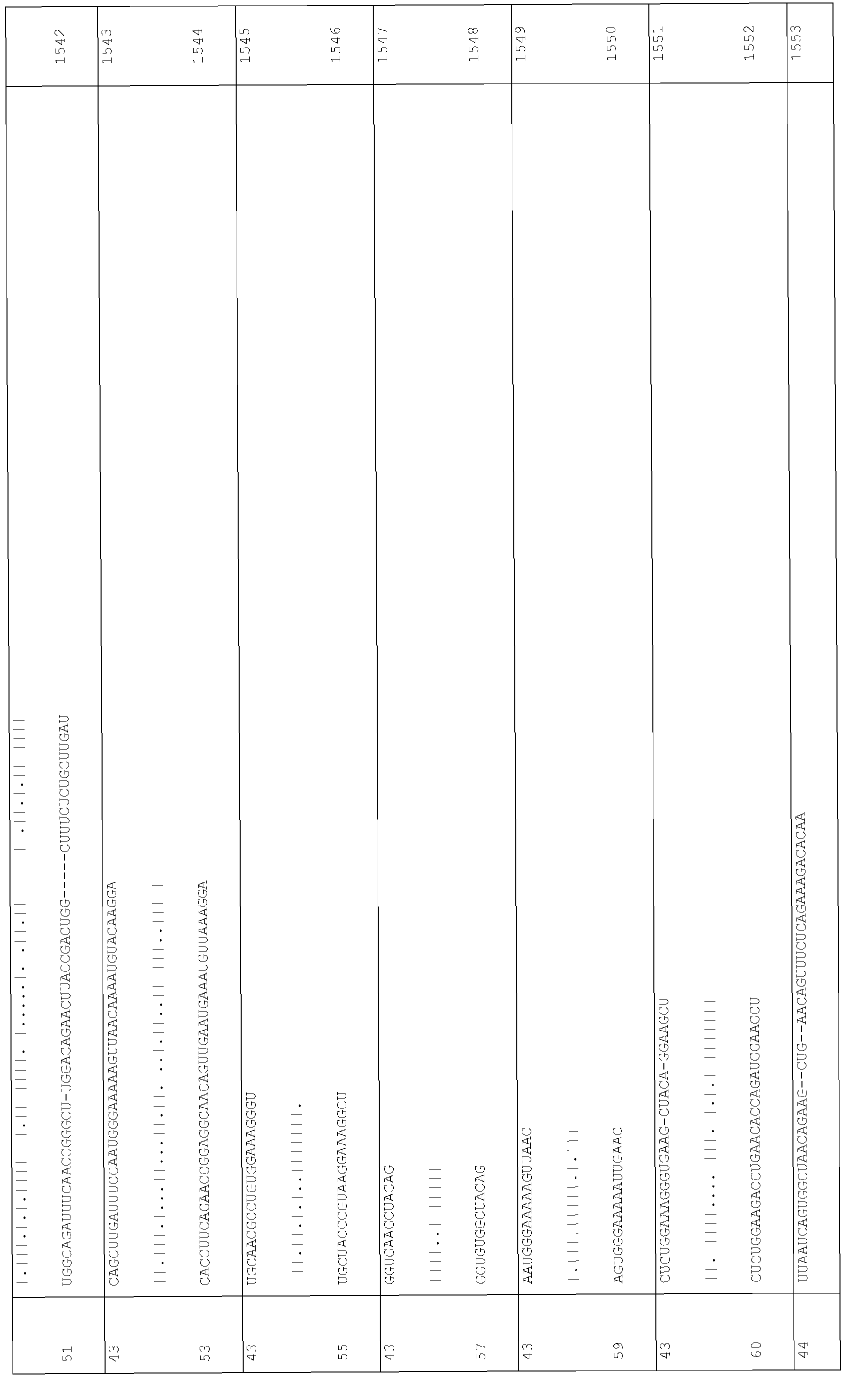

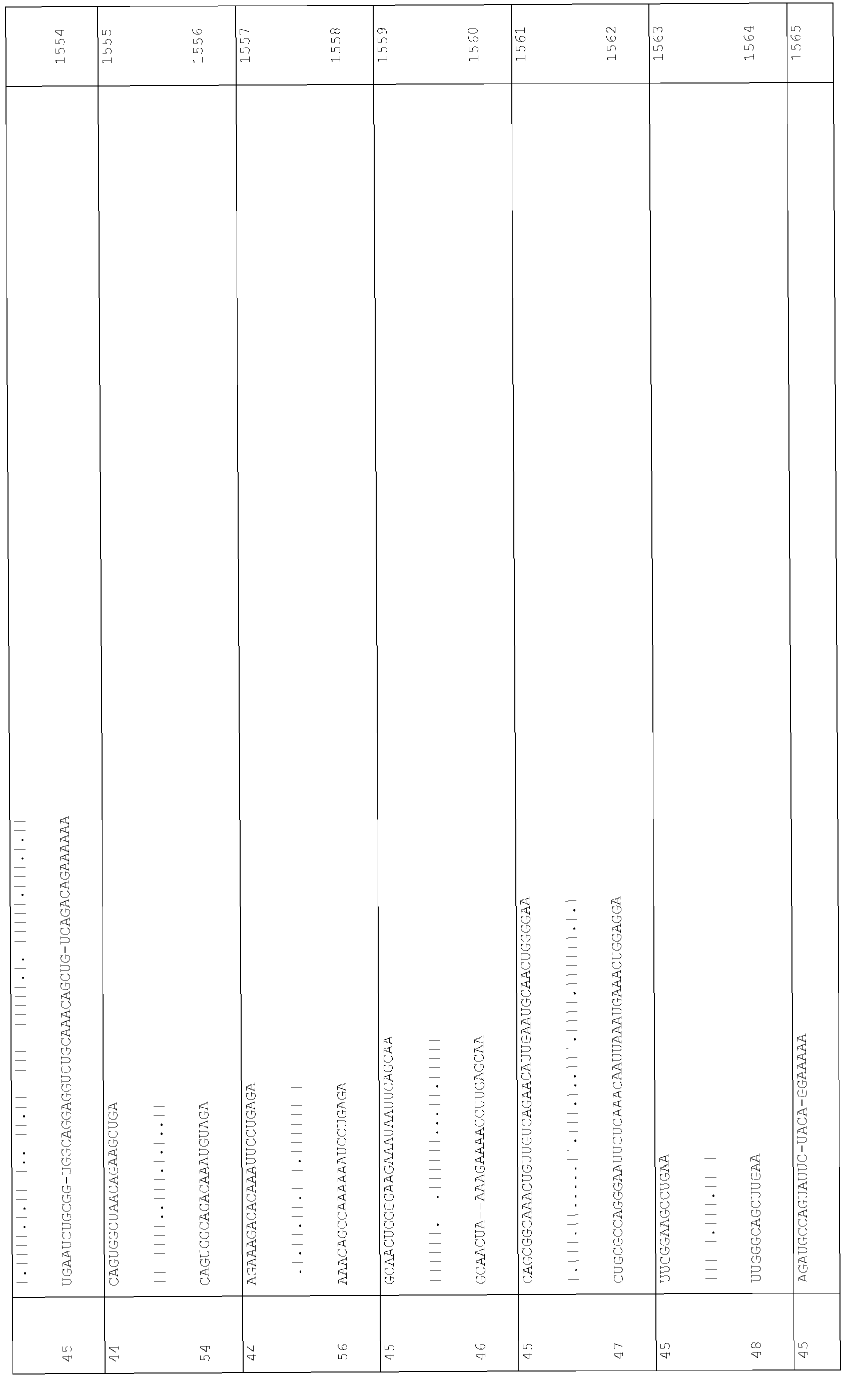

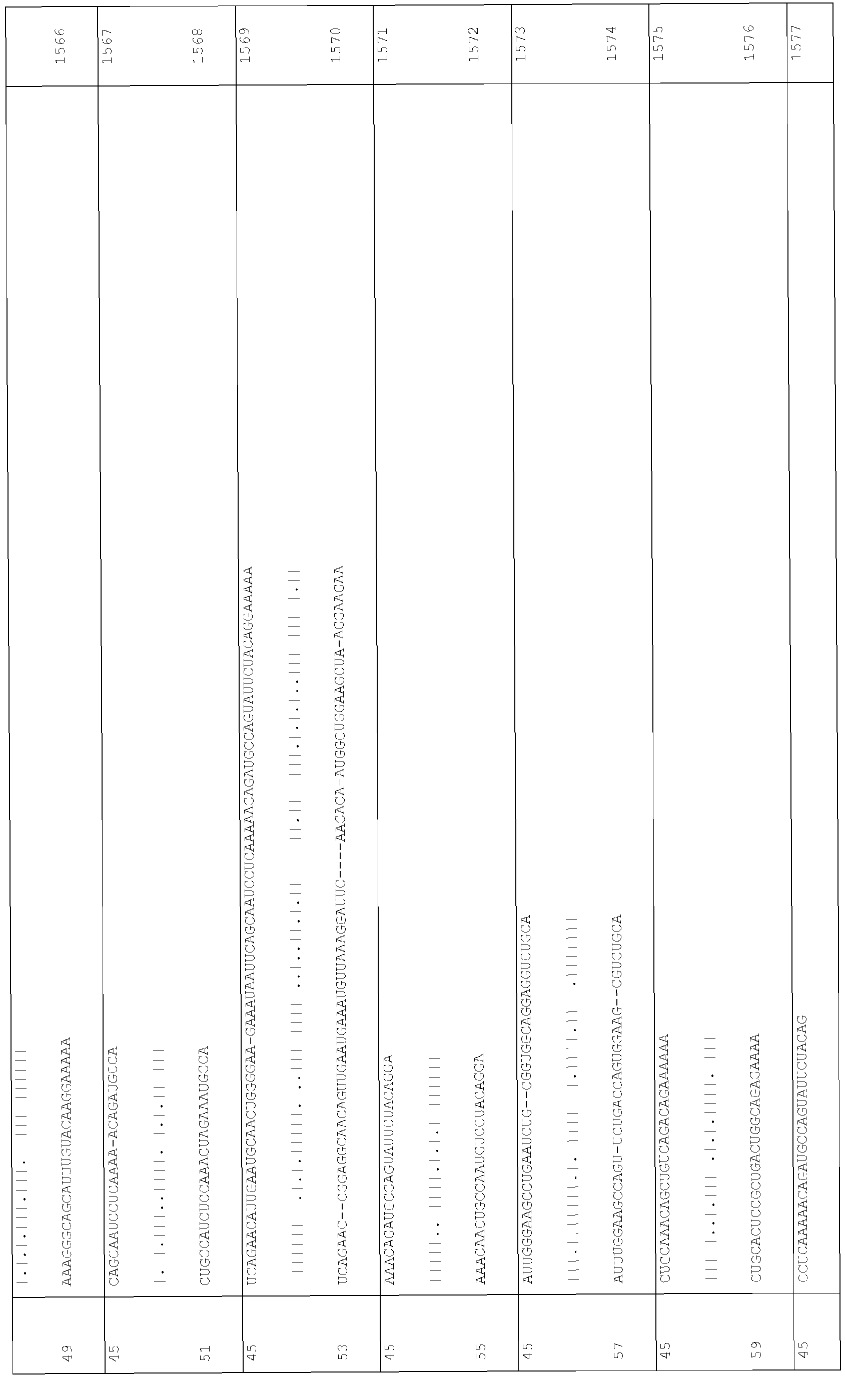

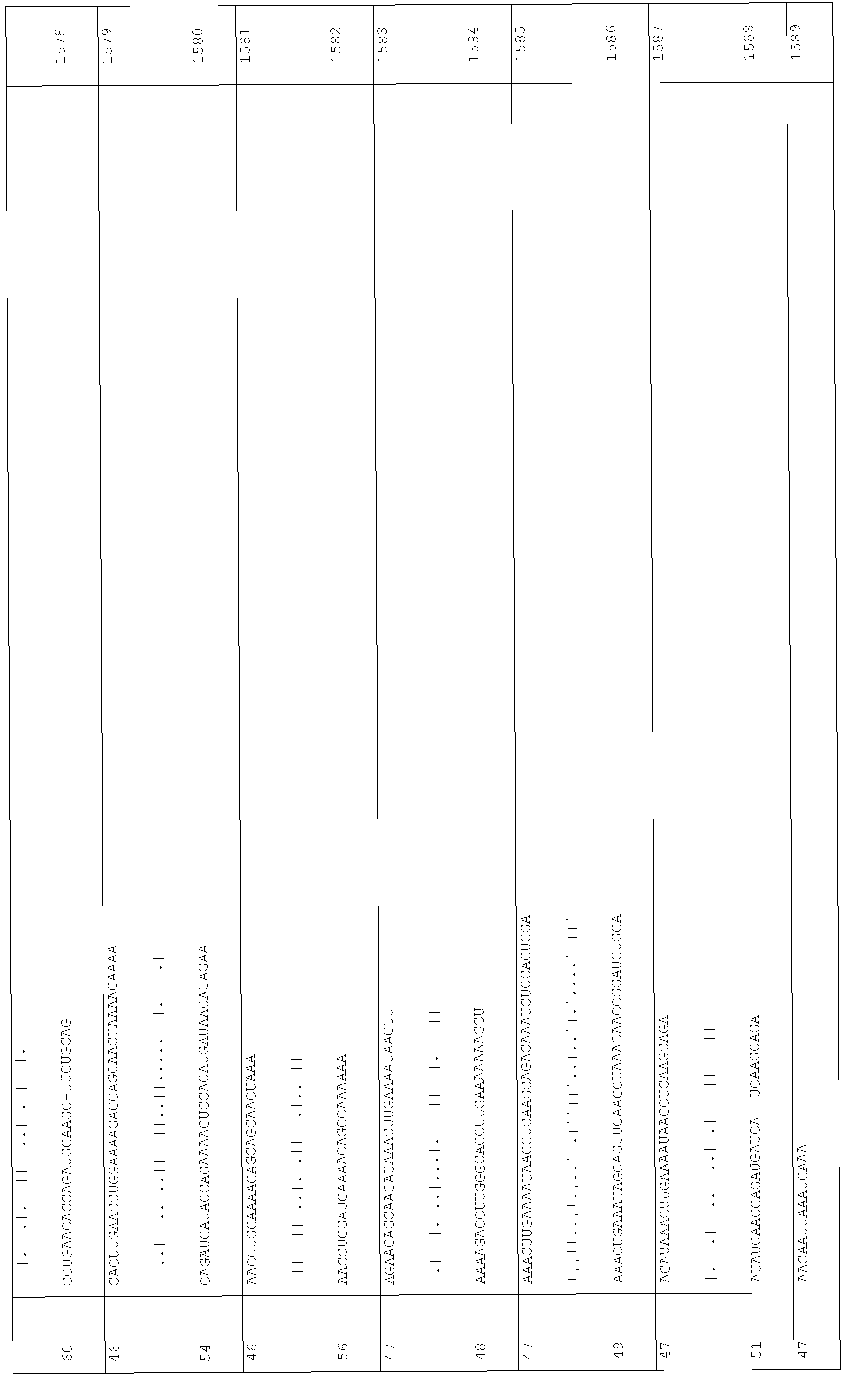

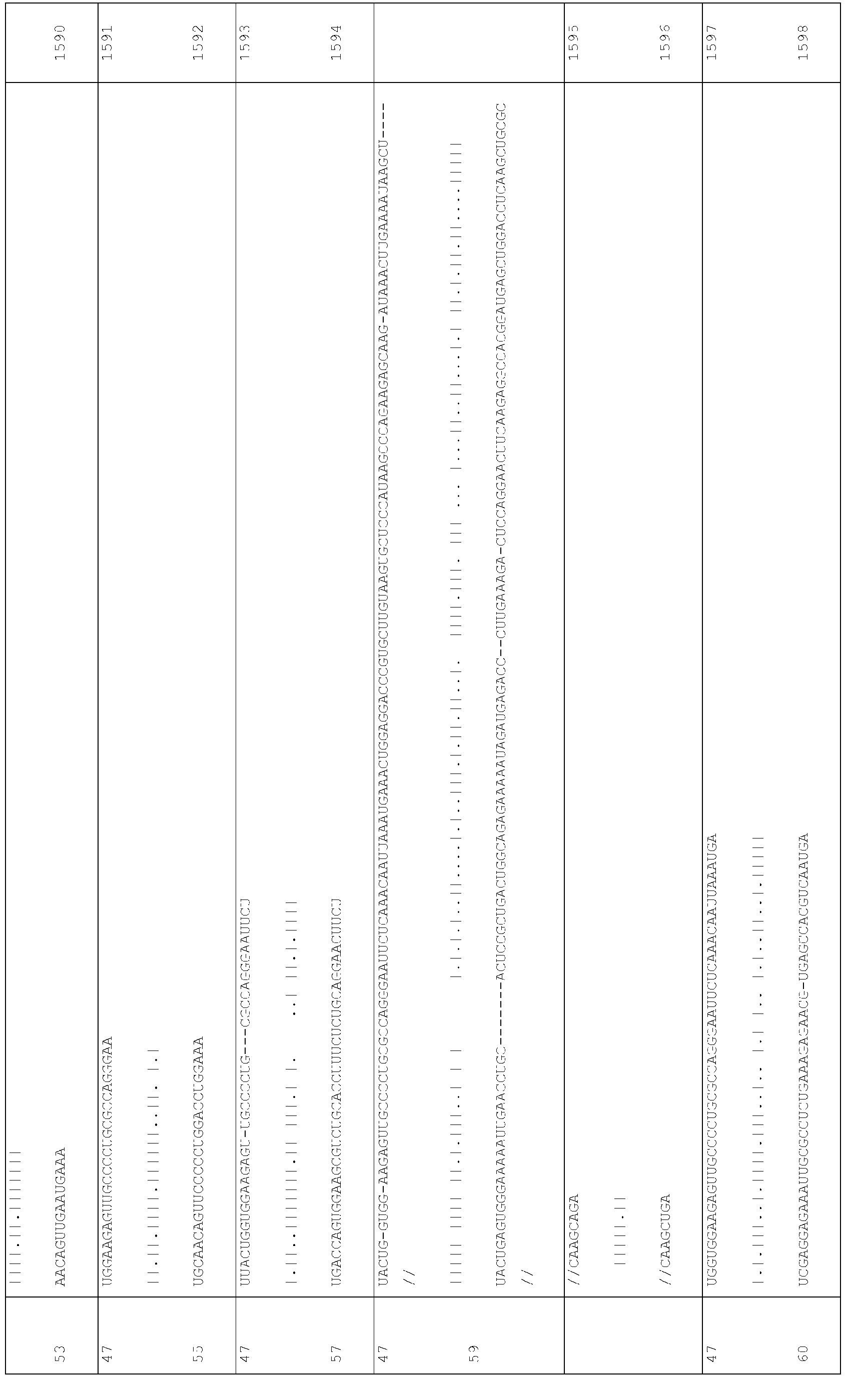

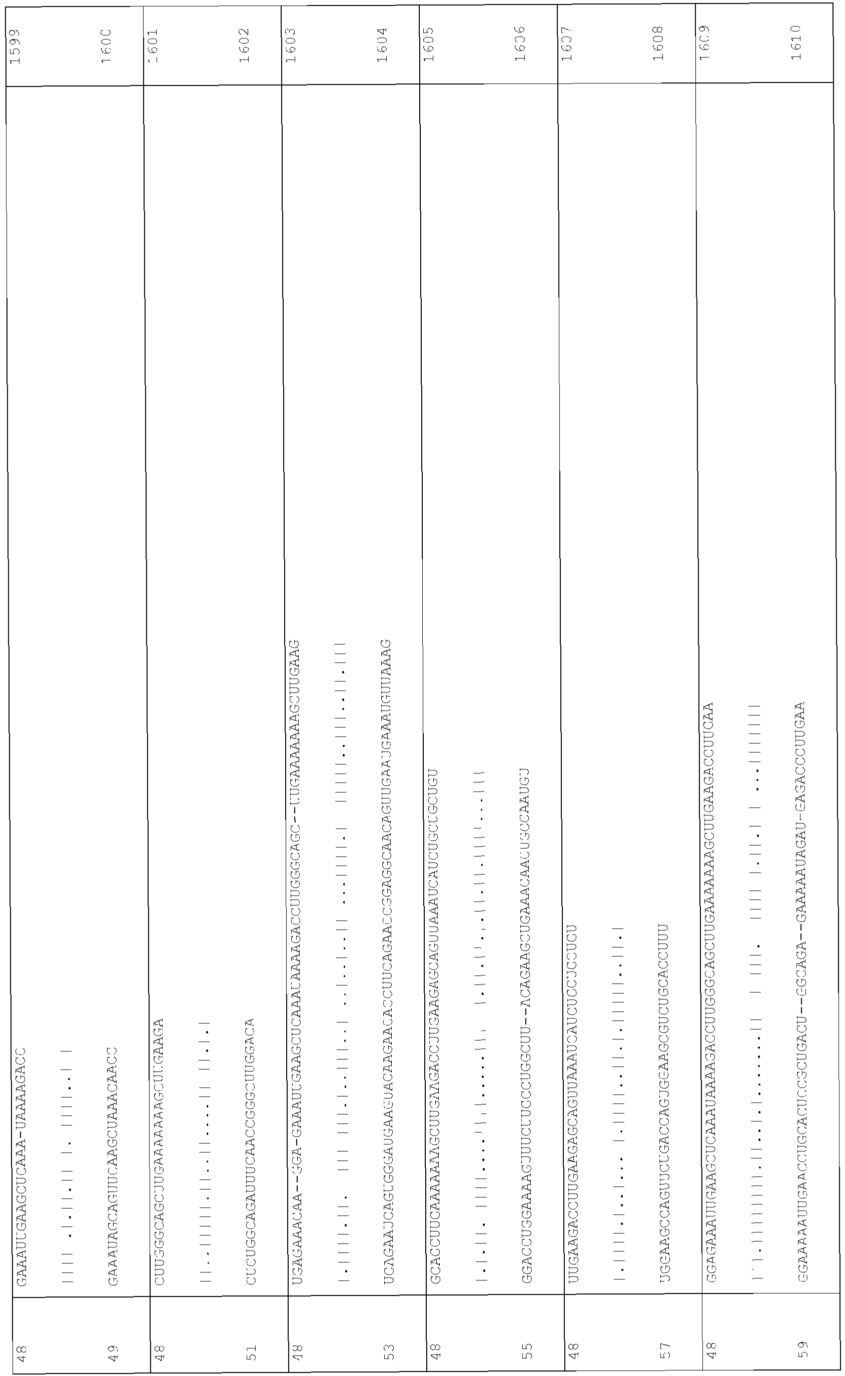

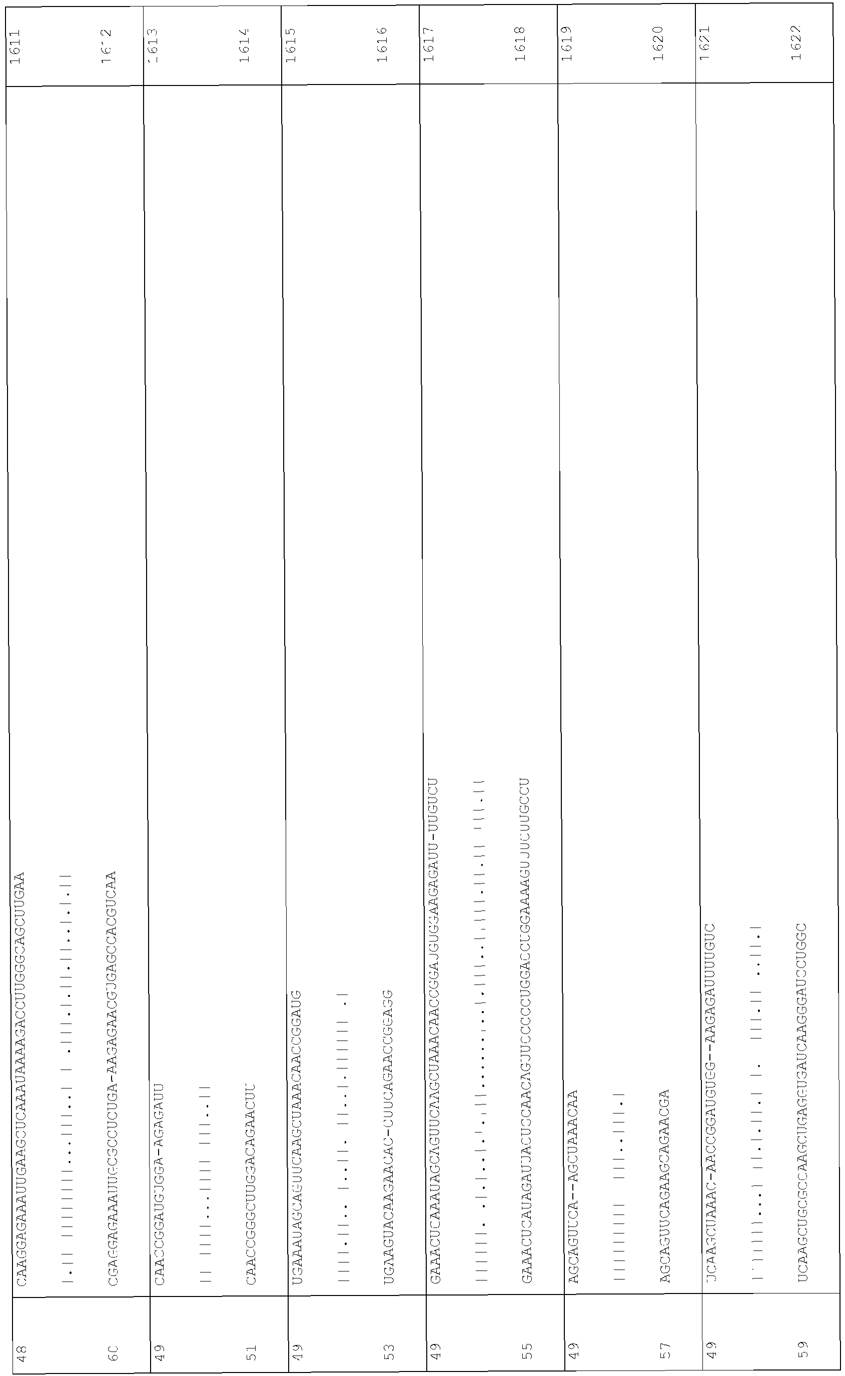

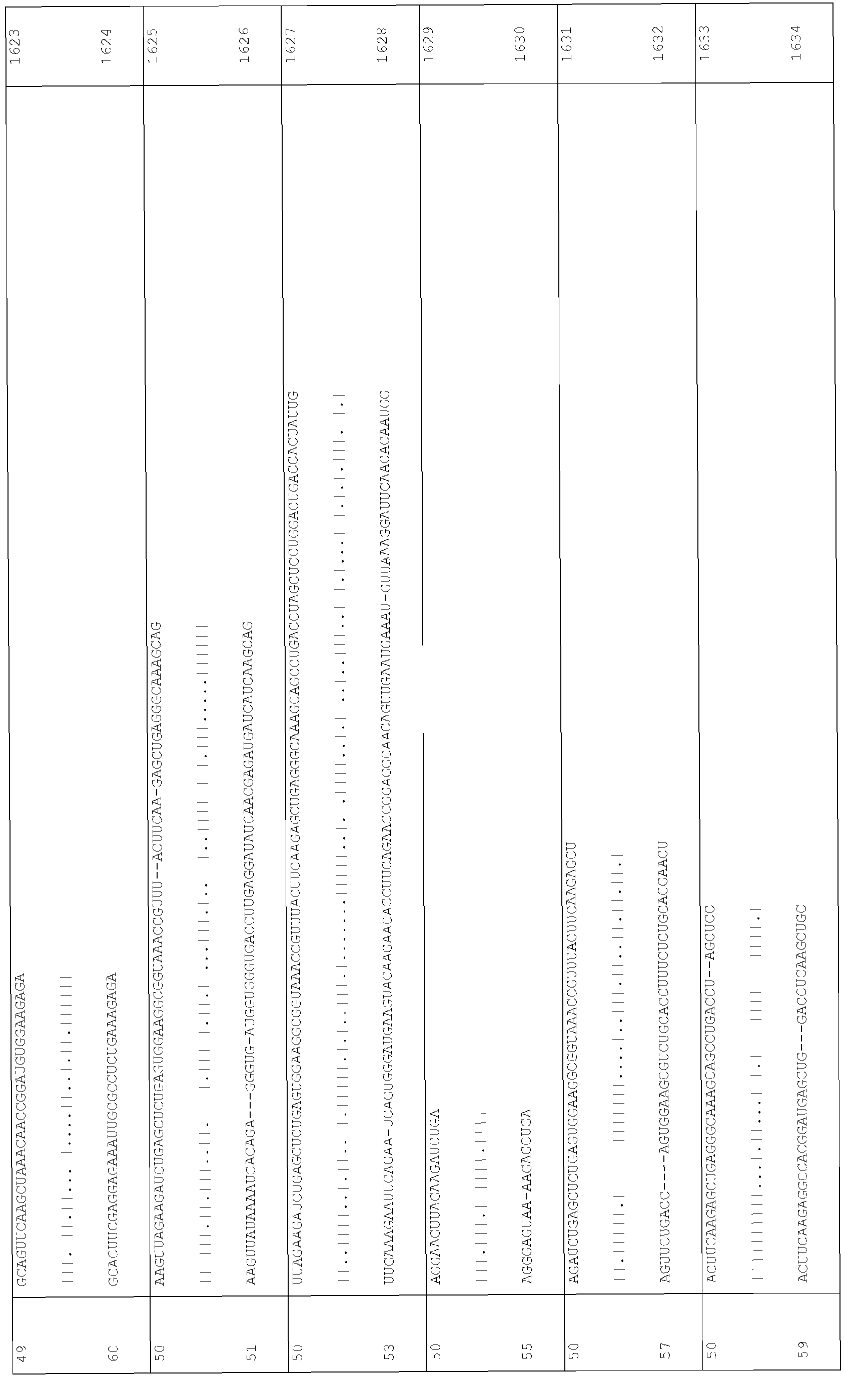

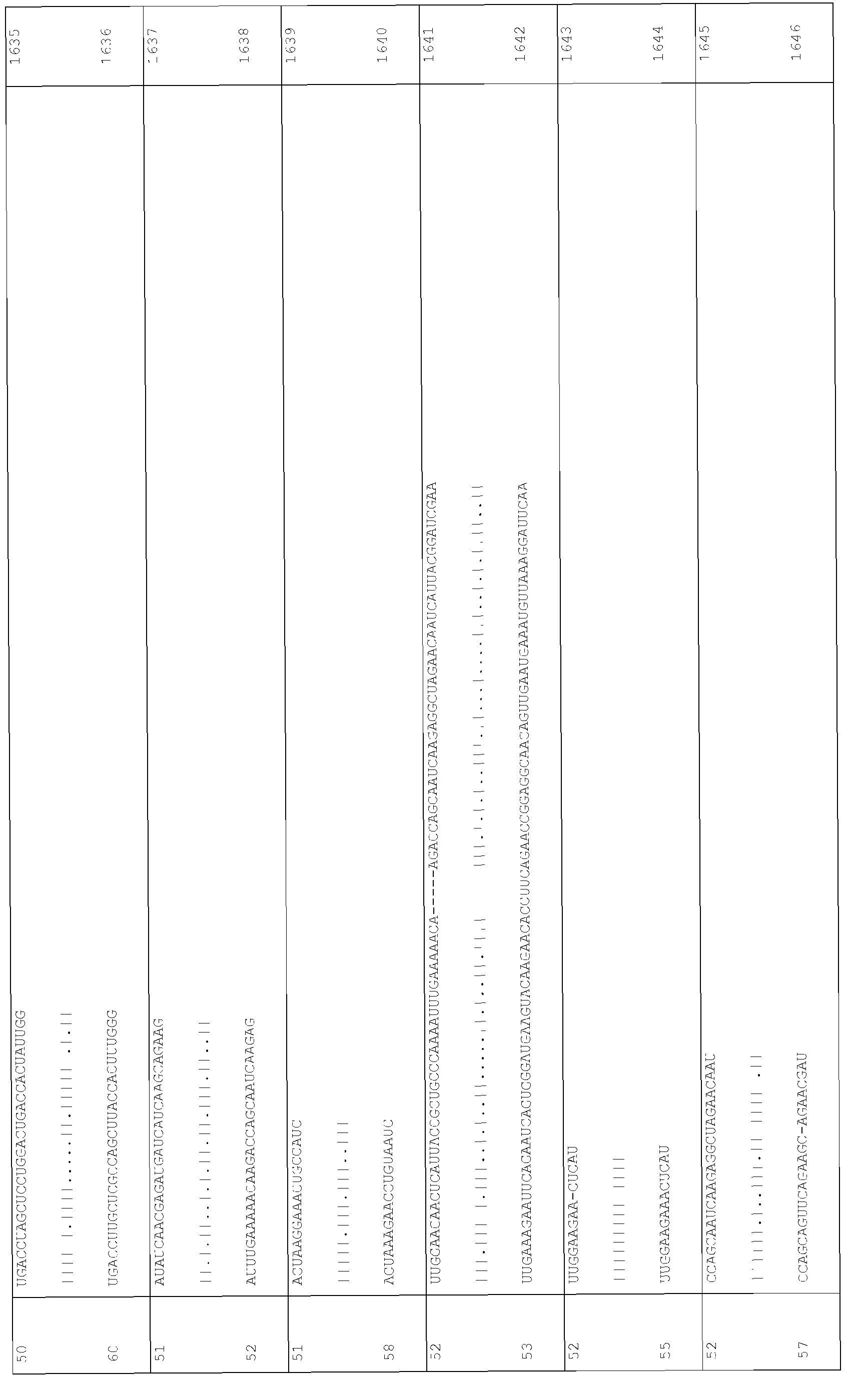

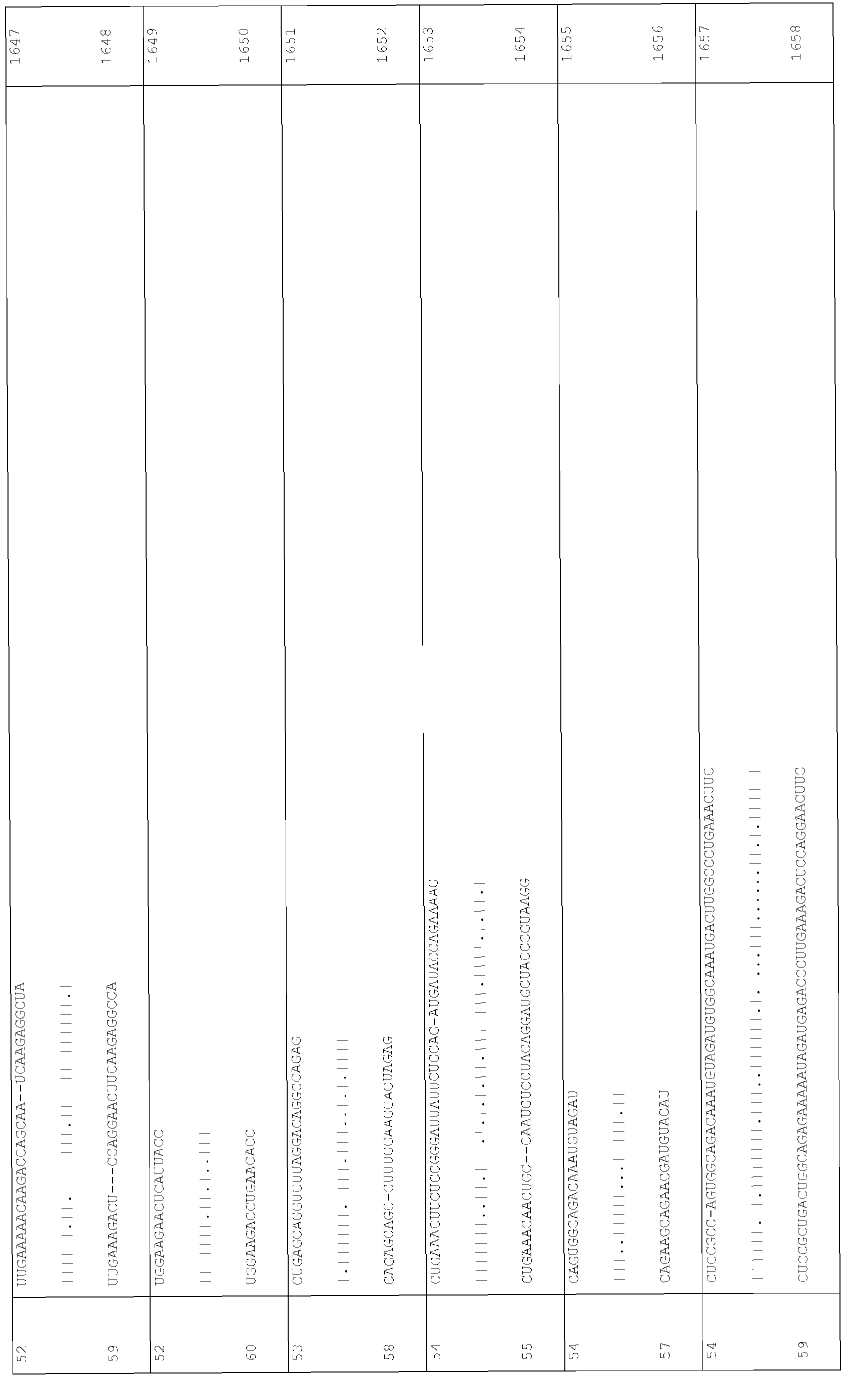

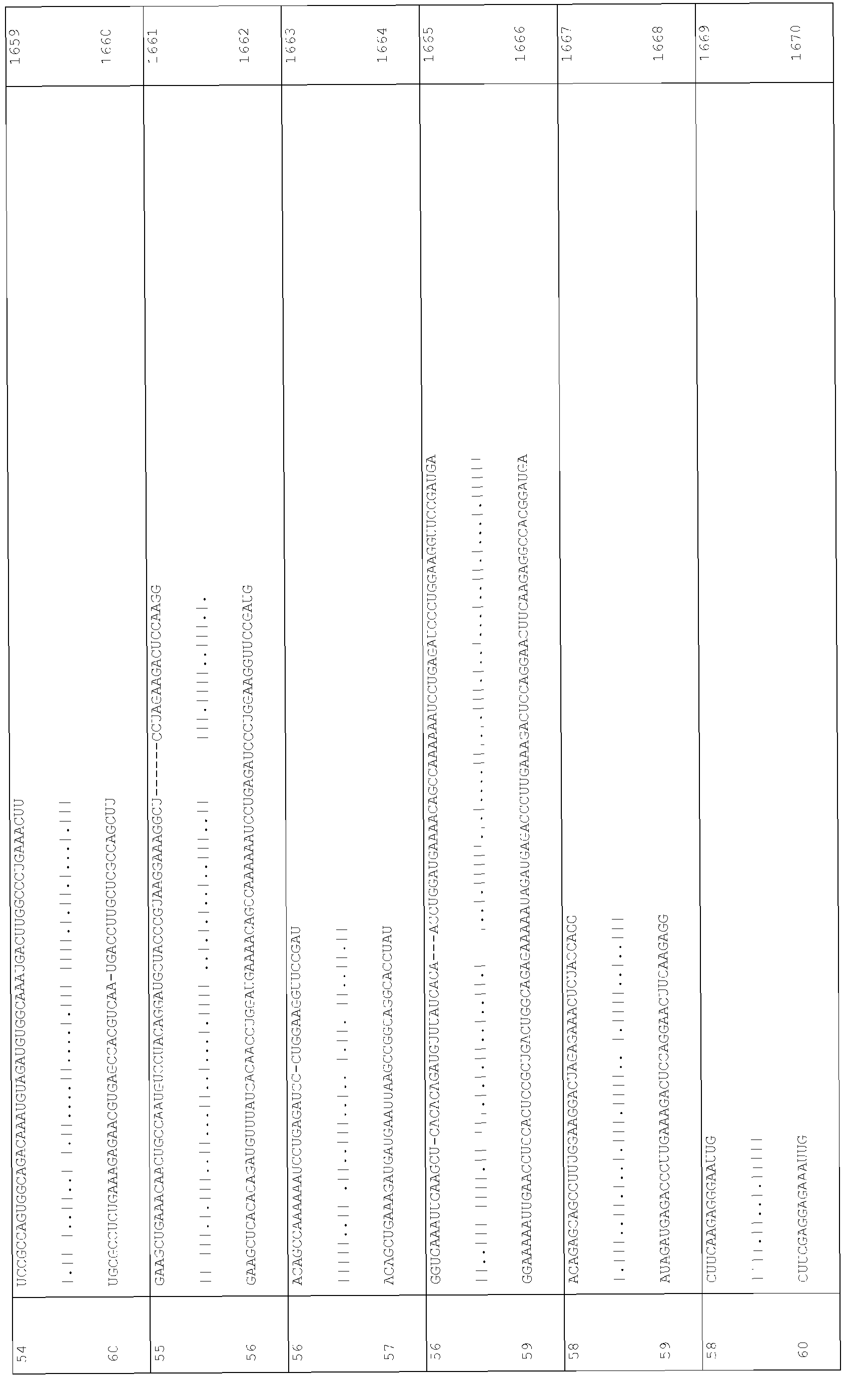

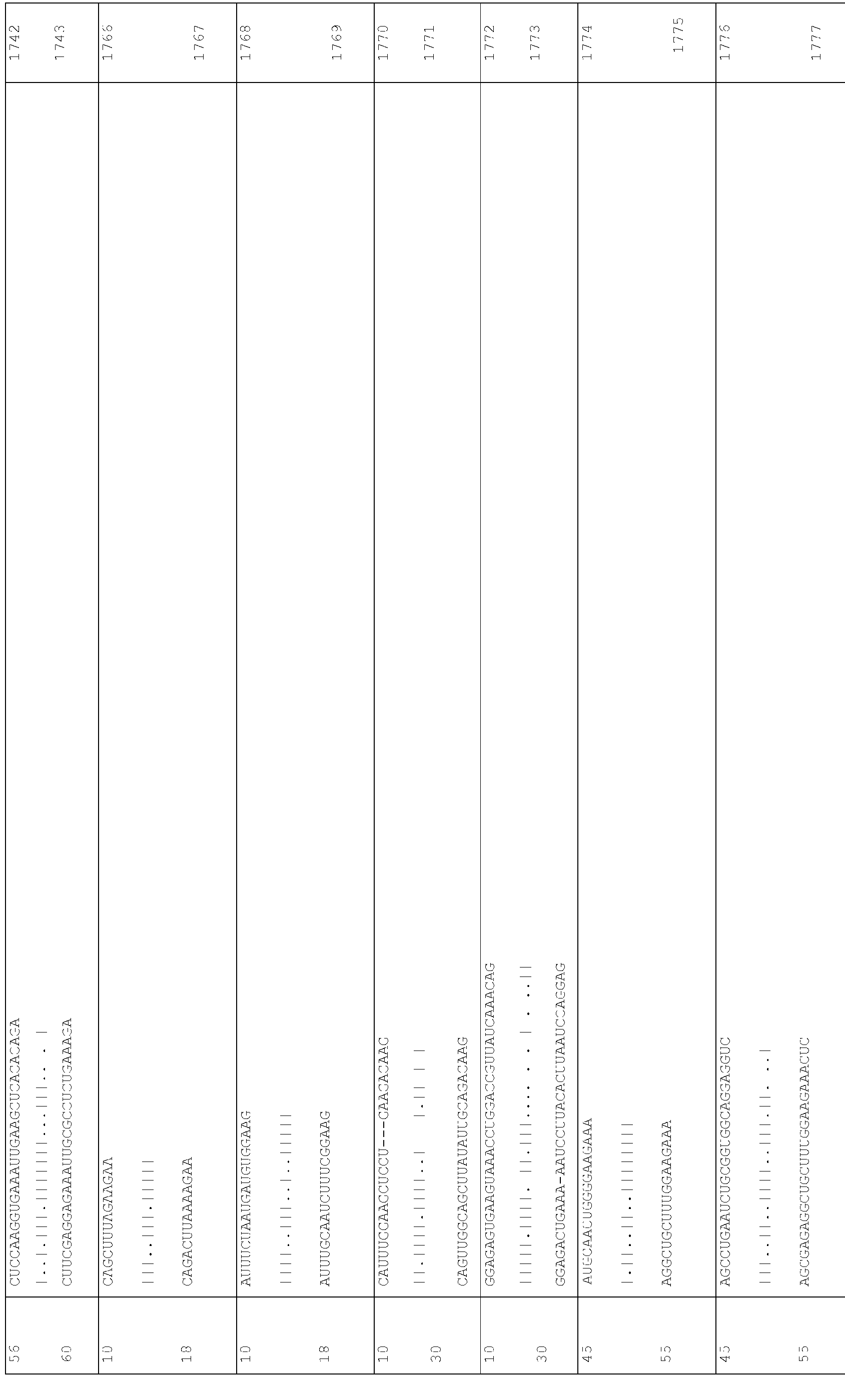

**Table 3. List of preferred base sequences of the oligonucleotides according to the invention. Oligonucleotides comprising these base sequences are capable of binding to highly similar regions (sequence stretches with >50% identity) in two different DMD exons (as exemplified in Table 2), and capable of inducing the skipping of these two exons, and any exons in between, to generate an in-frame DMD transcript.**

| Compound | Sequence* | SEQ ID NO |
|---|---|---|
| First exon 8 - Second exon 19 | | |
| PS830 | GUGAUGUACAUUAAGAUGGACUUC | 1671 |
| | GYGZYGYZXZYYZZGZYGGZXYYX | 1672 |
| | YGGZXYYXYYZYXYGGZY | 1722 |
| | YXYGZZXYYXYXZGXYY | 1723 |

| First exon 9 - Second exon 22 | | |
|---|---|---|
| | YXZGZGGYGGYGZXZYZZG | 1724 |
| | YXZYZGYXGGYGZXZXYZZG | 1725 |

| First exon 9 - Second exon 30 | | |
|---|---|---|
| | YXYXZYZYXXXYGYGXYZGZXYG | 1726 |
| | GZZYXGZGGXYYZGGYGZZGZZG | 1727 |
| | YYXZGYXYXXYGGGXZGZXYG | 1728 |
| | GZXYXXYGGZYYZZGYGYZZGG | 1729 |

| First exon 10 - Second exon 13, 14, 15, 18, 20, 27, 30, 31, 32, 35,42,44,47, 48, 55, 57, or 60 | | |
|---|---|---|
| PS811 | CUUCCUUCCGAAAGAUUGCAAAUUC | 1673 |
| | XYYXXYYXXGZZZGZYYGXZZZYYX | 1674 |
| PS814 | GACUUGUCUUCAGGAGCUUCC | 1675 |
| | GZXYYGYXYYXZGGZGXYYXX | 1676 |
| PS815 | CAAAUGACUUGUCUUCAGGAGCUUC | 1677 |
| | XZZZYGZXYYGYXYYXZGGZGXYYX | 1678 |
| PS816 | CUGCCAAAUGACUUGUCUUCAGGAG | 1679 |
| | XYGXXZZZYGZXYYGYXYYXZGGZG | 1680 |
| PS1168 | CUGCCDDDUGDCUUGUCUUCDGGDG | 1681 |
| PS1050 | CCAAAUGACUUGUCU | 1682 |
| | CCAAAYGACYYGYCY | 1683 |
| PS1059 | CAAAUGACUUGUCUUCAGGAG | 1684 |
| PS1138 | CAAAUGACUUGUCUUCAGGAG | 1685 |
| PS1170 | CDDDUGDCUUGUCUUCDGGDG | 1686 |
| | ZYGZXYYGYXYYXZGGZGGZG | 1687 |
| PS1016 | CAGUCUCCUGGGCAGACUGGAUGCUC | 1688 |
| | XZGYXYXXYGGGXZGZXYGGZYGXYX | 1689 |
| | ZYGZZXYGXXZZZYGZXYYGYX | 1690 |
| | ZYZZGXYGXXZZXYGXYYGYX | 1691 |
| | GYXXZGGYYYZXYYXZXY | 1692 |
| | GYXXZXXYYGYXYGXZZY | 1693 |
| | XYYXYZZZGXYGYYYGZ | 1694 |
| | XYYXXYGZGGXZYYYGZ | 1695 |
| | YYYGZYZZXGGYXXZGGYYYZXYYXZ | 1696 |
| | ZGGXZYYYGZGXYGXGYXXZ | 1697 |

| First exon 23 - Second exon 42 | | |
|---|---|---|
| | YXYYXGZXZYXYXYYYXZXZGY | 1698 |
| | GXGXYYYXYYXGZXZYXYX | 1699 |
| | ZZYYYXZGZGGGXGXYYYXYYX | 1700 |
| | YXYYXZGYXZYXZXXZYXZYXGY | 1701 |
| | GGXZYGYXYYXZGYXZYXZX | 1702 |
| | ZZYYYXXZZZGGXZYGYXYYX | 1703 |

| First exon 45 - Second exon 51 | | |
|---|---|---|
| | YGGXZYXYGYGYYYGZGGZYYGXYG | 1730 |
| | YGGXZYYYXYZGYYYGGZGZYGGXZG | 1731 |

| First exon 45 - Second exon 53 | | |
|---|---|---|
| | YYXXZXZGYYGXZYYXZZYGYYXYGZ | 1732 |
| | YYXZZXYGYYGXXYXXZGYYXYGZ | 1733 |
| | YYXXYGYZGZZYZXYGGXZYXYGY | 1734 |
| | YYXZZXYGYYGXXYXXGGYYXYGZ | 1735 |
| | XYYYZZXZYYYXZYYXZZXYGYYGX | 1736 |
| | YYXYYXXYYZGXYYXXZGXXZYYGYGYY | 1737 |

| First exon 45 - Second exon 55 | | |
|---|---|---|
| | UCCUGUAGAAUACUGGCAUCUGUUU | 1704 |
| | YXXYGYZGZZYZXYGGXZYXYGYYY | 1705 |
| PS1185 | UCCUGUDGDDUDCUGGCDUCUGUUU | 1706 |
| PS1186 | UCCUGUDGDDUDCUGGCDUCUGU | 1707 |
| | UCCUGUAGAAUACUGGCAUCUGU | 1708 |
| | YXXYGYZGZZYZXYGGXZYXYGY | 1709 |
| PS1187 | UCCUGUDGGDUDUUGGCDGUUGUUU | 1710 |
| | UCCUGUAGGAUAUUGGCAGUUGUUU | 1711 |
| | YXXYGYZGGZYZYYGGXZGYYGYYY | 1712 |
| PS1188 | UCCUGUDGGDUDUUGGCDGUUGU | 1713 |
| | UCCUGUAGGAUAUUGGCAGUUGU | 1714 |
| | YXXYGYZGGZYZYYGGXZGYYGY | 1715 |
| | UCCUGUAGGACAUUGGCAGUUGU | 1716 |
| | YXXYGYZGGZXZYYGGXZGYYGY | 1717 |
| | UCCUGUAGGACAUUGGCAGUUGUUU | 1718 |
| | YXXYGYZGGZXZYYGGXZGYYGYYY | 1719 |

| First exon 45 - Second exon 60 | | |
|---|---|---|
| | ZZYZXYGGXZYXYGYYGYYGZGG | 1738 |
| | XYGYZGZZYZXYGGXZYXYGYY | 1739 |
| | GXYYXXZYXYGGYGYYXZGG | 1740 |
| | XYGXZGZZGXYYXXZYXYGGY | 1741 |

| First exon 56 - Second exon 60 | | |
|---|---|---|
| | YXYGYGYGZGXYYXZZYYYXZXXYYGGZG | 1720 |
| | YCYYYXZGZGGXGXZZYYYXYXXYXGZZG | 1721 |

| | | |
|---|---|---|
| *D = 2,6-diaminopurine; C = 5-methylcytosine; X = C or 5-methylcytosine, Y = U or 5-methyluracil; Z = A or 2,6-diaminopurine | | |

**Table 6. List of most preferred and/or additional exon combinations, most preferred and/or additional exon identity regions, and most preferred and/or additional oligonucleotides with their base sequences, chemical modifications and sequence identification numbers, wherein *= LNA, C=5-methylcytosine, U=5-methyluracil, D=2,6-diaminopurine, X=C or 5-methylcytosine, Y=U or 5-methyluracil, Z=A or 2,6-diaminopurine, and < > all 2'-fluoro nucleotides (as in SEQ ID NO: 1844).**

| **Exon Combination** | **Exon Identity Region** | **Oligonucleotides** | | **SEQ ID NO** |
|---|---|---|---|---|
| 10-18 | 1)22/35 (62.9%) | **PS816 and derivatives (exon 10 origin)** | | |
| | 10 AUUUGGAAGCUC-CUGAAGACAAGUCAUUUGGCAG SEQ ID NO: 109 ;;;;;.;;.;;. ;.;;;;..;.;..;.;;..;;; 18 AUUUGCAAUCUUUCGGAAGGAAGGCAACUUCUCAG SEQ ID NO: 110 | **PS816** | CUGCCAAAUGACUUGUCUUCAGGAG | 1679 |
| | | | XYGXXZZZYGZXYYGYXYYXZGGZG | 1680 |
| | | **PS1465** | CUGCCDDAUGACUUGUCUUCAGGDG | 1812 |
| | | **PS1168** | CUGCCDDDUGDCUUGUCUUCDGGDG | 1681 |
| | | **PS1169** | CUGCCDDDUGDCUUGUCUUCDGGDG | 1813 |
| | | **PS1494** | C*UGCCAAAUGACUUGUCUUCAGGAG | 1778 |
| | | **PS1495** | CUGCCAAAUGACUUGUCUUCAGGAG* | 1884 |
| | | **PS1496** | C*UGCCAAAUGACUUGUCUUCAGGAG* | 1885 |
| | | **PS1497** | C*UGCCAAAUGACU*UGUCUUCAGGAG* | 1886 |
| | | | CUGC*CAAAUGACUUGUCUUCAGGAG | 1890 |
| | | | C*U*G*C*C*A*A*AU*G*AC*U*UG*U*CUUCAGGAG | 1891 |
| | | **PS813 and derivatives (exon 18 origin)** | | |
| | | **PS813** | GUCUGAGAAGUUGCCUUCCUUC | 1815 |
| | | **PS1413** | GUCUGDGDDGUUGCCUUCCUUC | 1816 |
| | | **PS1414** | GUCUGAGAAGUUGCCUUCCUUC | 1817 |
| | | **PS1415** | GUCUGAGAAGUUGCC UUCCUUC | 1818 |
| | | **PS1135** | GUCUGAGAAGUUGCCUUCCUUC | 1819 |
| | | **Additional** | | |
| | | **PS1498** | C*UGCCAAAU*ACUUGUU*UUCAGGAG* | 1887 |
| | | | CUGCCAAAUGACUUGUCUU*CAG*A*GA*G | 1888 |
| | | | C*U*G*CCA*A*AU*GAC*UUGUCU*U*CAG*A*GA*G | 1889 |
| | | **PS1125** | CUGAGAAGUUGCCUUCCUUCAGAAAG | 1814 |
| | | **PS1407** | CUGAGAAGUUGCCUUCCUUC | 1820 |
| | | **PS1408** | CUGAGAAGUUGCCUUCCUUCCG | 1821 |
| | | **PS1409** | CUGAGAAGUUGCCUUCCUUCCGAA | 1822 |
| | | **PS1410** | UAAGUCUGAGAAGUUGCCUUCCUUC | 1823 |
| | | **PS1411** | CUGCCAAAUGACUUGUCUUC | 1824 |
| | | **PS1412** | AACUGCCAAAUGACUUGUCUUC | 1825 |
| | | **PS1418** | GUCUGDGDDGUUGCCUUCCUU | 1826 |
| | | **PS1445** | GDCUUGUCUUCDGGDGCUUCC (PS814, ID NO: 1675) | 1827 |
| | | **PS1446** | CDDDUGDCUUGUCUUCDGGDGCUUC (PS815, ID NO: 1677) | 1828 |
| | | **PS1246** | AUGAACUGCCAAAUGACUUGUC | 1780 |
| | | **PS1457** | ACUUGUCUUCAGGAGCUUCCAAAUG | 1781 |
| | | **PS1459** | ACUUGUCUUCAGGDGCUUCCDDDUG (PS1457 mod) | 1829 |
| | | **PS1461** | ACUUGUCUUCAGGAGCUUCCAAAUG (PS1457 mod) | 1830 |
| | | **PS1462** | ACUUGUCUUCAGGDGCUUCCDDDUG (PS1457 mod) | 1831 |
| | | **PS1458** | GUCUUCAGGAGCUUCCAAAUG | 1782 |
| | | **PS1460** | GUCUUCAGGDGCUUCCDDDUG (PS1458 mod) | 1832 |
| | 2) 11/14 (78.6%) | **PS1249 and derivatives** | | |
| | 10 CAGCUUUAGAAGAA  SEQ ID NO: 1766 ;;;..;;;.;;;;; 18 CAGACUUAAAGAA  SEQ ID NO: 1767 | **PS1249** | CUUCUAAAGCUGUUUGA | 1783 |
| | | | XYYXYZZZGXYGYYYGZ | 1833 |
| | | | CUUCUAAAGCUGUUUGA | 1834 |
| | | | CUUCUDDDGCUGUUUGD | 1835 |
| | 3)13/19 (68.4%) | **PS811** | CUUCCUUCCGAAAGAUUGCAAAUUC | 1673,1674 |
| | 10 AUUUCUAAUGAUGUGGAAG  SEQ ID NO: 1768 ;;;;..;;;..;..;;;;; 18 AUUUGCAAUCUUUCGGAAG  SEQ ID NO: 1769 | | | |
| 10-30 | 1) 41/70 (58.6%) | **PS1245 and derivatives (exon 30 origin)** | | |
| | 10 GACAAGUCAUUUGGCAGUUCAUUGAUGGAGAGUGAAGUAAACCUGGACCG// ;;;;;; ;;.;;;;;;;.;.;;        ;.;;.;;..;;..;;;;;.. 30 GACAAG-CAGUUGGCAGCUUAU--------AUUGCAGACAAGGUGGACGC// //UUAUCAAACAGCUUUAGAAG SEQ ID NO: 127 ...;;;;;..;;...;;;; //AGCUCAAAUGCCUCAGGAAG SEQ ID NO: 128 | **PS1245** | AUAAGCUGCCAACUGCUUGUC | 1784 |
| | | | ZYZZGXYGXXZZXYGXYYGYX | 1836 |
| | | **PS1358 and derivatives (exon 30 origin)** | | |
| | | **PS1358** | CUGGGCUUCCUGAGGCAUUUGAGCU | 1786 |
| | | | XYGGGXYYXXYGZGGXZYYYGZGXY | 1838 |
| | | **PS816 and derivatives (exon 10 origin)** | | 1679-1681, 1812-1814 |
| | | **Additional** | | |
| | | **PS814** | | 1675, 1676 |
| | | **PS815** | | 1677, 1678 |
| | | **PS1246** | AUGAACUGCCAAAUGACUUGUC | 1780 |
| | | **PS1359** | AUUUGAGCUGCGUCCACCUUGUCUG | 1785 |
| | | | ZYYYGZGXYGXGYXXZXXYYGYXYG | 1837 |
| | 2) 22/35 (62.9%) | **PS1016 and derivatives (exon 30 origin)** | | |
| | 10 GGAGAGUGAAGUAAACCUGGACCGUUAUCAAACAG SEQ ID NO: 1772 ;;;;;.;;;;. ;;.;;;....;.;.;;;.;..;; 30 GGAGACUGAAA-AAUCCUUACACUUAAUCCAGGAG SEQ ID NO: 1773 | **PS1016** | CAGUCUCCUGGGCAGACUGGAUGCUC | 1688 |
| | | | XZGYXYXXYGGGXZGZXYGGZYGXYX | 1689 |
| | | **PS1451** | CDGUCUCCUGGGCDGDCUGGDUGCUC | 1839 |
| | | **PS1452** | CAGUCUCCUGGGCDGDCUGGAUGCUC | 1840 |
| | | **PS1453** | CDGUCUCCUGGGCAGACUGGDUGCUC | 1841 |
| | | **PS1448** | CAGUCUCCUGGGCAGACUGGAUGCUC | 1842 |
| | | **PS1449** | CDGUCUCCUGGGCDGDCUGGDUGCUC | 1843 |
| | | **PS1450** | <CAGUCUCCUGGGCAGACUGGAUGCUC> | 1844 |
| | | **Additional** | | |
| | | **PS1454** | GUCUCCUGGGCAGACUGGAUGCUC | 1845 |
| | | | GYXYXXYGGGXZGZXYGGZYGXYX | 1846 |
| | | **PS1455** | CAGUCUCCUGGGCAGACUGGAUGC | 1847 |
| | | | XZGYXYXXYGGGXZGZXYGGZYGX | 1848 |
| | | **PS1456** | GUCUCCUGGGCAGACUGGAUGC | 1849 |
| | | | GYXYXXYGGGXZGZXYGGZYGX | 1850 |
| | | **PS1357** | GACUCCUGGAUUAAGUGUAAGGAUUU | 1787 |
| | | | GZCYCCYGGZYYZZGYGYZZGGZYYY | 1851 |
| | | **PS535 and derivatives (exon 55 origin)** | | |
| 45-55 | 1) 20/25 (80.0%) | **PS535** | CAUCCUGUAGGACAUUGGCAGUUG | 1788 |
| | 45 AAACAGAUGCCAGUAUUCUACAGGA  SEQ ID NO: 1571 ;;;;;..;;;;;.;.;.;;;;;;;; 55 AAACAACUGCCAAUGUCCUACAGGA  SEQ ID NO: 1572 | | XZYXXYGYZGGZXZYYGGXZGYYG | 1852 |
| | | **Additional** | | |
| | | **PS537** | CUGUAGGACAUUGGCAGUUGUUUC | 1789 |
| | | | XYGYZGGZXZYYGGXZGYYGYYYX | 1853 |
| | | **PS1185** | | 1706 |
| | | **PS1186** | | 1707 |
| | | **PS1187** | | 1710 |
| | | **PS1188** | | 1713 |
| | 2)13/18 (72.2%) | **PS479 and derivatives** | | |
| | | **PS479** | CUGAAUUAUUUCUUCCCCAGUUGCA | 1790 |
| | 45 AUGCAACUGGGAAGAAA  SEQ ID NO: 1774 ;.;;..;;..;;;;;;;; 55 AGGCUGCUUGGAAGAAA  SEQ ID NO: 1775 | | XYGZZYYZYYYXYYXXXXZGYYGXZ | 1854 |
| | | | | |
| | | | | |
| | | **Additional** | | |
| | | **PS481** | UUAUUUCUUCCCCAGUUGCAUUCAA | 1792 |
| | | | YYZYYYXYYXXXXZGYYGXZYYXZZ | 1855 |

| | | | | |
|---|---|---|---|---|
| **ADDITIONAL EXON COMBINATIONS** | | | | |
| **Exon Combination** | **Exon Identity Region** | **Oligonucleotides** | | **SEQ ID NO** |
| | **(SEQ ID NO; see Table 2)** | | | |
| 11-23 | 191-192 | UCAGUUUCUUCAUCUUCUGAU | | 1794 |
| | | YXZGYYYXYYXZYXYYXYGZY | | 1861 |
| | | UCAAUUUCUUCAAAUUCUGAU | | 1795 |
| | | YXZZYYYXYYXZZZYYXYGZY | | 1862 |
| | | | CUUCAGUUUCUUCAUCUUCUGAU | 1796 |
| | | | XYYXZGYYYXYYXZYXYYXYGZY | 1863 |
| | | | CCUCAAUUUCUUCAAAUUCUGAU | 1797 |
| | | | XXYXZZYYYXYYXZZZYYXYGZY | 1864 |
| | | | UACUUCAGUUUCUUCAUCUUCUGAU | 1798 |
| | | | YZXYYXZGYYYXYYXZYXYYXYGZY | 1865 |
| | | | UCCCUCAAUUUCUUCAAAUUCUGAU | 1799 |
| | | | YXXXYXZZYYYXYYXZZZYYXYGZY | 1866 |
| 13-30 | 285-286 | | CUACCACCACCAUGUGAGUGA | 1808 |
| | | | XYZXXZXXZXXZYGYGZGYGZ | 1867 |
| | | | CUGCCAACUGCUUGUCAAUGA | 1809 |
| | | | XYGXXZZXYGXYYGYXZZYGZ | 1868 |
| | | | AGUUGCGUGAUCUCCACUAGA | 1810 |
| | | | ZGYYGXGYGZYXYXXZXYZGZ | 1869 |
| | | | AGCUGCGUCCACCUUGUCUGCA | 1811 |
| | | | ZGXYGXGYXXZXXYYGYXYGXZ | 1870 |
| | | | UGAGAGAAUUGACCCUGACUUGU | 1856 |
| | | | YGZGZGZZYYGZXXXYGZXYYGY | 1871 |
| | | | ACCAUGUGAGUGAGAGAAUUGACCCU | 1857 |
| | | | ZXXZYGYGZGYGZGZGZZYYGZXXXY | 1872 |
| | | | CACCACCAUGUGAGUGAGAGA | 1858 |
| | | | XZXXZXXZYGYGZGYGZGZGZ | 1873 |
| | | | CUCCACUAGAUUCAUCAACUAC | 1859 |
| | | | XYXXZXYZGZYYXZYXZZXYZX | 1874 |
| | | | UCUUCCAAAGCAGCAGUUGCGUG | 1860 |
| | | | YXYYXXZZZGXZGXZGYYGXGYG | 1875 |
| 34-53 | 1294-1295 | | UCUGUAGCUGCCAGCCAUU | 1800 |
| | | | YXYGYZGXYGXXZGXXZYY | 1876 |
| | | | UCCUUAGCUUCCAGCCAUU | 1801 |
| | | | YXXYYZGXYYXXZGXXZYY | 1877 |
| | | | UCUGUAGCUGCCAGCCAUUCUGU | 1802 |
| | | | YXYGYZGXYGXXZGXXZYYXYGY | 1878 |
| | | | UCCUUAGCUUCCAGCCAUUGUGU | 1803 |
| | | | YXXYYZGXYYXXZGXXZYYGYGY | 1879 |
| 40-53 | 1477-1478 | | UCUUGUACUGAUACCACUGAU | 1804 |
| | | | YXYYGYZXYGZYZXXZXYGZY | 1880 |
| | | | UCUUGUACUUCAUCCCACUGAU | 1805 |
| | | | YXYYGYZXYYXZYXXZXYGZY | 1881 |
| 44-56 | 1557-1558 | | UCUCAGGAAUUUGUGUCUUU | 1806 |
| | | | YXYXZGGZZYYYGYGYXYYY | 1882 |
| | | | UCUCAGGAUUUUUUGGCUGU | 1807 |
| | | | YXYXZGGZYYYYYYGGXYGY | 1883 |

### Examples 1-5

### Materials and methods

The design of the oligonucleotides was primarily based on reverse complementarity to specific, highly similar, sequence stretches in two different DMD exons, as identified by EMBOSS Matcher and as disclosed in Table 2. Further sequence parameters taken into account were the presence of partly open/closed secondary RNA structures in said sequence stretches (as predicted by RNA structure version 4.5 or RNA mfold version 3.5 (Zuker, M.) and/or the presence of putative SR-protein binding sites in said sequence stretches (as predicted by the ESE-finder software (Cartegni L, et al. 2002 and Cartegni L, et al, 2003). All AONs were synthesized by Prosensa Therapeutics B.V. (Leiden, Netherlands) or obtained from commercial source (ChemGenes, US), and contain 2'-*O*-methyl RNA and full-length phosphorothioate (PS) backbones. All oligonucleotides were 2'-*O*-methyl phosphorothioate RNA, and synthesized in 10µmol scale using an OP-10 synthesizer (GE/ÄKTA Oligopilot), through standard phosphoramidite protocols. Oligonucleotides were cleaved and deprotected in a two-step sequence (DIEA followed by conc. NH₄OH treatment), purified by HPLC and dissolved in water and an excess of NaCl was added to exchange ions. After evaporation, compounds were redissolved in water, desalted by FPLC and lyophilized. Mass spectrometry confirmed the identity of all compounds, and purity (determined by UPLC) was found acceptable for all compounds (>80%). For the *in vitro* experiments described herein, 50 µM working solutions of the AONs were prepared in phosphate buffer (pH 7.0).

### Tissue culturing, transfection and RT-PCR analysis

Differentiated human healthy control muscle cells (myotubes) were transfected in 6-wells plates at a standard AON concentration of 400 nM, according to non-GLP standard operating procedures. For transfection polyethylenimine (ExGen500; Fermentas Netherlands) was used (2 µl per µg AON, in 0.15M NaCl). Aforementioned transfection procedures were adapted from previously reported material and methods (Aartsma-Rus A., et al., 2003). At 24 hrs after transfection, RNA was isolated and analyzed by RT-PCR. Briefly, to generate cDNA, a random hexamer mixture (1.6 µg/µl; Roche Netherlands) was used in the reverse transcriptase (RT) reaction on 500-1000 ng input RNA. The PCR analysis was subsequently done on 3 µl of cDNA for each sample, and included a first and nested PCR using DMD gene specific primers (see Tables 4 and 5). The RNA isolation and RT-PCR analysis were performed according to non-GLP standard operating procedures adapted from previously reported material and methods (Aartsma-Rus A., et al., 2002; and Aartsma-Rus A., et al, 2003). RT-PCR products were analyzed by gel electrophoresis (2% agarose gels). The resulting RT-PCR fragments were quantified through DNA Lab-on-a-Chip analysis (Agilent Technologies USA; DNA 7500 LabChips). The data was processed by "Agilent 2100 Bioanalyzer" software and Excel 2007. The ratio of the smaller transcript products (containing the anticipated multiple exon skipping) to the total amount of transcript products was assessed (representing the skipping efficiencies in percentages), and directly compared to that in non-transfected cells. PCR fragments were also isolated from agarose gels (QIAquick gel extraction kit, Qiagen Netherlands) for sequence verification (Sanger sequencing, BaseClear, Netherlands).

**Table 4. PCR primer sets used for detection of the skipping of the targeted exons**

| **Target** | **1st PCR** | | **2nd PCR** | |
|---|---|---|---|---|
| **Exons** | **forw** | **rev** | **forw** | **rev** |
| 10 to 18 | h7f | h20r | h8f | hl9r |
| 10 to 30 | h7f | h32r | h8f | h31r |
| 10 to 47 | h8f | h50r | h9f | h49r |
| 10 to 57 | h8f | h64r | h9f | h63r2 |
| 45 to 55 | h43f2 | h57r | h44f | h56r |

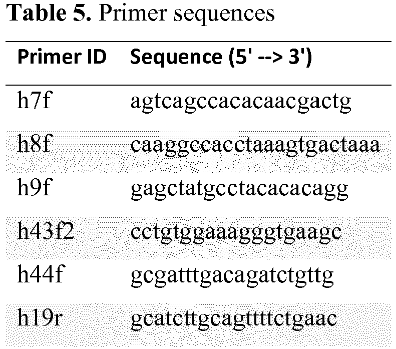

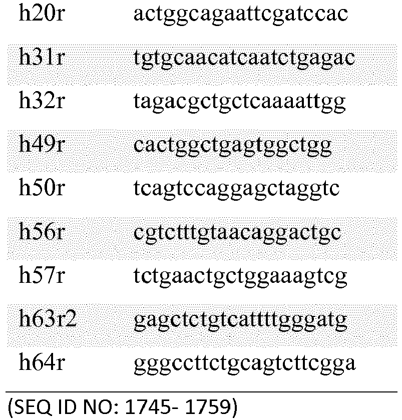

### Results

### Example 1. Targeting the sequence stretch with high similarity in exon 10 and 18 with AONs at different sites.

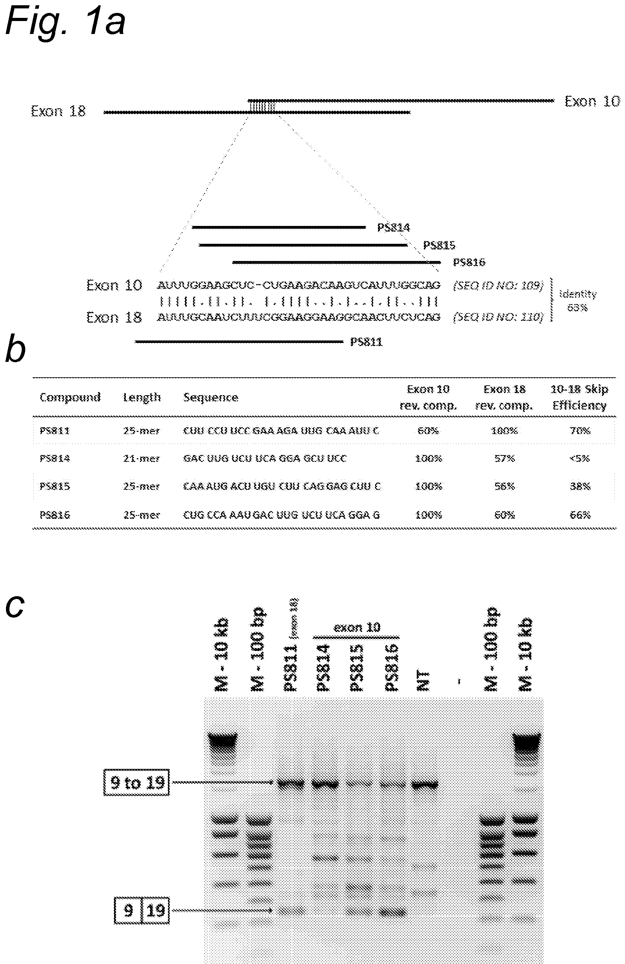

Based on a highly similar (63%) sequence stretch in exons 10 (SEQ ID NO:109) and 18 (SEQ ID NO:110) a series of AONs were designed dispersed over said sequence stretch, either 100% reverse complementary to exon 10 (PS814; SEQ ID NO:1675, PS815; SEQ ID NO:1677, PS816; SEQ ID NO:1679) or to exon 18 (PS811; SEQ ID NO:1673). Following transfection in healthy human control myotube cultures, RT-PCR analysis demonstrated that all four AONs were capable of inducing the skipping of exon 10 to 18 (confirmed by sequence analysis) (Fig.1). PS811 and PS816 have highest reverse complementarity percentages with both exons (Fig.1B) and were most efficient with exon 10 to 18 skipping efficiencies of 70% and 66% respectively (Fig.1C). PS814 was least efficient, which may have been inherent to its location and/or shorter length (21 versus 25 nucleotides) and thus lower binding affinity or stability. No exon 10 to 18 skipping was observed in non-treated cells (NT). These results were highly reproducible (exon 10 to 18 skipping by PS816 in 20/20 different transfections) and demonstrate that the skipping of a multi-exon stretch from exon 10 to 18 is feasible by using a single AON that is capable of binding to both exons 10 and 18, in a region with high sequence similarity (63%), and that is capable of inducing the skipping of these exons and all exons in between. Although additional multiple exon skipping fragments may be obtained in this transcript region, the resulting transcript in which exon 9 was directly spliced to 19 (an in frame transcript) was most abundant in all experiments.

### Example 2. Targeting the sequence stretch with high similarity in exon 10 and 18 with AONs of different lengths.

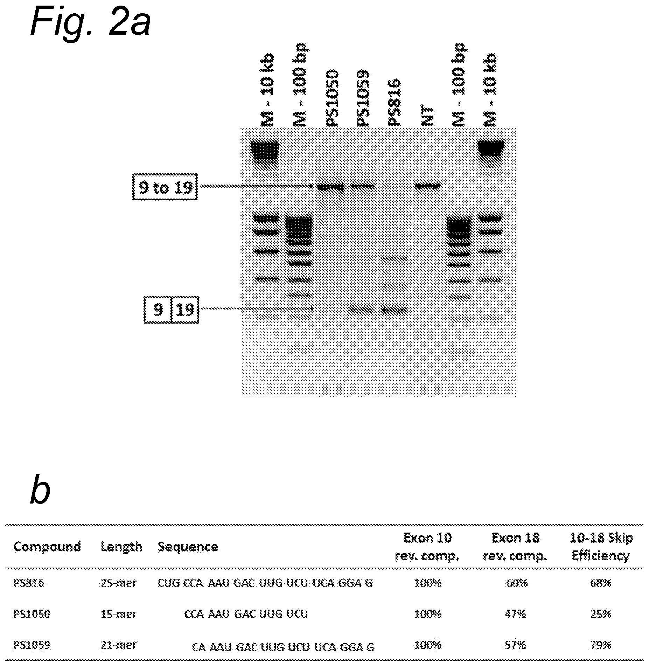

Within the highly similar sequence stretch in exons 10 (SEQ ID NO:109) and 18 (SEQ ID NO:110) we evaluated the effect of AONs with different lengths to identify the most effective minimal length. Following transfection of PS816 (a 25-mer; SEQ ID NO:1679), PS1059 (a 21-mer; SEQ ID NO:1684), or PS1050 (a 15-mer; SEQ ID NO:1682) in healthy human control muscle cells (i.e. differentiated myotubes), RT-PCR analysis demonstrated that all three AONs were capable of inducing the skipping of exon 10 to 18 (confirmed by sequence analysis) (Fig.2A, B). PS816 and PS1059 were most efficient (68% and 79% respectively). The shorter PS1050 was less effective (25%). No exon 10 to 18 skipping was observed in non-treated cells (NT). These results indicate that the skipping of a multi-exon stretch from exon 10 to 18 is feasible by using AONs of 15, 21 and 25 nucleotides. Longer AONs are anticipated to work as well. The 21-mer PS1059 was the most effective, shortest candidate. The 15-mer PS1050 was least efficient which may have been inherent to its reduced reverse complementarity to exon 18 (47%; Fig 2B) and/or reduced binding affinity or stability to the target RNA. Base modifications may be required to enhance the Tm, and thus binding affinity or duplex stability, of 15-mers in order to improve their bioactivity. The most abundant resulting transcript product in this experiment was again that in which exon 9 was directly spliced to 19 (an in frame transcript).

### Example 3. Targeting the sequence stretch with high similarity in exon 10 and 18 with AONs with modified base chemistry

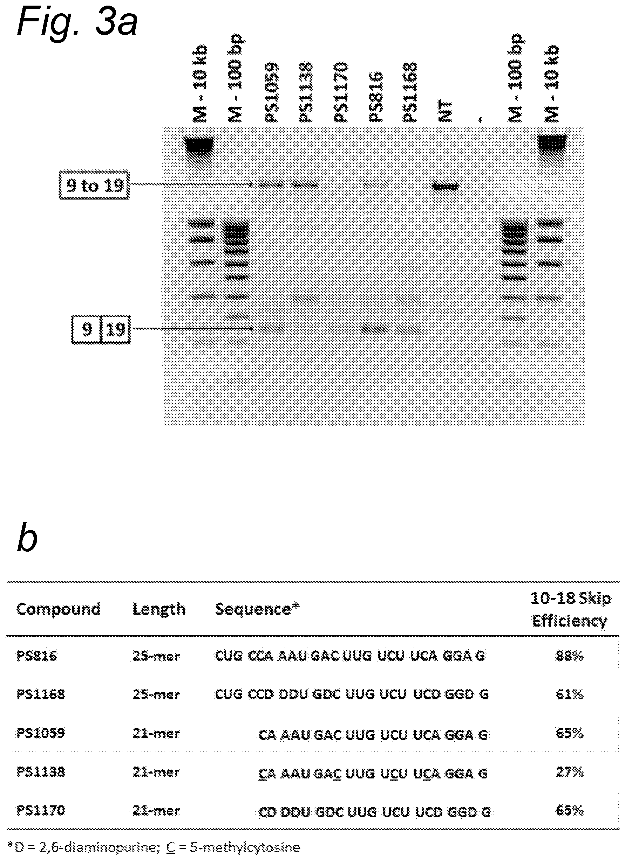

The particular characteristics of a chosen AON chemistry at least in part affects the delivery of an AON to the target transcript: administration route, biostability, biodistribution, intra-tissue distribution, and cellular uptake and trafficking. In addition, further optimization of oligonucleotide chemistry is conceived to enhance binding affinity and stability, enhance activity, improve safety, and/or to reduce cost of goods by reducing length or improving synthesis and/or purification procedures. Within the highly similar sequence stretch in exons 10 (SEQ ID NO:109) and 18 (SEQ ID NO:110) we here evaluated the effect of 2'-*O*-methyl RNA AONs with different modified bases(such as 5-substituted pyrimidines and 2,6-diaminopurines). Following transfection of PS816 (a regular 2'-*O*-methyl RNA AON; SEQ ID NO:1679), PS1168 (PS816 but with all As replaced by 2,6-diaminopurines; SEQ ID NO:1681), PS1059 (a regular 2'-*O*-methyl phosphorothioate RNA AON; SEQ ID NO:1684), PS1138 (PS1059 but with all Cs replaced by 5-methylcytosines; SEQ ID NO:1685), or PS1170 (PS1059 but with all As replaced by 2,6-diaminopurines; SEQ ID NO:1686) (Fig.3B) in healthy human control muscle cells (i.e. differentiated myotubes), RT-PCR analysis demonstrated that all five AONs were capable of inducing the skipping of exon 10 to 18 (confirmed by sequence analysis) (Fig.3). PS816 was most efficient (88%). Although the base modifications in these particular sequences did not further improve bioactivity, they may have a more positive effect on biodistribution, stability, and/or safety such that less efficient compounds are still favored for clinical development. These results indicate that the skipping of a multi-exon stretch from exon 10 to 18 is feasible by using AONs with modified bases. The most abundant resulting transcript product in this experiment was again that in which exon 9 was directly spliced to 19 (an in frame transcript).

### Example 4. PS816 induces the skipping of other in-frame multi-exon stretches

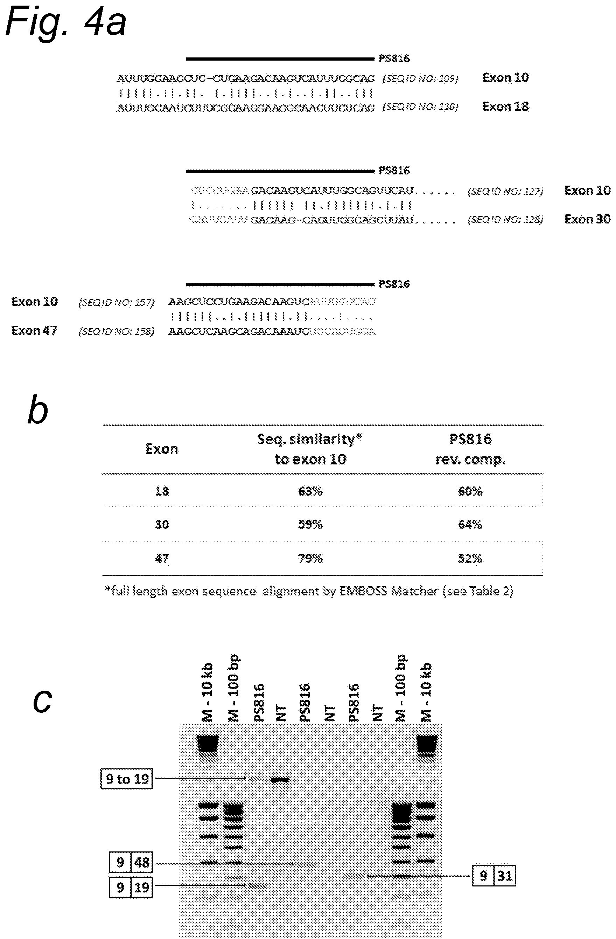

The highly similar sequence stretch in exons 10 (SEQ ID NO:109) and 18 (SEQ ID NO:110) is also (partly) present in exons 30 (SEQ ID NO:128), 31 (SEQ ID NO: 130), 32 (SEQ ID NO:132), 42 (SEQ ID NO:152), 47 (SEQ ID NO:158), 48 (SEQ ID NO:160), 57(SEQ ID NO:170), and 60 (SEQ ID NO:174) (Fig.4A, B, Table 2). We here thus focused on the detection of different multi-exon stretch skipping following transfection of 400 nM PS816 (SEQ ID NO:1679). We used different primer sets (Table 4 and 5) for that purpose. Indeed with RT-PCR analysis we observed the in-frame exon 10 to 30, exon 10 to 42, exon 10 to 47, exon 10 to 57, and/or exon 10 to 60 skipping in multiple experiments (confirmed by sequence analysis), which was not observed in non-treated cells. As an example Fig.4C shows the PS816-induced exon 10 to 18, exon 10 to 30, and exon 10 to 47 skipping. Despite additional multiple exon skipping events (either in-frame or out-of-frame), the resulting transcript in which exon 9 was directly spliced to 19 (an in frame transcript) was most reproducible and seemed most abundant in all experiments. These results confirm that multiple exon skipping can be induced across the DMD gene using a single AON that is capable of binding to a sequence stretch that is highly similar between two different exons and capable of inducing the skipping of these exons and all exons in between to generate an in-frame DMD transcript.

### Example 5. Targeting a sequence stretch with high similarity in exon 45 and 55 with AONs with modified base chemistry.

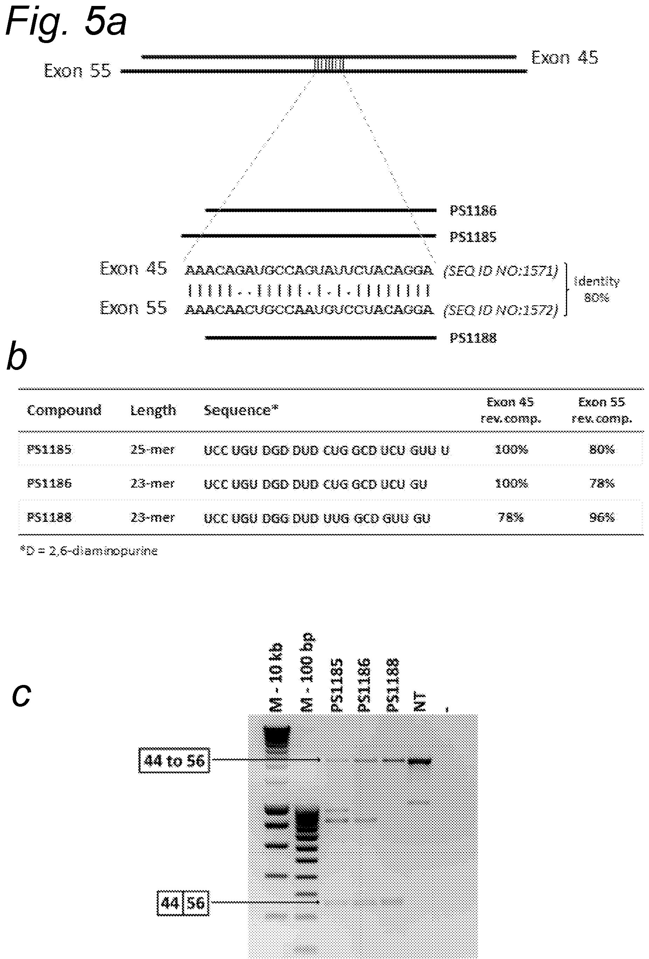

Based on a highly similar (80%) sequence stretch in exons 45 (SEQ ID NO:1571) and 55 (SEQ ID NO:1572) a series of AONs were designed dispersed over said sequence stretch, either 100% reverse complementary to exon 45 (PS1185; SEQ ID NO:1706, PS1186; SEQ ID NO:1707) or 96% to exon 55 (with one mismatch) (PS1188; SEQ ID NO:1713) (Fig.5A). In all three AONs the As were replaced by 2,6-diaminopurines. Following transfection in healthy human control myotube cultures, RT-PCR analysis demonstrated that PS1185, PS1186, and PS1188 were capable of inducing the skipping of exon 45 to 55 (confirmed by sequence analysis) (Fig.5B). No exon 45 to 55 skipping was observed in non-treated cells (NT). These results demonstrate that the skipping of another multi-exon stretch, from exon 45 to 55, is feasible by using a single AON that is capable of binding to both exons 45 and 55, in a region with high sequence similarity (80%), and that is capable of inducing the skipping of these exons and all exons in between. The resulting transcript in which exon 44 was directly spliced to 56 is in-frame was and was observed in multiple experiments. As for the exon 10 to 18 skipping experiments (example 3) we here show again that AONs with modified bases can be applied effectively. Furthermore, the results obtained with PS1188 indicate that AONs do not need to be 100% reverse complementary to one of the target exons, but can also be designed as hybrid structures, in which there is no 100% reverse complmentarity to either target exon.

### Figure legends

**Figure 1****.** A) Localisation of PS811 (SEQ ID NO:1673), PS814 (SEQ ID NO:1675), PS815 (SEQ ID NO:1677) and PS816 (SEQ ID NO:1679) in the sequence stretch that is highly similar (63%) in exon 10 and exon 18. B) AON characteristics and efficiencies. The percentage of reverse complementarity (rev. comp.) of each AON to either exon 10 or exon 18 is indicated. C) RT-PCR analysis. In healthy human muscle cells (i.e. differentiated myotubes) all four AONs were effective in inducing the skipping of a multi-exon stretch from exon 10 to exon 18. PS811 and PS816 were most efficient. Boxes on the left site of the figure represent the content of the PCR amplified transcript products. (M: DNA size marker; NT: non-treated cells)
**Figure 2****.** A) RT-PCR analysis. In healthy human muscle cells (i.e. differentiated myotubes), AONs with different lengths, but with the same core target sequence within the sequence stretch that is highly similar (63%) in exon 10 and exon 18, were tested. PS816 (SEQ ID NO:1679), a 25-mer, and PS1059 (SEQ ID NO:1684), a 21-mer, were most efficient (68% and 79% exon 10 to 18 skipping respectively). The 15-mer PS1050 (SEQ ID NO:1682) was less effective. Boxes on the left site of the figure represent the content of the PCR amplified transcript products. (M: DNA size marker; NT: non-treated cells) B) AON characteristics and efficiencies. The percentage of reverse complementarity (rev. comp.) of each AON to either exon 10 or exon 18 is indicated.
**Figure 3****.** A) RT-PCR analysis. In healthy human muscle cells (i.e. differentiated myotubes), AONs with different base chemistry were tested: PS816 (a regular 2'-*O-*methyl phosphorothioate RNA AON; SEQ ID NO:1679), PS1168 (PS816 but with all As replaced by 2,6-diaminopurines; SEQ ID NO:1681), PS1059 (a regular 2'-*O*-methyl phosphorothioate RNA AON; SEQ ID NO:1684), PS1138 (PS1059 but with all Cs replaced by 5-methylcytosines; SEQ ID NO:1685), or PS1170 (PS1059 but with all As replaced by 2,6-diaminopurines; SEQ ID NO:1686). The skipping of exons 10 to 18 was observed for all AONs tested. PS816 was most efficient (88%). In these specific sequences the base modifications did not further improve bioactivity. Boxes on the left site of the figure indicate the PCR amplified transcript products. (M: DNA size marker; NT: non-treated cells) B) AON characteristics and efficiencies.
**Figure 4****.** A) Highly similar sequence stretches in exons 10, 18, 30, and 47, as multiple targets for PS816. The SEQ ID NO's are referring to the EMBOSS full length exon alignments (as in Table 2). In grey font the sequence part that was not included in the EMBOSS alignment but that is adjacent to the part with high reverse complementarity to PS816. B) Overview of exon alignment and PS816 reverse complementarity (rev. comp.) percentages. C) RT-PCR analysis. Multiple exon stretch skipping may be induced by PS816, here identified as exon 10 to 18, exon 10 to 30, and exon 10 to 47 skipping. The resulting transcripts are in-frame. These were not detected in non-treated cells (NT). Boxes on the left and right site of the figure represent the content of the PCR amplified transcript products. (M: DNA size marker)
**Figure 5****.** A) Localisation of PS1185 (SEQ ID NO:1706), PS1186 (SEQ ID NO:1707), and PS1188 (SEQ ID NO:1713) in the sequence stretch that is highly similar (80%) in exon 45 and exon 55. The table summarizes AON characteristics. The percentage of reverse complementarity (rev. comp.) of each AON to either exon 45 or exon 55 is indicated. B) RT-PCR analysis. In healthy human muscle cells (i.e. differentiated myotubes) all three AONs were effective in inducing the skipping of a multi-exon stretch from exon 45 to exon 55. Boxes on the left site of the figure represent the content of the PCR amplified transcript products. (M: DNA size marker; NT: non-treated cells)

### Reference list

Aartsma-Rus A, Bremmer-Bout M, Janson AA, den Dunnen JT, van Ommen GJ, van Deutekom JC. Targeted exon skipping as a potential gene correction therapy for Duchenne muscular dystrophy. See 1 article found using an alternative search: Neuromuscul Disord. 2002; 12:S71-7.
Aartsma-Rus A, Janson AA, Kaman WE, Bremmer-Bout M, den Dunnen JT, Baas F, van Ommen GJ, van Deutekom JC. Therapeutic antisense-induced exon skipping in cultured muscle cells from six different DMD patients. Hum Mol Genet. 2003; 12(8):907-14.
Aartsma-Rus A, Janson AA, Kaman WE, Bremmer-Bout M, van Ommen GJ, den Dunnen JT, van Deutekom JC. Antisense-induced multiexon skipping for Duchenne muscular dystrophy makes more sense. Am. J. Hum. Genet. 2004; Jan 74(1):83-92.
Alloza I et al., Genes Immun 2012; 13(3):253-257
Abeliovich A et al., J Neurochem 2006; 99:1062-1072
van Ommen GJ, van Deutekom J, Aartsma-Rus A. The therapeutic potential of antisense-mediated exon skipping. Curr Opin Mol Ther. 2008; 10(2):140-9.
Andrade MA, Perez-Iratxeta C, Ponting CP. Protein repeats: structures, functions, and evolution. J Struct Biol. 2001; 134(2-3):1 17-31.
Arai K, Uchiyama N, Wada T. Synthesis and properties of novel 2'-O-alkoxymethyl-modified nucleic acids. Bioorg Med Chem Lett. 2011; 21(21):6285-7.
Béroud C, Tuffery-Giraud S, Matsuo M, Hamroun D, Humbertclaude V, Monnier N, Moizard MP, Voelckel MA, Calemard LM, Boisseau P, Blayau M, Philippe C, Cossée M, Pagès M, Rivier F, Danos O, Garcia L, Claustres M. Multiexon skipping leading to an artificial DMD protein lacking amino acids from exons 45 through 55 could rescue up to 63% of patients with Duchenne muscular dystrophy. Hum Mutat. 2007; Feb;28(2):196-202.
Bomont P et al., Nat Genet 2000; 26(3):370-374
Cartegni L, Chew SL, Krainer AR.Listening to silence and understanding nonsense: exonic mutations that affect splicing. Nat Rev Genet 2002; 3(4):285-98.
Cartegni L, Wang J, Zhu Z, Zhang MQ, Krainer AR. ESEfinder: A web resource to identify exonic splicing enhancers. Nucleic Acids Res 2003; 31(13):3568-71.
Chabriat H et al., Lancet Neurol 2009; 8(7):643-653
Cheng AJ and Van Dyke MW. Oligodeoxyribonucleotide length and sequence effects on intramolecular and intermolecular G-quartet formation. Gene. 1997; 197(1-2):253-60.
Cirak S, Arechavala-Gomeza V, Guglieri M, Feng L, Torelli S, Anthony K, Abbs S, Garralda ME, Bourke J, Wells DJ, Dickson G, Wood MJ, Wilton SD, Straub V, Kole R, Shrewsbury SB, Sewry C, Morgan JE, Bushby K, Muntoni F. Exon skipping and dystrophin restoration in patients with Duchenne muscular dystrophy after systemic phosphorodiamidate morpholino oligomer treatment: an open-label, phase 2, dose-escalation study. Lancet. 2011; 378(9791): 595-605.
Cirak S et al., Brain 2010; 133(Pt 7):2123-2135
Diebold SS, Massacrier C, Akira S, Paturel C, Morel Y, Reis e Sousa C. Nucleic acid agonists for Toll-like receptor 7 are defined by the presence of uridine ribonucleotides. Eur J Immunol; 2006; 36(12): 3256-67.
Dhanoa BS et al., Hum Genomics 2013; 7(1):13
Dorn A, Kippenberger S. Clinical application of CpG-, non-CpG-, and antisense oligodeoxynucleotides as immunomodulators. Curr Opin Mol Ther. 2008; 10(1):10-20.
Dubosq-Bidot L et al., Eur Heart J 2009; 30(17):2128-2136
Ehmsen J. et al, J. Cell Sci. 2002, 115 (Ptl4): 2801-2803.
Fiorillo C et al., Neurogenetics 2012; 13(3):195-203
Friedman JS et al., Am J Hum genet 2009; 84(6):792-800
Goemans NM, Tulinius M, van den Akker JT, Burm BE, Ekhart PF, Heuvelmans N, Holling T, Janson AA, Platenburg GJ, Sipkens JA, Sitsen JM, Aartsma-Rus A, van Ommen GJ, Buyse G, Darin N, Verschuuren JJ, Campion GV, de Kimpe SJ, van Deutekom JC. Systemic administration of PR0051 in Duchenne's muscular dystrophy. N Engl J Med. 2011;364(16):1513-22.
Goyenvalle A, Wright J, Babbs A, Wilkins V, Garcia L, Davies KE. Engineering Multiple U7snRNA Constructs to Induce Single and Multiexon-skipping for Duchenne Muscular Dystrophy. Mol Ther. 2012 Jun;20(6):1212-21.
Heemskerk H, de Winter C, van Kuik P, Heuvelmans N, Sabatelli P, Rimessi P, Braghetta P, van Ommen GJ, de Kimpe S, Ferlini A, Aartsma-Rus A, van Deutekom JC. Preclinical PK and PD studies on 2'-O-methyl-phosphorothioate RNA antisense oligonucleotides in the mdx mouse model. Mol Ther. 2010; 18(6):1210-7.
Heneka MT et al., Nature2013; 493(7434):674-678
Hodgetts S, Radley H, Davies M, Grounds MD. Reduced necrosis of dystrophic muscle by depletion of host neutrophils, or blocking TNFalpha function with Etanercept in mdx mice. Neuromuscul Disord. 2006; 16(9-10):591-602.
Hovnanian A, Cell Tissue Res 2013; 351(2):289-300
Huang JY et al., Hum Reprod 2013; 28(4):1127-1134
KnaufF et al., Kidney Int 2013; doi:10.1038/ki.2013.207
Krieg AM, Yi AK, Matson S, Waldschmidt TJ, Bishop GA, Teasdale R, Koretzky GA, Klinman DM. CpG motifs in bacterial DNA trigger direct B-cell activation. Nature. 1995; 374(6522):546-9.
Krieg, AM. The role of CpG motifs in innate immunity. Curr. Opin. Immunol. 2000; 12: 35-43.
Kumar L, Pharm. Technol. 2008, 3, 128.
Louis-Dit-Picard H et al., Nat Genet 2012; 44(4):456-460
Macaya RF, Waldron JA, Beutel BA, Gao H, Joesten ME, Yang M, Patel R, Bertelsen AH, Cook AF. Structural and functional characterization of potent antithrombotic oligonucleotides possessing both quadruplex and duplex motifs. Biochemistry. 1995; 34(13):4478-92.
Manzur AY, Kuntzer T, Pike M, Swan A. Glucocorticoid corticosteroids for Duchenne muscular dystrophy. Cochrane Database Syst Rev. 2008; (1):CD003725.
Monaco A.P., et al., Genomics 1988; 2: 90-95.
Peacock H, Fucini RV, Jayalath P, Ibarra-Soza JM, Haringsma HJ, Flanagan WM, Willingham A, Beal PA. Nucleobase and ribose modifications control immunostimulation by a microRNA-122-mimetic RNA. J. Am. Chem. Soc. 2011, 133(24): 9200-3.
Mollie A et al., Hum Genet 2011; 129(6):641-654
Moller RS et al., Epilepsia 2013; 54(2):256-264
Noris P et al., Blood 2011; 117(24):6673-6680
Popovic PJ, DeMarco R, Lotze MT, Winikoff SE, Bartlett DL, Krieg AM, Guo ZS, Brown CK, Tracey KJ, Zeh HJ 3rd. High mobility group B1 protein suppresses the human plasmacytoid dendritic cell response to TLR9 agonists.. J of Immunol 2006; 177: 8701-8707.
Puente XS et al., Nature 2011; 475(7354):101-105
Raciti GA et al., Diabetologia 2011; 54(11):2911-2922
Rantamaki T et al., Am J Hum Genet 1999; 64(4):993-1001
Remington: The Science and Practice of Pharmacy, 20th Edition. Baltimore, MD: Lippincott Williams & Wilkins, 2000
Sirmaci A et al., Am J Hum Genet 2011; 89(2):289-294
Stuart HM et al., Am J Hum Genet 2013; 92(2):259-264
Suzuki K, Doi T, Imanishi T, Kodama T, Tanaka T. Oligonucleotide aggregates bind to the macrophage scavenger receptor. Eur J Biochem. 1999; 260(3):855-60.
Uchida K et al., Immunology 2005; 116(1):53-63
van Deutekom JC, Janson AA, Ginjaar IB, Frankhuizen WS, Aartsma-Rus A, Bremmer-Bout M, den Dunnen JT, Koop K, van der Kooi AJ, Goemans NM, de Kimpe SJ, Ekhart PF, Venneker EH, Platenburg GJ, Verschuuren JJ, van Ommen GJ. Local dystrophin restoration with antisense oligonucleotide PRO051. N Engl J Med. 2007; 357(26):2677-86.
van Vliet L, de Winter CL, van Deutekom JC, van Ommen GJ, Aartsma-Rus A. Assessment of the feasibility of exon 45-55 multiexon skipping for Duchenne muscular dystrophy. BMC Med Genet. 2008, Dec 1; 9:105.
Wagner, H., Bacterial CpG DNA activates immune cells to signal infectious danger Adv. Immunol. 1999; 73: 329-368.
Yokota T, Duddy W, Partridge T. Optimizing exon skipping therapies for DMD. Acta Myol. 2007; 26(3):179-84.
Yokota T, Hoffman E, Takeda S. Antisense oligo-mediated multiple exon skipping in a dog model of duchenne muscular dystrophy. Methods Mol Biol. 2011;709:299-312.
Zuker M. Mfold web server for nucleic acid folding and hybridization prediction. Nucleic Acids Res. 2003; 31 (13), 3406-3415.

## Claims

1. A method for designing an oligonucleotide for producing a functional or semi-functional protein, wherein said method comprises the following steps:
(a) identifying an in-frame combination of a first and a second exon in a same pre-mRNA, wherein a region of said second exon has at least 50% identity with a region of said first exon;
(b) designing an oligonucleotide that is capable of binding to said region of said first exon and said region of said second exon;
(c) wherein said binding results in the skipping of said first and said second exon, preferably in the skipping of a multi-exon stretch starting with said first exon and encompassing one or more exons present between said first and said second exons and at the most in the skipping of the entire stretch of exons in between said first and said second exons, and
(d) wherein said method leads to an oligonucleotide.

2. A method according to claim 1, wherein the oligonucleotide is capable of binding to a part of a region within a first exon and a part of a region within a second region.

3. A method according to claim 1 or 2, wherein the binding of said oligonucleotide is capable of interfering with at least one splicing regulatory sequence in said regions of said first and second exons and/or with the secondary structure encompassing at least said first and/or said second exon in said pre-mRNA, preferably, wherein said splicing regulatory sequence comprises an exonic splicing enhancer (ESE), an exon recognition sequence (ERS) and/or a binding site for a serine-arginine (SR) protein.

4. A method according to any one of claims 1 to 3, wherein the oligonucleotide is capable of inducing the skipping of the entire stretch of exons between said first exon and said second exon.

5. A method according to any one of claims 1 to 4, wherein said oligonucleotide comprises 10 to 40 nucleotides.

6. A method according to any one of claim 1 to 5, wherein said oligonucleotide comprises at least one modification compared to a naturally occurring ribonucleotide- or deoxyribonucleotide-based oligonucleotide, more preferably
(a) at least one base modification, preferably selected from 2-thiouracil, 2-thiothymine, 5-methylcytosine, 5-methyluracil, thymine, 2,6-diaminopurine, more preferably selected from 5-methylpyrimidine and 2,6-diaminopurine; and/or
(b) at least one sugar modification, preferably selected from 2'-*O*-methyl, 2'-*O*-(2-methoxy)ethyl, 2'-*O*-deoxy (DNA), 2'-F, morpholino, a bridged nucleotide or BNA, or the oligonucleotide comprises both bridged nucleotides and 2'-deoxy modified nucleotides (BNA/DNA mixmers), more preferably the sugar modification is 2'-*O*-methyl; and/or
(c) at least one backbone modification, preferably selected from phosphorothioate or phosphorodiamidate, more preferably the backbone modification is phosphorothioate.

7. A method according to any one of claims 1 to 6, wherein said oligonucleotide comprises one or more conjugate groups, optionally protected, selected from the group consisting of peptides, proteins, carbohydrates, drugs, targeting moieties, uptake enhancing moieties, solubility enhancing moieties, pharmacodynamics enhancing moieties, pharmacokinetics enhancing moieties, polymers, ethylene glycol derivatives, vitamins, lipids, polyfluoroalkyl moieties, steroids, cholesterol, fluorescent moieties, reporter molecules, radioactively labeled moieties and combinations thereof, attached directly or *via* a divalent or multivalent linker, to a terminal or internal residue.

8. A method according to any one of claims 1 to 7, wherein said first and second exon are selected from exons 10, 18, 30, 8, 9 11, 13, 19, 22, 23, 34, 40, 42, 44, 45, 47, 51, 53, 55, 56, 57 or 60 of dystrophin pre-mRNA.

9. A method according to any one of claims 1 to 7, wherein said first exon is exon 10 and said second exon is exon 12, 13, 14, 15, 16, 18, 20, 22, 23, 25, 26, 27, 28, 29 30, 31, 32, 33, 34, 35, 36, 37, 38, 39, 40, 41, 42, 44, 46, 47, 48 ,49, 51, 53, 55, 57 ,59 or 60.

10. A method according to claim 8 or 9, wherein the oligonucleotide is capable of inducing the skipping of dystrophin exons 10 to 18, exons 10 to 30, exons 10 to 42, exons 10 to 47, exons 10 to 57, exons 10 to 60, exons 8 to 19, exons 9 to 22, exons 9 to 30, exons 11 to 23, exons 13 to 30, exons 23 to 42, exons 34 to 53, exons 40 to 53, exons 44 to 56, exons 45 to 51, exons 45 to 53, exons 45 to 55, exons 45 to 60, and/or exons 56 to 60.

11. An antisense oligonucleotide obtainable by the method of any one of claim 1 to 10, wherein said oligonucleotide is capable of binding to a region within a first exon and a region within a second exon, wherein said region of said second exon has at least 50% identity with said region of said first exon,
wherein said first and said second exons are within a same dystrophin pre-mRNA in said subject,
wherein said binding of said oligonucleotide is capable of interfering with at least one splicing regulatory sequence in said regions of said first and second exons and/or with the secondary structure encompassing at least said first and/or said second exon in said pre-mRNA,
wherein said splicing regulatory sequence comprises an exonic splicing enhancer (ESE), an exon recognition sequence (ERS) and/or a binding site for a serine-arginine (SR) protein, or and/or
wherein said binding of said oligonucleotide results in the skipping of said first exon and of said second exon, preferably in the skipping of a multi-exon stretch starting with said first exon and encompassing one or more exons present between said first and said second exons and at the most in the skipping of the entire stretch of exons in between said first and said second exons,
wherein an in-frame transcript is obtained allowing production of a functional or semi-functional dystrophin and
wherein said oligonucleotide sequence is not part of a cocktail or a combination of two or more distinct oligonucleotide sequences possibly linked with one or more linker(s), wherein said first and second exon are defined as follows:
- first exon is exon 10 and second exon is 18,
- first exon is exon 10 and second exon is 30,
- first exon is exon 10 and second exon is 42,
- first exon is exon 10 and second exon is 47,
- first exon is exon 10 and second exon is 57, or
- first exon is exon 10 and second exon is 60.

12. An oligonucleotide according to claim 11, wherein:
- region of exon 10 comprises or consists of SEQ ID NO: 109 and region of exon 18 comprises or consists of SEQ ID NO: 110 when said first exon is exon 10 and said second exon is exon 18;
- region of exon 10 comprises or consists of SEQ ID NO: 1766 and region of exon 18 comprises or consists of SEQ ID NO: 1767 when said first exon is exon 10 and said second exon is exon 18;
- region of exon 10 comprises or consists of SEQ ID NO: 1768 and region of exon 18 comprises or consists of SEQ ID NO: 1769 when said first exon is exon 10 and said second exon is exon 18;
- region of exon 10 comprises or consists of SEQ ID NO: 127 and region of exon 30 comprises or consists of SEQ ID NO: 128 when said first exon is exon 10 and said second exon is exon 30;
- region of exon 10 comprises or consists of SEQ ID NO: 1772 and region of exon 30 comprises or consists of SEQ ID NO: 1773 when said first exon is exon 10 and said second exon is exon 30;
- region of exon 10 comprises or consists of SEQ ID NO: 151 and region of exon 42 comprises or consists of SEQ ID NO: 152 when said first exon is exon 10 and said second exon is exon 42;
- region of exon 10 comprises or consists of SEQ ID NO: 157 and region of exon 47 comprises or consists of SEQ ID NO: 158 when said first exon is exon 10 and said second exon is exon 47;
- region of exon 10 comprises or consists of SEQ ID NO: 169 and region of exon 57 comprises or consists of SEQ ID NO: 170 when said first exon is exon 10 and said second exon is exon 57; or
- region of exon 10 comprises or consists of SEQ ID NO: 173 and region of exon 60 comprises or consists of SEQ ID NO: 174 when said first exon is exon 10 and said second exon is exon 60.

13. An oligonucleotide according to claim 11 or 12, comprising the base sequence as defined in any one of SEQ ID NO:1673-1681, 1684-1686, 1688, 1689, 1778, 1780-1787, 1812-1851 or 1884-1891, and having a length, which is defined by the number of nucleotides present in said base sequence or which is 1, 2, 3, 4, or 5 nucleotides longer.

14. An oligonucleotide according to any one of claims 11 to 13, comprising part of the base sequence as defined in any one of SEQ ID NO: 1673-1681, 1684-1686, 1688, 1689, 1778, 1780-1787, 1812-1851 or 1884-1891 wherein said part corresponds to the base sequence as defined in any of said sequences with 1, 2, 3, 4 or 5 nucleotides less than defined in said base sequence.

15. An oligonucleotide according to claim 14, comprising:
(a) the base sequence as defined in any one of SEQ ID NO: 1679 to 1681, 1778, 1812, 1813, 1884 to 1886, 1890, or 1891, and having a length of 25, 26, 27, 28, 29 or 30 nucleotides or SEQ ID NO: 1814 and having a length of 26, 27, 28, 29 or 30 nucleotides; or
(b) the base sequence as defined in SEQ ID NO: 1688, 1689, or 1839 to 1844, and having a length of 26, 27, 28, 29 or 30 nucleotides; or
(c) the base sequence as defined in SEQ ID NO: 1673 or 1674 and having a length of 25, 26, 27, 28, 29 or 30 nucleotides; or
(d) the base sequence as defined in SEQ ID NO: 1675 or 1676 and having a length of 21, 22, 23, 24, 25, 26, 27, 28, 29 or 30 nucleotides; or
(e) the base sequence as defined in SEQ ID NO: 1677 or 1678 and having a length of 25, 26, 27, 28, 29 or 30 nucleotides; or
(f) the base sequence as defined in any one of SEQ ID NO: 1684 to 1686 and having a length of 21, 22, 23, 24, 25, 26, 27, 28, 29 or 30 nucleotides; or
(g) the base sequence as defined in any one of SEQ ID NO: 1815 to 1819 and having a length of 22, 23, 24, 25, 26, 27, 28, 29 or 30 nucleotides, or
(h) the base sequence as defined in any one of SEQ ID NO: 1820, 1824, and having a length of 20, 21, 22, 23, 24, 25, 26, 27, 28, 29 or 30 nucleotides, or
(i) the base sequence as defined in any one of SEQ ID NO: 1826, 1780, 1782, 1832 and having a length of 21, 22, 23, 24, 25, 26, 27, 28, 29 or 30 nucleotides, or
(j) base sequence as defined in any one of SEQ ID NO: 1821, 1825 and having a length of 22, 23, 24, 25, 26, 27, 28, 29 or 30 nucleotides, or
(k) base sequence as defined in any one of SEQ ID NO: 1822 and having a length of 24, 25, 26, 27, 28, 29 or 30 nucleotides, or
(l) base sequence as defined in any one of SEQ ID NO: 1823, 1781, 1829, 1830, 1831 and having a length of 25, 26, 27, 28, 29 or 30 nucleotides, or
(m) base sequence as defined in anyone of SEQ ID NO: 1783, 1833, 1834, 1835 and having a length of 17, 18, 19, 20, 21, 22, 23, 24, 25, 26, 27, 28, 29 or 30 nucleotides, or
(n) base sequence as defined in any one of SEQ ID NO: 1887 and have a length of 24, 25, 26, 27, 28, 29 or 30 nucleotides, or
(o) base sequence as defined in any one of SEQ ID NO: 1888 or 1889 and have a length of 26, 27, 28, 29 or 30 nucleotides, or
(p) base sequence as defined in SEQ ID NO: 1827 and have a length of 21, 22, 23, 24, 25, 26, 27, 28, 29 or 30 nucleotides, or
(q) base sequence as defined in SEQ ID NO: 1828 and have a length of 25, 26, 27, 28, 29 or 30 nucleotides, or
(r) base sequence as defined in any one of SEQ ID NO: 1784, 1836 and having a length of 21, 22, 23, 24, 25, 26, 27, 28, 29 or 30 nucleotides, or
(s) base sequence as defined in any one of SEQ ID NO: 1786, 1838 and having a length of 25, 26, 27, 28, 29 or 30 nucleotides, or
(t) base sequence as defined in any one of SEQ ID NO: 1780 and having a length of 22, 23, 24, 25, 26, 27, 28, 29 or 30 nucleotides, or
(u) base sequence as defined in any one of SEQ ID NO: 1785, 1837 and having a length of 25, 26, 27, 28, 29 or 30 nucleotides, or
(v) base sequence as defined in any one of SEQ ID NO: 1845, 1846, 1847, 1848 and having a length of 24, 25, 26, 27, 28, 29 or 30 nucleotides, or
(w) base sequence as defined in any one of SEQ ID NO: 1849, 1850 and having a length of 22, 23, 24, 25, 26, 27, 28, 29 or 30 nucleotides, or
(x) base sequence as defined in any one of SEQ ID NO: 1787, 1851 and having a length of 26, 27, 28, 29 or 30 nucleotides.

16. An oligonucleotide according to any one of claims 11-15, for use as a medicament for preventing, delaying, ameliorating and/or treating a disease in a subject.

17. A composition comprising one single oligonucleotide according to any one of claims 11-15 and optionally further comprising a pharmaceutically acceptable carrier, diluent, excipient, salt, adjuvant and/or solvent, preferably wherein said composition comprises two or more identical oligonucleotides as defined in any one of claims 11 to 15 linked by a counterion, preferably calcium, more preferably wherein said composition is for use as a medicament for preventing, delaying, ameliorating and/or treating DMD or BMD in a subject.

## Patentansprüche

1. Verfahren zur Gestaltung eines Oligonucleotids zur Herstellung eines funktionellen oder semifunktionellen Proteins, wobei das Verfahren die folgenden Schritte umfasst:
(a) Identifizieren einer in-frame-Kombination eines ersten und eines zweiten Exons in einer gleichen prä-mRNA, wobei eine Region des zweiten Exons mindestens 50% Identität mit einer Region des ersten Exons aufweist;
(b) Gestalten eines Oligonucleotids, das in der Lage ist, an die Region des ersten Exons und die Region des zweiten Exons zu binden;
(c) wobei die Bindung zum Überspringen des ersten und des zweiten Exons führt, bevorzugt zum Überspringen von einer Multiexon-Strecke, die mit dem ersten Exon beginnt und ein oder mehrere Exons umfasst, die zwischen dem ersten und dem zweiten Exon vorhanden sind, und höchstens zum Überspringen der gesamten Strecke von Exons zwischen dem ersten und dem zweiten Exon, und
(d) wobei das Verfahren zu einem Oligonucleotid führt.

2. Verfahren nach Anspruch 1, wobei das Oligonucleotid in der Lage ist, an einen Teil einer Region innerhalb eines ersten Exons und einen Teil einer Region innerhalb einer zweiten Region zu binden.

3. Verfahren nach Anspruch 1 oder 2, wobei die Bindung des Oligonucleotids in der Lage ist, mindestens eine spleißregulatorische Sequenz in den genannten Regionen des ersten und des zweiten Exons und/oder die Sekundärstruktur, die mindestens das erste und/oder das zweite Exon umfasst, in der prä-mRNA zu beeinflussen, wobei die spleißregulatorische Sequenz bevorzugt einen exonischen Spleiß-Enhancer (ESE), eine Exon-Erkennungssequenz (ERS) und/oder eine Bindungsstelle für ein Serin-Arginin (SR)-Protein umfasst.

4. Verfahren nach irgendeinem der Ansprüche 1 bis 3, wobei das Oligonucleotid in der Lage ist, das Überspringen der gesamten Strecke der Exons zwischen dem ersten Exon und dem zweiten Exon zu induzieren.

5. Verfahren nach irgendeinem der Ansprüche 1 bis 4, wobei das Oligonucleotid 10 bis 40 Nucleotide umfasst.

6. Verfahren nach irgendeinem der Ansprüche 1 bis 5, wobei das Oligonucleotid im Vergleich zu einem natürlich vorkommenden Oligonucleotid auf Ribonucleotid- oder Desoxyribonucleotidbasis mindestens eine Modifikation umfasst, bevorzugter
(a) mindestens eine Basenmodifikation, bevorzugt ausgewählt aus 2-Thiouracil, 2-Thiothymin, 5-Methylcytosin, 5-Methyluracil, Thymin, 2,6-Diaminopurin, bevorzugter ausgewählt aus 5-Methylpyrimidin und 2,6-Diaminopurin; und/oder
(b) mindestens eine Zuckermodifikation, bevorzugt ausgewählt aus 2'-O-Methyl, 2'-O-(2-Methoxy)ethyl, 2'-O-Desoxy (DNA), 2'-F, Morpholino, einem verbrückten Nucleotid oder BNA, oder das Oligonucleotid umfasst sowohl verbrückte Nucleotide als auch 2'-Desoxy-modifizierte Nucleotide (BNA/DNA-Mixmere), wobei die Zuckermodifikation bevorzugter 2'-O-Methyl ist; und/oder
(c) mindestens eine Hauptkettenmodifikation, bevorzugt ausgewählt aus Phosphorthioat oder Phosphordiamidat, wobei die Hauptkettenmodifikation bevorzugter Phosphorthioat ist.

7. Verfahren nach irgendeinem der Ansprüche 1 bis 6, wobei das Oligonucleotid eine oder mehrere konjugierte Gruppen umfasst, die gegebenenfalls geschützt sind, ausgewählt aus der Gruppe bestehend aus Peptiden, Proteinen, Kohlenhydraten, Arzneimitteln, Zielkomponenten, die Aufnahme erhöhenden Komponenten, die Löslichkeit verstärkenden Komponenten, die Pharmakodynamik verstärkenden Komponenten, die Pharmakokinetik verstärkenden Komponenten, Polymeren, Ethylenglycolderivaten, Vitaminen, Lipiden, Polyfluoralkylkomponenten, Steroiden, Cholesterin, Fluoreszenzkomponenten, Reportermolekülen, radioaktiv markierten Komponenten und Kombinationen davon, die direkt oder über einen zweiwertigen oder mehrwertigen Linker an einen endständigen oder internen Rest gebunden sind.

8. Verfahren nach irgendeinem der Ansprüche 1 bis 7, wobei das erste und das zweite Exon aus den Exons 10, 18, 30, 8, 9, 11, 13, 19, 22, 23, 34, 40, 42, 44, 45, 47, 51, 53, 55, 56, 57 oder 60 von Dystrophin-prä-mRNA ausgewählt sind.

9. Verfahren nach irgendeinem der Ansprüche 1 bis 7, wobei das erste Exon Exon 10 ist und das zweite Exon Exon 12, 13, 14, 15, 16, 18, 20, 22, 23, 25, 26, 27, 28, 29, 30, 31, 32, 33, 34, 35, 36, 37, 38, 39, 40, 41, 42, 44, 46, 47, 48, 49, 51, 53, 55, 57, 59 oder 60 ist.

10. Verfahren nach Anspruch 8 oder 9, wobei das Oligonucleotid in der Lage ist, das Überspringen der Exons 10 bis 18, Exons 10 bis 30, Exons 10 bis 42, Exons 10 bis 47, Exons 10 bis 57, Exons 10 bis 60, Exons 8 bis 19, Exons 9 bis 22, Exons 9 bis 30, Exons 11 bis 23, Exons 13 bis 30, Exons 23 bis 42, Exons 34 bis 53, Exons 40 bis 53, Exons 44 bis 56, Exons 45 bis 51, Exons 45 bis 53, Exons 45 bis 55, Exons 45 bis 60 und/oder Exons 56 bis 60 von Dystrophin zu induzieren.

11. Antisense-Oligonucleotid, erhältlich durch das Verfahren nach irgendeinem der Ansprüche 1 bis 10,
wobei das Oligonucleotid in der Lage ist, an eine Region innerhalb eines ersten Exons und eine Region innerhalb einer zweiten Exons zu binden, wobei die Region des zweiten Exons mindestens 50% Identität mit der Region des ersten Exons aufweist,
wobei das erste und das zweite Exon innerhalb einer gleichen prä-mRNA von Dystrophin in dem Subjekt sind,
wobei die Bindung des Oligonucleotids in der Lage ist, mindestens eine spleißregulatorische Sequenz in den genannten Regionen des ersten und des zweiten Exons und/oder die Sekundärstruktur, die mindestens das erste und/oder das zweite Exon umfasst, in der prä-mRNA zu beeinflussen,
wobei die spleißregulatorische Sequenz einen exonischen Spleiß-Enhancer (ESE), eine Exon-Erkennungssequenz (ERS) und/oder eine Bindungsstelle für ein Serin-Arginin (SR)-Protein umfasst, und/oder
wobei die Bindung des Oligonucleotids zum Überspringen des ersten Exons und des zweiten Exons führt, bevorzugt zum Überspringen von einer Multiexon-Strecke, die mit dem ersten Exon beginnt und ein oder mehrere Exons umfasst, die zwischen dem ersten und dem zweiten Exon vorhanden sind, und höchstens zum Überspringen der gesamten Strecke von Exons zwischen dem ersten und dem zweiten Exon,
wobei ein in-frame-Transkript erhalten wird, das die Herstellung von einem funktionellen oder semifunktionellen Dystrophin ermöglicht, und
wobei die Oligonucleotidsequenz nicht Teil eines Cocktails oder einer Kombination von zwei oder mehr verschiedenen Oligonucleotidsequenzen, die möglicherweise mit einem oder mehreren Linker(n) verknüpft sind, ist,
wobei das erste Exon und das zweite Exon wie folgt definiert sind:
- das erste Exon ist Exon 10 und das zweite Exon ist 18,
- das erste Exon ist Exon 10 und das zweite Exon ist 30,
- das erste Exon ist Exon 10 und das zweite Exon ist 42,
- das erste Exon ist Exon 10 und das zweite Exon ist 47,
- das erste Exon ist Exon 10 und das zweite Exon ist 57 oder
- das erste Exon ist Exon 10 und das zweite Exon ist 60.

12. Oligonucleotid nach Anspruch 11, wobei:
- die Region von Exon 10 SEQ ID NO: 109 umfasst oder daraus besteht und die Region von Exon 18 SEQ ID NO: 110 umfasst oder daraus besteht, wenn das erste Exon Exon 10 ist und das zweite Exon Exon 18 ist;
- die Region von Exon 10 SEQ ID NO: 1766 umfasst oder daraus besteht und die Region von Exon 18 SEQ ID NO: 1767 umfasst oder daraus besteht, wenn das erste Exon Exon 10 ist und das zweite Exon Exon 18 ist;
- die Region von Exon 10 SEQ ID NO: 1768 umfasst oder daraus besteht und die Region von Exon 18 SEQ ID NO: 1769 umfasst oder daraus besteht, wenn das erste Exon Exon 10 ist und das zweite Exon Exon 18 ist;
- die Region von Exon 10 SEQ ID NO: 127 umfasst oder daraus besteht und die Region von Exon 30 SEQ ID NO: 128 umfasst oder daraus besteht, wenn das erste Exon Exon 10 ist und das zweite Exon Exon 30 ist;
- die Region von Exon 10 SEQ ID NO: 1772 umfasst oder daraus besteht und die Region von Exon 30 SEQ ID NO: 1773 umfasst oder daraus besteht, wenn das erste Exon Exon 10 ist und das zweite Exon Exon 30 ist;
- die Region von Exon 10 SEQ ID NO: 151 umfasst oder daraus besteht und die Region von Exon 42 SEQ ID NO: 152 umfasst oder daraus besteht, wenn das erste Exon Exon 10 ist und das zweite Exon Exon 42 ist;
- die Region von Exon 10 SEQ ID NO: 157 umfasst oder daraus besteht und die Region von Exon 47 SEQ ID NO: 158 umfasst oder daraus besteht, wenn das erste Exon Exon 10 ist und das zweite Exon Exon 47 ist;
- die Region von Exon 10 SEQ ID NO: 169 umfasst oder daraus besteht und die Region von Exon 57 SEQ ID NO: 170 umfasst oder daraus besteht, wenn das erste Exon Exon 10 ist und das zweite Exon Exon 57 ist; oder
- die Region von Exon 10 SEQ ID NO: 173 umfasst oder daraus besteht und die Region von Exon 60 SEQ ID NO: 174 umfasst oder daraus besteht, wenn das erste Exon Exon 10 ist und das zweite Exon Exon 60 ist.

13. Oligonucleotid nach Anspruch 11 oder 12, umfassend die Basensequenz wie in irgendeiner der SEQ ID NO: 1673-1681, 1684-1686, 1688, 1689, 1778, 1780-1787, 1812-1851 oder 1884-1891 definiert und mit einer Länge, die durch die Anzahl der in der Basensequenz vorhandenen Nucleotide definiert ist oder die 1, 2, 3, 4 oder 5 Nucleotide länger ist.

14. Oligonucleotid nach irgendeinem der Ansprüche 11 bis 13, umfassend einen Teil der Basensequenz wie in irgendeiner der SEQ ID NO: 1673-1681, 1684-1686, 1688, 1689, 1778, 1780-1787, 1812-1851 oder 1884-1891 definiert, wobei der Teil der Basensequenz wie in irgendeiner der Sequenzen mit 1, 2, 3, 4 oder 5 weniger Nucleotiden als in der Basensequenz definiert entspricht.

15. Oligonucleotid nach Anspruch 14, umfassend:
(a) die Basensequenz wie in irgendeiner der SEQ ID NO: 1679 bis 1681, 1778, 1812, 1813, 1884 bis 1886, 1890 oder 1891 definiert und mit einer Länge von 25, 26, 27, 28, 29 oder 30 Nucleotiden oder der SEQ ID NO: 1814 und mit einer Länge von 26, 27, 28, 29 oder 30 Nucleotiden; oder
(b) die Basensequenz wie in SEQ ID NO: 1688, 1689 oder 1839 bis 1844 definiert und mit einer Länge von 26, 27, 28, 29 oder 30 Nucleotiden; oder
(c) die Basensequenz wie in SEQ ID NO: 1673 oder 1674 definiert und mit einer Länge von 25, 26, 27, 28, 29 oder 30 Nucleotiden; oder
(d) die Basensequenz wie in SEQ ID NO: 1675 oder 1676 definiert und mit einer Länge von 21, 22, 23, 24, 25, 26, 27, 28, 29 oder 30 Nucleotiden; oder
(e) die Basensequenz wie in SEQ ID NO: 1677 oder 1678 definiert und mit einer Länge von 25, 26, 27, 28, 29 oder 30 Nucleotiden; oder
(f) die Basensequenz wie in irgendeiner der SEQ ID NO: 1684 bis 1686 definiert und mit einer Länge von 21, 22, 23, 24, 25, 26, 27, 28, 29 oder 30 Nucleotiden; oder
(g) die Basensequenz wie in irgendeiner der SEQ ID NO: 1815 bis 1819 definiert und mit einer Länge von 22, 23, 24, 25, 26, 27, 28, 29 oder 30 Nucleotiden, oder
(h) die Basensequenz wie in irgendeiner der SEQ ID NO: 1820, 1824 definiert und mit einer Länge von 20, 21, 22, 23, 24, 25, 26, 27, 28, 29 oder 30 Nucleotiden, oder
(i) die Basensequenz wie in irgendeiner der SEQ ID NO: 1826, 1780, 1782, 1832 definiert und mit einer Länge von 21, 22, 23, 24, 25, 26, 27, 28, 29 oder 30 Nucleotiden, oder
(j) die Basensequenz wie in irgendeiner der SEQ ID NO: 1821, 1825 definiert und mit einer Länge von 22, 23, 24, 25, 26, 27, 28, 29 oder 30 Nucleotiden, oder
(k) die Basensequenz wie in irgendeiner der SEQ ID NO: 1822 definiert und mit einer Länge von 24, 25, 26, 27, 28, 29 oder 30 Nucleotiden, oder
(l) die Basensequenz wie in irgendeiner der SEQ ID NO: 1823, 1781, 1829, 1830, 1831 definiert und mit einer Länge von 25, 26, 27, 28, 29 oder 30 Nucleotiden, oder
(m) die Basensequenz wie in irgendeiner der SEQ ID NO: 1783, 1833, 1834, 1835 definiert und mit einer Länge von 17, 18, 19, 20, 21, 22, 23, 24, 25, 26, 27, 28, 29 oder 30 Nucleotiden, oder
(n) die Basensequenz wie in irgendeiner der SEQ ID NO: 1887 definiert und mit einer Länge von 24, 25, 26, 27, 28, 29 oder 30 Nucleotiden, oder
(o) die Basensequenz wie in irgendeiner der SEQ ID NO: 1888 oder 1889 definiert und mit einer Länge von 26, 27, 28, 29 oder 30 Nucleotiden, oder
(p) die Basensequenz wie in SEQ ID NO: 1827 definiert und mit einer Länge von 21, 22, 23, 24, 25, 26, 27, 28, 29 oder 30 Nucleotiden, oder
(q) die Basensequenz wie in SEQ ID NO: 1828 definiert und mit einer Länge von 25, 26 27, 28, 29 oder 30 Nucleotiden, oder
(r) die Basensequenz wie in irgendeiner der SEQ ID NO: 1784, 1836 definiert und mit einer Länge von 21, 22, 23, 24, 25, 26, 27, 28, 29 oder 30 Nucleotiden, oder
(s) die Basensequenz wie in irgendeiner der SEQ ID NO: 1786, 1838 definiert und mit einer Länge von 25, 26, 27, 28, 29 oder 30 Nucleotiden, oder
(t) die Basensequenz wie in irgendeiner der SEQ ID NO: 1780 definiert und mit einer Länge von 22, 23, 24, 25, 26, 27, 28, 29 oder 30 Nucleotiden, oder
(u) die Basensequenz wie in irgendeiner der SEQ ID NO: 1785, 1837 definiert und mit einer Länge von 25, 26, 27, 28, 29 oder 30 Nucleotiden, oder
(v) die Basensequenz wie in irgendeiner der SEQ ID NO: 1845, 1846, 1847, 1848 definiert und mit einer Länge von 24, 25 26, 27, 28, 29 oder 30 Nucleotiden, oder
(w) die Basensequenz wie in irgendeiner der SEQ ID NO: 1849, 1850 definiert und mit einer Länge von 22, 23, 24, 25, 26, 27, 28, 29 oder 30 Nucleotiden, oder
(x) die Basensequenz wie in irgendeiner der SEQ ID NO: 1787, 1851 definiert und mit einer Länge von 26, 27, 28, 29 oder 30 Nucleotiden.

16. Oligonucleotid nach irgendeinem der Ansprüche 11-15 zur Verwendung als Medikament zum Verhindern, Verzögern, Verbessern und/oder Behandeln einer Krankheit bei einem Subjekt.

17. Zusammensetzung, umfassend ein einzelnes Oligonucleotid nach irgendeinem der Ansprüche 11-15 und gegebenenfalls ferner umfassend einen pharmazeutisch akzeptablen Träger, Verdünnungsmittel, Exzipient, Salz, Adjuvans und/oder Lösungsmittel, wobei die Zusammensetzung bevorzugt zwei oder mehr identische Oligonucleotide wie in irgendeinem der Ansprüche 11 bis 15 definiert umfasst, die durch ein Gegenion, vorzugsweise Calcium, verknüpft sind, wobei die Zusammensetzung bevorzugter zur Verwendung als Medikament zum Verhindern, Verzögern, Verbessern und/oder Behandeln von DMD oder BMD bei einem Subjekt ist.

## Revendications

1. Procédé de conception d'un oligonucléotide pour produire une protéine fonctionnelle ou semi-fonctionnelle, dans lequel ledit procédé comprend les étapes suivantes :
(a) identifier une combinaison à l'intérieur d'un cadre d'un premier et d'un second exon dans un même pré-ARNm, dans lequel une région dudit second exon présente une identité d'au moins 50% avec une région dudit premier exon ;
(b) concevoir un oligonucléotide capable de se lier à ladite région dudit premier exon et ladite région dudit second exon ;
(c) dans lequel ladite liaison entraîne le saut dudit premier et dudit second exons, de préférence le saut d'une suite de plusieurs exons commençant par ledit premier exon et englobant un ou plusieurs exons présents entre ledit premier et ledit second exons et au maximum le saut de toute la suite d'exons entre ledit premier et ledit second exons, et
(d) dans lequel ledit procédé aboutit à un oligonucléotide.

2. Procédé selon la revendication 1, dans lequel l'oligonucléotide est capable de se lier à une partie d'une région dans un premier exon et à une partie d'une région dans une seconde région.

3. Procédé selon la revendication 1 ou 2, dans lequel la liaison dudit oligonucléotide est capable d'interférer avec au moins une séquence régulatrice de l'épissage dans lesdites régions desdits premier et second exons et/ou avec la structure secondaire englobant au moins ledit premier et/ou ledit second exon dans ledit pré-ARNm, de préférence, dans lequel ladite séquence régulatrice de l'épissage comprend un activateur de l'épissage exonique (ESE), une séquence de reconnaissance d'exon (ERS) et/ou un site de liaison pour une protéine de sérine-arginine (SR).

4. Procédé selon l'une quelconque des revendications 1 à 3, dans lequel l'oligonucléotide est capable d'induire le saut de toute la suite d'exons comprise entre ledit premier exon et ledit second exon.

5. Procédé selon l'une quelconque des revendications 1 à 4, dans lequel ledit oligonucléotide comprend de 10 à 40 nucléotides.

6. Procédé selon l'une quelconque des revendications 1 à 5, dans lequel ledit oligonucléotide comprend au moins une modification comparée à un oligonucléotide naturel à base de ribonucléotides ou de désoxyribonucléotides, de préférence
(a) au moins une modification de base, de préférence choisie parmi la 2-thiouracile, la 2-thiothymine, la 5-méthylcytosine, le 5-méthyluracile, la thymine, la 2,6-diaminopurine, plus préférablement choisie parmi la 5-méthylpyrimidine et la 2,6-diaminopurine ; et/ou
(b) au moins une modification de sucre, de préférence choisi parmi le 2'-*O*-méthyle, le 2'-*O*-(2-méthoxy)éthyle, le 2'-*O*-désoxy(ADN), le 2'-F, le morpholino, un nucléotide ponté ou le BNA, ou l'oligonucléotide comprend à la fois des nucléotides pontés et des nucléotides modifiés en 2'-désoxy (mixtes de BNA/ADN), plus préférablement la modification de sucre est le 2'-*O*-méthyle ; et/ou
(c) au moins une modification de chaîne principale, de préférence choisie parmi la phosphorothioate ou la phosphorodiamidate, plus préférablement la modification de chaîne principale est la phosphorothioate.

7. Procédé selon l'une quelconque des revendications 1 à 6, dans lequel ledit oligonucléotide comprend un ou plusieurs groupes conjugués, éventuellement protégés, choisis dans le groupe constitué de peptides, protéines, glucides, médicaments, fractions de ciblage, fractions d'augmentation de l'absorption, fractions d'augmentation de la solubilité, fractions d'augmentation de la pharmacodynamique, fractions d'augmentation de la pharmacocinétique, polymères, dérivés d'éthylène glycol, vitamines, lipides, fractions polyfluoroalkyle, stéroïdes, cholestérol, fractions fluorescentes, molécules indicatrices, fractions marquées radioactivement et de leurs combinaisons, fixés directement ou par l'intermédiaire d'un lieur divalent ou multivalent, à un résidu terminal ou interne.

8. Procédé selon l'une quelconque des revendications 1 à 7, dans lequel lesdits premier et second exons sont choisis parmi les exons 10, 18, 30, 8, 9, 11, 13, 19, 22, 23, 34, 40, 42, 44, 45, 47, 51, 53, 55, 56, 57 ou 60 du pré-ARNm de la dystrophine.

9. Procédé selon l'une quelconque des revendications 1 à 7, dans lequel ledit premier exon est l'exon 10 et ledit second exon est l'exon 12, 13, 14, 15, 16, 18, 20, 22, 23, 25, 26, 27, 28, 29, 30, 31, 32, 33, 34, 35, 36, 37, 38, 39, 40, 41, 42, 44, 46, 47, 48, 49, 51, 53, 55, 56, 57, 59 ou 60.

10. Procédé selon la revendication 8 ou 9, dans lequel l'oligonucléotide est capable d'induire le saut des exons 10 à 18 de la dystrophine, des exons 10 à 30, des exons 10 à 42, des exons 10 à 47, des exons 10 à 57, des exons 10 à 60, des exons 8 à 19, des exons 9 à 22, des exons 9 à 30, des exons 11 à 23, des exons 13 à 30, des exons 23 à 42, des exons 34 à 53, des exons 40 à 53, des exons 44 à 56, des exons 45 à 51, des exons 45 à 53, des exons 45 à 55, des exons 45 à 60 et/ou des exons 56 à 60.

11. Oligonucléotide antisens pouvant être obtenu par le procédé selon l'une quelconque des revendications 1 à 10, dans lequel ledit oligonucléotide est capable de se lier à une région dans un premier exon et à une région dans un second exon, dans lequel ladite région dudit second exon présente une identité d'au moins 50 % avec ladite région dudit premier exon,
dans lequel ledit premier et ledit second exons se trouvent dans un même pré-ARNm de dystrophine dudit sujet,
dans lequel ladite liaison dudit oligonucléotide est capable d'interférer avec au moins une séquence régulatrice de l'épissage dans lesdites régions desdits premier et second exons et/ou avec la structure secondaire englobant au moins ledit premier et/ou ledit second exon dans ledit pré-ARNm,
dans lequel ladite séquence régulatrice de l'épissage comprend un activateur de l'épissage exonique (ESE), une séquence de reconnaissance d'exon (ERS) et/ou un site de liaison pour une protéine de sérine-arginine (SR), et/ou dans lequel ladite liaison dudit oligonucléotide entraîne le saut dudit premier exon et dudit second exon, de préférence le saut d'une suite de plusieurs exons commençant par ledit premier exon et englobant un ou plusieurs exons présents entre ledit premier et ledit second exons et au maximum par le saut de toute la suite d'exons entre ledit premier et ledit second exons,
dans lequel on obtient une transcription à l'intérieur d'un cadre en permettant la production d'une dystrophine fonctionnelle ou semi-fonctionnelle et
dans lequel ladite séquence d'oligonucléotides ne fait pas partie d'un cocktail ou d'une combinaison de deux séquences distinctes ou plus d'oligonucléotides éventuellement liées par un ou plusieurs lieur(s), dans lequel lesdits premier et second exons sont définis de la façon suivante :
- le premier exon est l'exon 10 et le second exon est 18,
- le premier exon est l'exon 10 et le second exon est 30,
- le premier exon est l'exon 10 et le second exon est 42,
- le premier exon est l'exon 10 et le second exon est 47,
- le premier exon est l'exon 10 et le second exon est 57, ou
- le premier exon est l'exon 10 et le second exon est 60.

12. Oligonucléotide selon la revendication 11, dans lequel :
- la région de l'exon 10 comprend ou est constituée de SEQ ID NO : 109 et la région de l'exon 18 comprend ou est constituée de SEQ ID NO : 110 quand ledit premier exon est l'exon 10 et ledit second exon est l'exon 18 ;
- la région de l'exon 10 comprend ou est constituée de SEQ ID NO : 1766 et la région de l'exon 18 comprend ou est constituée de SEQ ID NO : 1767 quand ledit premier exon est l'exon 10 et ledit second exon est l'exon 18 ;
- la région de l'exon 10 comprend ou est constituée de SEQ ID NO : 1768 et la région de l'exon 18 comprend ou est constituée de SEQ ID NO : 1769 quand ledit premier exon est l'exon 10 et ledit second exon est l'exon 18 ;
- la région de l'exon 10 comprend ou est constituée de SEQ ID NO : 127 et la région de l'exon 30 comprend ou est constituée de SEQ ID NO : 128 quand ledit premier exon est l'exon 10 et ledit second exon est l'exon 30 ;
- la région de l'exon 10 comprend ou est constituée de SEQ ID NO : 1722 et la région de l'exon 30 comprend ou est constituée de SEQ ID NO : 1773 quand ledit premier exon est l'exon 10 et ledit second exon est l'exon 30 ;
- la région de l'exon 10 comprend ou est constituée de SEQ ID NO : 151 et la région de l'exon 42 comprend ou est constituée de SEQ ID NO : 152 quand ledit premier exon est l'exon 10 et ledit second exon est l'exon 42 ;
- la région de l'exon 10 comprend ou est constituée de SEQ ID NO : 157 et la région de l'exon 47 comprend ou est constituée de SEQ ID NO : 158 quand ledit premier exon est l'exon 10 et ledit second exon est l'exon 47 ;
- la région de l'exon 10 comprend ou est constituée de SEQ ID NO : 169 et la région de l'exon 57 comprend ou est constituée de SEQ ID NO : 170 quand ledit premier exon est l'exon 10 et ledit second exon est l'exon 57 ; ou
- la région de l'exon 10 comprend ou est constituée de SEQ ID NO : 173 et la région de l'exon 60 comprend ou est constituée de SEQ ID NO : 174 quand ledit premier exon est l'exon 10 et ledit second exon est l'exon 60.

13. Oligonucléotide selon la revendication 11 ou 12, comprenant la séquence de base telle que définie par l'une quelconque de SEQ ID NO : 1673-1681, 1684-1686, 1688, 1689, 1778, 1780-1787, 1812-1851 ou 1884-1891 et présentant une longueur, qui est définie par le nombre de nucléotides présents dans ladite séquence de base ou qui est plus longue de 1, 2, 3, 4 ou 5 nucléotides.

14. Oligonucléotide selon l'une quelconque des revendications 11 à 13, comprenant une partie de la séquence de base telle que définie par l'une quelconque de SEQ ID NO : 1673-1681, 1684-1686, 1688, 1689, 1778, 1780-1787, 1812-1851 ou 1884-1891, dans lequel ladite partie correspond à la séquence de base telle que définie par l'une quelconque desdites séquences avec 1, 2, 3, 4 ou 5 nucléotides de moins que ce qui a été défini dans ladite séquence de base.

15. Oligonucléotide selon la revendication 14, comprenant :
(a) la séquence de base telle que définie par l'une quelconque de SEQ ID NO : 1679 à 1681, 1778, 1812, 1813, 1884 à 1886, 1890 ou 1891 et présentant une longueur de 25, 26, 27, 28, 29 ou 30 nucléotides ou SEQ ID NO : 1814 et présentant une longueur de 26, 27, 28, 29 ou 30 nucléotides ; ou
(b) la séquence de base telle que définie par SEQ ID NO : 1688, 1689 ou 1839 à 1844 et présentant une longueur de 26, 27, 28, 29 ou 30 nucléotides ; ou
(c) la séquence de base telle que définie par SEQ ID NO : 1673 ou 1674 et présentant une longueur de 25, 26, 27, 28, 29 ou 30 nucléotides ; ou
(d) la séquence de base telle que définie par SEQ ID NO : 1675 ou 1676 et présentant une longueur de 21, 22, 23, 24, 25, 26, 27, 28, 29 ou 30 nucléotides ; ou
(e) la séquence de base telle que définie par SEQ ID NO : 1677 ou 1678 et présentant une longueur de 25, 26, 27, 28, 29 ou 30 nucléotides ; ou
(f) la séquence de base telle que définie par l'une quelconque de SEQ ID NO : 1684 à 1686 et présentant une longueur de 21, 22, 23, 24, 25, 26, 27, 28, 29 ou 30 nucléotides ; ou
(g) la séquence de base telle que définie par l'une quelconque de SEQ ID NO : 1815 à 1819 et présentant une longueur de 22, 23, 24, 25, 26, 27, 28, 29 ou 30 nucléotides, ou
(h) la séquence de base telle que définie par l'une quelconque de SEQ ID NO : 1820, 1824 et présentant une longueur de 20, 21, 22, 23, 24, 25, 26, 27, 28, 29 ou 30 nucléotides, ou
(i) la séquence de base telle que définie par l'une quelconque de SEQ ID NO : 1826, 1780, 1782, 1832 et présentant une longueur de 21, 22, 23, 24, 25, 26, 27, 28, 29 ou 30 nucléotides, ou
(j) la séquence de base telle que définie par l'une quelconque de SEQ ID NO : 1821, 1825 et présentant une longueur de 22, 23, 24, 25, 26, 27, 28, 29 ou 30 nucléotides, ou
(k) la séquence de base telle que définie par l'une quelconque de SEQ ID NO : 1822 et présentant une longueur de 24, 25, 26, 27, 28, 29 ou 30 nucléotides, ou
(l) la séquence de bases telle que définie par l'une quelconque de SEQ ID NO : 1823, 1781, 1829, 1830, 1831 et présentant une longueur 25, 26, 27, 28, 29 ou 30 nucléotides, ou
(m) la séquence de base telle que définie par l'une quelconque de SEQ ID NO : 1783, 1833, 1834, 1835 et présentant une longueur de 17, 18, 19, 20, 21, 22, 23, 24, 25, 26, 27, 28, 29 ou 30 nucléotides, ou
(n) la séquence de base telle que définie par l'une quelconque de SEQ ID NO : 1887 et présentant une longueur de 24, 25, 26, 27, 28, 29 ou 30 nucléotides, ou
(o) la séquence de base telle que définie par l'une quelconque de SEQ ID NO : 1888 ou 1889 et présentant une longueur de 26, 27, 28, 29 ou 30 nucléotides, ou
(p) la séquence de base telle que définie par SEQ ID NO : 1827 et présentant une longueur de 21, 22, 23, 24, 25, 26, 27, 28, 29 ou 30 nucléotides, ou
(q) la séquence de base telle que définie par SEQ ID NO : 1828 et présentant une longueur de 25, 26, 27, 28, 29 ou 30 nucléotides, ou
(r) la séquence de base telle que définie à l'une quelconque de SEQ ID NO : 1784, 1836 et présentant une longueur de 21, 22, 23, 24, 25, 26, 27, 28, 29 ou 30 nucléotides, ou
(s) la séquence de base telle que définie par l'une quelconque de SEQ ID NO : 1786, 1838 et présentant une longueur de 25, 26, 27, 28, 29 ou 30 nucléotides, ou
(t) la séquence de base telle que définie par l'une quelconque de SEQ ID NO : 1780 et présentant une longueur de 22, 23, 24, 25, 26, 27, 28, 29 ou 30 nucléotides, ou
(u) la séquence de base telle que définie par l'une quelconque de SEQ ID NO : 1785, 1837 et présentant une longueur de 25, 26, 27, 28, 29 ou 30 nucléotides, ou
(v) la séquence de base telle que définie par l'une quelconque de SEQ ID NO : 1845, 1846, 1847, 1848 et présentant une longueur de 24, 25, 26, 27, 28, 29 ou 30 nucléotides, ou
(w) la séquence de base telle que définie par l'une quelconque de SEQ ID NO : 1849, 1850 et présentant une longueur de 22, 23, 24, 25, 26, 27, 28, 29 ou 30 nucléotides, ou
(x) la séquence de base telle que définie par l'une quelconque de SEQ ID NO : 1787, 1851 et présentant une longueur de 26, 27, 28, 29 ou 30 nucléotides.

16. Oligonucléotide selon l'une quelconque des revendications 11 à 15, destiné à être utilisé en tant que médicament pour prévenir, retarder, améliorer et/ou traiter une maladie chez un sujet.

17. Composition comprenant un oligonucléotide unique selon l'une quelconque des revendications 11 à 15 et comprenant éventuellement en outre un support, diluant, excipient, sel, adjuvant et/ou solvant pharmaceutiquement acceptable, de préférence dans laquelle ladite composition comprend au moins deux oligonucléotides identiques, tels que définis à l'une quelconque des revendications 11 à 15 liés par un contre-ion, de préférence un calcium, plus préférablement dans laquelle ladite composition est destinée à une utilisation en tant que médicament pour prévenir, retarder, améliorer et/ou traiter la DMD ou la BMD chez un sujet.
